(19) Europäisches Patentamt
European Patent Office
Office européen des brevets

(11) EP 4 491 625 A1

(12) EUROPEAN PATENT APPLICATION
published in accordance with Art. 153(4) EPC

(43) Date of publication:
**15.01.2025 Bulletin 2025/03**

(21) Application number: **23766014.7**

(22) Date of filing: **07.03.2023**

(51) International Patent Classification (IPC):
***C07D 405/14*** (2006.01) ***C07D 409/14*** (2006.01)
***C07D 407/14*** (2006.01) ***C07D 471/04*** (2006.01)
***C07D 487/04*** (2006.01) ***A61K 31/443*** (2006.01)
***A61K 31/4436*** (2006.01) ***A61K 31/4439*** (2006.01)
***A61K 31/444*** (2006.01) ***A61P 3/10*** (2006.01)
***A61P 9/00*** (2006.01) ***A61P 25/00*** (2006.01)
***A61P 13/12*** (2006.01) ***A61P 9/12*** (2006.01)
***A61P 25/08*** (2006.01) ***A61P 25/16*** (2006.01)
***A61P 25/28*** (2006.01)

(52) Cooperative Patent Classification (CPC):
**A61K 31/443; A61K 31/4436; A61K 31/4439; A61K 31/444; A61P 3/10; A61P 9/00; A61P 9/12; A61P 13/12; A61P 25/00; A61P 25/08; A61P 25/16; A61P 25/28; C07D 405/14; C07D 407/14; C07D 409/14;** (Cont.)

(86) International application number:
**PCT/CN2023/080149**

(87) International publication number:
**WO 2023/169436 (14.09.2023 Gazette 2023/37)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **08.03.2022 CN 202210219469**

(71) Applicants:
- **Guangzhou Unirise Pharmaceutical Co., Ltd.
Guangzhou, Guangdong 510000 (CN)**
- **Guangzhou Apichope Pharmaceutical Co., Ltd.
Guangzhou, Guangdong 510000 (CN)**
- **Guangzhou Runlin Pharmaceutical Technology Co., Ltd.
Guangzhou, Guangdong 510000 (CN)**

(72) Inventors:
- **YU, Tianzhu
Guangzhou, Guangdong 510000 (CN)**
- **ZHANG, Shiguo
Guangzhou, Guangdong 510000 (CN)**
- **YANG, Tiping
Guangzhou, Guangdong 510000 (CN)**
- **YANG, Wenqian
Guangzhou, Guangdong 510000 (CN)**
- **WANG, Hui
Guangzhou, Guangdong 510000 (CN)**
- **LI, Hanxiong
Guangzhou, Guangdong 510000 (CN)**

(74) Representative: **DREISS Patentanwälte PartG mbB
Friedrichstraße 6
70174 Stuttgart (DE)**

# (54) BENZO BICYCLIC COMPOUND, AND PREPARATION METHOD AND APPLICATION THEREOF

(57) The present invention discloses benzobicyclic compounds, preparation methods and applications thereof. Specifically, the present invention discloses a compound having formula (X) or a stereoisomer, a geometric isomer, a tautomer, an *N*-oxide, a hydrate, a solvate, a metabolite, a pharmaceutically acceptable salt or a prodrug thereof,



Processed by Luminess, 75001 PARIS (FR)

(Cont. next page)

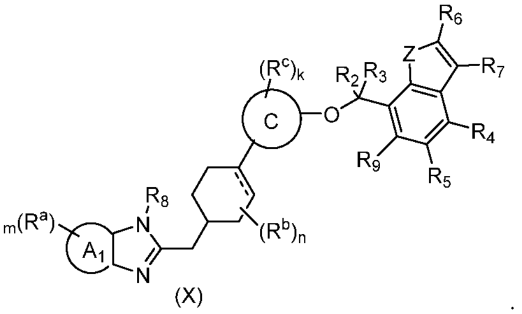

(X) .

(52) Cooperative Patent Classification (CPC): (Cont.)
**C07D 471/04; C07D 487/04**

## Description

This application claims the following priority:
CN202210219469.9, filed on March 8, 2022.

## FIELD OF THE INVENTION

The present invention relates to the technical field of chemical medicine, and specifically relates to a benzobicyclic compound, preparation method and use thereof.

## BACKGROUND O THE INVENTION

Diabetes mellitus is a multi-causal metabolic disease characterized by chronic hyperglycemia accompanied by disorders of glucose, lipid and protein metabolism caused by defective insulin secretion or action. Diabetes mellitus is an ancient disease that is characterized by increased blood glucose due to an absolute or relative deficiency of insulin in the body, and thus excessive glucose is excreted in the urine with symptoms such as polydipsia, polyuria, polyphagia, and weight loss.

According to the classification of the pathogenesis of diabetes, diabetes can usually be divided into type I diabetes and type II diabetes. Type I diabetes is characterized by a loss of function to secrete insulin due to autoimmune system destruction to the pancreatic β-cells. Type II diabetes is caused by insulin resistance or the inability of cells to respond to insulin; Obesity is one of the main reasons for insulin resistance, and therefore obesity can be said to be the main risk factor of type II diabetes. Type II diabetes patients accounts for approximately 90% of all diabetes patients, making it a major public health concern in developed countries where obesity is a serious problem, as well as in China, where the number of obese people is rising.

Glucagon-like peptide-1 (GLP-1) is an intestinal hypoglycemic hormone secreted by L-cells in the lower gastrointestinal tract. GLP-1 acts accordingly by binding to its widely available specific receptors. GLP-1 receptors are clearly present in pancreatic islet cells, gastrointestinal tract, lung, brain, kidney, hypothalamus and cardiovascular system, and may be present in the liver, adipose tissue and skeletal muscle. GLP-1 acts not only on β-cells to promote insulin secretion, but also acts on α-cells to inhibit glucagon secretion. There is generally no significant difference in serum GLP-1 levels among patients with normal glucose tolerance, impaired glucose tolerance and type II diabetes. However, there is a defective β-cell response to GLP-1 after eating, and under certain conditions this response is markedly enhanced by continuous infusion of GLP-1. Because the duration of action of human own GLP-1 is very brief (intravenous $t_{1/2} < 1.5$ min), human own GLP-1 is not suitable for clinical treatment of diabetes.

Peptide GLP-1 receptor agonists (e.g., liraglutide, exenatide, etc.) have been shown to reduce fasting and postprandial glucose as well as improve blood glucose in type II diabetic patients. However, because peptide GLP-1 has poor oral bioavailability and is inconvenient to take, there is an urgent clinical need for small molecule agonists ofGLP-1 receptor with good oral bioavailability.

Several studies have been already conducted to find therapeutic agents that can effectively agonize the GLP-1 receptor. PCT applications WO2018109607, WO2019239319, WO2019239371, WO2020103815, WO2020207474, WO2020263695, WO2021154796, WO2021112538, WO2021096304, WO2021096284, WO2021081207, WO2021018023 and WO2021254470 disclose numerous small molecule compounds, which are used as GLP-1 receptor agonists for the prevention or treatment of diabetes. However, there is still an urgent clinical need for more and better GLP-1 receptor agonists.

## SUMMARY OF THE INVENTION

The present invention provides a compound, or a pharmaceutical composition thereof, which acts as GLP-1 receptor agonist. The present invention further relates to the use of said compound, or pharmaceutical composition thereof in the preparation of a medicament for the treatment of diseases and/or conditions by

agonizing the GLP-1 receptor. The present invention further describes the synthetic method of the compound. The compounds of the present invention exhibit improved biological activity and pharmacokinetic advantages.

Specifically,
In one aspect, provided herein is a compound having formula (X), or a stereoisomer, a geometric isomer, a tautomer, an N-oxide, a hydrate, a solvate, a metabolite, a pharmaceutically acceptable salt or a prodrug thereof,

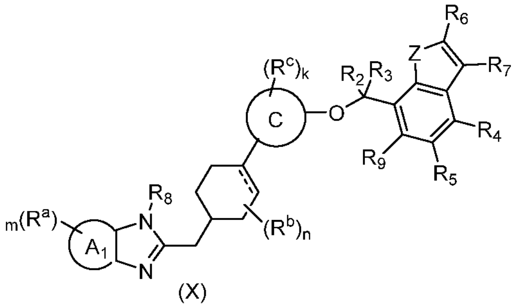

(X) ,

Wherein:

Ring $A_1$ is selected from 5-6 membered heteroaryl, phenyl, 5-6 membered heterocyclyl and $C_{5-6}$ cycloalkyl;

Ring C is 5-6 membered heteroaryl;

Z is selected from O, S, $N(R^z)$ and $C(R^z)_2$;

each of $R^c$, $R_2$ and $R_3$ is independently selected from H, D, F, Cl, Br, I, hydroxyl, cyano, nitro and $C_{1-6}$ alkyl, and wherein the $C_{1-6}$ alkyl is optionally substituted with 1, 2 or 3 substituents selected from F, Cl, Br, I, hydroxyl, cyano, amino and nitro;

each of $R_4$, $R_5$, $R_6$, $R_7$ and $R_9$ is independently selected from H, D, F, Cl, Br, I, hydroxyl, cyano, nitro and $C_{1-6}$ alkyl, and wherein the $C_{1-6}$ alkyl is optionally substituted with 1, 2 or 3 substituents selected from F, Cl, Br, I, hydroxyl, cyano, amino and nitro;

each $R^a$ is independently selected from H, F, Cl, Br, I, hydroxyl, cyano, amino, -C(=O)OH, - $C(=O)NHC(=O)C_{1-6}$ alkyl, $-C(=O)NHS(=O)_2C_{1-6}$ alkyl, $-C(=O)NHS(=O)_2NH_2$, $-C(=O)NHS(=O)_2NHC_{1-6}$ alkyl, $-C(=O)NHS(=O)_2C_{3-6}$ cycloalkyl, -C(=O)NHS(=O)z phenyl, 5-6 membered heteroaryl and

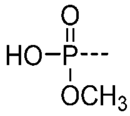

,

and wherein the $-C(=O)NHC(=O)C_{1-6}$ alkyl, $-C(=O)NHS(=O)_2C_{1-6}$ alkyl, $-C(=O)NHS(=O)_2NH_2$, - $C(=O)NHS(=O)_2NHC_{1-6}$ alkyl, $-C(=O)NHS(=O)_2C_{3-6}$ cycloalkyl, -C(=O)NHS(=O)z phenyl or 5-6 membered heteroaryl is optionally substituted with 1, 2, 3, 4 or 5 substituents selected from D, F, Cl, Br, I, hydroxyl, oxo, amino, nitro and cyano;

each $R^z$ is independently selected from H, F, Cl, Br, I, hydroxyl, cyano, nitro, $C_{1-6}$ alkyl, $C_{1-6}$ alkoxy, $C_{1-6}$ alkylthio, $C_{1-6}$ alkylamino, carboxyl, $-C(=O)OC_{1-6}$ alkyl, $-C(=O)NHC_{1-6}$ alkyl and $-C_{1-6}$ alkylene $C(=O)OC_{1-6}$ alkyl, and wherein the $C(=O)OC_{1-6}$ alkyl, $-C(=O)NHC_{1-6}$ alkyl or $-C_{1-6}$ alkylene $C(=O)OC_{1-6}$ alkyl is optionally substituted with 1, 2, 3, 4 or 5 substituents selected from D, F, Cl, Br, I, hydroxyl, oxo, amino, nitro and cyano;

each $R^b$ is independently selected from H, F, Cl, Br, I, hydroxyl, cyano, nitro, $C_{1-6}$ alkyl, $C_{1-6}$ alkoxy, $C_{1-6}$ alkylthio, $C_{1-6}$ alkylamino, carboxyl, $-C(=O)OC_{1-6}$ alkyl and $-C_{1-6}$ alkylene $C(=O)OC_{1-6}$ alkyl, and wherein the $C(=O)OC_{1-6}$ alkyl or $-C_{1-6}$ alkylene $C(=O)OC_{1-6}$ alkyl is optionally substituted with 1, 2, 3, 4 or 5 substituents selected from D, F, Cl, Br, I, hydroxyl, oxo, amino, nitro and cyano;

$R_8$ is selected from H, $C_{1-6}$ alkyl, $C_{2-6}$ alkenyl, $C_{2-6}$ alkynyl, $C_{3-6}$ cycloalkyl, 3-6 membered heterocyclyl, - $C_{1-6}$ alkylene $-R_{10}$ and $C_{1-6}$ alkylene $C(R_SR_MR_N)$, and wherein the $C_{1-6}$ alkyl, $C_{2-6}$ alkenyl, $C_{2-6}$ alkynyl, $C_{3-6}$ cycloalkyl, 3-6 membered heterocyclyl, $-C_{1-6}$ alkylene $-R_{10}$ or $C_{1-6}$ alkylene $C(R_SR_MR_N)$ is optionally substituted with 1, 2 or 3 $R^{8a}$;

each $R^{8a}$ is independently selected from D, F, Cl, Br, I, CN, $C_{1-6}$ alkyl, $C_{1-6}$ alkoxy, $C_{1-6}$ alkylthio, $C_{1-6}$ alkylamino, $C_{3-6}$ cycloalkyl and 3-6 membered heterocyclyl, and wherein the $C_{1-6}$ alkyl, $C_{1-6}$ alkoxy, $C_{1-6}$ alkylthio, $C_{1-6}$ alkylamino, $C_{3-6}$ cycloalkyl and 3-6 membered heterocyclyl is optionally substituted with 1, 2 or 3 $R^{8b}$;

each $R^{8b}$ is independently selected from D, F, Cl, Br, I, CN, $C_{1-6}$ alkyl, $C_{1-6}$ alkoxy, $C_{1-6}$ alkylthio, $C_{1-6}$ alkylamino, $C_{3-6}$ cycloalkyl and 3-6 membered heterocyclyl;

$R_{10}$ is selected from $C_{3-6}$ cycloalkyl, 3-8 membered heterocyclyl and 5-10 membered heteroaryl, and wherein the $C_{3-6}$ cycloalkyl, 3-8 membered heterocyclyl or 5-10 membered heteroaryl is optionally substituted with 1, 2, 3, 4 or 5 substituents selected from D, F, Cl, Br, I, oxo, hydroxyl, Amino, cyano, nitro, $C_{1-6}$ alkyl, $C_{1-6}$ alkoxy, $C_{1-6}$ alkylthio and $C_{1-6}$ alkylamino;

each of $R_S$, $R_M$ and $R_N$ is independently selected from D, F, Cl, Br, I, hydroxyl, oxo, amino, nitro and cyano;

or $R_S$ and $R_M$ join together to form $C_{3-6}$ cycloalkyl or 3-6 membered heterocyclyl;

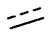

represents a single or double bond;
m is 0, 1, 2, 3 or 4;
k is 0, 1, 2, 3 or 4;
n is 0, 1, 2, 3, 4, 5, 6, 7 or 8.

In some embodiments, provided herein is a compound having formula (I-a), (I-b) or (I-c) or a stereoisomer, a geometric isomer, a tautomer, an *N*-oxide, a hydrate, a solvate, a metabolite, a pharmaceutically acceptable salt or a prodrug thereof,

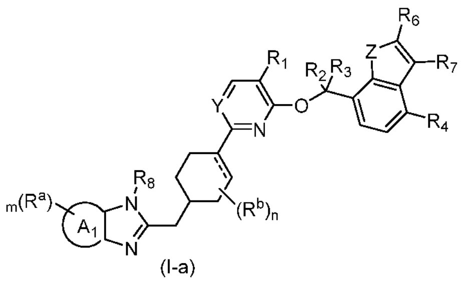

(I-a) ,

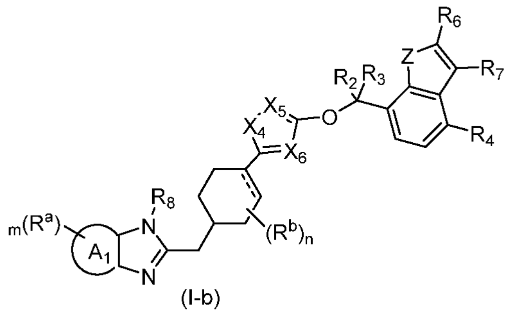

(I-b) ,

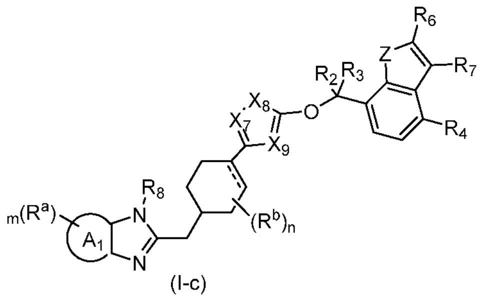

(I-c) ,

Wherein:

Ring $A_1$ is selected from 5-6 membered heteroaryl, phenyl, 5-6 membered heterocyclyl and $C_{5-6}$ cycloalkyl;
each of $X_5$, $X_6$, $X_7$ and $X_9$ is independently selected from $C(R^c)$ and N;
each of $X_4$ and $X_8$ is independently selected from O, S, $C(R^c)_2$ and $N(R^c)$;
each of Y is independently selected from $C(R^Y)$ and N;
each of Z is independently selected from O, S, $N(R^z)$ and $C(R^z)_2$;
each of $R^c$, $R^1$, $R_2$ and $R_3$ is independently selected from H, D, F, Cl, Br, I, hydroxyl, cyano, nitro and $C_{1-6}$ alkyl, and wherein the $C_{1-6}$ alkyl is optionally substituted with 1, 2 or 3 substituents selected from F, Cl, Br, I, hydroxyl, cyano, amino and nitro;
each of $R_4$, $R_6$ and $R_7$ is independently selected from H, D, F, Cl, Br, I, hydroxyl, cyano, nitro and $C_{1-6}$ alkyl, and wherein the $C_{1-6}$ alkyl is optionally substituted with 1, 2 or 3 substituents selected from F, Cl, Br, I, hydroxyl, cyano, amino and nitro;
each $R^a$ is independently selected from H, F, Cl, Br, I, hydroxyl, cyano, amino, -C(=O)OH, - C(=O)NHC(=O)$C_{1-6}$ alkyl, -C(=O)NHS(=O)$_2$$C_{1-6}$ alkyl, -C(=O)NHS(=O)$_2$$NH_2$, -C(=O)NHS(=O)$_2$NH$C_{1-6}$ alkyl, -C(=O)NHS(=O)$_2$$C_{3-6}$ cycloalkyl, -C(=O)NHS(=O)$_2$ phenyl, 5-6 membered heteroaryl and

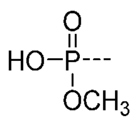

,

and wherein the -C(=O)NHC(=O)$C_{1-6}$ alkyl, -C(=O)NHS(=O)$_2$$C_{1-6}$ alkyl, -C(=O)NHS(=O)$_2$$NH_2$, - C(=O) NHS(=O)$_2$NH$C_{1-6}$ alkyl, -C(=O)NHS(=O)$_2$$C_{3-6}$ cycloalkyl, -C(=O)NHS(=O)$_2$ phenyl or 5-6 membered heteroaryl is

optionally substituted with 1, 2, 3, 4 or 5 substituents selected from D, F, Cl, Br, I, hydroxyl, oxo, amino, nitro and cyano;

each of $R^Y$ and $R^z$ is independently selected from H, F, Cl, Br, I, hydroxyl, cyano, nitro, $C_{1-6}$ alkyl, $C_{1-6}$ alkoxy, $C_{1-6}$ alkylthio, $C_{1-6}$ alkylamino, carboxyl, -C(=O)O$C_{1-6}$ alkyl, -C(=O)NH$C_{1-6}$ alkyl and -$C_{1-6}$ alkylene C(=O)O$C_{1-6}$ alkyl, and wherein the C(=O)O$C_{1-6}$ alkyl, -C(=O)NH$C_{1-6}$ alkyl or -$C_{1-6}$ alkylene C(=O)O$C_{1-6}$ alkyl is optionally substituted with 1, 2, 3, 4 or 5 substituents selected from D, F, Cl, Br, I, hydroxyl, oxo, amino, nitro and cyano;

each $R^b$ is independently selected from H, F, Cl, Br, I, hydroxyl, cyano, nitro, $C_{1-6}$ alkyl, $C_{1-6}$ alkoxy, $C_{1-6}$ alkylthio, $C_{1-6}$ alkylamino, carboxyl, -C(=O)O$C_{1-6}$ alkyl and -$C_{1-6}$ alkylene C(=O)O$C_{1-6}$ alkyl, and wherein the C(=O)O$C_{1-6}$ alkyl or -$C_{1-6}$ alkylene C(=O)O$C_{1-6}$ alkyl is optionally substituted with 1, 2, 3, 4 or 5 substituents selected from D, F, Cl, Br, I, hydroxyl, oxo, amino, nitro and cyano;

$R_8$ is selected from H, $C_{1-6}$ alkyl, $C_{2-6}$ alkenyl, $C_{2-6}$ alkynyl, $C_{3-6}$ cycloalkyl, 3-6 membered heterocyclyl, - $C_{1-6}$ alkylene -$R_{10}$ and $C_{1-6}$ alkylene C($R_SR_MR_N$), and wherein the $C_{1-6}$ alkyl, $C_{2-6}$ alkenyl, $C_{2-6}$ alkynyl, $C_{3-6}$ cycloalkyl, 3-6 membered heterocyclyl, -$C_{1-6}$ alkylene -$R_{10}$ or $C_{1-6}$ alkylene C($R_SR_MR_N$) is optionally substituted with 1, 2 or 3 $R^{8a}$;

each $R^{8a}$ is independently selected from D, F, Cl, Br, I, CN, $C_{1-6}$ alkyl, $C_{1-6}$ alkoxy, $C_{1-6}$ alkylthio, $C_{1-6}$ alkylamino, $C_{3-6}$ cycloalkyl and 3-6 membered heterocyclyl, and wherein the $C_{1-6}$ alkyl, $C_{1-6}$ alkoxy, $C_{1-6}$ alkylthio, $C_{1-6}$ alkylamino, $C_{3-6}$ cycloalkyl and 3-6 membered heterocyclyl is optionally substituted with 1, 2 or 3 $R^{8b}$;

each $R^{8b}$ is independently selected from D, F, Cl, Br, I, CN, $C_{1-6}$ alkyl, $C_{1-6}$ alkoxy, $C_{1-6}$ alkylthio, $C_{1-6}$ alkylamino, $C_{3-6}$ cycloalkyl and 3-6 membered heterocyclyl;

$R_{10}$ is selected from $C_{3-6}$ cycloalkyl, 3-8 membered heterocyclyl and 5-10 membered heteroaryl, and wherein the $C_{3-6}$ cycloalkyl, 3-8 membered heterocyclyl or 5-10 membered heteroaryl is optionally substituted with 1, 2, 3, 4 or 5 substituents selected from D, F, Cl, Br, I, oxo, hydroxyl, Amino, cyano, nitro, $C_{1-6}$ alkyl, $C_{1-6}$ alkoxy, $C_{1-6}$ alkylthio and $C_{1-6}$ alkylamino;

each of $R_S$, $R_M$ and $R_N$ is independently selected from D, F, Cl, Br, I, hydroxyl, oxo, amino, nitro and cyano;

or $R_S$ and $R_M$ join together to form $C_{3-6}$ cycloalkyl or 3-6 membered heterocyclyl;

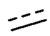

represents a single or double bond;

m is 0, 1, 2, 3 or 4;

k is 0, 1, 2, 3 or 4;

n is 0, 1, 2, 3, 4, 5, 6, 7 or 8.

In some embodiments, provided herein is a compound having formula (II-a), (II-b), (II-c), (II-d), (II-e), (II-f), (II-g), (II-h) or (II-i) or a stereoisomer, a geometric isomer, a tautomer, an *N*-oxide, a hydrate, a solvate, a metabolite, a pharmaceutically acceptable salt or a prodrug thereof,

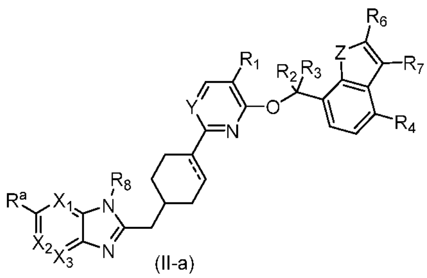

(II-a) ,

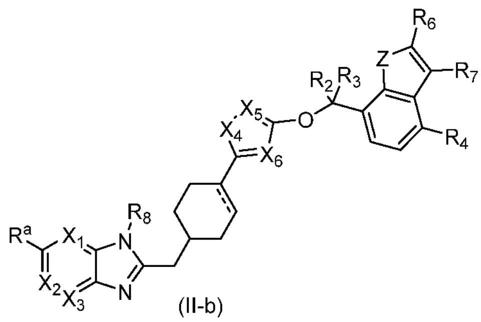

(II-b) ,

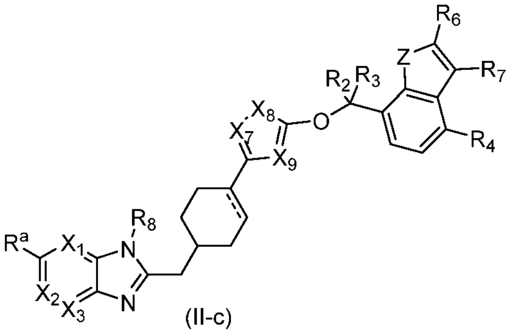
(II-c) ,

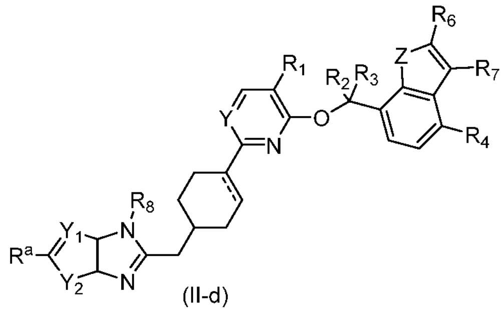
(II-d) ,

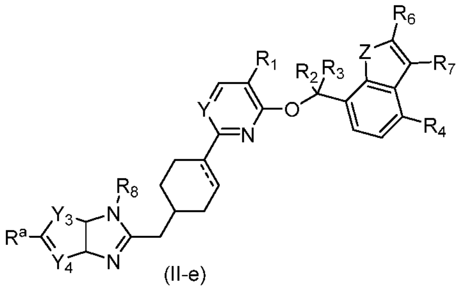
(II-e) ,

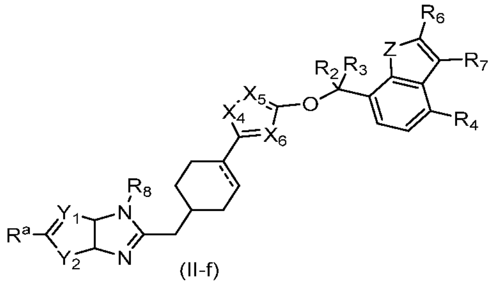
(II-f) ,

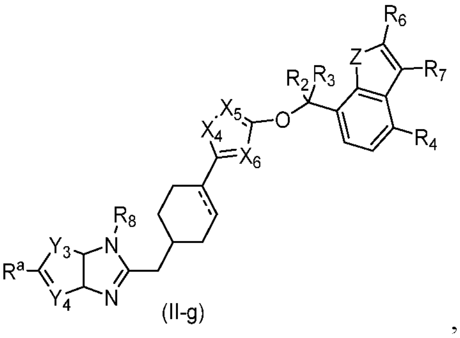
(II-g) ,

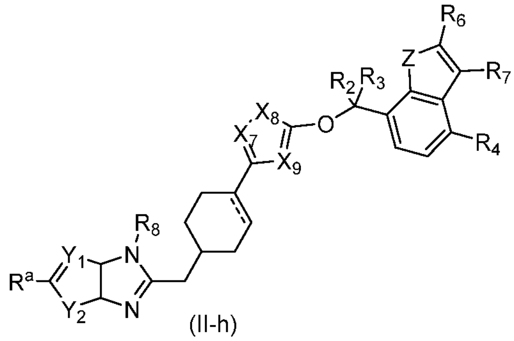
(II-h) ,

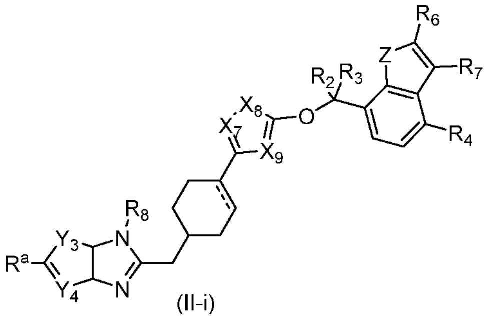
(II-i) ,

Wherein:

each of $X_1$, $X_2$ and $X_3$ is independently selected from $C(R^a)$ and N;
each of $X_5$, $X_6$, $X_7$, $X_9$, $Y_1$ and $Y_4$ is independently selected from $C(R^c)$ and N;
each of $X_4$, $X_8$, $Y_2$ and $Y_3$ is independently selected from O, S, $C(R^c)_2$ and $N(R^c)$;
Y is selected from $C(R^Y)$ and N;
Z is selected from O, S, $N(R^z)$ and $C(R^z)_2$;
each $R^a$ is independently selected from H, F, Cl, Br, I, hydroxyl, cyano, amino, -C(=O)OH, - C(=O)NHC(=O)$C_{1-6}$ alkyl, -C(=O)NHS(=O)$_2$$C_{1-6}$ alkyl, -C(=O)NHS(=O)$_2$$NH_2$, -C(=O)NHS(=O)$_2$NH$C_{1-6}$ alkyl, -C(=O)NHS(=O)$_2$$C_{3-6}$ cycloalkyl, -C(=O)NHS(=O)$_2$ phenyl, 5-6 membered heteroaryl and

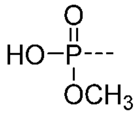,

and wherein the -C(=O)NHC(=O)$C_{1-6}$ alkyl, -C(=O)NHS$(=O)_2C_{1-6}$ alkyl, -C(=O)NHS$(=O)_2NH_2$, - C(=O) NHS$(=O)_2NHC_{1-6}$ alkyl, -C(=O)NHS$(=O)_2C_{3-6}$ cycloalkyl, -C(=O)NHS$(=O)_2$ phenyl or 5-6 membered heteroaryl is optionally substituted with 1, 2, 3, 4 or 5 substituents selected from D, F, Cl, Br, I, hydroxyl, oxo, amino, nitro and cyano;

each of $R^c$, $R_1$, $R_2$ and $R_3$ is independently selected from H, D, F, Cl, Br, I, hydroxyl, cyano, nitro and $C_{1-6}$ alkyl, and wherein the $C_{1-6}$ alkyl is optionally substituted with 1, 2 or 3 substituents selected from F, Cl, Br, I, hydroxyl, cyano, amino and nitro;

each of $R_4$, $R_6$ and $R_7$ is independently selected from H, D, F, Cl, Br, I, hydroxyl, cyano, nitro and $C_{1-6}$ alkyl, and wherein the $C_{1-6}$ alkyl is optionally substituted with 1, 2 or 3 substituents selected from F, Cl, Br, I, hydroxyl, cyano, amino and nitro;

each of $R^Y$ and $R^z$ is independently selected from H, F, Cl, Br, I, hydroxyl, cyano, nitro, $C_{1-6}$ alkyl, $C_{1-6}$ alkoxy, $C_{1-6}$ alkylthio, $C_{1-6}$ alkylamino, carboxyl, -C(=O)O$C_{1-6}$ alkyl, -C(=O)NH$C_{1-6}$ alkyl and -$C_{1-6}$ alkylene C(=O)O$C_{1-6}$ alkyl, and wherein the C(=O)O$C_{1-6}$ alkyl, -C(=O)NH$C_{1-6}$ alkyl or -$C_{1-6}$ alkylene C(=O)O$C_{1-6}$ alkyl is optionally substituted with 1, 2, 3, 4 or 5 substituents selected from D, F, Cl, Br, I, hydroxyl, oxo, amino, nitro and cyano;

each $R^b$ is independently selected from H, F, Cl, Br, I, hydroxyl, cyano, nitro, $C_{1-6}$ alkyl, $C_{1-6}$ alkoxy, $C_{1-6}$ alkylthio, $C_{1-6}$ alkylamino, carboxyl, -C(=O)O$C_{1-6}$ alkyl and -$C_{1-6}$ alkylene C(=O)O$C_{1-6}$ alkyl, and wherein the C(=O)O$C_{1-6}$ alkyl or -$C_{1-6}$ alkylene C(=O)O$C_{1-6}$ alkyl is optionally substituted with 1, 2, 3, 4 or 5 substituents selected from D, F, Cl, Br, I, hydroxyl, oxo, amino, nitro and cyano;

$R_8$ is selected from H, $C_{1-6}$ alkyl, $C_{2-6}$ alkenyl, $C_{2-6}$ alkynyl, $C_{3-6}$ cycloalkyl, 3-6 membered heterocyclyl, - $C_{1-6}$ alkylene -$R_{10}$ and $C_{1-6}$ alkylene $C(R_SR_MR_N)$, and wherein the $C_{1-6}$ alkyl, $C_{2-6}$ alkenyl, $C_{2-6}$ alkynyl, $C_{3-6}$ cycloalkyl, 3-6 membered heterocyclyl, -$C_{1-6}$ alkylene -$R_{10}$ or $C_{1-6}$ alkylene $C(R_SR_MR_N)$ is optionally substituted with 1, 2 or 3 $R^{8a}$;

each $R^{8a}$ is independently selected from D, F, Cl, Br, I, CN, $C_{1-6}$ alkyl, $C_{1-6}$ alkoxy, $C_{1-6}$ alkylthio, $C_{1-6}$ alkylamino, $C_{3-6}$ cycloalkyl and 3-6 membered heterocyclyl, and wherein the $C_{1-6}$ alkyl, $C_{1-6}$ alkoxy, $C_{1-6}$ alkylthio, $C_{1-6}$ alkylamino, $C_{3-6}$ cycloalkyl and 3-6 membered heterocyclyl is optionally substituted with 1, 2 or 3 $R^{8b}$;

each $R^{8b}$ is independently selected from D, F, Cl, Br, I, CN, $C_{1-6}$ alkyl, $C_{1-6}$ alkoxy, $C_{1-6}$ alkylthio, $C_{1-6}$ alkylamino, $C_{3-6}$ cycloalkyl and 3-6 membered heterocyclyl;

$R_{10}$ is selected from $C_{3-6}$ cycloalkyl, 3-8 membered heterocyclyl and 5-10 membered heteroaryl, and wherein the $C_{3-6}$ cycloalkyl, 3-8 membered heterocyclyl or 5-10 membered heteroaryl is optionally substituted with 1, 2, 3, 4 or 5 substituents selected from D, F, Cl, Br, I, oxo, hydroxyl, Amino, cyano, nitro, $C_{1-6}$ alkyl, $C_{1-6}$ alkoxy, $C_{1-6}$ alkylthio or $C_{1-6}$ alkylamino substitution;

each of $R_S$, $R_M$ and $R_N$ is independently selected from D, F, Cl, Br, I, hydroxyl, oxo, amino, nitro and cyano;

or $R_S$ and $R_M$ join together to form $C_{3-6}$ cycloalkyl or 3-6 membered heterocyclyl;

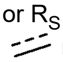 represents a single or double bond.

In some embodiments, $R_8$ is selected from H, $C_{1-3}$ alkyl, $C_{2-3}$ alkenyl, $C_{2-3}$ alkynyl, $C_{3-6}$ cycloalkyl, 3-6 membered heterocyclyl and -$C_{1-3}$ alkylene -$R_{10}$, and wherein the $C_{1-3}$ alkyl, $C_{2-3}$ alkenyl, $C_{2-3}$ alkynyl, $C_{3-6}$ cycloalkyl, 3-6 membered heterocyclyl or -$C_{1-3}$ alkylene -$R_{10}$ is optionally substituted with 1, 2 or 3 $R^{8a}$, the remaining variables are as defined in the present invention;

In some embodiments, each $R^{8a}$ is independently selected from D, F, Cl, Br, I, CN, $C_{1-3}$ alkyl, $C_{1-3}$ alkoxy, $C_{1-3}$ alkylthio, $C_{1-3}$ alkylamino, $C_{4-6}$ cycloalkyl and 5-6 membered heterocyclyl, and wherein the $C_{1-3}$ alkyl, $C_{1-3}$ alkoxy, $C_{1-3}$ alkylthio, $C_{1-3}$ alkylamino, $C_{4-6}$ cycloalkyl and 5-6 membered heterocyclyl is optionally substituted with 1, 2 or 3 $R^{8b}$, the remaining variables are as defined in the present invention;

In some embodiments, each $R^{8b}$ is independently selected from D, F, Cl, Br, I, CN, $C_{1-3}$ alkyl, $C_{1-3}$ alkoxy, $C_{1-3}$ alkylthio, $C_{1-3}$ alkylamino, $C_{5-6}$ cycloalkyl and 5-6 membered heterocyclyl, the remaining variables are as defined in the present invention;

In some embodiments, $R_{10}$ is optionally selected from $C_{5-6}$ cycloalkyl, 5-6 membered heterocyclyl and 5-10 membered heteroaryl, the remaining variables are as defined in the present invention;

In some embodiments, $R_{10}$ is optionally selected from cyclopropyl, cyclobutyl, cyclopentyl, cyclohexyl, azetidinyl, pyrrolidinyl, tetrahydrofuranyl, piperidyl, piperazinyl, morpholinyl,

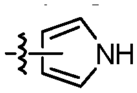, 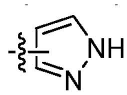,

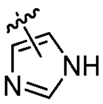, 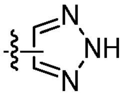, 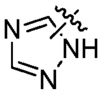, 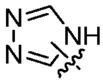, 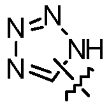, 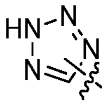, 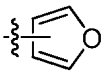, 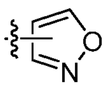, 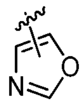,

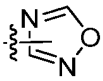, 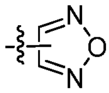, 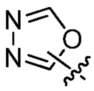, 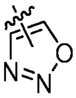, 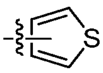, 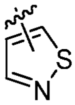, 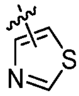, 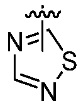, 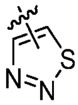, 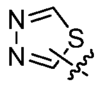, 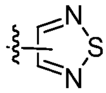,

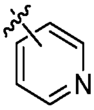, 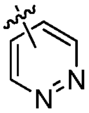, 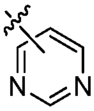, 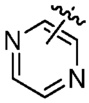, 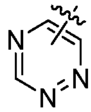, 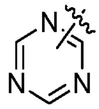 and 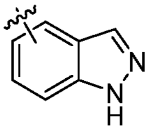,

the remaining variables are as defined in the present invention;

In some embodiments, $R_8$ is optionally selected from H,

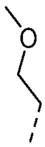, 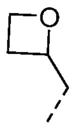, 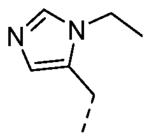, 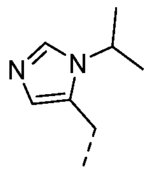,

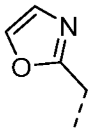, 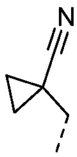, 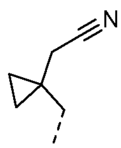 and 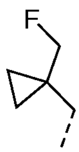,

the remaining variables are as defined in the present invention;

In some embodiments, each of $R^1$, $R_2$ and $R_3$ is optionally independently selected from H, D, F, Cl, Br and I, the remaining variables are as defined in the present invention;

In some embodiments, each of $R_4$, $R_6$ and $R_7$ is optionally independently selected from H, D, F, Cl, Br and I, the remaining variables are as defined in the present invention;

In some embodiments, each $R^a$ is optionally independently selected from -C(=O)OH, -C(=O)NHC(=O)$C_{1-3}$ alkyl, -C(=O)NHS(=O)$_2C_{1-3}$ alkyl, -C(=O)NHS(=O)$_2NH_2$, -C(=O)NHS(=O)$_2NHC_{1-3}$ alkyl, -C(=O)NHS(=O)$_2C_{3-6}$ cycloalkyl, -C(=O)NHS(=O)$_2$ phenyl, 5-6 membered heteroaryl and

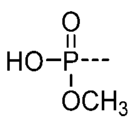,

and wherein the - C(=O)NHC(=O)$C_{1-3}$ alkyl, -C(=O)NHS(=O)$_2C_{1-3}$ alkyl, -C(=O)NHS(=O)$_2NH_2$, -C(=O)NHS(=O)$_2NHC_{1-3}$ alkyl, -C(=O)NHS(=O)$_2C_{3-6}$ cycloalkyl, -C(=O)NHS(=O)$_2$ phenyl or 5-6 membered heteroaryl is optionally substituted with 1, 2, 3, 4 or 5 substituents selected from D, F, Cl, Br, I, hydroxyl, oxo, amino, nitro and cyano, the remaining variables are as defined in the present invention;

In some embodiments, each $R^a$ is optionally independently selected from

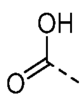, 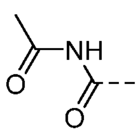,

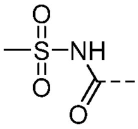, 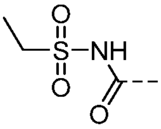, 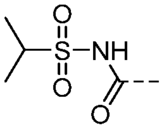, 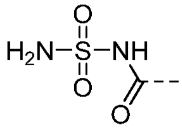, 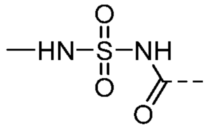, 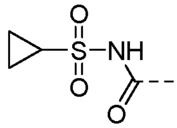,

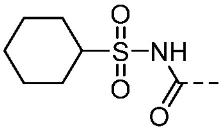, 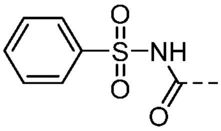, 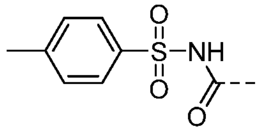, 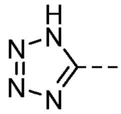 and 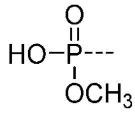,

the remaining variables are as defined in the present invention.

In some embodiments, each $R^a$ is optionally independently selected from H, F, Cl, Br, I, hydroxyl, cyano, amino, -C(=O)OH, -C(=O)NHC(=O)$C_{1-3}$ alkyl, -C(=O)NHS(=O)$_2$$C_{1-3}$ alkyl, -C(=O)NHS(=O)$_2$$NH_2$, - C(=O)NHS(=O)$_2$NH $C_{1-3}$ alkyl, -C(=O)NHS(=O)$_2$$C_{3-6}$ cycloalkyl, -C(=O)NHS(=O)$_2$ phenyl, 5-6 membered heteroaryl and

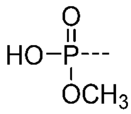,

and wherein the -C(=O)NHC(=O)$C_{1-3}$ alkyl, -C(=O)NHS(=O)$_2$$C_{1-3}$ alkyl, - C(=O)NHS(=O)$_2$$NH_2$, -C(=O)NHS(=O)$_2$NH $C_{1-3}$ alkyl, -C(=O)NHS(=O)$_2$$C_{3-6}$ cycloalkyl, -C(=O)NHS(=O)$_2$ phenyl or 5-6 membered heteroaryl is optionally substituted with 1, 2, 3, 4 or 5 substituents selected from D, F, Cl, Br, I, hydroxyl, oxo, amino, nitro and cyano, the remaining variables are as defined in the present invention;

In some embodiments, each $R^a$ is optionally independently selected from H, F, Cl, Br, I, hydroxyl, cyano, amino,

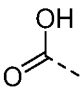, 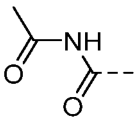, 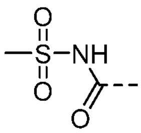, 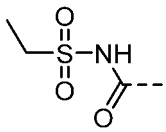, 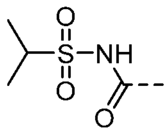, 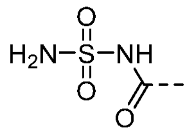,

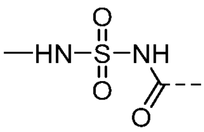, 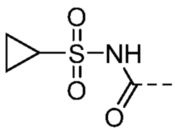, 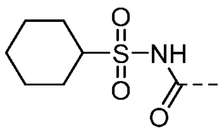, 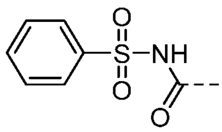, 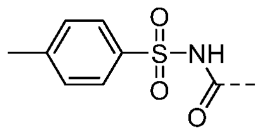,

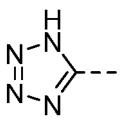 and 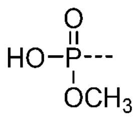,

the remaining variables are as defined in the present invention.

In one aspect, provided herein is the compound having one of the following structures or a stereoisomer, a geometric isomer, a tautomer, an *N*-oxide, a hydrate, a solvate, a metabolite, a pharmaceutically acceptable salt or a prodrug thereof:

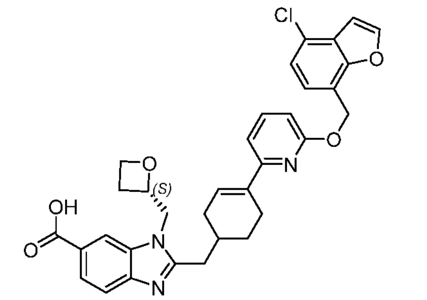
,
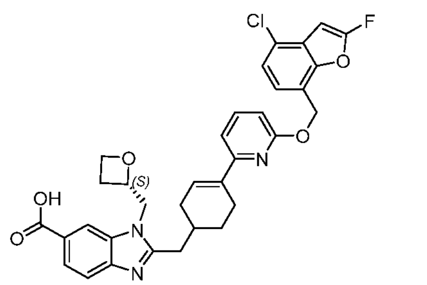
,

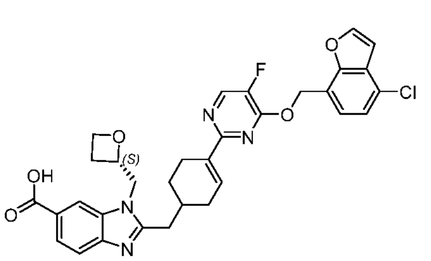
,
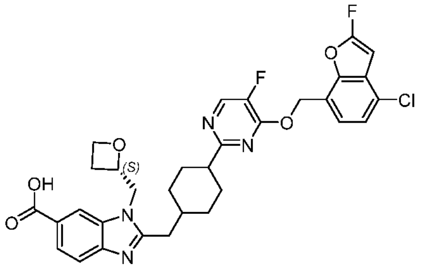
,

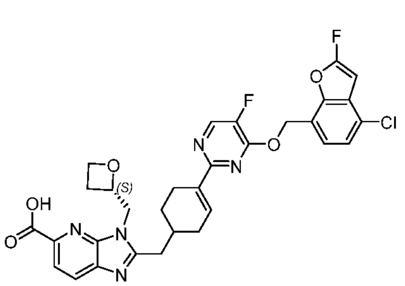
,
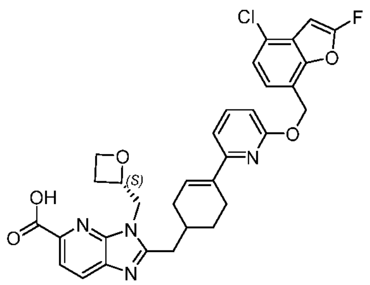
,
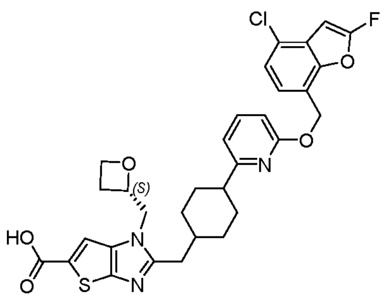
,

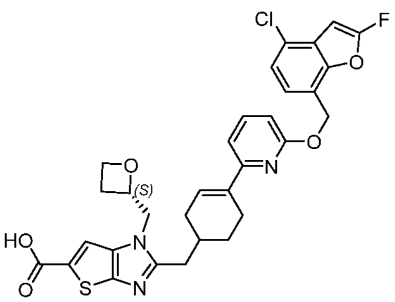
,
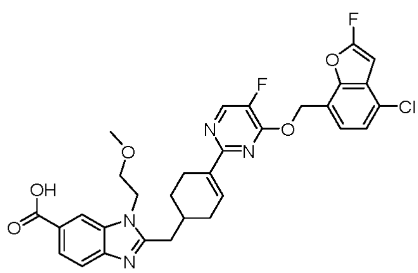
,
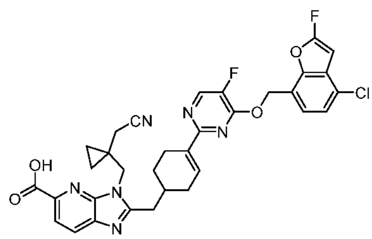
,

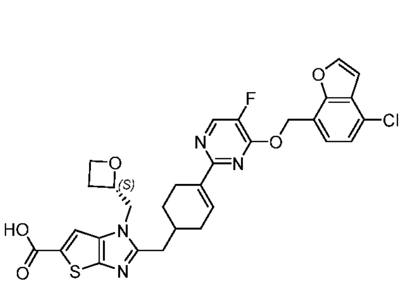
,
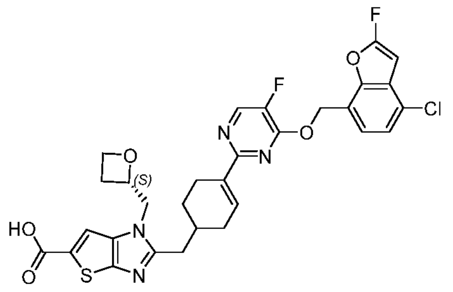
,

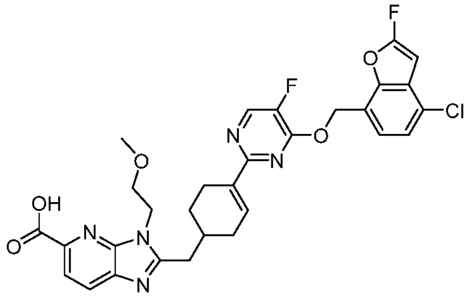 ,

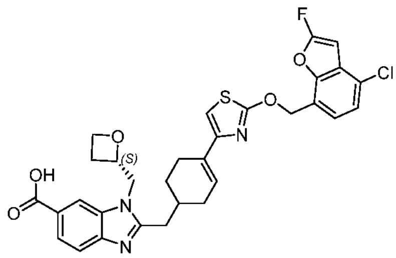 ,

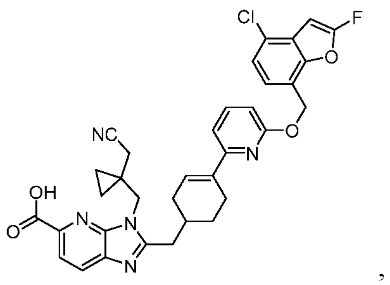 ,

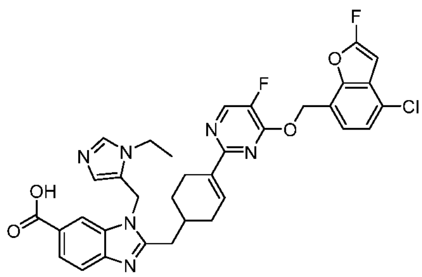 ,

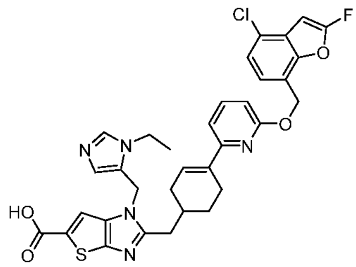 ,

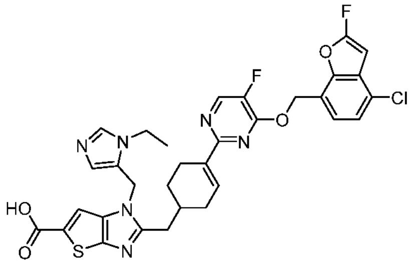 ,

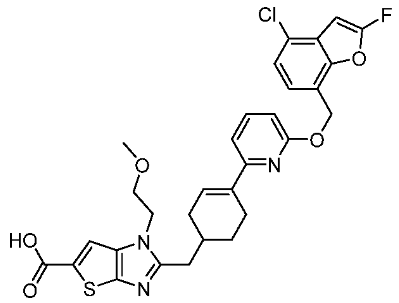 ,

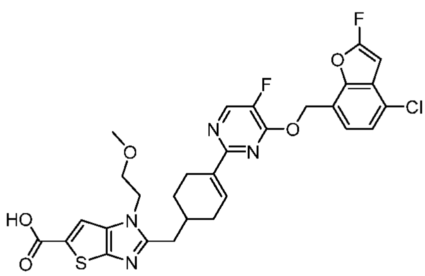 ,

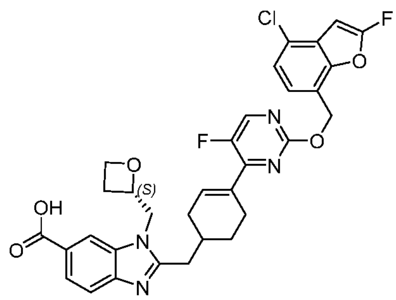 ,

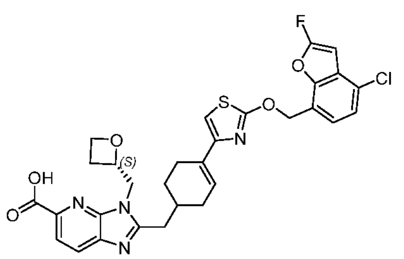 ,

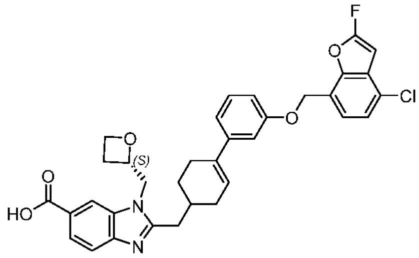 ,

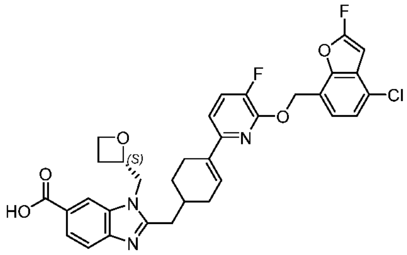,

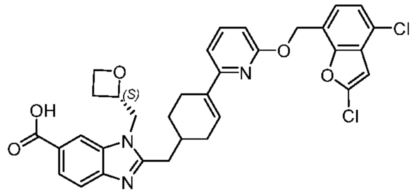,

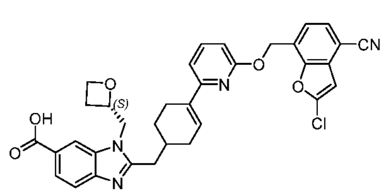,

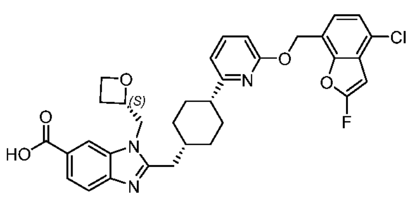,

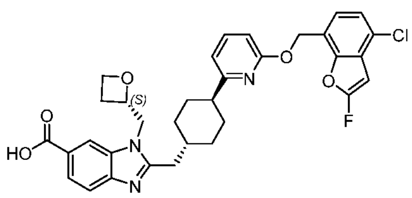,

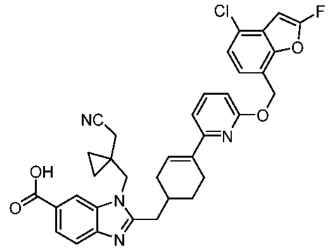,

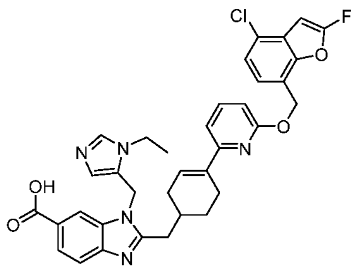,

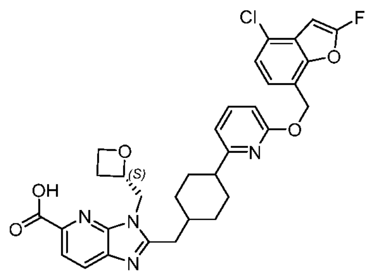,

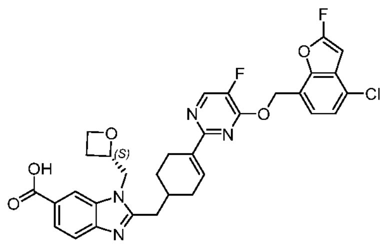,

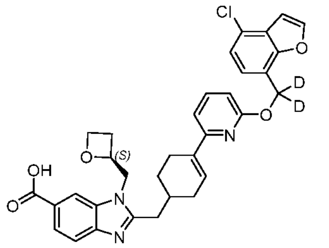,

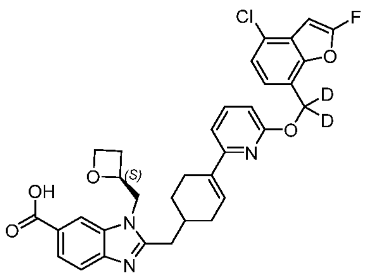,

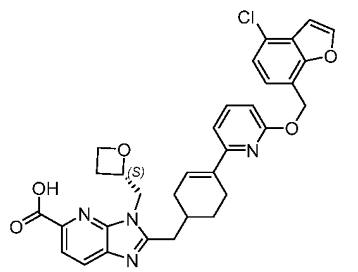,

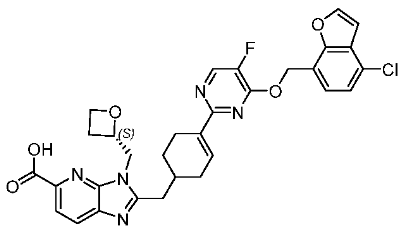,

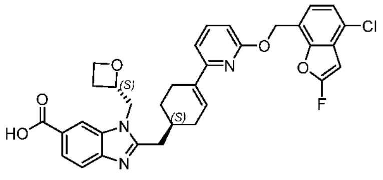,

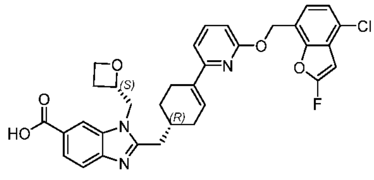,

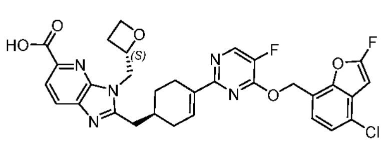,

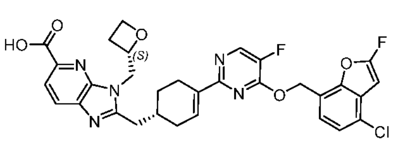,

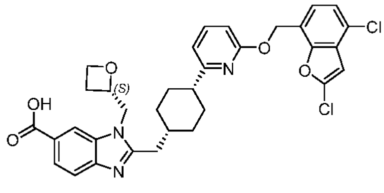,

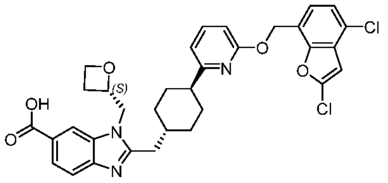
,
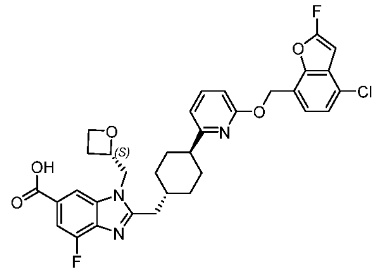
,
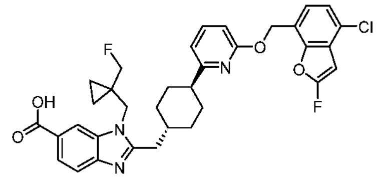
,
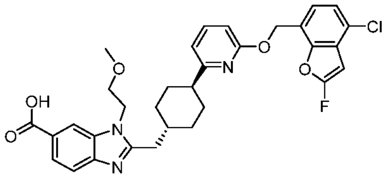
,
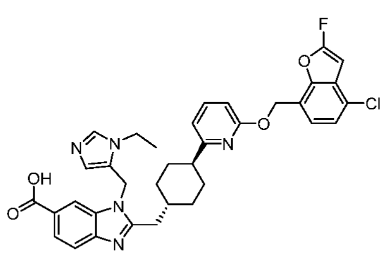
,
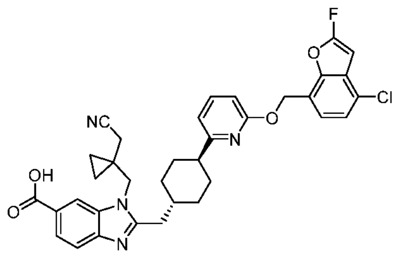
,
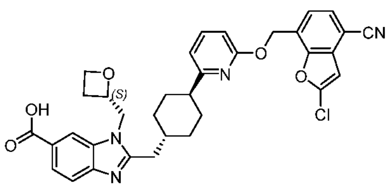
,
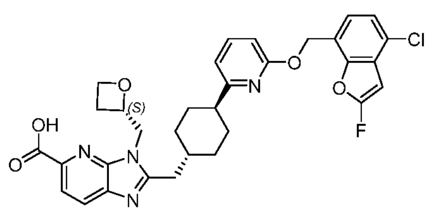
,
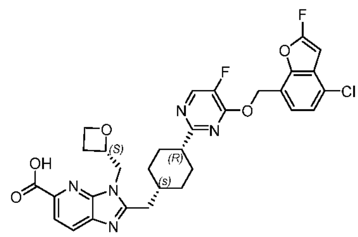
,
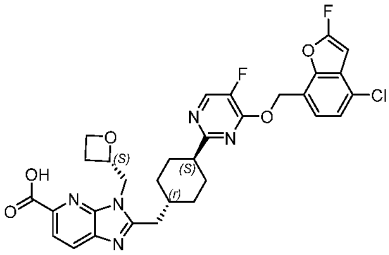
,
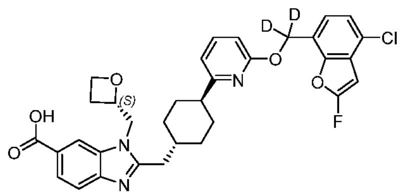
,
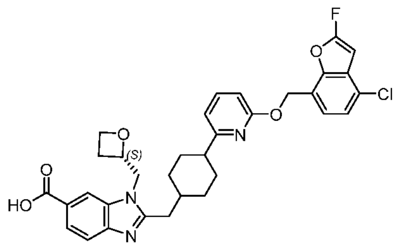
,
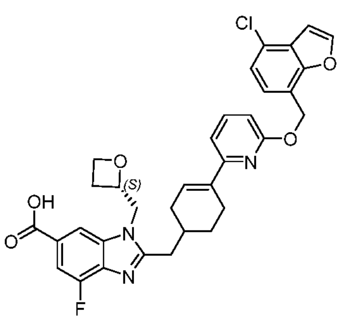
,
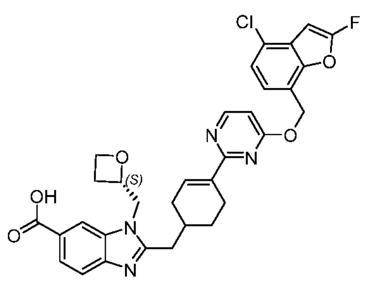
,
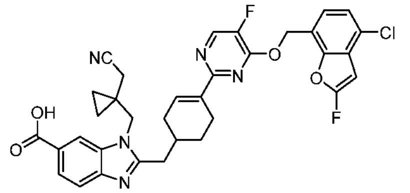
,

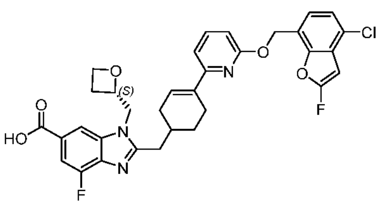
,
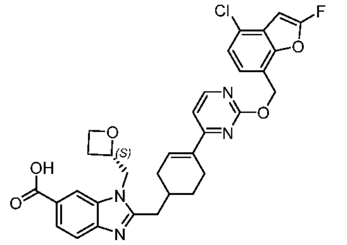
,
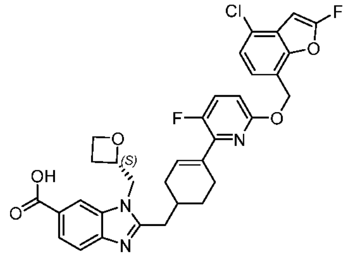
,

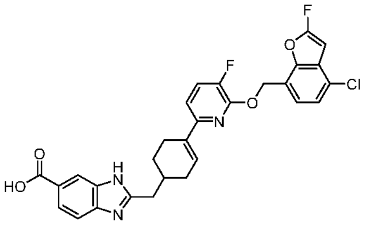
,
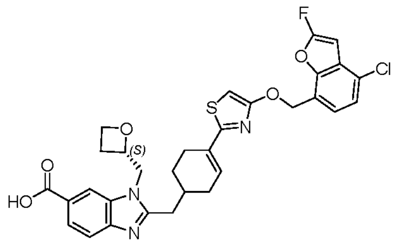
,
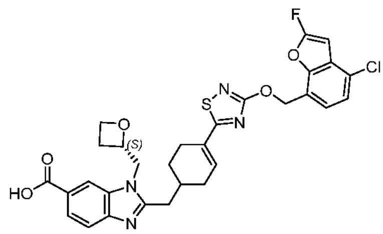
,

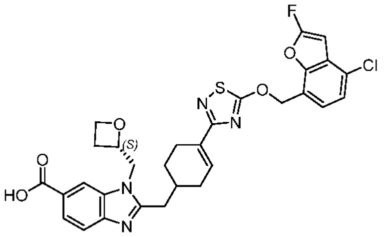
,
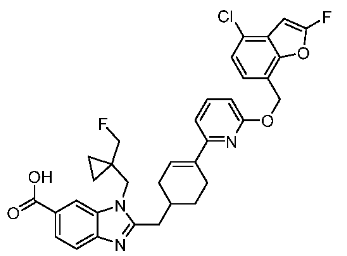
,
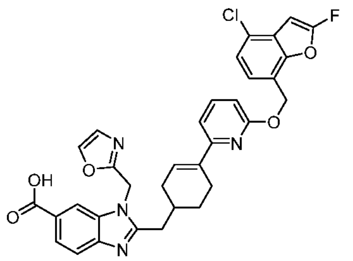
,

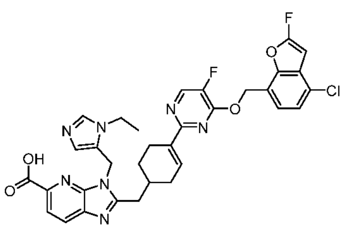
,
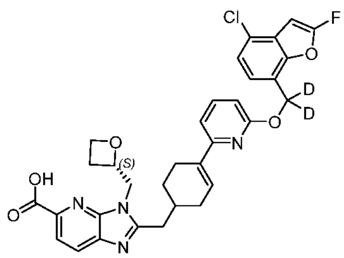
,
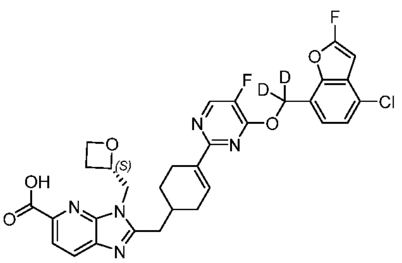
,

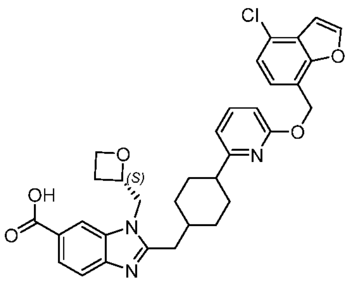
,
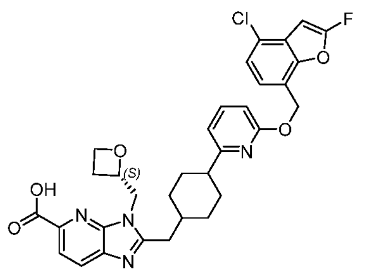
,
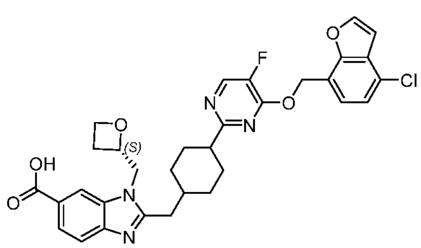
,

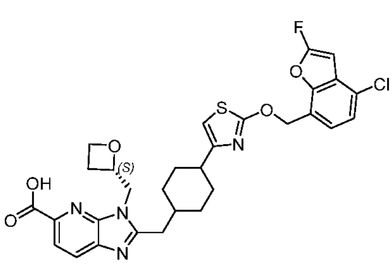,

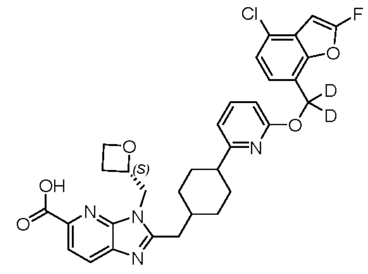,

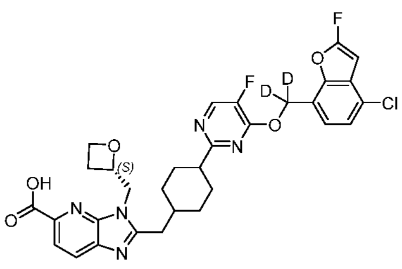,

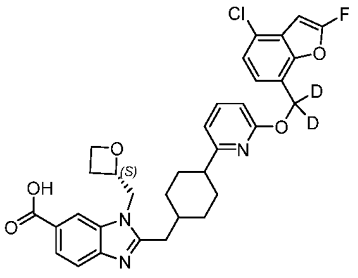,

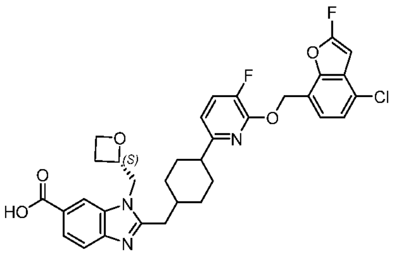,

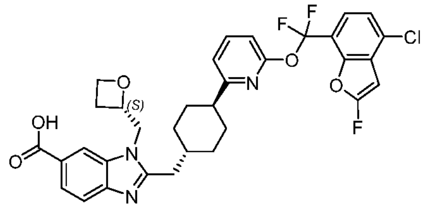,

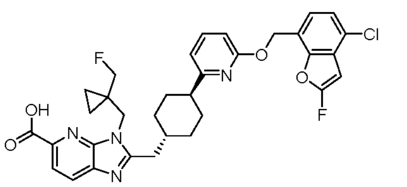,

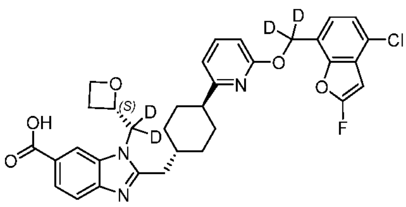,

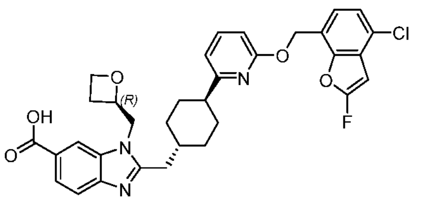,

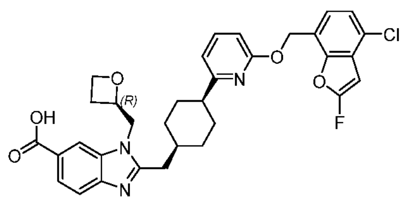,

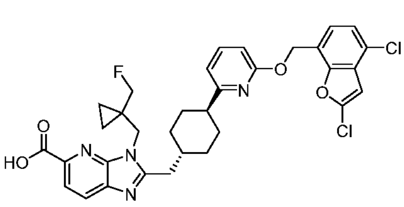,

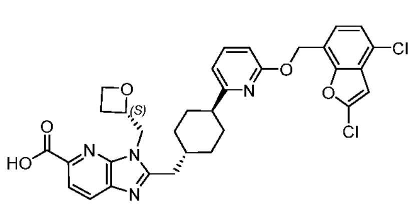,

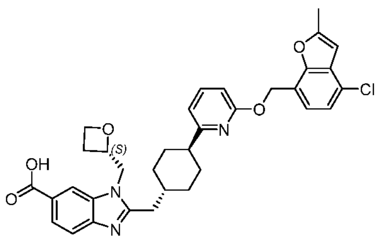,

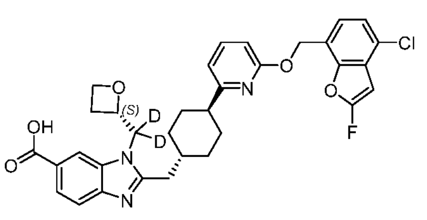,

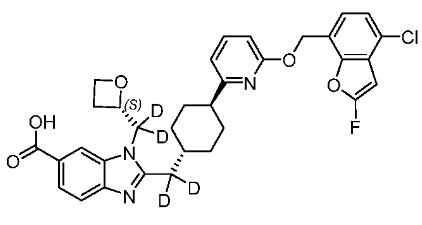,

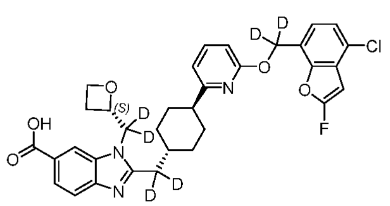,
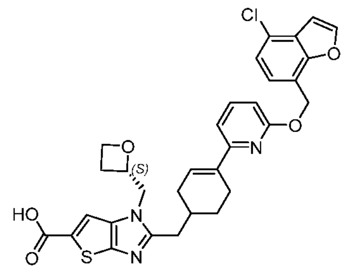,
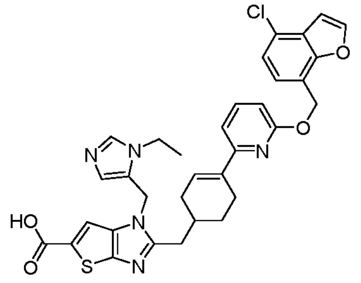,

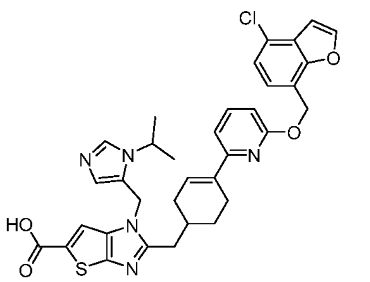,
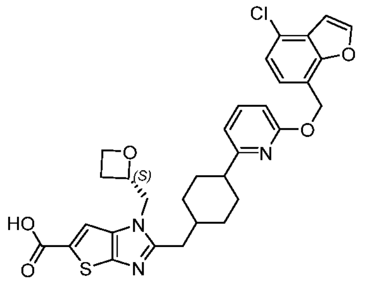,
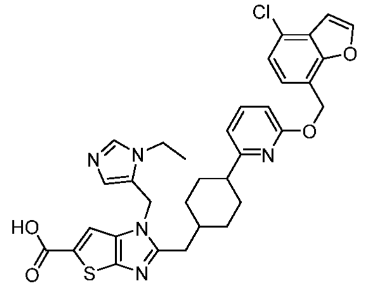,

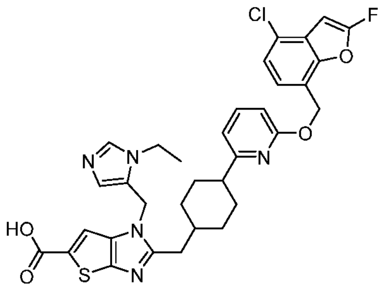,
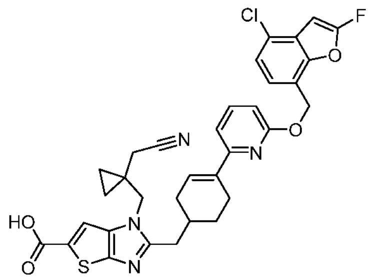,
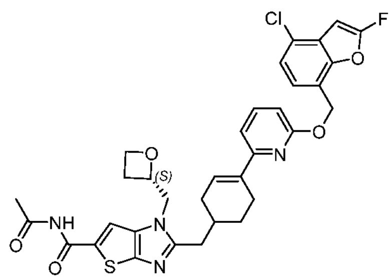,

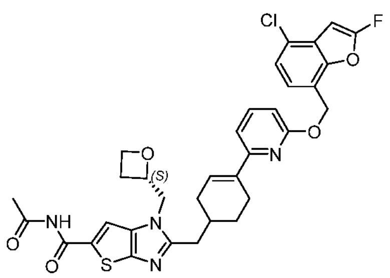,
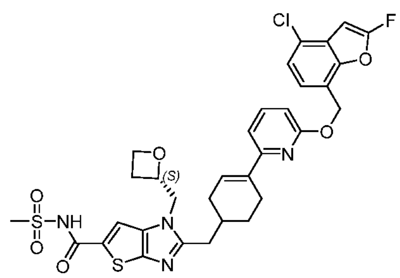,
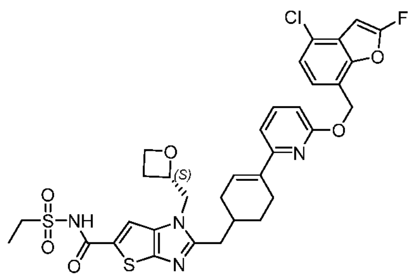,

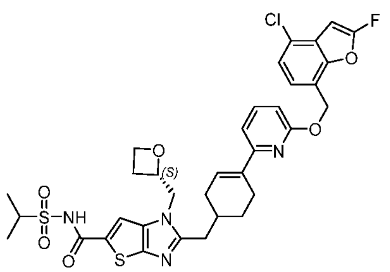,
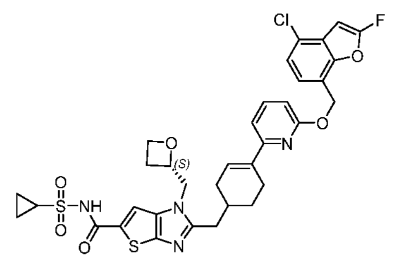,
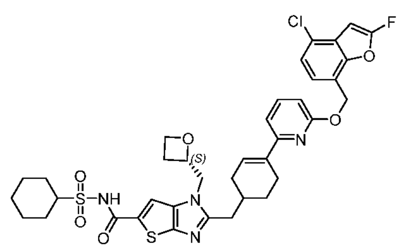,

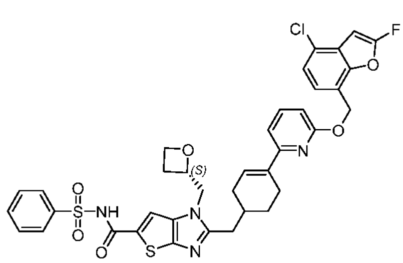
,
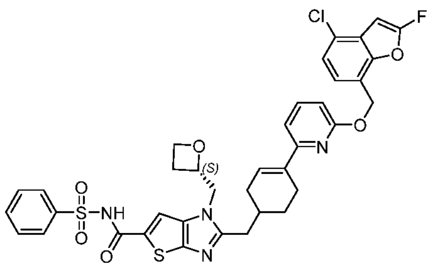
,
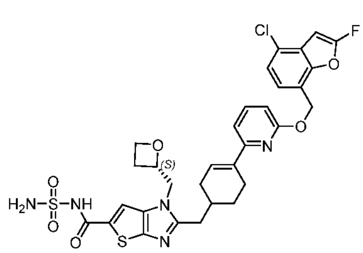
,
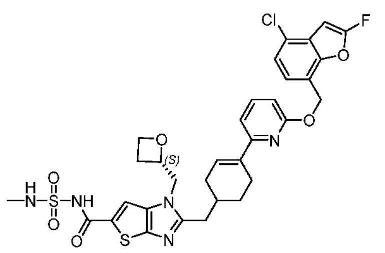
,
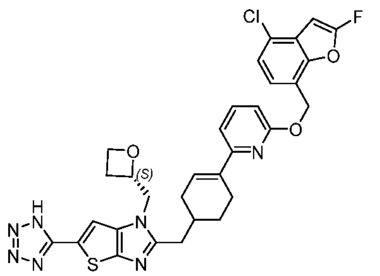
,
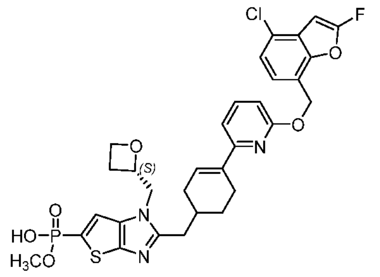
,
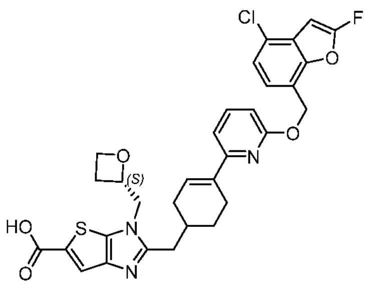
,
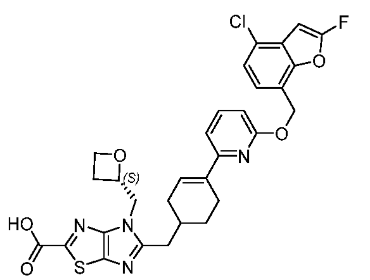
,
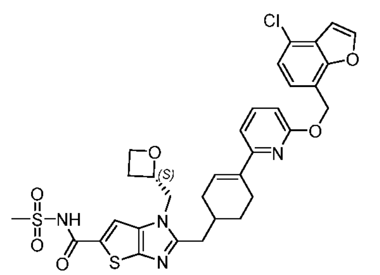
,
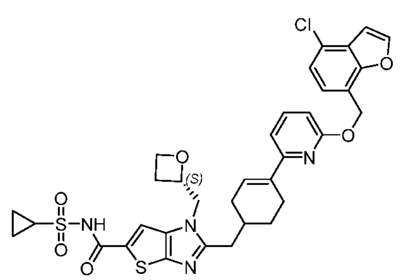
,
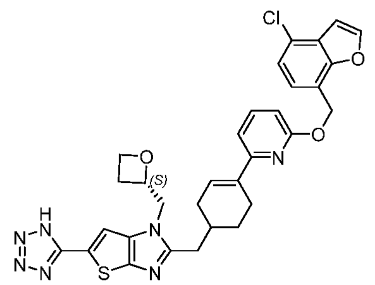
,
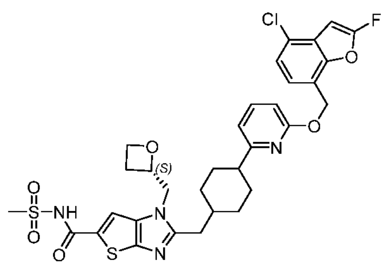
,
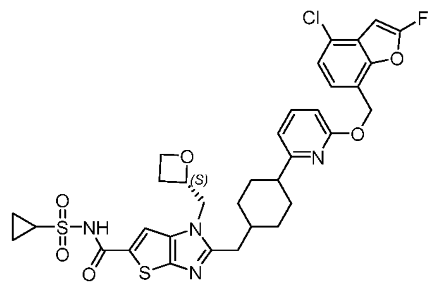
,
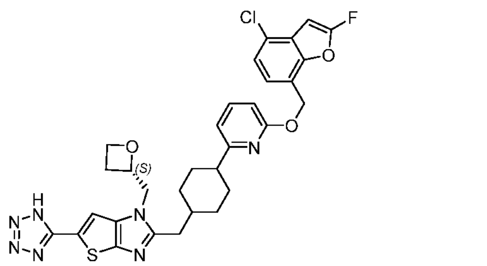
,

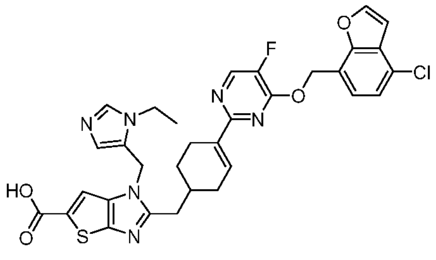
,
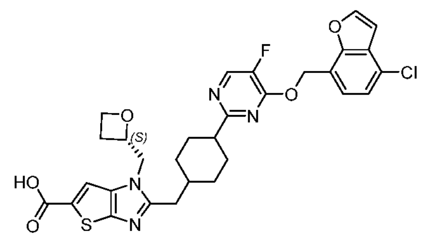
,
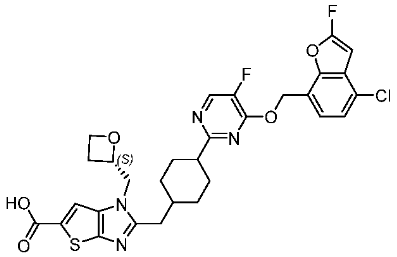
,
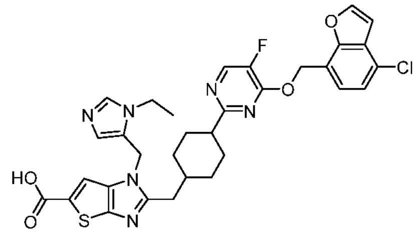
,
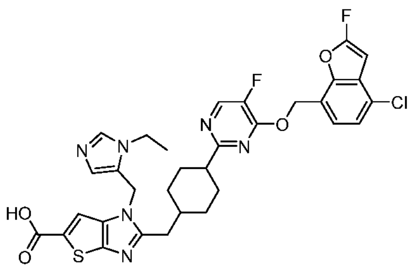
,
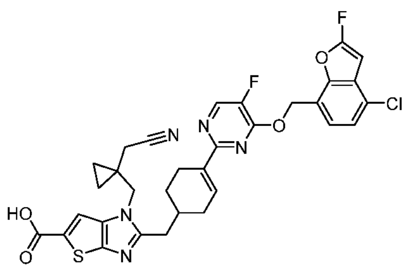
,
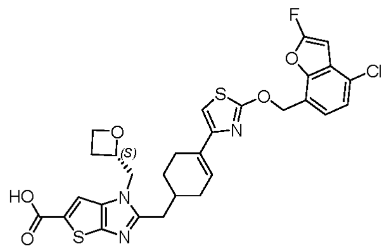
,
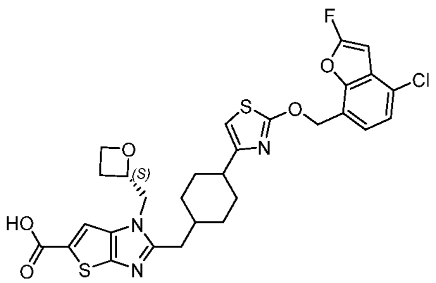
,
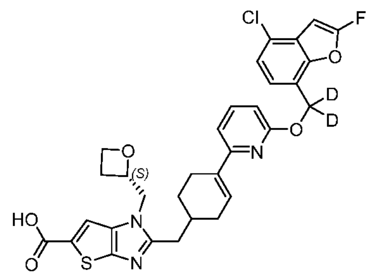
,
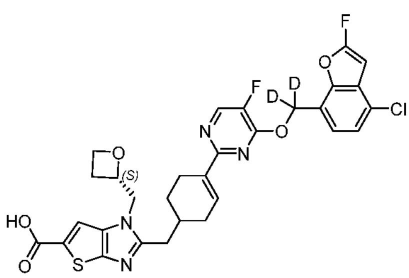
,
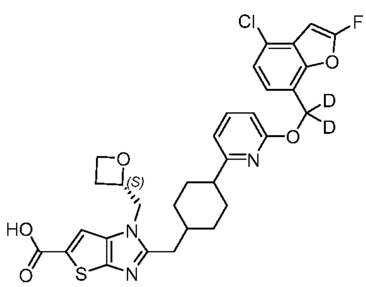
,
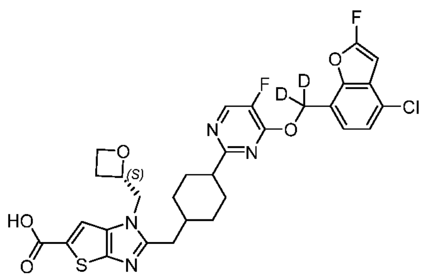
,

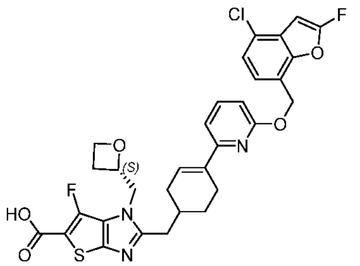,
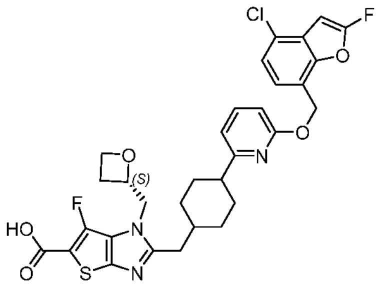,
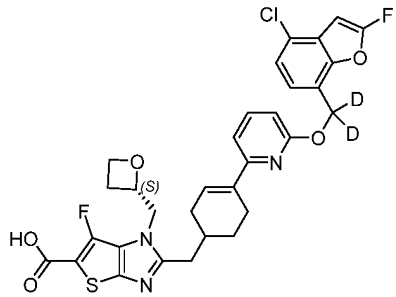,
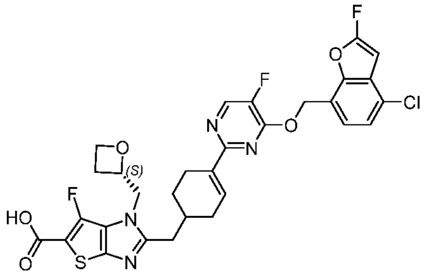,
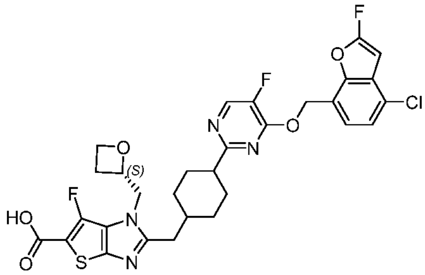,
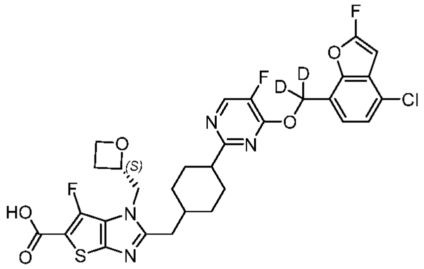,
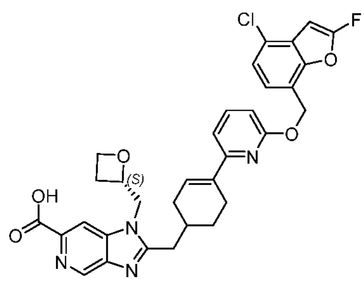,
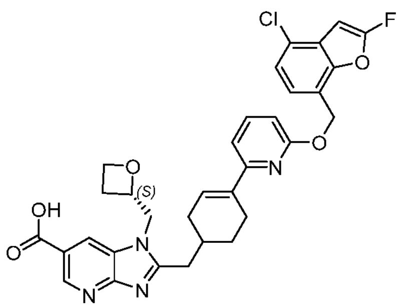,
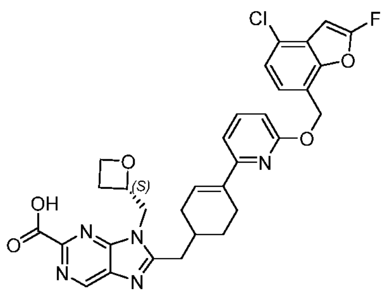,
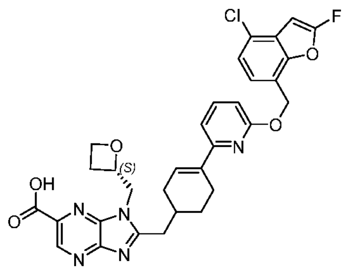,
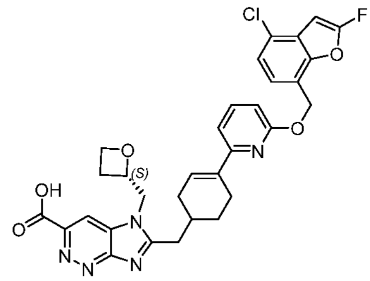,
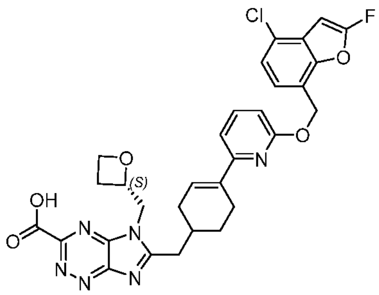,
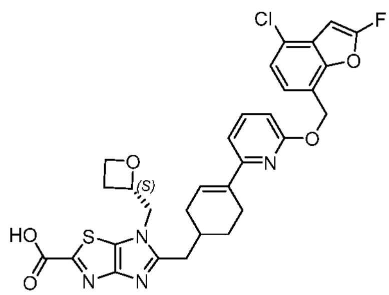,
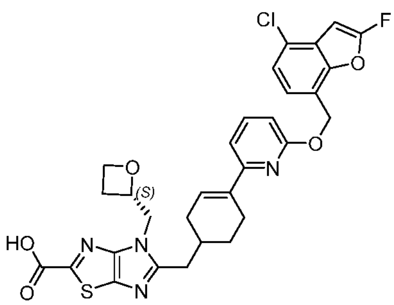.

In one aspect, provided herein is a pharmaceutical composition comprising the aforementioned compound the stereoisomer, the geometric isomer, the tautomer, the *N*-oxide, the hydrate, the solvate, the metabolite, the pharmaceutically acceptable salt or the prodrug thereof.

In some embodiments, provided herein is the aforementioned pharmaceutical composition further comprising a pharmaceutically acceptable carrier, excipient, diluent, auxiliary agent, vehicle or any combination thereof.

In one aspect, provided herein is a use of the aforementioned compound or the stereoisomer, the geometric isomer, the tautomer, the *N*-oxide, the hydrate, the solvate, the metabolite, the pharmaceutically acceptable salt or the prodrug thereof or the aforementioned pharmaceutical compositionin the manufacture of a medicament for preventing, managing, treating or lessening a disease mediated by GLP-1 receptor agonist.In one aspect, provided herein is a use of the aforementioned compound or the stereoisomer, the geometric isomer, the tautomer, the *N*-oxide, the hydrate, the solvate, the metabolite, the pharmaceutically acceptable salt or the prodrug thereof or the aforementioned pharmaceutical composition in the manufacture of a medicament for preventing and/or treating a disease associated with signaling pathways downstream of the GLP-1 receptor.

In some embodiments, the disease associated with signaling pathways downstream of the GLP-1 receptor is selected from: diabetes, diabetic retinopathy, diabetic cerebrovascular disease, diabetic neuropathy, insulin resistance, hyperglycemia, diabetic nephropathy, hypertension, cataracts, osteoporosis, hyperuricemia and infections caused by diabetes, obesity, metabolic syndrome, dyslipidemia, non-alcoholic fatty liver disease, non-alcoholic fat hepatitis, fibrosis, heart disease, stroke, cirrhosis, liver cancer, metabolic acidosis, ketosis, cardiovascular complaints, epilepsy, atherosclerosis, Parkinson's disease and Alzheimer's disease.

In another aspect, provided herein is a use of the aforementioned compound or the stereoisomer, the geometric isomer, the tautomer, the *N*-oxide, the hydrate, the solvate, the metabolite, the pharmaceutically acceptable salt or the prodrug thereof or the aforementioned pharmaceutical composition in the manufacture of a medicament for promoting insulin secretion or lowering blood glucose.

In another aspect, provided herein is a use of the compound or the stereoisomer, the geometric isomer, the tautomer, the *N*-oxide, the hydrate, the solvate, the metabolite, the pharmaceutically acceptable salt or the prodrug thereof or the aforementioned pharmaceutical composition in the manufacture of a medicament for preventing and/or treating diabetes.

In some embodiments, the diabetes is type I diabetes, type II diabetes, gestational diabetes, idiopathic type I diabetes, early-onset type II diabetes, adult-onset diabetes of the young, juvenile-onset diabetes atypical diabetes, malnutrition-related diabetes or latent autoimmune diabetes in adults.

In one aspect, provided herein is the aforementioned compound or a stereoisomer, a geometric isomer, a tautomer, an *N*-oxide, a hydrate, a solvate, a metabolite, a pharmaceutically acceptable salt or a prodrug thereof or a pharmaceutical composition for use in preventing, managing, treating or lessening a disease mediated by GLP-1 receptor agonist.

In one aspect, provided herein is the aforementioned compound or the stereoisomer, the geometric isomer, the tautomer, the *N*-oxide, the hydrate, the solvate, the metabolite, the pharmaceutically acceptable salt or the prodrug thereof or the aforementioned pharmaceutical composition for use in preventing and/or treating a disease associated with signaling pathways downstream of the GLP-1 receptor.

In some embodiments, the disease associated with signaling pathways downstream of the GLP-1 receptor is selected from: diabetes, diabetic retinopathy, diabetic cerebrovascular disease, diabetic neuropathy, insulin resistance, hyperglycemia, diabetic nephropathy, hypertension, cataracts, osteoporosis, hyperuricemia and infections caused by diabetes, obesity, metabolic syndrome, dyslipidemia, non-alcoholic fatty liver disease, non-alcoholic fat hepatitis, fibrosis, heart disease, stroke, cirrhosis, liver cancer, metabolic acidosis, ketosis, cardiovascular complaints, epilepsy, atherosclerosis, Parkinson's disease and Alzheimer's disease.

In another aspect, provided herein is the aforementioned compound or the stereoisomer, the geometric isomer, the tautomer, the *N*-oxide, the hydrate, the solvate, the metabolite, the pharmaceutically acceptable salt or the prodrug thereof or the aforementioned pharmaceutical composition for use in promoting insulin secretion or lowering blood glucose.

In another aspect, provided herein is a use of the compound or the stereoisomer, the geometric isomer, the tautomer, the *N*-oxide, the hydrate, the solvate, the metabolite, the pharmaceutically acceptable salt or the prodrug thereof or the aforementioned pharmaceutical composition in the manufacture of a medicament for preventing and/or treating diabetes.

In some embodiments, the diabetes is type I diabetes, type II diabetes, gestational diabetes, idiopathic type I diabetes, early-onset type II diabetes, adult-onset diabetes of the young, juvenile-onset diabetes atypical diabetes, malnutrition-related diabetes or latent autoimmune diabetes in adults.

In one aspect, provided herein is a method of preventing, managing, treating or lessening a disease mediated by GLP-1 receptor agonist comprising administering to a subject in need thereof the aforementioned compound or the stereoisomer, the geometric isomer, the tautomer, the *N*-oxide, the hydrate, the solvate, the metabolite, the pharmaceutically acceptable salt or the prodrug thereof or the aforementioned pharmaceutical compositionin.

In one aspect, provided herein is a method of preventing and/or treating a disease associated with signaling pathways downstream of the GLP-1 receptor comprising administering to a subject in need thereof the aforementioned

compound or the stereoisomer, the geometric isomer, the tautomer, the *N*-oxide, the hydrate, the solvate, the metabolite, the pharmaceutically acceptable salt or the prodrug thereof or the aforementioned pharmaceutical composition.

In some embodiments, the disease associated with signaling pathways downstream of the GLP-1 receptor is selected from: diabetes, diabetic retinopathy, diabetic cerebrovascular disease, diabetic neuropathy, insulin resistance, hyperglycemia, diabetic nephropathy, hypertension, cataracts, osteoporosis, hyperuricemia and infections caused by diabetes, obesity, metabolic syndrome, dyslipidemia, non-alcoholic fatty liver disease, non-alcoholic fat hepatitis, fibrosis, heart disease, stroke, cirrhosis, liver cancer, metabolic acidosis, ketosis, cardiovascular complaints, epilepsy, atherosclerosis, Parkinson’s disease and Alzheimer’s disease.

In another aspect, provided herein is a method of promoting insulin secretion or lowering blood glucose comprising administering to a subject in need thereof the aforementioned compound or the stereoisomer, the geometric isomer, the tautomer, the *N*-oxide, the hydrate, the solvate, the metabolite, the pharmaceutically acceptable salt or the prodrug thereof or the aforementioned pharmaceutical composition.

In another aspect, provided herein is a method of preventing and/or treating diabetes comprising administering to a subject in need thereof the compound or the stereoisomer, the geometric isomer, the tautomer, the *N*-oxide, the hydrate, the solvate, the metabolite, the pharmaceutically acceptable salt or the prodrug thereof or the aforementioned pharmaceutical composition.

In some embodiments, the diabetes is type I diabetes, type II diabetes, gestational diabetes, idiopathic type I diabetes, early-onset type II diabetes, adult-onset diabetes of the young, juvenile-onset diabetes atypical diabetes, malnutrition-related diabetes or latent autoimmune diabetes in adults.

## DEFINITIONS AND GENERAL TERMINOLOGY

The present invention will list the references corresponding to the determined content in detail. Examples are accompanied by structural and chemical formulas. The present invention is expected to cover all alternatives, variants and equivalents, which may be included within the scope of the invention as defined in claim. Those skilled in the art will recognize many methods and materials similar or equivalent to those described herein, which may be applied to the practice of the present invention. The present invention is by no means limited to the methods and materials described herein. There are a lot of literatures and similar materials distinguished or inconsistent with the present invention, including but not limited to the definitions of terms, the use of terminology, described techniques, or the like, this application controls.

The following terms are used in the description unless otherwise stated. For purposes of this invention, the chemical elements are defined in accordance with the Periodic Table of the elements, CAS version and chemical manuals, 75, thEd, 1994. Additionally, general principles of organic chemistry are described in "Organic Chemistry", Thomas Sorrell, University Science Books, Sausalito: 1999, and Smith et al., and "March’s Advanced Organic Chemistry", John Wiley&Sons, New York: 2007, all of which are incorporated herein by reference in their entireties.

The term "comprise" is an open-ended expression, it includes the contents disclosed herein, but don’t exclude other contents.

As described herein, compounds disclosed herein may optionally be substituted with one or more substituents, such as general formula compound in the invention, or as exemplified by particular classes, subclasses, and species of the invention. It will be appreciated that the phrase "optionally substituted" is used interchangeably with the phrase "substituted or unsubstituted". In general, the term "optionally" whether or not located before the term "substituted" refers to the replacement of one or more hydrogen radicals in a given structure with the radical of a specified substituent. Unless otherwise indicated, an optionally substituted group may have a substituent at each substitutable position of the group. When more than one position in a given structure can be substituted with more than one substituent selected from a specified group, the substituent may be either the same or different at each position. Wherein the substituents may be, but are not limited to H, F, Cl, Br, I, nitro, cyano, oxo(=O), hydroxy, alkyl, hydroxyalkyl, alkylamino, aminoalkyl, haloalkoxy, cycloalkyl, amino, aryl, heterocyclyl, heteroaryl, alkenyl, alkynyl, cycloalkyloxy, alkoxy, alkoxyalkyl, haloalkyl, -COOH, -alkylene-C(=O)O-alkyl, -alkylene-$S(=O)_2$-alkyl, -alkylene-$S(=O)_2$-amino, -$S(=O)_2$-alkyl, -$S(=O)_2$-amino, - $S(=O)_2OH$, -O-alkylene-C(=O)O-alkyl, -O-alkylene-$S(=O)_2$-alkyl, -O-alkylene-$S(=O)_2$-amino, -O-alkylene$S(=O)_2OH$, -C(=O)$NH_2$, -C(=O)NH-alkyl, -C(=O)N(alkyl)-alkyl, -C(=O)NH$S(=O)_2$-alkyl, -C(=O)NH$S(=O)_2$-amino, -C(=O)NH$S(=O)_2OH$, -N(haloalkyl)-alkyl, -N(alkyl)-$S(=O)_2$-alkyl, -NH$S(=O)_2$-alkyl, -NHS(=O)z-haloalkyl, -N(alkyl)$S(=O)_2$-haloalkyl, -N(alkyl) $S(=O)_2$-alkylamino, -NHC(=O)-alkyl, -NHC(=O)-haloalkyl, - N(alkyl)C(=O)-haloalkyl, -N(alkyl)C(=O)-alkylamino, -N(alkyl)C(=O)O-alkyl, -NHC(=O)O-alkyl, - NHC(=O)O-haloalkyl, -N(alkyl)C(=O)O-haloalkyl, -N(alkyl)C(=O) O-aminoalkyl, -NHC(=O)-NH2, - NHC(=O)NH-(alkyl), -NHC(=O)NH(haloalkyl), -NHC(=O)N(alkyl)-alkyl, -OC(=O)-alkyl, -OC(=O)-amino, - OC(=O)-alkylamino, -OC(=O)-aminoalkyl, -OC(=O)-alkoxy, -C(=O)N(alkyl)$S(=O)_2$-alkyl, - C(=O)N(alkyl)$S(=O)_2$-amino, -C(=O)NH-$S(=O)_2OH$, -C(=NH)$NH_2$, -C(=NH)NH-alkyl, -C(=NH)N(alkyl)-alkyl, -C(=N-alkyl)-$NH_2$, -C(=O)NH-alkylene-$S(=O)_2OH$, -C(=O)NHC(=O)OH, -C(=O)NHC(=O)O-alkyl, - C(=O)N(alkyl)C(=O)O-alkyl, -C(=O)NH-alkylene-C(=O)OH and -C(=O)NH-alkylene-C(=O)O-alkyl, and the like.

The term "alkyl" refers to a saturated linear or branched-chain monovalent hydrocarbon radical of 1 to 20 carbon

atoms, or 1 to 10 carbon atoms, or 1 to 6 carbon atoms, or 1 to 4 carbon atoms, or 1 to 3 carbon atoms, or 1 to 2 carbon atoms, wherein the alkyl radical may be optionally and independently substituted with one or more substituents described herein. Some non-limiting examples of the alkyl group include, methyl (Me, $-CH_3$), ethyl (Et, $-CH_2CH_3$), n-propyl (n-Pr, $-CH_2CH_2CH_3$), isopropyl (i-Pr, $-CH(CH_3)_2$), n-butyl (n-Bu, $-CH_2CH_2CH_2CH_3$), isobutyl (i-Bu, $-CH_2CH(CH_3)_2$), sec-butyl (s-Bu, $-CH(CH_3)CH_2CH_3$), tert-butyl (t-Bu, $-C(CH_3)_3$), n-pentyl ($-CH_2CH_2CH_2CH_2CH_3$), 2-pentyl ($-CH(CH_3)CH_2CH_2CH_3$), 3-pentyl ($-CH(CH_2CH_3)_2$), 2-methyl-2-butyl($-C(CH_3)_2CH_2CH_3$), 3-methyl-2-butyl($-CH(CH_3)CH(CH_3)_2$), 3-methyl-1-butyl ($-CH_2CH_2CH(CH_3)_2$), 2-methyl-1-butyl($-CH_2CH(CH_3)CH_2CH_3$), n-hexyl ($-CH_2CH_2CH_2CH_2CH_2CH_3$), 2-hexyl ($-CH(CH_3)CH_2CH_2CH_2CH_3$), 3-hexyl ($-CH(CH_2CH_3)(CH_2CH_2CH_3)$), 2-methyl-2-pentyl ($-C(CH_3)_2CH_2CH_2CH_3$), 3-methyl-2-pentyl ($-CH(CH_3)CH(CH_3)CH_2CH_3$), 4-methyl-2-pentyl ($-CH(CH_3)CH_2CH(CH_3)_2$), 3-methyl-3-pentyl ($-C(CH_3)(CH_2CH_3)_2$), 2-methyl-3-pentyl ($-CH(CH_2CH_3)CH(CH_3)_2$), 2,3-dimethyl-2-butyl ($-C(CH_3)_2CH(CH_3)_2$), 3,3-dimethyl-2-butyl ($-CH (CH_3)C(CH_3)_3$, n-heptyl and n-octyl, etc. The term "alkyl" or the prefix "alk-" is inclusive of both straight chain and branched saturated carbon chain. The term "alkylene" used herein refers to a saturated divalent hydrocarbon group derived from a straight or branched chain saturated hydrocarbon by the removal of two hydrogen atoms, such exaples include, but are not limited to methylene, ethylidene and isopropylidene, and the like.

The term "alkenyl" refers to a linear or branched chain monovalent hydrocarbon radical of 2 to 12 carbon atoms, or 2 to 8 carbon atoms, or 2 to 6 carbon atoms, or 2 to 4 carbon atoms, with at least one site of unsaturation, i.e., a carbon-carbon, $sp^2$ double bond, wherein the alkenyl radical may be independently and optionally substituted with one or more substituents described herein, and includes radicals having "cis" and "trans" orientations, or alternatively, "E" and "Z" orientations. Examples include, but are not limited to, ethenyl or vinyl ($-CH=CH_2$), allyl ($-CH_2CH=CH_2$), and the like.

The term "alkynyl" refers to a linear or branched chain monovalent hydrocarbon radical of 2 to 12 carbon atoms, or 2 to 8 carbon atoms, or 2 to 6 carbon atoms, or 2 to 4 carbon atoms, with at least one site of unsaturation, i.e., a carbon-carbon, sp triple bond, wherein the alkynyl radical is independently and optionally substituted with one or more substituents described herein. Examples include, but are not limited to, ethynyl ($-C\equiv CH$), propargyl ($-CH_2C\equiv CH$), and the like.

The term "heteroatom" refers to one or more of O, S, N, P and Si, including any oxidized form of C, N, S, or P; the quaternized form of any basic N; or a substitutable nitrogen of a heterocyclic ring, for example, N (as in 3, 4-dihydro-2H-pyrrolyl), NH (as in pyrrolidinyl) or NR (as in N-substituted pyrrolidinyl); or $-CH_2-$ of a heterocyclic ring is oxidized to form -C(=O)- form.

The term "halogen" or "halo" refers to fluoro (F), chloro (Cl), bromo (Br), or iodo (I).

The term "deuterium" refers to heavy hydrogen (D).

The term "unsaturated" refers to a moiety having one or more units of unsaturation.

The term "alkoxy" refers to an alkyl group, as defined herein, attached to the remaining part of the molecule through an oxygen atom. In some embodiments, alkoxy is $C_{1-4}$ alkoxy. Examples include, but are not limited to, methoxy, ethoxy, propoxy, butoxy, and the like. The alkoxy group may be optionally substituted with one or more substituents disclosed herein.

The term "alkylthio" refers to an alkyl group, as defined herein, attached to the remaining part of the molecule through an sulfur atom. In some embodiments, alkylthio is $C_{1-4}$ alkylthio. Examples include, but are not limited to, methylthio, ethylthio, propylthio, and butylthio, and the like. The alkylthio group may be optionally substituted with one or more substituents disclosed herein.

The term "alkylamino" as defined herein, relates to an alkyl group, as defined in the present invention, attached to the rest of the compound molecule by an N atom. In some embodiments, the alkylamino group is $C_{1-4}$ alkylamino. Some non-limiting examples of such group include, N-methylamino, *N*-ethylamino, *N, N*-dimethylamino, *N, N*-diethylamino, and the like. And wherein the alkylamino radical is optionally substituted with one or more substituents described herein.

The term "cycloalkyl" or "cycloalkane" refers to a monovalent or multivalent saturated ring having 3 to 12 carbon atoms as a monocyclic, bicyclic, or tricyclic carbon ring system, but not an aromatic ring. In some embodiments, the cycloalkyl group contains 3 to 12 carbon atoms. In other embodiments, the cycloalkyl group contains 3 to 8 carbon atoms. In still other embodiments, the cycloalkyl group contains 3 to 6 carbon atoms. Examples include, but are not limited to, cyclopropyl, cyclobutyl, cyclopentyl, cyclohexyl, and the like. The cycloalkyl group may be optionally substituted with one or more substituents disclosed herein.

The term "heterocycle", "heterocyclyl", or "heterocyclic ring" as used interchangeably herein refers to a saturated or partially unsaturated monocyclic, bicyclic or tricyclic ring containing 3 to 12 ring atoms, but not an aromatic ring, of which at least one ring atom is a heteroatom. In some embodiments, the heterocycle group contains 3 to 10 ring atoms. In other embodiments, the cycloalkyl group contains 3 to 8 ring atoms. In other embodiments, the cycloalkyl group contains 5 to 8 ring atoms. In other embodiments, the cycloalkyl group contains 3 to 6 ring atoms. In other embodiments, the cycloalkyl group contains 5 to 6 ring atoms. In other embodiments, the cycloalkyl group contains 4 to 6 ring atoms. Unless otherwise specified, the heterocyclyl group may be carbon or nitrogen linked, and the heteroatom is as defined herein. Some non-limiting examples of the heterocyclyl group include oxiranyl, azetidinyl, oxetanyl, thietanyl, pyrrolidinyl, 2-pyrrolinyl, 3-pyrrolinyl, pyrazolinyl, pyrazolidinyl, imidazolinyl, imidazolidinyl, tetrahydrofuranyl, dihydrofuranyl, tetrahydrothienyl, dihydrothienyl, 1, 3-dioxolanyl, dithiolanyl, tetrahydropyranyl, dihydropyranyl, 2H-pyranyl, 4H-pyranyl, tetrahydrothiopyranyl, piperidinyl, morpholinyl, thiomorpholinyl, piperazinyl, dioxanyl, dithianyl, thioxanyl, homopiperazinyl, homopiperidinyl, oxepanyl, thiepanyl, oxazepinyl, diazepinyl, thiazepinyl and 2-oxa-5-azabicyclo [2.2.1] hept-5-yl. Some non-limiting examples of heterocyclyl wherein-$CH_2$-group is replaced by-C (=O) -moiety include 2-oxopyrrolidinyl, oxo-1, 3-thiazolidinyl, 2-piperidinonyl, 3, 5-dioxopiperidinyl and pyrimidinedionyl. Some non-limited examples of heterocyclyl wherein the ring sulfur atom is oxidized include sulfolanyl, 1, 1-dioxo-thiomorpholinyl. The heterocyclyl group may be optionally substituted with one or more substituents disclosed herein.

The term "aryl" refers to monocyclic, bicyclic and tricyclic carbocyclic ring systems having a total of 6 to 14 ring members, or 6 to 12 ring members, or 6 to 10 ring members, wherein at least one ring in the system is aromatic, wherein each ring in the system contains 3 to 7 ring members, and that has a single point or multipoint of attachment to the rest of the molecule. The term "aryl" and "aromatic ring" can be used interchangeably herein. Examples of aryl ring may include phenyl, naphthyl and anthracene. The aryl group may be optionally and independently substituted with one or more substituents disclosed herein.

The term "heteroaryl" refers to monocyclic, bicyclic and tricyclic carbocyclic ring systems having a total of 5 to 12 ring members, or 5 to 10 ring members, or 5 to 6 ring members, wherein at least one ring system is an aromatic ring, and at least one ring system contains one or more hetero atoms, and wherein each ring in the system contains 5 to 7 ring members and that has a single point or multipoint of attachment to the rest of the molecule. The term "hetreroaryl" and "heteroaromatic ring" or "heteroaromatic compound" can be used interchangeably herein. The heteroaryl group is optionally substituted with one or more substituents disclosed herein. In one embodiment, a 5-10 membered heteroaryl group comprises 1, 2, 3 or 4 heteroatoms independently selected from O, S and N, wherein the nitrogen atom can be further oxidized.

Some non-limiting examples of heteroaryl rings include furanyl, imidazolyl (e.g., N-imidazolyl, 2-imidazolyl, 4-imidazolyl, 5-imidazolyl), isoxazolyl, oxazolyl (e.g. 2-oxazolyl, 4-oxazolyl, 5-oxazolyl), pyrrolyl (e.g., N-pyrrolyl, 2-pyrrolyl, 3-pyrrolyl), pyridyl, pyrimidinyl (e.g. 2-pyrimidinyl, 4-pyrimidinyl, 5-pyrimidinyl), pyridazinyl, thiazolyl (e.g., 2-thiazolyl, 4-thiazolyl, 5-thiazolyl), tetrazolyl (e.g., 5-tetrazolyl), triazolyl, thienyl (e.g., 2-thienyl, 3-thienyl), pyrazolyl, isothiazolyl, 1, 2, 3-oxadiazolyl, 1, 2, 5-oxadiazolyl, 1, 2, 4-oxadiazolyl, 1, 2, 3-triazolyl, 1, 2, 3-thiadiazolyl, 1, 3, 4-thiadiazolyl, 1, 2, 5-thiadiazolyl, pyrazinyl, 1, 3, 5-triazinyl; and the following bicycles: benzimidazolyl, benzofuryl, benzothiophenyl, indolyl (e.g., 2-indolyl), purinyl, quinolinyl (e.g., 2-quinolinyl, 3-quinolinyl, 4-quinolinyl), 1, 2, 3, 4-tetrahydroisoquinolinyl, 1, 3-benzodioxolyl, indolinyl, isoquinolinyl (e.g., 1-isoquinolinyl, 3-isoquinolinyl or 4-isoquinolinyl), imidazo [1, 2-*a*] pyridyl, pyrazolo [1, 5-*a*] pyridyl, pyrazolo [1, 5-*a*] pyrimidyl, imidazo [1, 2-b] pyridazinyl, [1, 2, 4] triazolo [4, 3-*b*] pyridazinyl, [1, 2, 4] triazolo [1, 5-*a*] pyrimidinyl, and [1, 2, 4] triazolo [1, 5-*a*] pyridyl, and the like.

As described herein, substituents connected to the center position of the ring through a bond represent that the ring to which substituents connected may be substituted in any reasonable and connectable position. For example, formula (a) represents pyridine ring can be substituted with one or more R substituents in any reasonable and substitutable position.

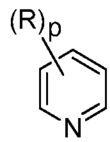
(a)

As described herein, a linkage bond connected to the center position of the ring through a bond represent that the ring to which linkage bond connected may be connected in any reasonable and connectable position. For example, formula (b) represents octahydrocyclopenta[c]pyrrole can be connected to the rest of the molecule in any reasonable and substitutable position.

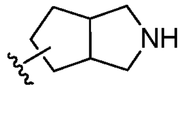
(b)

Furthermore, unless otherwise stated, the phrase "each... is independently" is used interchangeably with the phrase "each (of) ... and... is independently". It should be understood broadly that the specific options expressed by the same symbol are independent of each other in different radicals; or the specific options expressed by the same symbol are independent of each other in same radicals.

Unless otherwise stated, structures depicted herein are also meant to include all isomeric (e.g., enantiomeric, diastereomeric, geometric or conformational) forms of the structure; for example, the R and S configurations for each asymmetric center, (*Z*) and (*E*) double bond isomers, and (*Z*) and (*E*) conformational isomers. Therefore, single stereochemical isomers as well as enantiomeric, diastereomeric, geometric, or conformational mixtures of the present

compounds are within the scope disclosed herein.

Unless otherwise stated, structures and compounds depicted herein are also meant to include all isomeric (e.g., enantiomeric, diastereomeric, geometric or conformational) forms, N-oxide, hydrate, solvate, metabolite, pharmaceutically acceptable salt or a prodrug thereof. Therefore, single stereochemical isomers as well as enantiomer, diastereomer, geometric isomer, conformer, *N*-oxide, anhydrate, solvate, metabolite, pharmaceutically acceptable salt or prodrug of the compounds disclosed herein are within the scope of the present invention. Additionally, unless otherwise stated, structures depicted herein are also meant to include compounds that differ only in the presence of one or more isotopically enriched atoms.

A "metabolite" refers to a product produced through metabolism in the body of a specified compound, a pharmaceutically acceptable salt, analog or derivative, which exhibits similar activity of the compound of Formula (X) in vivo or in vitro. The metabolites of a compound may be identified using routine techniques known in the art and their activities can be determined using tests such as those described herein. Such products may result for example from oxidation, reduction, hydrolysis, amidation, deamidation, esterification, deesterification, or enzyme cleavage, and the like, of the administered compound. Accordingly, the invention includes metabolites of the compounds disclosed herein, including metabolites produced by contacting a compound disclosed herein with a mammal for a sufficient time period.

Stereochemical definitions and conventions used herein generally follow S. P. Parker, Ed., McGraw-Hill Dictionary of Chemical Terms (1984) McGraw-Hill Book Company, New York; and Eliel, E. and Wilen, S., "Stereochemistry of Organic Compounds", John Wiley&Sons, Inc., New York, 1994. The compounds disclosed herein may contain asymmetric or chiral centers, and therefore exist in different stereoisomeric forms. It is intended that all stereoisomeric forms of the compounds disclosed herein, including, but not limited to, diastereomers, enantiomers and atropisomers, as well as mixtures thereof such as racemic mixtures, form part of the present invention. Many organic compounds exist in optically active forms, i.e., they have the ability to rotate the plane of plane-polarized light. In describing an optically active compound, the prefixes D andL, orR and S, are used to denote the absolute configuration of the molecule about its chiral center (s). The prefixes d and 1 or (+) and (-) are employed to designate the sign of rotation of plane-polarized light by the compound, with (-) or l meaning that the compound is levorotatory. A compound prefixed with (+) or d is dextrorotatory. For a given chemical structure, these stereoisomers are identical except that they are mirror images of one another. A specific stereoisomer may also be referred to as an enantiomer, and a mixture of such isomers is often called an enantiomeric mixture. A 50: 50 mixture of enantiomers is referred to as a racemic mixture or a racemate, which may occur where there has been no stereoselection or stereospecificity in a chemical reaction or process. The term "racemic mixture" or "racemate" refers to an equimolar mixture of two enantiomeric species, devoid of optical activity.

The term "tautomer" or "tautomeric form" refers to structural isomers of different energies which are interconvertible via a low energy barrier. Some non-limiting examples of proton tautomers (also known as prototropic tautomers) include interconversions via migration of a proton, such as keto-enol and imine-enamine isomerizations. Valence tautomers include interconversions by reorganization of some of the bonding electrons.

A "pharmaceutically acceptable salt" refers to organic or inorganic salt of a compound disclosed herein. Pharmaceutically acceptable salts are well known in the art. For example, Berge et al., describe pharmaceutically acceptable salts in detail in J. Pharmacol Sci, 1977, 66: 1-19, which is incorporated herein by reference. Some non-limiting examples of pharmaceutically acceptable and nontoxic salts include salts of an amino group formed with inorganic acids such as hydrochloric acid, hydrobromic acid, phosphoric acid, sulfuric acid and perchloric acid or with organic acids such as acetic acid, oxalic acid, maleic acid, tartaric acid, citric acid, succinic acid and malonic acid or by using other methods used in the art such as ion exchange. Other pharmaceutically acceptable salts include adipate, malate, 2-hydroxy propionate, alginate, ascorbate, aspartate, benzenesulfonate, benzoate, bisulfate, borate, butyrate, camphorate, camphorsulfonate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, laurylsulfate, malate, methanesulfonate, 2-naphthalenesulfonate, nicotinate, nitrate, oleate, palmitate, pamoate, pectinate, persulfate, 3-phenylpropionate, picrate, pivalate, propionate, stearate, thiocyanate,p-toluenesulfonate, undecanoate, valerate salts, and the like. Salts derived from appropriate bases include alkali metal, alkaline earth metal, ammonium and $N^+$ $(C_{1-4}$ alkyl$)_4$ salts. This invention also envisions the quaternization of any basic nitrogen-containing groups of the compounds disclosed herein. Water or oil soluble or dispersable products may be obtained by such quaternization. Representative alkali or alkaline earth metal (formed) salts include sodium, lithium, potassium, calcium, magnesium, and the like. Further pharmaceutically acceptable salts include, when appropriate, nontoxic ammonium, quaternary ammonium, and amine cations formed using counterions such as halide, hydroxide, carboxylate, sulfate, phosphate, nitrate, $C_{1-8}$ sulfonate or aryl sulfonate.

The term "hydrate" refers to the complex where the solvent molecule is water.

The term "solvate" refers to an association or complex of one or more solvent molecules and a compound disclosed herein. Some non-limiting examples of the solvent that form solvates include water, isopropanol, ethanol, methanol, dimethylsulfoxide (DMSO), ethyl acetate, acetic acid and ethanolamine.

An "ester" refers to an in vivo hydrolysable ester of a compound of the Formula (X) containing hydroxy group, for

example, a pharmaceutically acceptable ester which is hydrolysed in the human or animal body to produce the parent alcohol. Some non-limiting examples of in vivo) hydrolysable ester forming groups of a compound of the Formula (X) containing hydroxy group include phosphate, acetoxymethoxy, 2, 2-dimethylpropionyloxymethoxy, alkanoyl, benzoyl, phenylacetyl, alkoxycarbonyl, dialkylcarbamoyl, *N*-(dialkylaminoethyl) -*N*-alkylcarbamoyl, and the like.

An "*N*-oxide" refers to one or more than one nitrogen atoms oxidised to form *N*-oxides, where a compound contains several amine functions. Particular examples of N-oxides are the *N*-oxides of a tertiary amine or a nitrogen atom of a nitrogen-containing heterocycle. A-oxides can be formed by treatment of the corresponding amine with an oxidizing agent such as hydrogen peroxide or a per-acid (e.g. a peroxycarboxylic acid) (See, Advanced Organic Chemistiy, by Jerry March, 4th Edition, Wiley Interscience, pages). More particularly, *N*-oxides can be made by the procedure of L. W Deady (Syn. Comm. 1977, 7, 509-514), in which the amine compound is reacted with m-chloroperoxybenzoic acid (MCPBA), for example, in an inert solvent such as dichloromethane.

The term "prodrug" refers to a compound that is transformed in vivo into a compound of Formula (I). Such a transformation can be affected, for example, by hydrolysis of the prodrug form in blood or enzymatic transformation to the parent form in blood or tissue. Prodrugs of the compounds disclosed herein may be, for example, esters. Some common esters which have been utilized as prodrugs are phenyl esters, aliphatic ($C_1$-$C_{24}$) esters, acyloxymethyl esters, carbonates, carbamates and amino acid esters. For example, a compound disclosed herein that contains a hydroxy group may be acylated at this position in its prodrug form. Other prodrug forms include phosphates, such as, those phosphate compounds derived from the phosphonation of a hydroxy group on the parent compound. A thorough discussion of prodrugs is provided in T. Higuchi and V. Stella, Pro-drugs as Novel Delivery Systems, Vol. 14 of the A.C.S. Symposium Series, Edward B. Roche, ed., Bioreversible Carriers in Drug Design, American Pharmaceutical Association and Pergamon Press, 1987, J. Rautio et al., Prodrugs: Design and Clinical Applications, Nature Review Drug Discovery, 2008, 7, 255-270, and S. J. Hecker et al, Prodrugs of Phosphates and Phosphonates, Journal of Medicinal Chemistry, 2008, 51, 2328-2345.

Unless otherwise indicated herein or clearly contradicted by the context, the terms "a", "an", "the" and similar terms used in the context of the present invention (particularly in the context of the claims) are to be construed to cover both the singular and the plural.

As used herein, the term "GLP-1 receptor agonist" refers to a substance that agonizes the activity of the GLP-1 receptor.

## GENERAL SYNTHETIC PROCEDURES

To describe the present invention, examples are listed below. It is to be understood, however, that the present invention is not limited to these examples, but merely provides methods for practicing the invention.

Generally, the compounds disclosed herein may be prepared by methods described herein, wherein the substituents are as defined in the present invention, except where further noted. The following non-limiting schemes and examples are presented to further exemplify the invention.

Persons skilled in the art will recognize that the chemical reactions described may be readily adapted to prepare a number of other compounds disclosed herein, and alternative methods for preparing the compounds disclosed herein are deemed to be within the scope disclosed herein. For example, the synthesis of non-exemplified compounds according to the invention may be successfully performed by modifications apparent to those skilled in the art, e.g., by appropriately protecting interfering groups, by utilizing other suitable reagents known in the art other than those described, and/or by making routine modifications of reaction conditions. Alternatively, the known reaction conditions or the reaction disclosed in the present invention will be recognized as having applicability for preparing other compounds disclosed herein.

In the examples described below, unless otherwise indicated all temperatures are set forth in degrees Celsius. Reagents were purchased from commercial suppliers such as Anhui Zesheng Tech Co., Ltd., ShangHai Shaoyuan Reagent Co., Ltd., Shanghai Meryer Chemical Technology Co., Ltd., Shanghai Macklin Biochemical Technology Co., Ltd., and were used without further purification unless otherwise indicated. Common solvents were purchased from commercial suppliers such as Shantou XiLong Chemical Factory, Guangdong Guanghua Reagent Chemical Factory Co., Ltd., Guangzhou Reagent Chemical Factory, Damao Chemical Reagent Factory, Yantai Jiangyou Silica gel Development Co., Ltd., and Qingdao Ocean Chemical Factory.

Anhydrous THF, *N,N*-dimethylformamide, 1,4-dioxane, toluene, and acetonitrile were obtained by molecular sieve drying. The Dichloromethane, ethyl acetate, petroleum ether, 1,2-dichloroethane, methanol and isopropanol used are analytically pure grade reagent.

The reactions set forth below were done generally under a positive pressure of nitrogen or argon or with a drying tube (unless otherwise stated) in anhydrous solvents, and the reaction flasks were typically fitted with rubber septa for the introduction of substrates and reagents via syringe. Glassware was oven dried and/or heat dried.

Silica gel columns were used Flash silica gel columns purchased from Bonna Agela Tech Co., Ltd. Silica gel (300-400 mesh) was purchased from Qingdao Ocean Chemical Factory.

$^1H$ NMR spectra were recorded with a Bruker 500 MHz spectrometer using $CDCl_3$, $d_6$-DMSO, $CD_3OD$ or $d_6$-acetone as solvents (reported in ppm), and using TMS (0 ppm) or chloroform (7.26 ppm) as the reference standard. For multiple peaks, the following abbreviations used: s (singlet), d (doublet), t (triplet), m (multiplet), br (broadened), dd (doublet of doublets), q (quartet), dt (doublet of triplets), tt (triplet of triplets), dddd (doublet of doublet of doublet of doublets), qd (quartet of doublets), ddd (doublet of doublet of doublets), td (triplet of doublets), dq (doublet of quartets), ddt (doublet of doublet of triplets), tdd (triplet of doublet of doublets), dtd (doublet of triplet of doublets). Coupling constants, when given, were reported in Hertz (Hz).

Low-resolution mass spectral (MS) data were determined by an Agilent 6125C Series LC-MS, column model: XBridge BEH C18, 4.6 × 50 mm, 2.5 μm, 6 min, flow rate of 1 mL/min. mobile phase: 0% - 95% ($CH_3CN$) in ($H_2O$ containing 0.1% formic acid:$CH_3CN$ = 90:10) using electrospray ionization (ESI) at 210 nm/254 nm with DAD detection.

Compound purification was performed using Cheetah Pro (BonnaAgela Tech Co.) medium pressure rapid purification preparation chromatography, and UV detection was performed at 210 nm/254 nm.

The following abbreviations are used throughout the specification:

| | | | |
|---|---|---|---|
| PE | petroleum ether | EtOAc | ethyl acetate |
| mg | milligramme | mmol | millimole |
| mL | milliliter | g | gramme |
| M | mole/liter | rpm | rev/min |
| μM | micromole/liter | h | hour |
| DCM | dichloromethane | MeOH | methanol |
| THF | tetrahydrofuran | DIPEA | *N,N*-Diisopropylethylamine |
| DMF | *N*, *N*-dimethylformamide | LiHMDS | Lithium bis(trimethylsilyl)amide |
| HEPES | 2-[4-(2-hydroxyethyl)piperazin-1-yl]ethanesulfonic acid | BSA | Bovine albumin |
| IBMX | 3-Isobutyl-1-methylxanthine | HBSS | Hank's Balanced Salt Solution |
| HATU | 2-(7-Azabenzotriazol-1-yl)-*N,N,N',N'*-tetramethyluronium hexafluorophosphate | | |

Typical synthetic procedures for preparing the compounds of the present invention disclosed are shown in the following synthetic schemes. Unless otherwise specified, each $R^a$, $R^b$, $R^c$, $R_4$, $R_5$, $R_6$, $R_7$, $R_8$, $R_9$, m, n and k are as defined herein.

**Scheme 1**

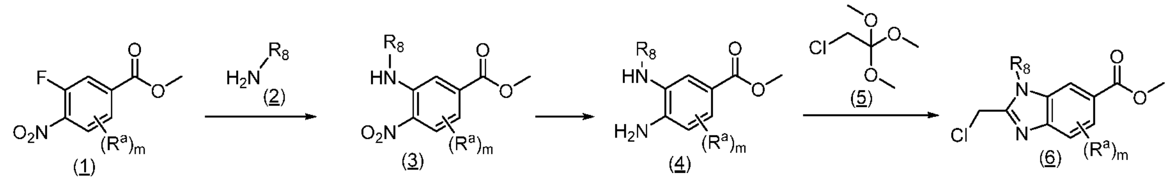

The compounds shown in formula (6) can be prepared by this reaction scheme 1: the compound shown in formula (1) and the compound shown in formula (2) were reacted in the presence of anhydrous potassium carbonate to give the compound shown in formula (3). The compound shown in formula (3) was reacted in the presence of palladium carbon and hydrogen to give the compound shown in formula (4). The compound shown in formula (4) and the compound shown in formula (5) were reacted in the presence of *p*-toluenesulfonic acid to give the compound shown in formula (6).

**Scheme 2**

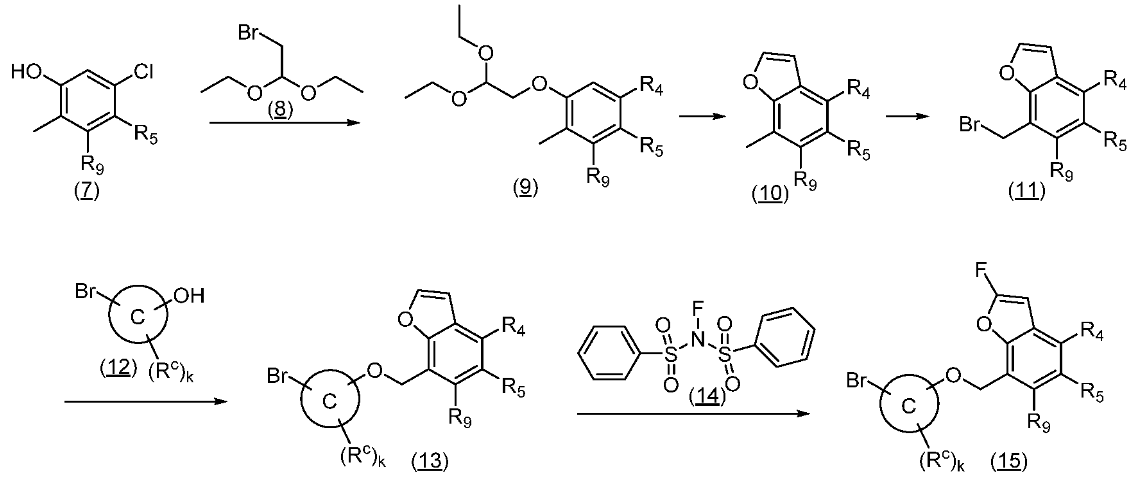

(7) (8) (9) (10) (11) (12) (13) (14) (15)

The compounds shown in formula (15) can be prepared by this reaction scheme 2: the compound shown in formula (7) and the compound shown in formula (8) were reacted in the presence of potassium carbonate to give the compound shown in formula (9). The compound shown in formula (9) is reacted in the presence of polyphosphoric acid to give the compound shown in formula (10). The compound shown in formula (10) was reacted in the presence of 2,2'-Azobis(2-methylpropionitrile) and *N*-bromosuccinimide to give the compound shown in formula (11). The compound shown in formula (11) and the compound shown in formula (12) were reacted in the presence of potassium carbonate to give the compound shown in formula (13). The compound shown in formula (13) and the compound shown in formula (14) were reacted in the presence of lithium diisopropylammonium to give the compound shown in formula (15).

**Scheme 3**

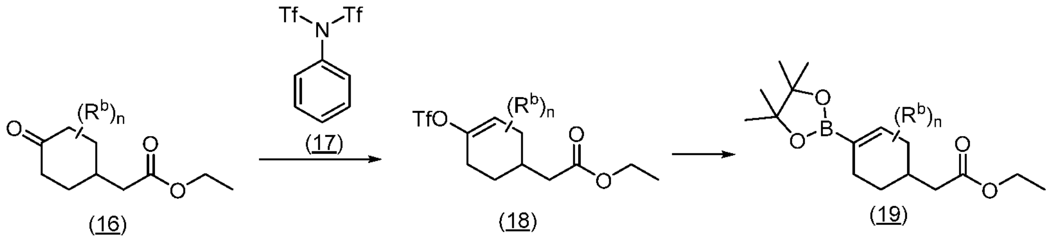

(16) (17) (18) (19)

The compound shown in formula (19) can be prepared by this reaction scheme 3: the compound shown in formula (16) and the compound shown in formula (17) were reacted in the presence of LiHMDS to give the compound shown in formula (18). Reaction of the compound shown in formula (18) in the presence of pinacol bis(boronic acid) ester, palladium tetrakis(triphenylphosphine) and potassium acetate gives the compound shown in formula (19).

**Scheme 4**

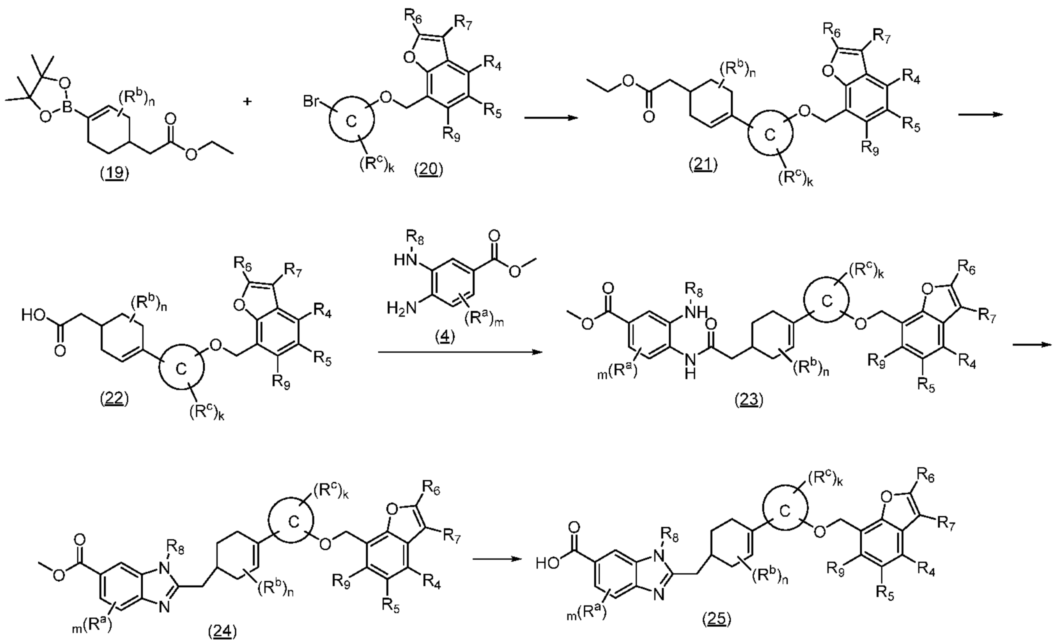

The compound shown in formula (25) can be prepared by this reaction scheme 4: The compound shown in formula (19) and the compound shown in formula (20) are reacted in the presence of 1,1'-Bis(diphenylphosphino) ferrocene-palladium(II)dichloride dichloromethane complex and potassium carbonate to give the compound shown in formula (21). The compound shown in formula (21) was reacted in the presence of lithium hydroxide monohydrate to give the compound shown in formula (22). Reaction of the compound shown in formula (22) and the compound shown in formula (4) in the presence of HATU, DIPEA and DMF gives the compound shown in formula (23). The compound shown in formula (23) was reacted in the presence of glacial acetic acid to give the compound shown in formula (24). The compound shown in formula (24) was reacted in the presence of lithium hydroxide monohydrate to give the compound shown in formula (25).

**Scheme 5**

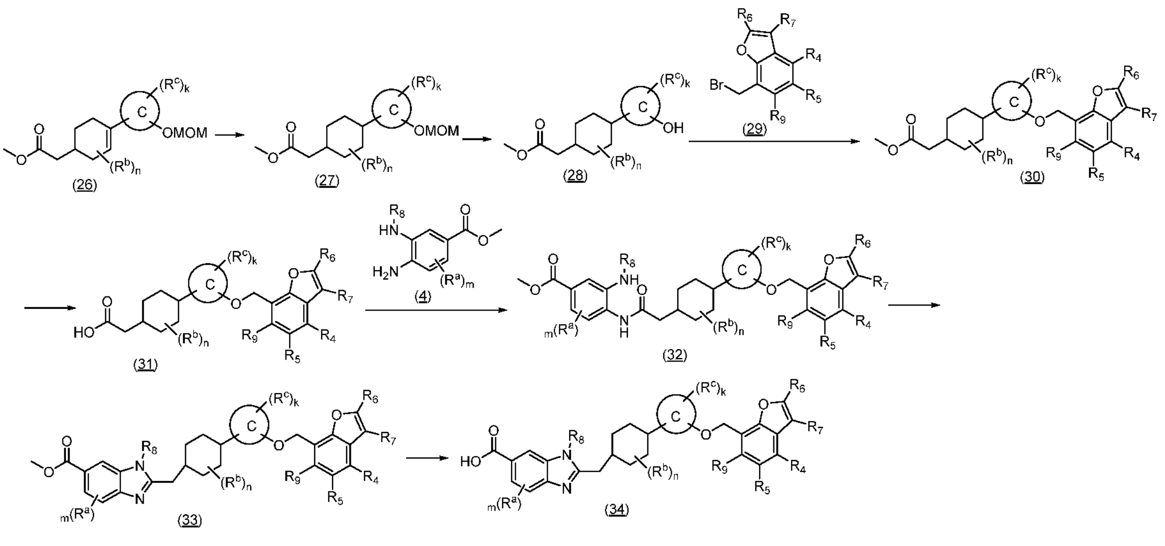

The compounds shown in formula (34) can be prepared by this reaction scheme 5: palladium-carbon hydrogenation reduction of the compound shown in formula (26) gives the compound shown in formula (27). The compound shown in formula (28) is obtained by reaction of the compound shown in formula (27) under acidic conditions. The compound shown in formula (28) and the compound shown in formula (29) were reacted to give the compound shown in formula (30). The compound shown in formula (30) was reacted under alkaline condition to give the compound shown in formula (31). The compound shown in formula (31) and the compound shown in formula (4) were reacted to give the

compound shown in formula (32). The ring closure reaction of the compound shown in formula (32) gives the compound shown in formula (33). The compound shown in formula (33) was reacted under basic conditions to give the compound shown in formula (34).

## DESCRIPTION OF THE PREFERRED EMBODIMENTS

The invention is described below with reference to specific examples, it being noted that these examples are merely descriptive and do not limit the invention in any way.

### Example 1 Synthesis of 2-((4-(6-((4-chlorobenzofuran-7-yl)methoxy)pyridin-2-yl)cyclohex-3-en-1-yl) methyl)-1-(((*S*)-oxetan-2-yl)methyl)-1*H*-benzo[*d*]imidazole-6-carboxylic acid (compound 1)

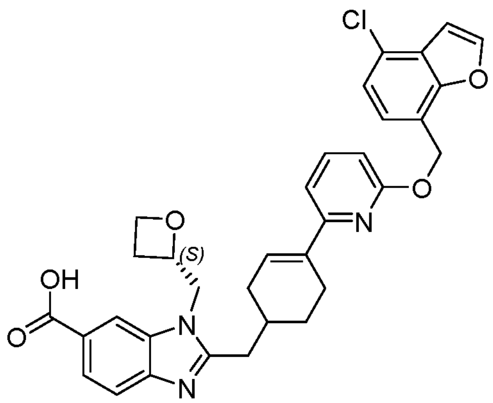

### Step 1: Synthesis of ethyl 2-(4-(6-((4-chlorobenzofuran-7-yl)methoxy)pyridin-2-yl)cyclohex-3-en-1-yl)acetate

Ethyl 2-(4-(4,4,5,5-tetramethyl-1,3,2-dioxaborolan-2-yl)cyclohex-3-en-1-yl)acetate (191 mg, 0.65 mmol), 2-bromo-6-((4-chlorobenzofuran-7-yl)methoxy)pyridine (200 mg, 0.59 mmol), $Pd(dppf)Cl_2$ (22 mg, 0.03 mmol) and $K_2CO_3$ (163 mg, 1.18 mmol) were added into the mixture of the 1,4-dioxane (15 mL) and water (4 mL), the reaction mixture was stirred under nitrogen protection at 85 °C for 6 h. The reaction was cooled down to room temperature, water (50 mL) was added, and the mixture was extracted with EtOAc (20 mL×3), the organic phases were combined, and the solution was dried with anhydrous sodium sulfate, concentrated under reduced pressure and purified by column chromatography eluted with PE:EtOAc (v/v)=15:1 to give a yellow mucilage (200 mg, 79.5%).

$^1H$ NMR (500 MHz, DMSO-$d_6$) δ 8.15 (dd, *J* = 2.4, 1.0 Hz, 1H), 7.68 - 7.61 (m, 1H), 7.41 (d, *J* = 8.0 Hz, 1H), 7.33 (dd, *J* = 8.0, 1.1 Hz, 1H), 7.07 - 7.02 (m, 2H), 6.70 *(d, J*= 8.1 Hz, 2H), 5.64 (s, 2H), 4.08 (qd, *J* = 7.2, 1.2 Hz, 2H), 2.54 - 2.50 (m, 1H), 2.37 - 2.28 (m, 4H), 2.01-1.96 (m, 1H), 1.94 - 1.82 (m, 2H), 1.41 - 1.31 (m, 1H), 120 (td, *J* = 7.1, 1.2 Hz, 3H).

LC-MS (ESI): $[M+H]^+$ =426.2.

### Step 2: Synthesis of 2-(4-(6-((4-chlorobenzofuran-7-yl)methoxy)pyridin-2-yl)cyclohex-3-en-1-yl)acetic acid

Ethyl 2-(4-(6-((4-chlorobenzofuran-7-yl)methoxy)pyridin-2-yl)cyclohex-3-en-1-yl)acetate (1.18 g, 2.77 mmol) was dissolved in the mixture of 1,4-dioxane (20 mL) and water (6 mL), and lithium hydroxide monohydrate (350 mg, 8.31 mmol) was added, and the reaction was carried out at 45°C for 2 h. Then cooled in an ice bath, the pH was adjusted to 2 with dilute hydrochloric acid, extracted with ethyl acetate (30 mL×3), The combined organic phases weredried with anhydrous sodium sulfate, concentrated under reduced pressure, and the residue was purified by column chromatography eluted with PE:EtOAc (v/v)=3:1 to give an oily-yellow viscous material (1.03 g, 93.4%).

$^1H$ NMR (500 MHz, DMSO-$d_6$) δ 12.04 (s, 1H), 8.16 (d, *J* = 2.2 Hz, 1H), 7.65 (t, *J* = 7.8 Hz, 1H), 7.42 (d, *J* = 8.0 Hz, 1H), 7.34 (d, *J* = 7.9 Hz, 1H), 7.06 (d, *J* = 7.1 Hz, 2H), 6.70 (d, *J* = 8.0 Hz, 2H), 5.65 (s, 2H), 2.53 (d, *J* = 5.1 Hz, 1H), 2.39 - 2.30 (m, 2H), 2.23 (d, *J* = 6.8 Hz, 2H), 2.01 - 1.95 (m, 1H), 1.92 - 1.83 (m, 2H), 1.46 - 1.32 (m, 1H).

LC-MS (ESI): [M+ H] =398.2.

**Step 3: Synthesis of methyl 4-(2-(4-(6-((4-chlorobenzofuran-7-yl)methoxy)pyridin-2-yl)cyclohex-3-en-1-yl)acetamido)-3-((((S)-oxetan-2-yl)methyl)amino)benzoate**

2-(4-(6-((4-chlorobenzofuran-7-yl)methoxy)pyridin-2-yl)cyclohex-3-en-1-yl)acetic acid (525 mg, 1.32 mmol) and methyl (*S*)-4-amino-3-((oxetan-2-ylmethyl)amino)benzoate (327 mg, 1.39 mmol) were added to dichloromethane (25 mL), HATU (752 mg, 1.98 mmol) and DIPEA (341 mg, 2.64 mmol) were added, the reaction was carried out at room temperature for 6 hours. The solvent was removed under reduced pressure, water (30 mL) was added, extracted with ethyl acetate (15 mL×3), the combined organic phases were dried with anhydrous sodium sulfate, concentrated under reduced pressure, and the residue was purified by column chromatography eluted with PE:EtOAc (v/v) = 3:2 to give a pale yellow viscous material (380 mg, 46.7%).

LC-MS (ESI): $[M+H]^+$ =616.5.

**Step 4: Synthesis of methyl 2-((4-(6-((4-chlorobenzofuran-7-yl)methoxy)pyridin-2-yl)cyclohex-3-en-1-yl) methyl)-1-(((*S*)-oxetan-2-yl)methyl)-1*H*-benzo[*d*]imidazole-6-carboxylate**

Methyl 4-(2-(4-(6-((4-chlorobenzofuran-7-yl)methoxy)pyridin-2-yl)cyclohex-3-en-1-yl)acetamido)-3-((((S)-oxetan-2-yl)methyl)amino)benzoate (380 mg, 0.62 mmol) was added to glacial acetic acid (15 mL), and the reaction was carried out at 70 °C for 6 h. The solvent was removed under reduced pressure, water (60 mL) was added, the pH was adjusted to 8 with sodium bicarbonate solution (2M). The organic phases were extracted with ethyl acetate (30 mL×3), the combined organic phases were dried with anhydrous sodium sulfate, concentrated under reduced pressure, and the residue was purified by column chromatography eluted with PE:EtOAc (v/v) =1:1 to give a white solid (210 mg, 56.9%).

$^1H$ NMR (500 MHz, DMSO-$d_6$) δ 8.23 (d, *J* = 1.6 Hz, 1H), 8.15 (d, *J* = 2.2 Hz, 1H), 7.81 (dd, *J* = 8.4, 1.6 Hz, 1H), 7.66 (dt, *J* = 7.9, 3.6 Hz, 2H), 7.42 (d, *J* = 7.9 Hz, 1H), 7.34 (d, *J* = 8.0 Hz, 1H), 7.09 - 7.01 (m, 2H), 6.72 (dd, *J* = 11.9, 6.2 Hz, 2H), 5.65 (s, 2H), 5.06 - 4.99 (m, 1H), 4.65 (dd, *J* = 15.6, 7.1 Hz, 1H), 4.53 (dt, *J* = 15.3, 2.3 Hz, 1H), 4.45 (td, *J* = 8.0, 5.8 Hz, 1H), 4.29 (dt, *J* = 9.1, 5.9 Hz, 1H), 3.87 (s, 3H), 3.04- 2.93 (m, 2H), 2.74 - 2.65 (m, 1H), 2.57 (d, *J* = 17.6 Hz, 1H), 2.47 - 2.30 (m, 4H), 2.10 - 2.01 (m, 1H), 2.00 - 1.95 (m, 1H), 1.54- 1.46 (m, 1H).

LC-MS (ESI): $[M+H]^+$ =598.4.

**Step 5: Synthesis of 2-((4-(6-((4-chlorobenzofuran-7-yl)methoxy)pyridin-2-yl)cyclohex-3-en-1-yl) methyl)-1-(((*S*)-oxetan-2-yl)methyl)-1*H*-benzo[*d*]imidazole-6-carboxylic acid**

Methyl 2-((4-(6-((4-chlorobenzofuran-7-yl)methoxy)pyridin-2-yl)cyclohex-3-en-1-yl)methyl)-1-(((*S*)-oxetan-2-yl)methyl)-1*H*-benzo[*d*]imidazole-6-carboxylate (145 mg, 0.24 mmol) and lithium hydroxide hydrate (31 mg, 0.73 mmol) were added to the mixture of 1,4-dioxane (10 mL) and water (3 mL), and the reaction was carried out at 40 °C for 2 h. The reaction was cooled to room temperature. Diluted with water (20 mL), the pH was adjusted to 5 with hydrochloric acid (2M), extracted with ethyl acetate (15 mL×3), the combined the organic phases were dried with anhydrous sodium sulfate, concentrated under reduced pressure, and the residue was purified by column chromatography eluted with DCM:MeOH (v/v)=10:1 to give the title compound (85 mg, 60%).

$^1H$ NMR (500 MHz, DMSO-$d_6$) δ 12.66 (s, 1H), 8.21 (d, *J* = 1.5 Hz, 1H), 8.15 (d, *J* = 2.3 Hz, 1H), 7.80 (dd, V = 8.3, 1.6 Hz, 1H), 7.64 (q, *J* = 8.2 Hz, 2H), 7.42 (d, *J* = 8.0 Hz, 1H), 7.34 (d, *J* = 8.0 Hz, 1H), 7.12 - 7.01 (m, 2H), 6.72 (dd, *J* = 13.0, 6.3 Hz, 2H), 5.65 (s, 2H), 5.05 -5.0 (m, 1H), 4.63 (dd, *J* = 15.6, 7.2 Hz, 1H), 4.57 - 4.42 (m, 2H), 4.30 (dt, *J* = 9.1, 5.9 Hz, 1H), 3.05 - 2.91 (m, 2H), 2.72 -2.65 (m, 1H), 2.57 (d, *J* = 17.7 Hz, 1H), 2.48 - 2.28 (m, 4H), 2.09 -2.01 (m, 1H), 2.00-1.95 (m, 1H), 1.53 -1.45 (m, 1H).

LC-MS (ESI): $[M+H]^+$ =584.4.

**Example 2 Synthesis of 2-((4-(6-((4-chloro-2-fluorobenzofuran-7-yl)methoxy)pyridin-2-yl)cyclohex-3-en-1-yl) methyl)-1-(((*S*)-oxetan-2-yl)methyl)-1*H*-benzo[*d*]imidazole-6-carboxylic acid (compound 2)**

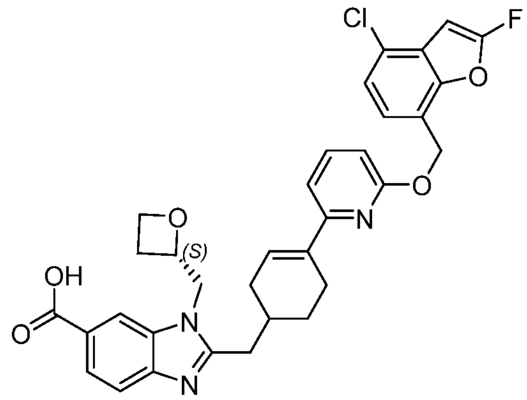

**Step 1: Synthesis of methyl (*S*)-4-nitro-3-((oxetan-2-ylmethyl)amino)benzoate**

Methyl 3-fluoro-4-nitrobenzoate (500 mg, 2.51 mmol), anhydrous potassium carbonate (521 mg, 3.77 mmol) were added to anhydrous tetrahydrofuran (6 mL), and then (*S*)-oxetan-2-ylmethanamine (241 mg, 2.76 mmol) was added and the reaction was carried out at room temperature for 15 h. Water (25 mL) and ethyl acetate (10 mL) were added, separated and extracted with ethyl acetate (5 mL×2), the combined organic phases were dried with anhydrous sodium sulfate, concentrated under reduced pressure, and the residue was purified by column chromatography eluted with EtOAc:PE (v/v)=1:4 to give a reddish-brown solid (522 mg, 78.1%).

$^1$HNMR (500 MHz, DMSO-$d_6$) δ 8.32 (t, *J* = 5.8 Hz, 1H), 8.17 (d, *J* = 8.9 Hz, 1H), 7.67 (d, *J* = 1.7 Hz, 1H), 7.16 (dd, *J* = 8.9, 1.8 Hz, 1H), 5.03 - 4.96 (m, 1H), 4.58 - 4.54 (m, 1H), 4.46 - 4.42 (m, 1H), 3.88 (s, 3H), 3.71 - 3.61 (m, 2H), 2.71 - 2.64 (m, 1H), 2.56 - 2.50 (m, 1H).

LC-MS (ESI): $[M+H]^+$ =267.1.

**Step 2: Synthesis of methyl (*S*)-4-amino-3-((oxetan-2-ylmethyl)amino)benzoate**

Methyl (*S*)-4-nitro-3-((oxetan-2-ylmethyl)amino)benzoate (3.6 g, 13.52 mmol) was dissolved in the mixture of methanol (30 mL) and tetrahydrofuran (15 mL), 10% palladium carbon (60% water content, 1.43 g) was added, and the reaction was replaced with hydrogen for three times and protected by hydrogen at room temperature overnight. The reaction solution was filtered with diatomaceous earth, and the filtrate was concentrated under reduced pressure to give a light yellow liquid (3.18 g, 99.5%).

$^1$HNMR (500 MHz, DMSO-$d_6$) δ 7.17 (dd, *J* = 8.1, 1.9 Hz, 1H), 7.04 (d, *J* = 1.9 Hz, 1H), 6.56 (d, *J* = 8.1 Hz, 1H), 5.44 (s, 2H), 4.95 - 4.90 (m, 1H), 4.67 (t, *J* = 5.7 Hz, 1H), 4.56 - 4.52 (m, 1H), 4.50 - 4.46 (m, 1H), 3.73 (s, 3H), 3.37 - 3.25 (m, 2H), 2.70 - 2.63 (m, 1H), 2.48 - 2.43 (m, 1H).

LC-MS (ESI): $[M+H]^+$ =237.1.

**Step 3: Synthesis of methyl (*S*)-2-(chloromethyl)-1-(oxetan-2-ylmethyl)-1*H*-benzo[*d*]imidazole-6-carboxylate**

Methyl (*S*)-4-amino-3-((oxetan-2-ylmethyl)amino)benzoate (1.0 g, 4.23 mmol), trimethyl chloroethyl protoacetate (790 mg, 5.08 mmol), and p-toluenesulfonic acid monohydrate (40 mg, 0.21 mmol) were dissolved in anhydrous tetrahydrofuran (8 mL), and the reaction was stirred at 45 °C for 5 h. The reaction was cooled, and concentrated to remove the solvent. Dissolved by adding dichloromethane (6 mL), and purified by column chromatography eluted with EtOAc:PE (v/v)=1:2 to give a white solid (1.1 g, 88.2%).

$^1$HNMR (500 MHz, DMSO-$d_6$) δ 8.35 (s, 1H), 7.87 (dd, *J* = 8.5, 1.6 Hz, 1H), 7.74 (d, *J* = 8.5 Hz, 1H), 5.20 - 5.08 (m, 2H), 5.09 - 5.04 (m, 1H), 4.78 - 4.74 (m, 1H), 4.66 - 4.63 (m, 1H), 4.50 - 4.45 (m, 1H), 4.35 - 4.30 (m, 1H), 3.89 (s, 3H), 2.73 - 2.66 (m, 1H), 2.41 - 2.34 (m, 1H).

LC-MS (ESI): $[M+H]^+$ =295.2.

**Step 4: Synthesis of 4-chloro-2-(2,2-diethoxyethoxy)-1-methylbenzene**

5-Chloro-2-methylphenol (15 g, 105.2 mmol), 2-bromo-1,1-diethoxyethane (25 g, 126.9 mmol) and potassium carbonate (29.1 g, 210.4 mmol) were added to *N,N*-dimethylformamide (50 mL), and the reaction was carried out under nitrogen protection at 120 °C for 11 h. The reaction was cooled down to room temperature, and then water (100 mL) was added. Extracted with ethyl acetate (35 mL×3), the combined organic phases were washed with saturated saline (30 mL), the organic phases were dried with anhydrous sodium sulfate, concentrated under reduced pressure, and the residue was

purified by column chromatography eluted with EtOAc:PE (v/v)=1% to give a light brown liquid (21.1 g, 77.5%).

$^1$HNMR (500 MHz, $CDCl_3$) δ 7.03 (d, *J* = 7.9 Hz, 1H), 6.84 (dd, *J* = 7.9, 2.0 Hz, 1H), 6.81 (d, *J* = 2.0 Hz, 1H), 4.84 (t, *J* = 5.2 Hz, 1H), 3.98 (d, *J* = 5.2 Hz, 2H), 3.81 - 3.74 (m, 2H), 3.68 - 3.62 (m, 2H), 2.18 (s, 3H), 1.25 (t, *J* = 7.0 Hz, 6H).

**Step 5: Synthesis of 4-chloro-7-methylbenzofuran**

4-Chloro-2-(2,2-diethoxyethoxy)-1-methylbenzene (21 g, 81.2 mmol) and polyphosphoric acid (16.6 g, 202.9 mmol) were added to 1,2-dichloroethane (50 mL), and the reaction was carried out at 85 °C under nitrogen protection overnight. Cooled to room temperature, the solvent was removed under reduced pressure, water (80 mL) was added, extracted with ethyl acetate (50 mL×2), the combined organic phases were dried with anhydrous sodium sulfate, concentrated under reduced pressure, and the residue was purified by column chromatography eluted with PE=100% to give a colorless liquid (9.2 g, 68.0%).

$^1$HNMR (500 MHz, $CDCl_3$) δ 7.63 (d, *J* = 2.3 Hz, 1H), 7.12 (d, *J* = 7.9 Hz, 1H), 7.00 (d, *J* = 7.9 Hz, 1H), 6.84 (d, *J* = 2.3 Hz, 1H), 2.48 (s, 3H).

**Step 6: Synthesis of 7-(bromomethyl)-4-chlorobenzofuran**

4-Chloro-7-methylbenzofuran (4.1 g, 24.61 mmol), azobisisobutyronitrile (810 mg, 4.92 mmol) and *N*-bromosuccinimide (5.26 g, 29.53 mmol) were added to 1,2-dichloroethane (30 mL) and reacted at 75 °C under nitrogen protection overnight. Cooled to room temperature, water (50 mL) was added, extracted with dichloromethane (15 mL×3), the combined organic phases were dried with anhydrous sodium sulfate, concentrated under reduced pressure, and the residue was purified by column chromatography eluted with PE=100% to give a colorless liquid (4.3 g, 71.2%).

$^1$HNMR (500 MHz, $CDCl_3$) δ 7.71 (s, 1H), 7.24 - 7.20 (m, 2H), 6.89 (s, 1H), 4.75 (s, 2H).

**Step 7: Synthesis of 2-bromo-6-((4-chlorobenzofuran-7-yl)methoxy)pyridine**

7-(Bromomethyl)-4-chlorobenzofuran (600 mg, 2.44 mmol), 2-bromo-6-pyridine (467 mg, 2.68 mmol) and potassium carbonate (675 mg, 4.88 mmol) were added to DMF (6 mL), and the reaction was carried out at 60 °C for 1.5 h. The reaction was cooled down to room temperature, water (50 mL) was added, and the organic phases were extracted with ethyl acetate (15 mL×3), the combined organic phases were dried with anhydrous sodium sulfate, concentrated under reduced pressure, and the residue was purified by column chromatography eluted with EtOAc:PE (v/v)=8% to give a white solid (756 mg, 91.4%).

$^1$HNMR (500 MHz, $CDCl_3$) δ 7.68 (s, 1H), 7.41 (td, *J* = 7.9, 2.9 Hz, 1H), 7.36 (dd, *J* = 8.0, 2.8 Hz, 1H), 7.27 - 7.21 (m, 1H), 7.08 (dd, *J* = 7.5, 2.9 Hz, 1H), 6.88 (s, 1H), 6.73 (dd, *J* = 8.2, 2.9 Hz, 1H), 6.54 (s, 2H).

**Step 8: Synthesis of 2-bromo-6-((4-chloro-2-fluorobenzofuran-7-yl)methoxy)pyridine**

2-Bromo-6-((4-chlorobenzofuran-7-yl)methoxy)pyridine (600 mg, 1.77 mmol) and N fluoro-N-(phenylsulfonyl) benzenesulfonamide (670 mg, 2.12 mmol) were added to anhydrous tetrahydrofuran (30 mL), cooled under nitrogen protection at -40 °C, lithium diisopropylammonium (2.0 mol/L THF solution) (1.4 mL, 2.80 mmol) was added, and the reaction was carried out at -40 °C for 3 h. Water (50 mL) was added, extracted with dichloromethane (20 mL×2), and the combined organic phases were dried with anhydrous sodium sulfate, concentrated under reduced pressure, and the residue was purified by column chromatography eluted with EtOAc:PE (v/v) =10% to give a white solid (211 mg, 33.4%).

$^1$HNMR (500 MHz, $CDCl_3$) δ 7.43 (t, *J* = 7.8 Hz, 1H), 7.33 (d, *J* = 8.1 Hz, 1H), 7.25 (d, *J* = 8.5 Hz, 1H), 7.09 (d, *J* = 7.5 Hz, 1H), 6.74 (d, *J* = 8.1 Hz, 1H), 6.01 (d, *J* = 6.7 Hz, 1H), 5.57 (s, 2H).

**Step 9: Synthesis of ethyl 2-(4-(((trifluoromethyl)sulfonyl)oxy)cyclohex-3-en-1-yl)acetate**

Ethyl 2-(4-oxocyclohexyl)acetate (3.0 g, 16.28 mmol) and 1,1,1-trifluoro-*N*-phenyl-*N*-((trifluoromethyl)sulfonyl) methanesulfonamide (6.98 g, 19.54 mmol) were added to anhydrous tetrahydrofuran (30 mL), cooled at -40 °C under nitrogen protection, LiHMDS (1 mol/L THF solution) (21.2 mL, 21.2 mmol) was added, and the reaction was carried out at this temperature for 2 h. The reaction was quenched by the addition of water (50 mL), extracted with ethyl acetate (30 mL × 2), the combined organic phases were dried with anhydrous sodium sulfate, concentrated under reduced pressure, and the residue was purified by column chromatography eluted with EtOAc:PE (v/v) =2% to give a colorless liquid (4.1 g, 79.6%).

$^1$HNMR (500 MHz, $CDCl_3$) δ 5.72 (s, 1H), 4.15 (q, *J* = 7.1 Hz, 2H), 2.49 - 2.39 (m, 1H), 2.38 - 2.31 (m, 2H), 2.30 (d, *J* = 7.1 Hz, 2H), 2.18 - 2.09 (m, 1H), 1.97 - 1.89 (m, 2H), 1.57 - 1.48 (m, 1H), 1.26 (t, *J* = 7.1 Hz, 3H).

**Step 10: Synthesis of ethyl 2-(4-(4,4,5,5-tetramethyl-1,3,2-dioxaborolan-2-yl)cyclohex-3-en-1-yl)acetate**

Ethyl 2-(4-(((trifluoromethyl)sulfonyl)oxy)cyclohex-3-en-1-yl)acetate (3.9 g, 12.33 mmol), pinacol ester of bis(boronic acid) (3.29 g, 12.95 mmol), palladium tetrakis(triphenylphosphine) (710 mg, 0.62 mmol), and potassium acetate (2.42 g, 24.66 mmol) were added to 1,4- dioxane (30 mL), the reaction was carried out at 90 °C for 1 h under nitrogen protection, cooled to room temperature, the reaction was quenched by adding water (100 mL), the reaction was extracted with ethyl acetate (50 mL×3), the combined organic phases were dried with anhydrous sodium sulfate, concentrated under reduced pressure, and the residue was purified by column chromatography eluted with EtOAc:PE (v/v) = 5% to give a colorless liquid (2.0 g, 55.1%).

$^1$HNMR (500 MHz, $CDCl_3$) δ 6.50 (s, 1H), 4.15 - 4.10 (m, 2H), 2.30 - 2.19 (m, 4H), 2.15 - 2.03 (m, 2H), 1.84 - 1.73 (m, 2H), 1.40 - 1.28 (m, 1H), 1.25 (s, 12H), 1.28 - 1.23 (m, 3H).

**Step 11: Synthesis of ethyl 2-(4-(6-((4-chloro-2-fluorobenzofuran-7-yl)methoxy)pyridin-2-yl)cyclohex-3-en-1-yl) acetate**

Ethyl 2-(4-(4-(4,4,5,5-tetramethyl-1,3,2-dioxaborolan-2-yl)cyclohex-3-en-1-yl)acetate (799 mg, 2.47 mmol), 2-bromo-6-((4-chloro-2-fluorobenzofuran-7-yl)methoxy)pyridine (880 mg, 2.72 mmol), [1,1'-bis(diphenylphosphine) ferrocene]palladium dichloride dichloromethane complex (101 mg, 0.12 mmol) and potassium carbonate (682 mg, 4.93 mmol) were added to the mixture of 1,4-dioxane (12 mL) and water (3 mL), the reaction was carried out at 85 °C for 6 h under nitrogen protection, cooled to room temperature, water (50 mL) was added, and the organic phases were extracted with ethyl acetate (20 mL×3), the combined organic phases were dried with anhydrous sodium sulfate, concentrated under reduced pressure, and the residue was purified by column chromatography eluted with EtOAc:PE (v/v)=5% to give a colorless liquid (905 mg, 82.6%).

$^1$HNMR (500 MHz, $CDCl_3$) δ 7.52 (t, *J* = 7.8 Hz, 1H), 7.32 (d, *J* = 8.2 Hz, 1H), 7.23 (d, *J* = 8.2 Hz, 1H), 6.94 (d, *J* = 7.5 Hz, 1H), 6.84 - 6.69 (m, 1H), 6.64 (d, *J* = 8.1 Hz, 1H), 6.00 (d, *J* = 6.6 Hz, 1H), 5.62 (s, 2H), 4.16 (q, *J* = 7.0 Hz, 2H), 2.64 - 2.57 (m, 1H), 2.50 - 2.39 (m, 2H), 2.34 (d, *J* = 7.1 Hz, 2H), 2.22 - 2.13 (m, 1H), 2.02 - 1.91 (m, 2H), 1.51 - 1.42 (m, 1H), 1.28 (t, *J* = 7.2 Hz, 3H).

LC-MS (ESI): $[M+H]^+$ =444.2.

**Step 12: Synthesis of 2-(4-(6-((4-chloro-2-fluorobenzofuran-7-yl)methoxy)pyridin-2-yl)cyclohex-3-en-1-yl) acetic acid**

Ethyl 2-(4-(6-((4-chloro-2-fluorobenzofuran-7-yl)methoxy)pyridin-2-yl)cyclohex-3-en-1-yl)acetate (680 mg, 1.53 mmol) was dissolved in the mixture of 1,4-dioxane (6 mL) and water (3 mL), lithium hydroxide monohydrate (322 mg, 7.67 mmol) was added, and the reaction was carried out at 40 °C for 4 h. The reaction was cooled in an ice bath, the pH was adjusted to 2 with dilute hydrochloric acid, extracted with ethyl acetate (20 mL×3), the combined organic phases were dried with anhydrous sodium sulfate, and concentrated under reduced pressure to give a light yellow solid (635 mg, 99.7%).

$^1$HNMR (500 MHz, $CDCl_3$) δ 7.49 (t, *J* = 7.8 Hz, 1H), 7.30 (d, *J* = 8.2 Hz, 1H), 7.21 (d, *J* = 8.2 Hz, 1H), 6.90 (d, *J* = 7.5 Hz, 1H), 6.73 (s, 1H), 6.62 (d, *J* = 8.2 Hz, 1H), 5.98 (d, *J* = 6.6 Hz, 1H), 5.60 (s, 2H), 2.64 - 2.54 (m, 1H), 2.50 - 2.41 (m, 2H), 2.38 (d, *J* = 7.1 Hz, 2H), 2.22 - 2.13 (m, 1H), 2.05 - 1.93 (m, 3H), 1.53 - 1.43 (m, 1H).

LC-MS (ESI): $[M-H]^-$=414.2.

**Step 13: Synthesis of methyl 4-(2-(4-(6-((4-chloro-2-fluorobenzofuran-7-yl)methoxy)pyridin-2-yl)cyclohex-3-en-1-yl)acetamido)-3-((((*S*)-oxetan-2-yl)methyl)amino)benzoate**

2-(4-(6-((4-chloro-2-fluorobenzofuran-7-yl)methoxy)pyridin-2-yl)cyclohex-3-en-1-yl)acetic acid (300 mg, 0.72 mmol) and methyl (S)-4-amino-3-((oxetan-2-ylmethyl)amino)benzoate (170 mg, 0.72 mmol) were added to the mixture of dichloromethane (5 mL) and *N,N*-dimethylformamide (2 mL), then HATU (411 mg, 1.08 mmol) and DIPEA (233 mg, 1.80 mmol) were added and the reaction was carried out at 45 °C overnight. The solvent was removed under reduced pressure, water (30 mL) was added, extracted with ethyl acetate (15 mL×3), the combined organic phases were dried with anhydrous sodium sulfate, concentrated under reduced pressure, and the residue was purified by column chromatography eluted with EtOAc:DCM (v/v)=1:5 to give a white solid (402 mg, 87.9%).

$^1$HNMR (500 MHz, $CDCl_3$) δ 8.01 (s, 1H), 7.87 - 7.75 (m, 2H), 7.60 - 7.48 (m, 3H), 7.32 (d, *J* = 7.8 Hz, 1H), 7.22 (d, *J* = 8.8 Hz, 1H), 6.94 (d, *J* = 7.4 Hz, 1H), 6.75 (s, 1H), 6.64 (d, *J*= 8.1 Hz, 1H), 5.99 (d, *J* = 6.6 Hz, 1H), 5.62 (s, 2H), 5.08 - 5.01 (m, 1H), 4.75 - 4.68 (m, 1H), 4.62 - 4.55 (m, 1H), 3.89 (s, 3H), 3.75 - 3.65 (m, 1H), 3.50 - 3.37 (m, 1H), 3.36 - 3.28 (m, 1H), 3.21 - 3.12 (m, 1H), 2.77 - 2.68 (m, 1H), 2.65 - 2.56 (m, 1H), 2.55 - 2.40 (m, 4H), 2.34 - 2.24 (m, 1H), 2.09 - 1.95 (m, 2H).

LC-MS (ESI): $[M+H]^+$ =634.4.

**Step 14: Synthesis of methyl 2-((4-(6-((4-chloro-2-fluorobenzofuran-7-yl)methoxy)pyridin-2-yl)cyclohex-3-en-1-yl)methyl)-1-(((*S*)-oxetan-2-yl)methyl)-1*H*-benzo[*d*]imidazole-6-carboxylate**

Methyl 4-(2-(4-(4-(6-((4-chloro-2-fluorobenzofuran-7-yl)methoxy)pyridin-2-yl)cyclohex-3-en-1-yl)acetamido)-3-((((*S*)-oxetan-2-yl)methyl)amino)benzoate (390 mg, 0.62 mmol) was added to glacial acetic acid (10 mL), and the reaction was carried out at 80 °C for 2 h. The solvent was removed by decompression. The solvent was removed under reduced pressure, water (20 mL) was added, extracted with ethyl acetate (20 mL×3), the combined organic phases were dried with anhydrous sodium sulfate, concentrated under reduced pressure, and the residue was purified by column chromatography eluted with EtOAc:PE (v/v)=45% to give a white solid (169 mg, 44.6%).

$^1$HNMR (500 MHz, $CDCl_3$) δ 8.09 (d, *J* = 7.2 Hz, 1H), 7.96 (t, *J* = 7.9 Hz, 1H), 7.74 (t, *J* = 8.2 Hz, 1H), 7.51 (q, *J* = 7.8 Hz, 1H), 7.30 (t, *J* = 8.0 Hz, 1H), 7.21 (t, *J* = 8.1 Hz, 1H), 6.93 (t, *J* = 7.6 Hz, 1H), 6.77 - 6.71 (m, 1H), 6.63 (t, *J* = 7.9 Hz, 1H), 5.98 (t, *J* = 7.1 Hz, 1H), 5.61 (d, *J* = 7.6 Hz, 2H), 5.21 - 5.13 (m, 1H), 4.63 - 4.56 (m, 1H), 4.50 - 4.32 (m, 3H), 3.94 (d, *J* = 7.6 Hz, 3H), 3.08 - 2.95 (m, 2H), 2.76 - 2.68 (m, 1H), 2.66 - 2.58 (m, 1H), 2.54 - 2.37 (m, 4H), 2.14 - 1.98 (m, 2H), 1.65 - 1.54 (m, 1H).

LC-MS (ESI): $[M+H]^+$ =616.4.

**Step 15: Synthesis of 2-((4-(6-((4-chloro-2-fluorobenzofuran-7-yl)methoxy)pyridin-2-yl)cyclohex-3-en-1-yl)methyl)-1-(((*S*)-oxetan-2-yl)methyl)-1*H*-benzo[*d*]imidazole-6-carboxylic acid**

Methyl 2-((4-(6-((4-chloro-2-fluorobenzofuran-7-yl)methoxy)pyridin-2-yl)cyclohex-3-en-1-yl)methyl)-1-(((*S*)-oxetan-2-yl)methyl)-1*H*-benzo[*d*]imidazole-6-carboxylate (160 mg, 0.26 mmol) and lithium hydroxide hydrate (54 mg, 1.29 mmol) were added to the mixture of 1,4-dioxane (4 mL) and water (1.5 mL), and the reaction was carried out at 40 °C for 40 min. Some solvent was removed under reduced pressure, the pH was adjusted to 6 with glacial acetic acid, extracted with ethyl acetate (20 mL×3), and the combined organic phases were dried with anhydrous sodium sulfate, concentrated under reduced pressure, and the residue was purified by column chromatography eluted with MeOH: DCM (v/v)=3% to give the title compound (91 mg, 51.8%).

$^1$HNMR (500 MHz, $CDCl_3$) δ 8.27 - 8.15 (m, 1H), 8.07 (d, *J* = 8.4 Hz, 1H), 7.84 (d, *J* = 8.5 Hz, 1H), 7.60 - 7.46 (m, 1H), 7.37 - 7.16 (m, 2H), 6.94 - 6.91 (m, 1H), 6.81 - 6.71 (m, 1H), 6.62 (d, *J* = 8.0 Hz, 1H), 6.04 - 5.93 (m, 1H), 5.60 (s, 2H), 5.26 - 5.13 (m, 1H), 4.70 - 4.56 (m, 1H), 4.54 - 4.29 (m, 3H), 3.09 (s, 2H), 2.80 - 2.59 (m, 2H), 2.57 - 2.35 (m, 4H), 2.20 - 1.97 (m, 2H), 1.47 - 1.28 (m, 1H).

LC-MS (ESI): $[M+H]^+$ =602.4.

**Example 3 Synthesis of 2-((4-(4-((4-chlorobenzofuran-7-yl)methoxy)-5-fluoropyrimidin-2-yl)cyclohex-3-en-1-yl)methyl)-1-(((*S*)-oxetan-2-yl)methyl)-1*H*-benzo[*d*]imidazole-6-carboxylic acid**

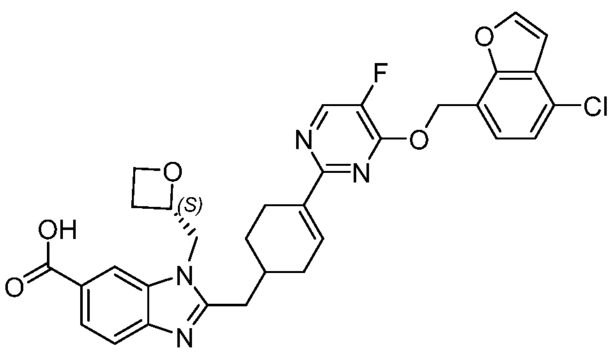

**Step 1: Synthesis of (4-chlorobenzofuran-7-yl)methyl acetate**

7-(Bromomethyl)-4-chlorobenzofuran (2.50 g, 10.18 mmol) was added to DMF (20 mL), then potassium acetate (7.00 g, 71.28 mmol) was added, and the reaction was carried out at room temperature for 3 h. The reaction was stopped, and quenched by adding water (80 mL), and the reaction was extracted with ethyl acetate (30 mL×3), the organic phases were combined, and the organic phase was washed with saturated brine (30 mL×3), the organic phase was dried with anhydrous sodium sulfate, and concentrated under reduced pressure to give a brown oil (2.0 g, 87.4%).

**Step 2: Synthesis of (4-chlorobenzofuran-7-yl)methanol**

(4-chlorobenzofuran-7-yl)methyl acetate (2.0 g, 8.90 mmol) was added to the mixture of 1,4-dioxane (30 mL) and water (8 mL), and then lithium hydroxide monohydrate (1.12 g, 26.71 mmol) was added, and the reaction was heated to 42 ° C for 2 h. Stopped and cooled to room temperature, diluted with water (60 ml), extracted with ethyl acetate (30 mL×3), the combined organic phases were dried with anhydrous sodium sulfate, filtered, concentrated the filtrate, and the residue was purified by column chromatography eluted with PE:EtOAc (v/v)=3:1 to give a white solid (1.6 g, 98.4%).

$^1H$ NMR (500 MHz, DMSO-$d_6$) δ 8.10 (d, *J* = 2.2 Hz, 1H), 7.33 (q, *J* = 8.0 Hz, 2H), 6.99 (d, *J* = 2.2 Hz, 1H), 5.36 (d, *J* = 5.3 Hz, 1H), 4.79 (d, *J* = 2.8 Hz, 2H).

LC-MS (ESI): $[M-17]^-$=165.0.

**Step 3: Synthesis of 2-chloro-4-((4-chlorobenzofuran-7-yl)methoxy)-5-fluoropyrimidine**

(4-Chlorobenzofuran-7-yl)methanol (850 mg, 4.65 mmol) and 2,4-dichloro-5-fluoropyrimidine (816 mg, 4.89 mmol) were dissolved in acetonitrile (20 mL), and under the condition of ice-bath, cesium carbonate (2.275 g, 6.98 mmol) was added, and the reaction was brought to room temperature for 17 h. Stopped and filtered by diatomaceous earth, the filtrate was concentrated and the residue was purified by column chromatography eluted with PE:EtOAc (v/v)=10:1 to give a white solid (1.31 g, 89.9%).

$^1H$ NMR (500 MHz, DMSO-$d_6$) δ 8.63 (d, *J* = 2.6 Hz, 1H), 8.18 (d, *J* = 2.3 Hz, 1H), 7.49 (d, *J* = 8.0 Hz, 1H), 7.40 (d, *J* = 8.0 Hz, 1H), 7.07 (d, *J* = 2.2 Hz, 1H), 5.77 (s, 2H).

**Step 4: Synthesis of ethyl 2-(4-(4-((4-chlorobenzofuran-7-yl)methoxy)-5-fluoropyrimidin-2-yl)cyclohex-3-en-1-yl)acetate**

Ethyl 2-(4-(4-(4,4,5,5-tetramethyl-1,3,2-dioxaborolan-2-yl)cyclohex-3-en-1-yl)acetate (1.35 g, 4.60 mmol), 2-chloro-4-((4-chlorobenzofuran-7-yl)methoxy)-5-fluoropyrimidine (1.31 g, 4.18 mmol), [1,1'-bis(diphenylphosphine) ferrocene]palladium dichloride (310 mg, 0.42 mmol) and potassium carbonate (1.16 g, 8.37 mmol) were added to the mixture of 1,4-dioxane (32 mL) and water (8 mL), the reaction was carried out under nitrogen protection at 85 °C for 11 h. The reaction was cooled down to room temperature, water (100 mL) was added, and the mixture was extracted with ethyl acetate (30 mL×3), the combined organic phases were dried with anhydrous sodium sulfate, concentrated under reduced pressure, and the residue was purified by column chromatography eluted with PE:EtOAc (v/v)=10:1 to give a yellow viscous substance (1.24 g, 66.6%).

$^1H$ NMR (500 MHz, DMSO-$d_6$) δ 8.53 (d, *J* = 2.8 Hz, 1H), 8.17 (d, *J* = 2.2 Hz, 1H), 7.46 (d, *J* = 8.0 Hz, 1H), 7.36 (d, *J* = 8.0 Hz, 1H), 7.06 (dd, *J* = 5.8, 2.4 Hz, 2H), 5.80 (s, 2H), 4.08 (q, *J* = 7.1 Hz, 2H), 2.67 - 2.57 (m, 1H), 2.41 - 2.28 (m, 4H), 2.02 - 1.90 (m, 2H), 1.86- 1.81 (m, 1H), 1.36- 1.29 (m, 1H), 1.19 (t, *J* = 7.1 Hz, 3H).

LC-MS (ESI): $[M+H]^+$ =445.2.

**Step 5: Synthesis of 2-(4-(4-((4-chlorobenzofuran-7-yl)methoxy)-5-fluoropyrimidin-2-yl)cyclohex-3-en-1-yl) acetic acid**

Ethyl 2-(4-(4-((4-chlorobenzofuran-7-yl)methoxy)-5-fluoropyrimidin-2-yl)cyclohex-3-en-1-yl)acetate (1.24 g, 2.79 mmol) was dissolved in the mixture of 1,4-dioxane (30 mL) and water (10 mL), and lithium hydroxide monohydrate (350 mg, 8.36 mmol) was added, and the reaction was carried out at 40 °C for 2 h. The reaction was cooled to room temperature, diluted with water (60 mL), the pH was adjusted to 5 with dilute hydrochloric acid, extracted with ethyl acetate (30 mL×3), and the combined organic phases were dried with anhydrous sodium sulfate, and the residue was concentrated under reduced pressure to give an oily-yellow viscous (1.1 g, 94.7%).

LC-MS (ESI): $[M-H]^-$=417.2.

**Step 6: Synthesis of methyl 4-(2-(4-(4-((4-chlorobenzofuran-7-yl)methoxy)-5-fluoropyrimidin-2-yl)cyclohex-3-en-1-yl)acetamido)-3-((((*S*)-oxetan-2-yl)methyl)amino)benzoate**

2-(4-(4-((4-chlorobenzofuran-7-yl)methoxy)-5-fluoropyrimidin-2-yl)cyclohex-3-en-1-yl)acetic acid (500 mg, 1.2 mmol) and methyl (S)-4-amino-3-((oxetan-2-ylmethyl)amino)benzoate (285 mg, 1.2 mmol) were added to the mixture of dichloromethane (12 mL) and DMF (3 mL), then HATU (684 mg, 1.8 mmol) and DIPEA (310 mg, 2.4 mmol) were added, and the reaction was carried out at 60 °C overnight. The solvent was removed under reduced pressure, water (30 mL) was added, extracted with ethyl acetate (15 mL×3), the combined organic phases were dried with anhydrous sodium sulfate, concentrated under reduced pressure, and the residue was purified by column chromatography eluted with DCM/MeOH (v/v) =25:1 to give a pale yellow viscous material (600 mg, 78.6%).

LC-MS (ESI): $[M+H]^+$ =636.6.

**Step 7: Synthesis of methyl 2-((4-(4-((4-chlorobenzofuran-7-yl)methoxy)-5-fluoropyrimidin-2-yl)cyclohex-3-en-1-yl)methyl)-1-(((*S*)-oxetan-2-yl)methyl)-1*H*-benzo[*d*]imidazole-6-carboxylate**

Methyl 4-(2-(4-(4-(6-((4-chloro-2-fluorobenzofuran-7-yl)methoxy)pyridin-2-yl)cyclohex-3-en-1-yl)acetamido)-3-((((*S*)-oxetan-2-yl)methyl)amino)benzoate (600 mg, 0.94 mmol) was added to glacial acetic acid (20 mL), and the reaction was carried out at 70 °C for 6 h. The solvent was removed under reduced pressure, water (60 mL) was added, the pH was adjusted to 8 with sodium bicarbonate solution (2M). The organic phases were extracted with ethyl acetate (30 mL×3), the combined organic phases were dried with anhydrous sodium sulfate, concentrated under reduced pressure, and the residue was purified by column chromatography eluted with DCM/MeOH (v/v) =18:1 to give a white solid (70 mg, 12.0%).

$^1$H NMR (500 MHz, DMSO-$d_6$) δ 8.55 (t, *J* = 2.8 Hz, 1H), 8.24 (d, *J* = 2.4 Hz, 1H), 8.18 (d, *J* = 2.8 Hz, 1H), 7.85 - 7.78 (m, 1H), 7.66 (dd, *J* = 8.5, 2.5 Hz, 1H), 7.48 (dd, *J* = 8.0, 2.4 Hz, 1H), 7.39 (dd, *J* = 8.0, 2.4 Hz, 1H), 7.11 (s, 1H), 7.09 - 7.03 (m, 1H), 5.82 (d, *J* = 2.5 Hz, 2H), 5.03 (q, *J* = 7.5 Hz, 1H), 4.66 (dd, *J* = 15.6, 7.1 Hz, 1H), 4.53 (d, *J* = 15.4 Hz, 1H), 4.46 (q, *J* = 7.5 Hz, 1H), 4.34 - 4.26 (m, 1H), 3.88 (d, *J* = 2.4 Hz, 3H), 3.08 - 2.93 (m, 2H), 2.70 (q, *J* = 8.0 Hz, 2H), 2.49 - 2.29 (m, 4H), 2.11 (t, *J* = 13.5 Hz, 1H), 2.00 - 1.95 (m, 1H), 1.48 (s, 1H).

LC-MS (ESI): $[M+H]^+$ =617.4.

**Step 8: Synthesis of 2-((4-(4-((4-chlorobenzofuran-7-yl)methoxy)-5-fluoropyrimidin-2-yl)cyclohex-3-en-1-yl)methyl)-1-(((*S*)-oxetan-2-yl)methyl)-1*H*-benzo[*d*]imidazole-6-carboxylic acid**

Methyl 2-((4-(4-((4-chlorobenzofuran-7-yl)methoxy)-5-fluoropyrimidin-2-yl)cyclohex-3-en-1-yl)methyl)-1-(((*S*)-oxetan-2-yl)methyl)-1*H*-benzo[*d*]imidazole-6-carboxylate (50 mg, 0.08 mmol) and lithium hydroxide hydrate (11 mg, 0.24 mmol) were added to the mixture of 1,4-dioxane (10 mL) and water (3 mL), and the reaction was carried out at 40 °C for 2 h. Stopped and cooled to room temperature. Diluted with water (15 mL), the pH was adjusted to 5 with dilute hydrochloric acid (2M), extracted with ethyl acetate (15 mL×3), the combined organic phases were dried with anhydrous sodium sulfate, filtered, and concentrated the filtrate. And the residue was purified by column chromatography eluted with DCM/MeOH (v/v) =10:1 to give the title compound (10 mg 20.5%).

$^1$H NMR (500 MHz, DMSO-$d_6$) δ 12.65 (s, 1H), 8.55 (d, *J* = 2.7 Hz, 1H), 8.24 - 8.16 (m, 2H), 7.79 (d, *J* = 8.4 Hz, 1H), 7.62 (d, *J* = 8.4 Hz, 1H), 7.48 (d, *J* = 8.0 Hz, 1H), 7.38 (d, *J* = 8.2 Hz, 1H), 7.11 (s, 1H), 7.06 (d, *J* = 2.3 Hz, 1H), 5.82 (s, 2H), 5.05- 5.00 (m, 1H), 4.64 (dd, *J* = 15.5, 7.1 Hz, 1H), 4.51 (d, *J* = 15.4 Hz, 1H), 4.45 (t, *J* = 7.2 Hz, 1H), 4.30 (dt, *J* = 9.1, 5.8 Hz, 1H), 3.04 - 2.93 (m, 2H), 2.69 (d, *J* = 16.0 Hz, 2H), 2.36 (dd, *J* = 18.9, 9.1 Hz, 4H), 2.15 - 2.05 (m, 1H), 2.02 - 1.93 (m, 1H), 1.51 -1.45 (m, 1H).

LC-MS (ESI): $[M+H]^+$ =603.5.

**Example 4 Synthesis of (*S*)-2-((4-(4-((4-chloro-2-fluorobenzofuran-7-yl)methoxy)-5-fluoropyrimidin-2-yl)cyclohexyl)methyl)-1-(oxetan-2-ylmethyl)-1*H*-benzo[d]imidazole-6-carboxylic acid (compound 4)**

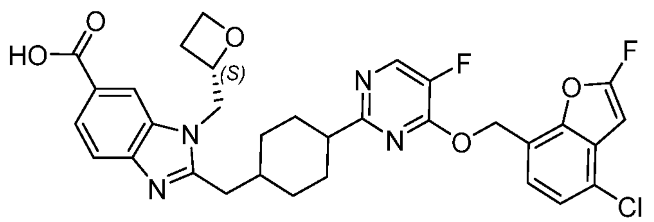

**Step 1: Synthesis of methyl 2-(4-(5-fluoro-4-(methoxymethoxy)pyrimidin-2-yl)cyclohexyl)acetate**

Methyl 2-(4-(5-fluoro-4-(methoxymethoxy)pyrimidin-2-yl)cyclohex-3-en-1-yl)acetate (500 mg, 1.61 mmol) was added to ethyl acetate (5 mL), then 10% palladium-carbon (50 mg) was added, replaced 3 times with hydrogen and stirred at room temperature for 3 hours. The reaction solution was filtered with diatomaceous earth and washed with dichloromethane (10 mL × 3). The organic phases were combined and concentrated under reduced pressure to give a colorless oil (350 mg, 69.5%).

LC-MS (ESI): $[M+H]^+$=313.3.

**Step 2: Synthesis of methyl 2-(4-(5-fluoro-4-hydroxypyrimidin-2-yl)cyclohexyl)acetate**

Methyl 2-(4-(5-fluoro-4-(methoxymethoxy)pyrimidin-2-yl)cyclohexyl)acetate (350 mg, 1.12 mmol) was added to a methanolic solution of hydrochloric acid (4 N, 5 mL) and stirred at 50 °C for 2 hours. Concentrated under reduced pressure to give a colorless oil (260 mg, 86.5% ), which was used directly in the next step of the reaction.
LC-MS (ESI): $[M+H]^+$=269.3.

**Step 3: Synthesis of methyl 2-(4-(4-((4-chloro-2-fluorobenzofuran-7-yl)methoxy)-5-fluoropyrimidin-2-yl)cyclohexyl)acetate**

Methyl 2-(4-(5-fluoro-4-hydroxypyrimidin-2-yl)cyclohexyl)acetate (200 mg, 0.75 mmol) was added to *N,N*-dimethylformamide (3 mL), then potassium carbonate (310 mg, 2.25 mmol) and 7-(bromomethyl)-4-chloro-2-fluorobenzofuran (237 mg, 0.9 mmol) was added, and stirred at 60 °C for 3 hours. Concentrated under reduced pressure and the residue was purified by column chromatography eluted with EtOAc:PE (v/v)=1:5 to give a colorless oil (90 mg, 33.5%).
LC-MS (ESI): $[M+H]^+$=451.3.

**Step 4: Synthesis of 2-(4-(4-((4-chloro-2-fluorobenzofuran-7-yl)methoxy)-5-fluoropyrimidin-2-yl)cyclohexyl) acetic acid**

Methyl 2-(4-(4-((4-chloro-2-fluorobenzofuran-7-yl)methoxy)-5-fluoropyrimidin-2-yl)cyclohexyl)acetate (90 mg, 0.2 mmol) was added to the mixture of 1,4-dioxane (0.6 mL) and water (0.6 mL), then lithium hydroxide (42 mg, 1.75 mmol) was added, and the mixture was stirred for 2 hours at 40 °C. Water (3 mL) was added to dilute and the pH was adjusted to 6 by slowly dropping in aqueous hydrochloric acid (1 N). dichloromethane (5 mL) was added and extracted with methanol (0.5 mL). The organic phases were combined, the organic phase was washed with saturated brine, dried with anhydrous sodium sulfate, and concentrated under reduced pressure to give a colorless oil (110 mg) and used directly in the next step of the reaction.
LC-MS (ESI): $[M+H]^+$=437.3.

**Step 5: Synthesis of methyl (*S*)-4-(2-(4-(4-((4-chloro-2-fluorobenzofuran-7-yl)methoxy)-5-fluoropyrimidin-2-yl) cyclohexyl)acetamido)-3-((oxetan-2-ylmethyl)amino)benzoate**

2-(4-(4-((4-chloro-2-fluorobenzofuran-7-yl)methoxy)-5-fluoropyrimidin-2-yl)cyclohexyl)acetic acid (100 mg, crude) was added to *N,N*-dimethylformamide (3 mL), and 2-(7-azobenzotriazolyl)-N, *N, N, N,* - tetramethyluronium hexafluorophosphate (261 mg, 0.69 mmol) was added. *N*, *N*-diisopropylethylamine (0.2 mL, 1.15 mmol) was added and stirred for 5 min, then methyl (*S*)-4-amino-3-((oxetan-2-yl)methyl)aminobenzoate (54 mg, 0.23 mmol) was added and stirred overnight. Concentrated under reduced pressure, diluted with water (5 mL), extracted with dichloromethane (5 mL $\times$ 3), the combined organic phases were washed with saturated brine, dried with anhydrous sodium sulfate, concentrated under reduced pressure, and the residue was purified by column chromatography eluted with EtOAc:PE (v/v)=1:1 to give a light brown oil (72 mg, 48%).
LC-MS (ESI): $[M+H]^+$=655.5.

**Step 6: Synthesis of methyl (*S*)-2-((4-(4-((4-chloro-2-fluorobenzofuran-7-yl)methoxy)-5-fluoropyrimidin-2-yl)cyclohexyl)methyl)-1-(oxetan-2-ylmethyl)-1*H*-benzo[*d*]imidazole-6-carboxylate**

Methyl (*S*)-4-(2-(4-(4-(4-((4-chloro-2-fluorobenzofuran-7-yl)methoxy)-5-fluoropyrimidin-2-yl)cyclohexyl)acetamido)-3-((oxetan-2-ylmethyl)amino)benzoate (72 mg, 0.12 mmol) was added to acetic acid (3 mL) and stirred at 70 °C for 4 hours. Concentrated under reduced pressure and the residue was purified by column chromatography eluted with DCM:MeOH (v/v)=10:1 to give a light brown oil (50 mg, 64.3%).
LC-MS (ESI): $[M+H]^+$=637.0;
$^1$HNMR (400 MHz, DMSO-$d_6$) $\delta$ 8.57 (s, 1H), 8.24 - 8.21 (m, 1H), 7.81 - 7.78 (m, 1H), 7.65 - 7.63 (m, 1H), 7.47 - 7.42 (m, 2H), 6.56 - 6.53 (m, 1H), 5.75 - 5.74 (m, 2H), 5.0 - 4.98 (m, 1H), 4.97 - 4.24 (m, 4H), 3.86 (s, 3H), 2.93 - 2.88 (m, 2H), 2.68 - 2.64 (m, 2H), 2.37 - 2.3 (m, 2H), 2.29 - 2.11 (m, 2H), 1.87 - 1.31 (m, 6H).

**Step 7: Synthesis of (S)-2-((4-(4-((4-chloro-2-fluorobenzofuran-7-yl)methoxy)-5-fluoropyrimidin-2-yl)cyclohexyl)methyl)-1-(oxetan-2-ylmethyl)-1*H*-benzo[*d*]imidazole-6-carboxylic acid**

Methyl (*S*)-2-((4-(4-((4-((4-chloro-2-fluorobenzofuran-7-yl)methoxy)-5-fluoropyrimidin-2-yl)cyclohexyl)methyl)-1-(oxetan-2-ylmethyl)-1*H*-benzo[*d*]imidazole-6-carboxylate (50 mg, 0.08 mmol) was added to the mixture of 1,4-dioxane (0.8 mL) and water (0.2 mL). Lithium hydroxide (16 mg, 0.38 mmol) was then added and stirred at 40 °C for 8 hours. Concentrated under reduced pressure and the pH was adjusted to 6-7 with hydrochloric acid (1 N). Concentrated under reduced pressure and purified by Prep-HPLC eluted with $CH_3CN$: $H_2O$ (v/v) = 60% to give a white solid (21.9 mg, 46.1%).

LC-MS (ESI): $[M+H]^+$=623.2;

$^1$HNMR (400 MHz, DMSO-$d_6$) δ 12.69 (s, 1H), 8.60 - 8.53 (m, 1H), 8.19 (d, *J* = 8.3 Hz, 1H), 7.81 - 7.76 (m, 1H), 7.61 (d, *J* = 7.4 Hz, 1H), 7.50 - 7.42 (m, 2H), 6.55 - 6.53 (m, 1H), 5.75 (d, *J* = 8.8 Hz, 2H), 4.99 - 4.83 (m, 1H), 4.61 - 4.25 (m, 4H), 2.97 - 2.81 (m, 3H), 2.74 - 2.61 (m, 2H), 2.40 - 2.25 (m, 2H), 2.16 - 1.85 (m, 2H), 1.72 - 1.54 (m, 3H), 1.45 - 1.18 (m, 2H).

**Example 5 Synthesis of 2-((4-(4-((4-chloro-2-fluorobenzofuran-7-yl)methoxy)-5-fluoropyrimidin-2-yl)cyclohex-3-en-1-yl)methyl)-3-(((*S*)-oxetan-2-yl)methyl)-3*H*-imidazo[4,5-*b*]pyridine-5-carboxylic acid (compound 5)**

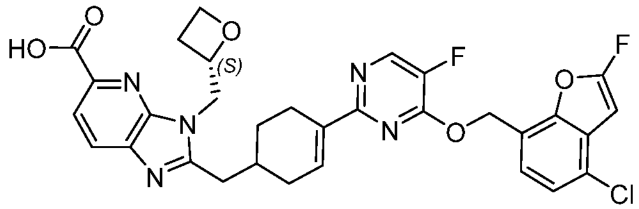

**Step 1: Synthesis of 6-chloro-5-nitropicolinic acid**

2-Chloro-6-methyl-3-nitropyridine (8.0 g, 46.36 mmol) was added to concentrated sulfuric acid (40 mL), and potassium dichromate (20.46 g, 69.54 mmol) was added slowly at room temperature, and the reaction was stirred at room temperature overnight. Under the condition of ice bath, ice water (500 mL) was added slowly, extracted with ethyl acetate (150 mL×3), the combined organic phases were dried with anhydrous sodium sulfate, and concentrated under reduced pressure to give a light yellow solid (8.26 g, 88.0% ).

LC-MS (ESI): $[M+H]^+$ =203.0.

**Step 2: Synthesis of methyl 6-chloro-5-nitropicolinate**

6-chloro-5-nitropicolinic acid (200 mg, 0.99 mmol) was dissolved in methanol (15 mL), then concentrated sulfuric acid (0.5 mL, 0.99 mmol) was added, and the reaction was heated to 68 °C overnight. Concentrated under reduced pressure, diluted with water (30 mL), extracted with ethyl acetate (15 mL×3), the combined organic phases were dried with anhydrous sodium sulfate, filtrated, concentrated under reduced pressure, the residue was purified by column chromatography eluted with PE:EtOAc (v/v)=2:1 to give a white solid (185 mg, 86.5%).

LC-MS (ESI): $[M+H]^+$ =217.1;
$^1$H NMR (500 MHz, $CDCl_3$) δ 8.31 (dd, *J* = 8.3, 0.8 Hz, 1H), 8.22 (dd, *J* = 8.2, 0.8 Hz, 1H), 4.04 (d, *J* = 0.9 Hz, 3H).

**Step 3: Synthesis of methyl (*S*)-5-nitro-6-((oxetan-2-ylmethyl)amino)picolinate**

methyl 6-chloro-5-nitropicolinate (1.3 g, 6.0 mmol) and (S)-oxetane-2-methanamine (0.58 g, 6.6 mmol) were dissolved in tetrahydrofuran (20 mL). Then potassium carbonate (1.24 g, 9.00 mmol) was added and stirred at room temperature overnight. The reaction mixture was filtered with diatomaceous earth and the filtrate was concentrated. And the residue was was purified by column chromatography eluted with PE:EtOAc (v/v)=2:1) to give a yellow solid (1.09 g, 68.0% ).

LC-MS (ESI): $[M+ H]^+$ =268.2;
$^1$H NMR (500 MHz, DMSO-d6) δ 8.58 (dd, *J* = 8.4, 1.0 Hz, 1H), 8.51 (t, *J* = 5.8 Hz, 1H), 7.31 (dd, *J* = 8.4, 1.0 Hz, 1H), 5.01 - 4.93 (m, 1H), 4.56 -4.52 (m, 1H), 4.48 - 4.45 (m, 1H), 3.96 - 3.87 (m, 4H), 3.82 (dt, *J* = 13.8, 5.2 Hz, 1H), 2.68 - 2.58 (m, 1H), 2.53 - 2.49 (m, 1H).

**Step 4: Synthesis of methyl (*S*)-5-amino-6-((oxetan-2-ylmethyl)amino)picolinate**

Methyl (S)-5-nitro-6-((oxetan-2-ylmethyl)amino)pyridinecarboxylate (1.09 g, 4.08 mmol) was dissolved in the mixture of tetrahydrofuran (10 mL) and methanol (10 mL) under the protection of hydrogen, and then palladium-carbon

(10% Pd, 40-60% water) (185 mg, 1.74 mmol) was added and stirred at room temperature. The reaction solution was filtered with diatomaceous earth and the filtrate was concentrated. The residue was was purified by column chromatography eluted with DCM:MeOH (v/v)=20:1 to give an oily-yellow viscous material (885 mg, 91.5%).

LC-MS (ESI): $[M+H]^+$ =238.1;
$^1$H NMR (500 MHz, DMSO-$d_6$) δ 7.23 *(d, J* = 7.8 Hz, 1H), 6.68 (d, *J* = 7.8 Hz, 1H), 5.95 (t, *J* = 5.6 Hz, 1H), 5.61 (s, 2H), 4.92 - 4.87 (m, 1H), 4.54 - 4.50 (m, 1H), 4.45 (dt, *J* = 9.0, 5.9 Hz, 1H), 3.72 (s, 4H), 3.62 (dt, *J* = 13.8, 5.2 Hz, 1H), 2.65 - 2.57 (m, 1H), 2.44 - 2.39 (m, 1H).

**Step 5: Synthesis of 2-chloro-5-fluoro-4-(methoxymethoxy)pyrimidine**

2-Chloro-5-fluoropyrimidin-4-ol (20 g, 127.93 mmol) was added to the mixture of dichloromethane (100 mL) and *N,N*-dimethylformamide (100 mL), and stirred for 10 minutes under ice bath conditions. *N,N*-diisopropylethylamine (63.9 mL, 386.85 mmol) was added slowly dropwise and stirring was continued for 10 minnutes. Bromomethyl methyl ether (33 g, 250.86 mmol) was added and stirred overnight. Diluted with water (100 mL), extracted with ethyl acetate (35 mL × 3), the combined organic phases were washed with saturated saline (50 mL), dried with anhydrous sodium sulfate, concentrated under reduced pressure, and the residue was purified by column chromatography eluted with EtOAc:PE (v/v) = 25% to give a white solid (10 g, 40.6%).

$^1$HNMR (400 MHz, DMSO-$d_6$) δ 8.08 (d, *J* = 1.6 Hz, 1H), 5.46 (s, 2H), 3.36 (s, 3H).

**Step 6: Synthesis of ethyl 2-(4-(5-fluoro-4-(methoxymethoxy)pyrimidin-2-yl)cyclohex-3-en-1-yl)acetate**

2-chloro-5-fluoro-4-(methoxymethoxy)pyrimidine (8 g, 41.54 mmol), methyl 2-(4-(4,4,5,5-tetramethyl-1,3,2-dioxaborolan-2-yl)cyclohex-3-en-1-yl)acetate (13.44 g, 45.7 mmol), potassium carbonate (11.48 g, 83.09 mmol) were added to the mixture of 1,4-dioxane (100 mL) and water (25 mL), then 1,1'-bis(diphenylphosphino)ferrocene palladium(II) dichloride (0.76 g, 1.04 mmol) was added to the reaction mixture and it was replaced with nitrogen for three times again, and the reaction was carried out at 85 °C overnight. Cooled to room temperature, removed the solvent under reduced pressure, diluted with water (80 mL), extracted with ethyl acetate (50 mL×2), the combined organic phases were dried with anhydrous sodium sulfate, concentrated under reduced pressure, and the residue was purified by column chromatography eluted with EtOAc:PE (v/v)=33% to give a colorless oil (8.1 g, 60.1%).

LC-MS (ESI): $[M+H]^+$ =311.3;
$^1$HNMR (400 MHz, $CDCl_3$) δ 7.83 (d, *J* = 1.6 Hz, 1H), 6.12 (s, 1H), 5.40 - 5.35 (m, 2H), 3.70 (s, 3H), 3.50 (s, 3H), 2.44 - 2.43 (m, 2H), 2.43 - 2.34 (m, 2H), 2.19 - 1.91 (m, 4H), 1.50 - 1.44 (m, 1H).

**Step 7: Synthesis of 2-(4-(5-fluoro-4-(methoxymethoxy)pyrimidin-2-yl)cyclohex-3-en-1-yl)ethan-1-ol**

ethyl 2-(4-(5-fluoro-4-(methoxymethoxy)pyrimidin-2-yl)cyclohex-3-en-1-yl)acetate (8.1 g, 26.1 mmol) was dissolved in tetrahydrofuran (250 mL) and stirred at -78 °C for five minutes. Diisobutylaluminum hydride hexane solution (1 mol / L, 78.3 mL) was added dropwise to the reaction mixture and the reaction was carried out for 4 h. Quenched with saturated ammonium chloride solution (300 mL), extracted with dichloromethane (200 mL), and the separated organic phase was dried with anhydrous sodium sulfate, concentrated under reduced pressure, and the residue was purified by column chromatography eluted with EtOAc:PE (v/v) = 75%) to give a light yellow oil (5 g, 68.8%).

LC-MS (ESI): $[M+H]^+$ =283.5.

**Step 8: Synthesis of 2-(4-(5-fluoro-4-(methoxymethoxy)pyrimidin-2-yl)cyclohex-3-en-1-yl)acetaldehyde**

2-(4-(5-fluoro-4-(methoxymethoxy)pyrimidin-2-yl)cyclohex-3-en-1-yl)ethan-1-ol (5 g, 17.73 mmol) was added to dichloromethane (200 mL) and dissolved completely, then Days-Martin oxidizer (22.56 g, 53.19 mmol) was added with stirring at room temperature overnight. The precipitate was filtered with diatomaceous earth, and the organic layer was concentrated under reduced pressure and the residue was purified by column chromatography eluted with EtOAc:PE (v/v) =50% to give a colorless oily product (3 g, 60.4%).

LC-MS (ESI): $[M+H]^+$ =281.4.

**Step 9: Synthesis of methyl 2-((4-(5-fluoro-4-(methoxymethoxy)pyrimidin-2-yl)cyclohex-3-en-1-yl) methyl)-3-(((*S*)-oxetan-2-yl)methyl)-3*H*-imidazo[4,5-*b*]pyridine-5-carboxylate**

2-(4-(5-fluoro-4-(methoxymethoxy)pyrimidin-2-yl)cyclohex-3-en-1-yl)acetaldehyde (101 mg, 0.43 mmol) and

methyl methyl (*S*)-5-amino-6-((oxetan-2-ylmethyl)amino)picolinate (102 mg, 0.43 mmol) were added to acetic acid (1.5 mL) and reacted for 1 h at 60 °C. The reaction mixture was concentrated under reduced pressure and the residue was purified by column chromatography eluted with MeOH:DCM (v/v)=3% to give a light yellow oil (84 mg, 47.3%).

LC-MS (ESI): $[M+H]^+$ =498.5;
$^1$HNMR (400 MHz, DMSO-$d_6$) δ 8.14 (d, *J* = 8.4 Hz, 1H), 8.09 (d, *J* = 2.4 Hz, 1H), 8.0 (d, *J* = 8.4 Hz, 1H), 6.07 (s, 1H), 5.29 (s, 2H), 5.12 - 5.09 (m, 1H), 4.68 - 4.31 (m, 4H), 3.90 (s, 3H), 3.35 (s, 3H), 3.10 - 3.02 (m, 2H), 2.75 - 2.66 (m, 1H), 2.44 - 2.36 (m, 5H), 2.06 - 1.91 (m, 2H), 1.54 - 1.46 (m, 1H).

**Step 10: Synthesis of methyl 2-((4-(5-fluoro-4-hydroxypyrimidin-2-yl)cyclohex-3-en-1-yl)methyl)-3-(((*S*)-oxetan-2-yl)methyl)-3*H*-imidazo[4,5-*b*]pyridine-5-carboxylate**

methyl 2-((4-(5 -fhioro-4-(methoxymethoxy)pyrimidin-2-yl)cyclohex-3 -en-1 -yl)methyl)-3 -(((*S*)-oxetan-2-yl) methyl)-3H imidazo[4,5-b]pyridine-5-carboxylate (80 mg, 0.16 mmol) was dissolved in dichloromethane (8 mL), trifluoroacetic acid (2.4 mL) was droped slowly and stirred for 12 hours at -50 °C. Concentrated under reduced pressure and the residue was purified by column chromatography eluted with DCM:MeOH (v/v)=5% to give a light yellow solid (63 mg, 86.4%).

LC-MS (ESI): $[M+H]^+$ =454.6.

**Step 11: Synthesis of methyl 2-((4-(4-((4-chloro-2-fluorobenzofuran-7-yl)methoxy)-5-fluoropyrimidin-2-yl)cyclohex-3-en-1-yl)methyl)-3-(((*S*)-oxetan-2-yl)methyl)-3*H*-imidazo[4,5-*b*]pyridine-5-carboxylate**

2-((4-(5-fluoro-4-hydroxypyrimidin-2-yl)cyclohex-3-en-1-yl)methyl)-3-(((*S*)-oxetan-2-yl)methyl)-3*H*-imidazo [4,5-*b*]pyridine-5-carboxylate (63 mg, 0.14 mmol) was added to *N,N*-dimethylformamide (2.7 mL). After complete dissolution, potassium carbonate (58 mg, 0.42 mmol) and 7-(bromomethyl)-4-chloro-2-fluorobenzofuran (44 mg, 0.17 mmol) were added, and the reaction was carried out at 60 °C for 2 h. The reaction was concentrated under reduced pressure, and the residue was purified by column chromatography eluted with EtOAc:PE (v/v) = 50% to afford a light-yellow solid (67 mg, 75.8%).

LC-MS (ESI): $[M+H]^+$=636.6.

**Step 12: Synthesis of 2-((4-(4-((4-chloro-2-fluorobenzofuran-7-yl)methoxy)-5-fluoropyrimidin-2-yl)cyclohex-3-en-1-yl)methyl)-3-(((*S*)-oxetan-2-yl)methyl)-3*H*-imidazo[4,5-*b*]pyridine-5-carboxylic acid**

methyl 2-((4-(4-((4-chloro-2-fluorobenzofuran-7-yl)methoxy)-5-fluoropyrimidin-2-yl)cyclohex-3-en-1-yl) methyl)-3-(((*S*)-oxetan-2-yl)methyl)-3*H*-imidazo[4,5-*b*]pyridine-5-carboxylate (67 mg, 0.11 mmol) was added to the mixture of dioxane (0.8 mL) and water (0.2 mL). After complete dissolution, lithium hydroxide (13 mg, 0.54 mmol) was added and the reaction was carried out at 40 °C for 2 h. The pH was adjusted to 6 with acetic acid (0.2 mL), concentrated under reduced pressure, and the residue was purified by pre-HPLC eluted with $CH_3CN$: $H_2O$ (v/v) = 60% to give a white solid (12.5 mg, 19.1%).

LC-MS (ESI): $[M+H]^+$ =622.5;
$^1$HNMR (400 MHz, DMSO-$d_6$) δ 12.81 (s, 1H), 8.58 (s, 1H), 8.12 (d, *J*= 8.4 Hz, 1H), 7.99 (d, *J*= 8.4 Hz, 1H), 7.49 - 7.43 (m, 2H), 7.11 (s, 1H), 6.55 (d, *J* = 6.4 Hz, 1H), 5.77 (s, 2H), 5.12 (m, 1H), 4.68 - 4.30 (m, 4H), 3.11 - 3.06 (m, 2H), 2.71 - 2.67 (m, 2H), 2.43 - 2.34 (m, 4H), 2.33 - 2.32 (m, 2H), 1.99 - 1.87 (m, 1H).

**Example 6 Synthesis of 2-((4-(6-((4-chloro-2-fluorobenzofuran-7-yl)methoxy)pyridin-2-yl)cyclohex-3-en-1-yl) methyl)-3-(((*S*)-oxetan-2-yl)methyl)-3*H*-imidazo[4,5-*b*]pyridine-5-carboxylic acid (comound 6)**

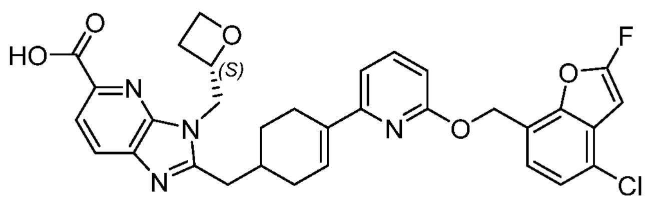

**Step 1: Synthesis of methyl 2-((4-(6-hydroxypyridin-2-yl)cyclohex-3-en-1-yl)methyl)-3-(((*S*)-oxetan-2-yl)methyl)-3*H*-imidazo[4,5-*b*]pyridine-5-carboxylate**

2-(4-(6-(methoxymethoxy)pyridin-2-yl)cyclohex-3-en-1-yl)acetaldehyde (300 mg, 1.15 mmol) (synthesis method refer to Step 7 of Example 8), methyl (*S*)-5-amino-6-((oxetan-2-ylmethyl)amino)picolinate (286 mg, 1.21 mmol) (synthesis method refer to Step 4 of Example 5) were added to acetic acid (10 mL) and stirred at 60°C for 2 hours. Concentrated under reduced pressure and the residue was purified by column chromatography eluted with DCM:MeOH (v/v)=20:1 to give a light brown solid (160 mg 47.6%).

LC-MS (ESI): $[M+H]^+$=435.4;
$^1$H NMR (400 MHz, DMSO-$d_6$) δ 11.27 (s, 1H), 8.14 (d, *J* = 8.2 Hz, 1H), 8.00 (d, *J* = 8.2 Hz, 1H), 7.39 (dd, *J* = 9.0, 7.1 Hz, 1H), 6.46 (s, 1H), 6.21 (t, *J* = 7.3 Hz, 2H), 5.09 (d, *J* = 6.2 Hz, 1H), 4.65 (dd, *J* = 15.0, 6.6 Hz, 1H), 4.49 (m, 2H), 4.36 - 4.27 (m, 1H), 3.90 (s, 3H), 3.07 (dd, *J* = 11.4, 7.0 Hz, 2H), 2.75 - 2.65 (m, 1H), 2.47 - 2.28 (m, 5H), 2.09 - 1.93 (m, 2H), 1.48 (m, 1H).

**Step 2: Synthesis of methyl 2-((4-(6-((4-chloro-2-fluorobenzofuran-7-yl)methoxy)pyridin-2-yl)cyclohex-3-en-1-yl)methyl)-3-(((*S*)-oxetan-2-yl)methyl)-3*H*-imidazo[4,5-*b*]pyridine-5-carboxylate**

methyl 2-((4-(6-hydroxypyridin-2-yl)cyclohex-3-en-1-yl)methyl)-3-(((*S*)-oxetan-2-yl)methyl)-3*H*-imidazo[4,5-*b*]pyridine-5-carboxylate (200 mg, 0.46 mmol), 7-(bromomethyl)-4-chloro-2-fluorobenzofuran (182 mg, 0.69 mmol) were added to *N,N*-dimethylformamide (10 mL), then potassium carbonate (190 mg, 1.38 mmol) was added, and stirred at 60 °C for 1 hour. Water (10 mL) was added and extracted with ethyl acetate (20 mL×3), the combined organic phases were washed with saturated brine, dried with anhydrous sodium sulfate, and the residue was purified by column chromatography eluted with DCM:MeOH (v/v)=10:1 to give a light yellow solid (200 mg, 70.3%).

LC-MS (ESI): $[M+H]^+$=617.6.

**Step 3: Synthesis of 2-((4-(6-((4-chloro-2-fluorobenzofuran-7-yl)methoxy)pyridin-2-yl)cyclohex-3-en-1-yl)methyl)-3-(((*S*)-oxctan-2-yl)mcthyl)-3*H*-imidazo[4,5-*b*]pyridinc-5-carboxylic acid**

methyl 2-((4-(6-((4-chloro-2-fluorobenzofuran-7-yl)methoxy)pyridin-2-yl)cyclohex-3-en-1-yl)methyl)-3-(((*S*)-oxetan-2-yl)methyl)-3*H*-imidazo[4,5-*b*]pyridine-5-carboxylate (200 mg, 0.32 mmol) was dissolved in the mixture of dioxane (16 mL) and water (4 mL). Lithium hydroxide (31 mg, 1.3 mmol) was added and stirred for 4 h at room temperature. The pH was adjusted to 6-7 with hydrochloric acid (0.5 N), extracted with ethyl acetate (10 mL × 3), and the combined organic phases were washed once with saturated saline, and dried with anhydrous sodium sulfate. The residue was concentrated under reduced pressure and purified by prep-HPLC eluted with acetonitrile:water (v/v)=50% to give a white solid (66.2 mg, 33.9%).

LC-MS (ESI): $[M+H]^+$=603.6;
$^1$HNMR (400 MHz, DMSO-$d_6$) δ 12.80 (s, 1H), 8.11 (d, *J* = 8.2 Hz, 1H), 7.99 (d, *J* = 8.2 Hz, 1H), 7.67 (t, *J* = 7.8 Hz, 1H), 7.41 (q, *J* = 8.2 Hz, 2H), 7.07 (d, *J* = 7.5 Hz, 1H), 6.72 (d, *J* = 8.2 Hz, 2H), 6.52 (dd, *J* = 6.4, 2.7 Hz, 1H), 5.59 (s, 2H), 5.16 - 5.07 (m, 1H), 4.66 (dd, *J* = 15.0, 6.6 Hz, 1H), 4.54 (dd, *J* = 15.0, 3.6 Hz, 1H), 4.46 (dd, *J* = 13.7, 7.6 Hz, 1H), 4.36 - 4.27 (m, 1H), 3.15 - 3.01 (m, 2H), 2.75 - 2.65 (m, 1H), 2.57 (d, *J* = 17.3 Hz, 1H), 2.47 - 2.31 (m, 4H), 2.13 - 1.94 (m, 2H), 1.57 - 1.42 (m, 1H).

**Example 7 Synthesis of (5)-2-((4-(6-((4-chloro-2-fluorobenzofuran-7-yl)methoxy)pyridin-2-yl)cyclohexyl)methyl)-1-(oxetan-2-ylmethyl)-1*H*-benzo[*d*]imidazole-6-carboxylic acid (compound 7)**

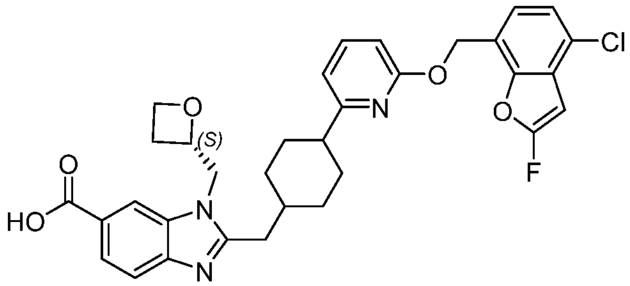

**Step 1: Synthesis of methyl (*S*)-4-(2-(4-(6-((4-chloro-2-fluorobenzofuran-7-yl)methoxy)pyridin-2-yl)cyclohexyl) acetamido)-3-((oxetan-2-ylmethyl)amino)benzoate**

2-(4-(6-((4-chloro-2-fluorobenzofuran-7-yl)methoxy)pyridin-2-yl)cyclohexyl)acetic acid (700 mg, 1.68 mmol) and methyl (S)-4-amino-3-((oxetan-2-ylmethyl)amino)benzoate (400 mg, 1.68 mmol) were added to *N,N*-dimethylformamide (10 mL). Then HOAT (300 mg, 2.18 mmol), EDCI (480 mg, 2.51 mmol) and *N,N*-diisopropylethylamine (430 mg, 3.35 mmol) were added and the reaction was carried out at 40 °C overnight. The solvent was removed under reduced pressure, water (40 mL) was added, extracted with ethyl acetate (30 mL×3), the combined organic phases were dried with anhydrous sodium sulfate, concentrated under reduced pressure, and the residue was purified by column chromatography eluted with EtOAc:PE (v/v)=45% to give a white solid (700 mg, 65.7%).

LC-MS (ESI): $[M+H]^+$=636.4.

**Step 2: Synthesis of methyl (*S*)-2-((4-(6-((4-chloro-2-fluorobenzofuran-7-yl)methoxy)pyridin-2-yl)cyclohexyl) methyl)-1-(oxetan-2-ylmethyl)-1*H*-benzo[*d*]imidazole-6-carboxylate**

methyl (S)-4-(2-(4-(6-((4-chloro-2-fluorobenzofuran-7-yl)methoxy)pyridin-2-yl)cyclohexyl)acetamido)-3-((oxetan-2-ylmethyl)amino)benzoate (700 mg, 1.10 mmol) was added to glacial acetic acid (20 mL), and the reaction was carried out at 80 °C for 4 h. The solvent was removed under reduced pressure, the pH was adjusted to 8 with saturated aqueous sodium bicarbonate, extracted with ethyl acetate (30 mL×3), the combined organic phases were dried with anhydrous sodium sulfate, concentrated under reduced pressure, and the residue was purified by column chromatography eluted with EtOAc:PE (v/v)=2:3 to give a light yellow solid (600 mg, 88.2%).

$^1$HNMR (500 MHz, $CDCl_3$) δ 8.09-8.07 (m, 1H), 7.97 - 7.94 (m, 1H), 7.75 - 7.72 (m, 1H), 7.53 - 7.46 (m, 1H), 7.32 (d, *J* = 8.7 Hz, 1H), 7.24 - 7.21 (m, 1H), 6.82 - 6.70 (m, 1H), 6.62 - 6.58 (m, 1H), 6.00 - 5.98 (m, 1H), 5.60 (d, *J* = 14.9 Hz, 2H), 5.20 - 5.13 (m, 1H), 4.65 - 4.55 (m, 1H), 4.45 - 4.30 (m, 3H), 3.94 - 3.93 (m, 3H), 3.01 - 2.94 (m, 2H), 2.82 - 2.60 (m, 2H), 2.57 - 2.35 (m, 2H), 1.99 - 1.89 (m, 2H), 21.63 - 1.58 (m, 6H).

LC-MS (ESI): $[M+H]^+$=618.4.

**Step 3: Synthesis of (*S*)-2-((4-(6-((4-chloro-2-fluorobenzofuran-7-yl)methoxy)pyridin-2-yl)cyclohexyl)methyl)-1-(oxetan-2-ylmethyl)-1*H*-benzo[*d*]imidazole-6-carboxylic acid**

methyl (*S*)-2-((4-(6-((4-chloro-2-fluorobenzofuran-7-yl)methoxy)pyridin-2-yl)cyclohexyl)methyl)-1-(oxetan-2-ylmethyl)-1*H*-benzo[d]imidazole-6-carboxylate (600 mg, 0.97 mmol) and lithium hydroxide monohydrate (200 mg, 4.85 mmol) were added to the mixture of isopropanol (18 mL) and water (3 mL), the reaction was carried out at 50 °C for 2 h. The pH was adjusted to 4 with dilute hydrochloric acid (2 M) in an ice bath, diluted with water (30 mL), extracted with ethyl acetate (30 mL×3), the combined organic phases were dried with anhydrous sodium sulfate, concentrated under reduced pressure, and the residue was purified by column chromatography eluted with MeOH: DCM (v/v)=5% to give a light yellow solid (200 mg, 31.9%).

$^1$HNMR (500 MHz, $CDCl_3$) δ 8.19 - 8.17 (m, 1H), 8.07 - 8.04 (m, 1H), 7.83 - 7.81 (m, 1H), 7.51 - 7.45 (m, 1H), 7.33 - 7.30 (m, 1H), 7.23 - 7.20 (m, 1H), 6.82 - 6.69 (m, 1H), 6.61 - 6.57 (m, 1H), 6.00 - 5.98 (m, 1H), 5.00 - 5.57 (m, 2H), 5.23 - 5.14 (m, 1H), 4.66 - 4.57 (m, 1H), 4.51 - 4.32 (m, 3H),3.06 - 2.98 (m, 2H), 2.81 - 2.55 (m, 2H), 2.48 - 2.39 (m, 1H), 2.16 - 1.93 (m, 4H), 1.80 - 1.58 (m, 3H), 1.44 - 1.24 (m, 3H).

LC-MS (ESI): $[M+H]^+$=604.5.

**Example 8 Synthesis of 2-((4-(6-((4-chloro-2-fluorobenzofuran-7-yl)methoxy)pyridin-2-yl)cyclohex-3-en-1-yl) methyl)-1-(((*S*)-oxetan-2-yl)methyl)-1*H*-thieno[2,3-*d*]imidazole-5-carboxylic acid (compound 8)**

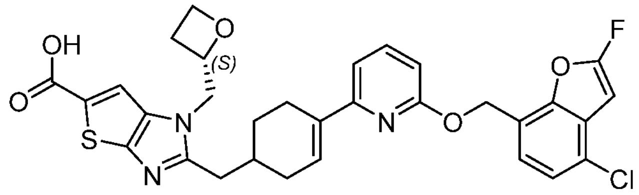

**Step 1: Synthesis of methyl 4-bromo-5-nitrothiophene-2-carboxylate**

Methyl 4-bromothiophene-2-carboxylate (6 g, 27.1 mmol) was added to concentrated sulfuric acid (20 mL) and the mixture was cooled to -5 °C. Nitric acid (4 mL) was added to concentrated sulfuric acid (12 mL) and then added slowly dropwise to the reaction mixture and stirred for 30 minutes. Ice water (50 mL) was added and a large amount of solid precipitated. Filtered and the filter cake was washed with water (50 mL × 3). The filtrate was further concentrated under reduced pressure to give a light yellow solid (6.7 g, 90.9%).

LC-MS: $[M+H]^+$=267.1;
$^1$HNMR (400 MHz, $CDCl_3$) δ 7.71 (s, 1H), 3.96 (s, 3H).

**Step 2: Synthesis of methyl (*S*)-5-nitro-4-((oxetan-2-ylmethyl)amino)thiophene-2-carhoxylate**

Methyl 4-bromo-5-nitrothiophene-2-carboxylate (6.35 g, 23.9 mmol), (S)-oxetan-2-ylmethylamine (2.50 g, 28.6 mmol) were added to 1,4-dioxane (120 mL), then triethylamine (10.0 mL, 71.6 mmol) was added, and the reaction was stirred under nitrogen protection heated to 110 °C overnight. The reaction mixture was concentrated under reduced pressure and the residue was purified by column chromatography eluted with EtOAc:PE (v/v)=1:2 to give a yellow solid (3.5 g 53.9%).

LC-MS: $[M+H]^+$=273.3;
$^1$HNMR (400 MHz, $CDCl_3$) δ 8.02 (s, 1H), 7.29 (s, 1H), 4.99 (s, 1H), 4.63 (dd, *J* = 14.3, 7.4 Hz, 1H), 4.48 (dd, *J* = 14.8, 6.1 Hz, 1H), 3.86 (s, 3H), 3.56 (t, *J* = 9.7 Hz, 2H), 2.78 - 2.63 (m, 1H), 2.56 - 2.41 (m, 1H).

**Step 3: Synthesis of methyl (5)-5-amino-4-((oxetan-2-ylmethyl)amino)thiophene-2-carboxylate**

Methyl (*S*)-5-nitro-4-((oxetan-2-ylmethyl)amino)thiophene-2-carboxylate (150 mg, 0.55 mmol) was added to acetic acid (5 mL), then zinc powder (360 mg, 5.5 mmol) was added under an ice bath, and stirred for 5 min at room temperature. The reaction solution was filtered solid with diatomaceous earth, concentrated under reduced pressure and the residue was purified by column chromatography eluted with DCM:MeOH (v/v)=10:1 to give a dark brown solid (80 mg, 59.9%).

LC-MS: $[M+H]^+$=243.4;
$^1$HNMR(400 MHz, DMSO-$d_6$) δ 7.15 (s, 1H), 5.92 (s, 2H), 4.84 - 4.75 (m, 1H), 4.51 (dd, *J* = 13.8, 7.6 Hz, 1H), 4.43 (dt, *J* = 9.1, 5.9 Hz, 1H), 4.24 (s, 1H), 3.66 (s, 3H), 3.18 (qd, *J* = 13.4, 4.9 Hz, 2H), 2.65 - 2.55 (m, 1H), 2.46 - 2.34 (m, 1H).

**Step 4: Synthesis of 2-bromo-6-(methoxymethoxy)pyridine**

6-Bromopyridin-2-ol (16 g, 92.0 mmol) was added to dichloromethane (160 mL), triethylamine (63.9 mL, 459.5 mmol) was added and stirred for 10 minutes under ice bath conditions. Bromo(methoxy)methane (23 g, 184.0 mmol) was added and stirred at room temperature for 2 hours. Dilute with Water (30 mL), extracted with dichloromethane (30 mL×3), the combined organic phases were washed with saturated brine, dried with anhydrous sodium sulfate, concentrated under reduced pressure, and the residue was purified by column chromatography eluted with EtOAc:PE (v/v)=1:20 to give a colorless oil (5.2 g, 25.9%).

$^1$HNMR (400 MHz, DMSO-$d_6$) δ 7.72 (d, *J* = 7.8 Hz, 1H), 7.28 (d, *J* = 7.3 Hz, 1H), 6.93 (d, *J* = 7.9 Hz, 1H), 5.42 (s, 2H), 3.42 (s, 3H).

**Step 5: Synthesis of methyl 2-(4-(6-(methoxymethoxy)pyridin-2-yl)cyclohex-3-en-1-yl)acetate**

2-bromo-6-(methoxymethoxy)pyridine (5.2 g, 23.8 mmol), methyl 2-(4-(4,4,5,5-tetramethyl-1,3,2-dioxaborolan-2-yl)cyclohex-3-en-1-yl)acetate (7.68 g, 27.4 mmol) was added to the mixture of 1,4-dioxane (60 mL) and water (15

mL). Potassium carbonate (6.59 g, 47.7 mmol) was added, it was replaced with nitrogen for three times, Pd(dppf)$Cl_2$ (0.52 g, 0.72 mmol) was added, replaced for three times with nitrogen again, and the reaction was heated to 85 °C and stirred overnight. The reaction mixture was concentrated under reduced pressure and the residue was purified by column chromatography eluted with EtOAc:PE (v/v)=1:15 to give a colorless oil (4.0 g 57.6%).

$^1$HNMR (400 MHz, $CDCl_3$) δ 7.48 (t, *J* = 7.8 Hz, 1H), 6.90 (d, *J* = 7.5 Hz, 1H), 6.68 (dd, *J* = 3.1, 1.9 Hz, 1H), 6.57 (d, *J* = 8.1 Hz, 1H), 5.49 (s, 2H), 3.62 (s, 3H), 3.45 (d, *J* = 3.2 Hz, 3H), 2.53 (dd, *J* = 16.5, 3.0 Hz, 1H), 2.46 - 2.31 (m, 2H), 2.28 (d, *J* = 7.2 Hz, 2H), 2.17 - 2.03 (m, 1H), 1.96 - 1.84 (m, 2H), 1.38 (m, 1H).

**Step 6: Synthesis of 2-(4-(6-(methoxymethoxy)pyridin-2-yl)cyclohex-3-en-1-yl)ethan-1-ol**

methyl 2-(4-(6-(methoxymethoxy)pyridin-2-yl)cyclohex-3-en-1-yl)acetate (1.5 g, 5.2 mmol) was added to tetrahydrofuran (30 mL) and stirred at -20 °C for 5 min. Lithium tetrahydroaluminum (0.39 g, 10.3 mmol) was added and stirred at 0 °C for 2 hours. Quenched with water (10 mL), extracted with ethyl acetate (30 mL × 3), the combined organic phases were washed with saturated brine, dried with anhydrous sodium sulfate, concentrated under reduced pressure, and the residue was purified by column chromatography eluted with EtOAc:PE (v/v)=1:5 to give a colorless oil (1.3 g, 95.9%).

LC-MS (ESI): $[M+H]^+$=264.3.

**Step 7: Synthesis of 2-(4-(6-(methoxymethoxy)pyridin-2-yl)cyclohex-3-en-1-yl)acetaldehyde**

2-(4-(6-(methoxymethoxy)pyridin-2-yl)cyclohex-3-en-1-yl)ethan-1-ol (600 mg, 2.2 mmol) was added to dichloromethane (20 mL), Desmartin's reagent (1.45 g, 3.4 mmol) was added, and stirred for 3 hours under icebath condition, large amount of solid was precipitated. Water (4 mL) was added, filtered and concentrated under reduced pressure, and the residue was purified by column chromatography eluted with EtOAc:PE (v/v)=1:5 to give a colorless oil (260 mg, 43.7%).

LC-MS (ESI): $[M+H]^+$=262.1.

**Step 8: Synthesis of methyl 2-((4-(6-hydroxypyridin-2-yl)cyclohex-3-en-1-yl)methyl)-1-(((*S*)-oxetan-2-yl) methyl)-1*H*-thieno[2,3-*d*]imidazole-5-carboxylate**

2-(4-(6-(methoxymethoxy)pyridin-2-yl)cyclohex-3-en-1-yl)acetaldehyde (200 mg, 0.76 mmol), methyl (S)-5-amino-4-((oxetan-2-ylmethyl)amino)thiophene-2-carboxylate (280 mg, 1.1 mmol) were added to acetic acid (5 mL), and the reaction was stirred by heating to 60 °C for 2 hours. Stopped and concentrated under reduced pressure and the residue was purified by column chromatography eluted with DCM:MeOH (v/v)=20:1 to give a light brown solid (160 mg, 47.6%).

LC-MS (ESI): $[M+H]^+$=440.6.

**Step 9: Synthesis of methyl 2-((4-(6-((4-chloro-2-fluorobenzofuran-7-yl)methoxy)pyridin-2-yl)cyclohex-3-en-1-yl)methyl)-1-(((*S*)-oxetan-2-yl)methyl)-1*H*-thieno[2,3-*d*]imidazole-5-carboxylate**

Methyl 2-((4-(6-hydroxypyridin-2-yl)cyclohex-3-en-1-yl)methyl)-1-(((*S*)-oxetan-2-yl)methyl)-1*H*-thieno[2,3-*d*]imidazole-5-carboxylate (150 mg, 0.34 mmol), 7-(bromomethyl)-4-chloro-2-fluorobenzofuran (117 mg, 0.44 mmol) were added to *N*,*N*-dimethylformamide (5 mL). Potassium carbonate (118 mg, 0.85 mmol) was added and stirred at 60 °C for 1 hour. Diluted with water (5 mL), extracted with ethyl acetate (15 mL × 3), the combined organic phases were washed with saturated brine, dried with anhydrous sodium sulfate, and the residue was purified by column chromatography eluted with DCM:MeOH (v/v)=10:1 to give a white solid (85 mg, 40.0%).

LC-MS (ESI): $[M+H]^+$=622.2.

**Step 10: Synthesis of 2-((4-(6-((4-chloro-2-fluorobenzofuran-7-yl)methoxy)pyridin-2-yl)cyclohex-3-en-1-yl) methyl)-1-(((*S*)-oxetan-2-yl)methyl)-1*H*-thieno[2,3-*d*]imidazole-5-carboxylic acid**

Methyl 2-((4-(6-((4-chloro-2-fluorobenzofuran-7-yl)methoxy)pyridin-2-yl)cyclohex-3-en-1-yl) methyl)-1-(((*S*)-oxetan-2-yl)methyl)-1*H*-thieno[2,3-*d*]imidazole-5-carboxylate (75 mg, 0.12 mmol) was dissolved in the mixture of dioxane (8 mL) and water (2 mL). Lithium hydroxide (12 mg, 0.50 mmol) was added and stirred at room temperature for 2 hours. The pH was adjusted to 6-7 with hydrochloric acid (0.5 N), extracted with ethyl acetate (5 mL×3), and the combined organic phases were washed with saturated brine, dried with anhydrous sodium sulfate, concentrated under reduced pressure, and the residue was purified by prep-HPLC eluted with acetonitrile:water (v/v)=50% to give a white solid (9.8 mg, 13.4%).

LC-MS (ESI): $[M+H]^+$=608.5;

$^1$HNMR (400 MHz, DMSO-$d_6$) δ 12.87 (s, 1H), 7.79 (s, 1H), 7.66 (t, *J* = 7.8 Hz, 1H), 7.46 - 7.36 (m, 2H), 7.06 (d, *J* = 7.5 Hz, 1H), 6.72 (d, *J* = 8.1 Hz, 2H), 6.52 (d, *J* = 6.4 Hz, 1H), 5.59 (s, 2H), 5.00 (d, *J* = 7.1 Hz, 1H), 4.58 - 4.37 (m, 4H), 2.97 - 2.86 (m, 2H), 2.76 - 2.62 (m, 1H), 2.57 - 2.52 (m, 1H), 2.42 - 2.28 (m, 3H), 2.24 - 2.19 (m, 1H), 2.10 - 1.89 (m, 2H), 1.46 - 1.38 (m, 1H).

**Example 9 Synthesis of 2-((4-(4-((4-chloro-2-fluorobenzofuran-7-yl)methoxy)-5-fluoropyrimidin-2-yl)cyclohex-3-en-1-yl)methyl)-1-(2-methoxyethyl)-1*H*-benzo[*d*]imidazole-6-carboxylic acid (compound 9)**

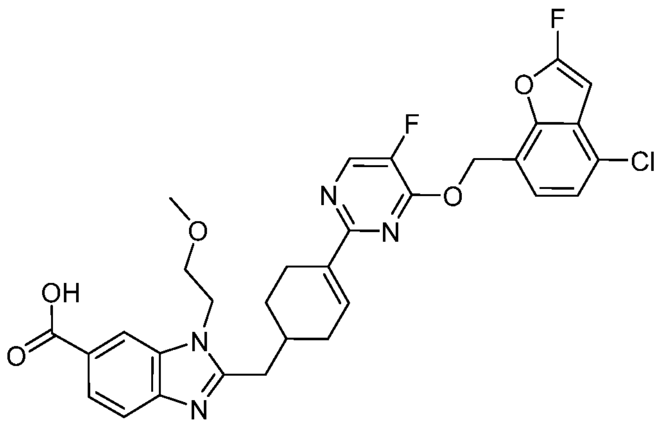

**Step 1: Synthesis of methyl 3-((2-methoxyethyl)amino)-4-nitrobenzoate**

Methyl 3-fluoro-4-nitrobenzoate (2 g, 10.04 mmol), 2-methoxyethan-1-atnine (1.2 g, 15.98 mmol) and *N,N*-diisopropylethylamine (2.6 g, 10.04 mmol) were added to tetrahydrofuran (30 mL), and the reaction was heated to 50°C and stirred for 2 hours. The reaction solution was cooled down to room temperature, concentrated under reduced pressure, and the residue was purified by column chromatography eluted with MeOH:DCM (v/v)=1:20) to give a light yellow solid (2.4 g, 98%).

$^1$H NMR (400 MHz, DMSO-$d_6$) δ 8.17 (d, *J* = 8.8 Hz, 2H), 7.57 (s, 1H), 7.16 (d, *J* = 8.9 Hz, 1H), 3.89 (s, 3H), 3.69 - 3.50 (m, 4H), 3.32 (s, 3H).

**Step 2: Synthesis of methyl 4-amino-3-((2-methoxyethyl)amino)benzoate**

Methyl 3-((2-methoxyethyl)amino)4-nitrobenzoate (2.4 g, 9.44 mmol) and 10% Pd/C (240 mg, 2.26 mmol) were added to MeOH (20 mL), and the reaction was carried out for 2 h at room temperature under hydrogen protection. Stopped and the palladium carbon was removed by filtration with diatomaceous earth and concentrated to give a gray solid (2.1 g, 99.2%).

LC-MS (ESI): $[M+H]^+$=225.2.

**Step 3: Synthesis of methyl 2-((4-(5-fluoro-4-(methoxymethoxy)pyrimidin-2-yl)cyclohex-3-en-1-yl)methyl)-1-(2-methoxyethyl)-1*H*-benzo[*d*]imidazole-6-carboxylate**

2-(4-(5-fluoro-4-(methoxymethoxy)pyrimidin-2-yl)cyclohex-3-en-1-yl)acetaldehyde (200 mg, 0.71 mmol) and methyl 4-amino-3-((2-methoxyethyl)amino)benzoate (160 mg, 0.71 mmol) were added into acetic acid (10 mL), and the reaction was heated to 60°C and stirred for 1 hour under nitrogen protection. The reaction mixture was cooled down to room temperature and concentrated under reduced pressure, and the residue was purified by column chromatography eluted with MeOH:DCM (v/v)=1:20) to give a yellow oil (280 mg, 81.0%).

LC-MS (ESI): $[M+H]^+$=485.1;

$^1$H NMR (400 MHz, DMSO-$d_6$) δ 8.18 (d, *J* = 1.1 Hz, 1H), 8.09 (d, *J* = 2.2 Hz, 1H), 7.80 (dd, *J* = 8.4, 1.5 Hz, 1H), 7.65 (d, *J* = 8.4 Hz, 1H), 6.07 (s, 1H), 5.29 (s, 2H), 4.50 (t, *J* = 5.0 Hz, 2H), 3.88 (s, 3H), 3.65 (t, *J* = 5.0 Hz, 2H), 3.35 (s, 3H), 3.18 (d, *J* = 2.9 Hz, 3H), 2.95 (d, *J* = 6.3 Hz, 2H), 2.36 (s, 3H), 2.05 - 1.88 (m, 2H), 1.56 - 1.43 (m, 1H), 1.23 (s, 1H).

**Step 4: Synthesis of methyl 2-((4-(5-fluoro-4-hydroxypyrimidin-2-yl)cyclohex-3-en-1-yl)methyl)-1-(2-methoxyethyl)-1H-benzo[*d*]imidazole-6-carboxylate**

Methyl 2-((4-(5-fluoro-4-(methoxymethoxy)pyrimidin-2-yl)cyclohex-3-en-1-yl)methyl)-1-(2-methoxyethyl)-1*H*-benzo[*d*]imidazole-6-carboxylate (260 mg, 0.54 mmol) was added to DCM (13 mL), cooled down to -10 °C, and then TFA (6.5 mL, 87.51 mmol) was added and kept the reaction at -10 °C overnight. The reaction mixture was concentrated under reduced pressure and the residue was purified by column chromatography eluted with MeOH:DCM (v/v)=1:20 to give a brown oil (230 mg, 97.3%).

LC-MS (ESI): $[M+H]^+$=441.4;
$^1$H NMR (400 MHz, DMSO-$d_6$) δ 12.67 (s, 1H), 8.17 (s, 1H), 8.03 (s, 1H), 7.84-7.77 (m, 1H), 7.65 (d, *J* = 8.2 Hz, 1H), 6.78 (s, 1H), 4.49 (s, 2H), 3.88 (s, 3H), 3.63 (d, *J* = 5.1 Hz, 2H), 3.17 (d, *J* = 4.7 Hz, 3H), 2.93 (d, *J* = 7.1 Hz, 2H), 2.33 (s, 3H), 1.99 (d, *J* = 70.6 Hz, 2H), 1.44 (s, 1H), 1.19 (d, *J* = 35.1 Hz, 1H).

**Step 5: Synthesis of methyl 2-((4-(4-((4-chloro-2-fluorobenzofuran-7-yl)methoxy)-5-fluoropyrimidin-2-yl)cyclohex-3-en-1-yl)methyl)-1-(2-methoxyethyl)-1*H*-benzo[*d*]imidazole-6-carboxylate**

Methyl 2-((4-(5-fluoro-4-hydroxypyrimidin-2-yl)cyclohex-3-en-1-yl)methyl)-1-(2-methoxyethyl)-1*H*-benzo[*d*]imidazole-6-carboxylate (210 mg, 0.48 mmol) and 7-(bromomethyl)-4-chloro-2-fluorobenzofuran (253 mg, 0.96 mmol) were added to DMF (15 mL) and stirred to dissolve, potassium carbonate (198 mg, 1.43 mmol) was added, and the reaction solution was heated to 60 °C and kept for 2 h under nitrogen protection. The reaction mixture was cooled down to room temperature, concentrated under reduced pressure, and the residue was purified by column chromatography eluted with MeOH:DCM (v/v)=1:20 to give a off-white solid (220 mg, 74.1%).

LC-MS (ESI): $[M+H]^+$=624.0.

**Step 6: Synthesis of 2-((4-(4-((4-chloro-2-fluorobenzofuran-7-yl)methoxy)-5-fluoropyrimidin-2-yl)cyclohex-3-en-1-yl)methyl)-1-(2-methoxyethyl)-1*H*-benzo[*d*]imidazole-6-carboxylic acid**

Methyl 2-((4-(4-((4-chloro-2-fluorobenzofuran-7-yl)methoxy)-5-fluoropyrimidin-2-yl)cyclohex-3-en-1-yl)methyl)-1-(2-methoxyethyl)-1*H*-benzo[*d*]imidazole-6-carboxylate (220 mg, 0.35 mmol) was added to 1,4-dioxane (5 mL), and lithium hydroxide (74 mg , 1.76 mmol) dissolved in water (2 mL) was added to the mixture. The reaction mixture was heated to 40 °C and kept for 2 h under nitrogen protection. The reaction mixture was reduced to room temperature, the pH was adjusted to about 4 with acetic acid, concentrated under reduced pressure, and the residue was purified by Prep-HPLC eluted with acetonitrile: water (v/v) = 50% to give a white solid (22.1 mg, 10.3%).

LC-MS (ESI): $[M+H]^+$=609.6;
$^1$H NMR (400 MHz, DMSO-$d_6$) δ 12.74 (s, 1H), 8.57 (d, *J = 2.9* Hz, 1H), 8.15 (d, *J* = 0.9 Hz, 1H), 7.80 (dd, *J* = 8.4, 1.4 Hz, 1H), 7.62 (d, *J* = 8.4 Hz, 1H), 7.49 - 7.42 (m, 2H), 7.12 (s, 1H), 6.55 (d, *J* = 6.4 Hz, 1H), 5.76 (s, 2H), 4.48 (t, *J* = 4.9 Hz, 2H), 3.65 (t, *J* = 5.0 Hz, 2H), 3.18 (s, 3H), 2.93 (t, *J* = 11.5 Hz, 2H), 2.68 (d, *J* = 17.7 Hz, 1H), 2.49 - 2.46 (m, 1H), 2.33 (s, 2H), 2.18 - 1.90 (m, 2H), 1.54 - 1.39 (m, 1H).

**Example 10 Synthesis of 2-((4-(4-((4-chloro-2-fluorobenzofuran-7-yl)methoxy)-5-fluoropyrimidin-2-yl)cyclohex-3-en-1-yl)methyl)-3-((1-(cyanomethyl)cyclopropyl)methyl)-3*H*-imidazo[4,5-*b*]pyridine-5-carboxylic acid (compound 10)**

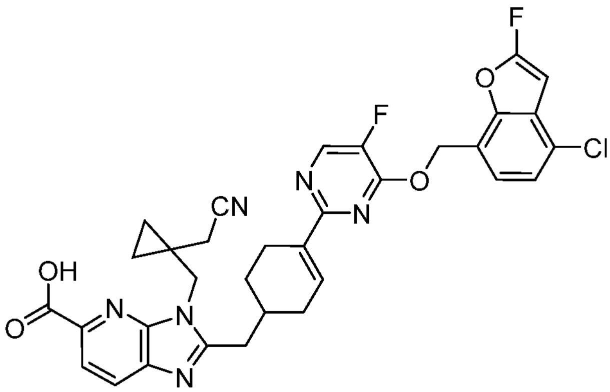

**Step 1: Synthesis of 6-chloro-5-nitropicolinic acid**

2-Chloro-6-methyl-3-nitropyridine (8.0 g, 46.36 mmol) was added to concentrated sulfuric acid (40 mL), potassium dichromate (20.46 g, 69.54 mmol) was added slowly at room temperature and the reaction was stirred at room temperature overnight. Under the condition of ice bath, ice water (500 mL) was added slowly, extracted with ethyl acetate (150 mL×3), the combined organic phases were dried with anhydrous sodium sulfate, and concentrated under reduced pressure to give a light yellow solid (8.26 g, 88.0%).
LC-MS (ESI): $[M+H]^+$=203.0.

**Step 2: Synthesis of methyl 6-chloro-5-nitropicolinate**

6-chloro-5-nitropicolinic acid (200 mg, 0.99 mmol) was dissolved in methanol (15 mL), concentrated sulfuric acid (0.5 mL, 0.99 mmol) was added, and the reaction was heated to 68 °C overnight. The solvent was concentrated, diluted with water (30 mL), extracted with ethyl acetate (15 mL×3), the combined organic phases were dried with anhydrous sodium sulfate, filtered, and the filtrate was concentrated. The residue was purified by column chromatography eluted with PE:EtOAc (v/v)=2:1 to give a white solid (185 mg, 86.5%).

LC-MS (ESI): $[M+H]^+$=217.1;
$^1$H NMR (500 MHz, $CDCl_3$) δ 8.31 (dd, *J* = 8.3, 0.8 Hz, 1H), 8.22 (dd, *J* = 8.2, 0.8 Hz, 1H), 4.04 (d, *J* = 0.9 Hz, 3H).

**Step 3: Synthesis of tert-butyl ((1-(hydroxymethyl)cyclopropyl)methyl)carbamate**

(1-(Aminomethyl)cyclopropyl)methanol (2.5 g, 24.72 mmol) and di-tert-butyl dicarbonate (2.5 g, 24.72 mmol) were dissolved in DCM (50 mL), and then *N*,*N*-diisopropylethylamine (8.2 mL, 49.43 mmol) was added, and the reaction was stirred at room temperature for 3 h. stopped and the reaction was diluted with water (40 mL), extracted with methylene chloride (15 mL×3), the combined organic phases were washed with dilute hydrochloric acid (1 M) twice, and the organic phases were dried with anhydrous sodium sulfate, filtered and concentrated. The residue was purified by column chromatography eluted with PE:EtOAc (v/v)=3:2 to give a light yellow viscous material (4.24 g, 85.2%).
$^1$H NMR (500 MHz, $CDCl_3$) δ 4.98 (s, 1H), 3.37 (d, *J* = 6.5 Hz, 2H), 3.25 (s, 1H), 3.10 (d, *J* = 6.4 Hz, 2H), 1.44 (d, *J* = 5.8 Hz, 9H), 0.45-0.40 (m, 4H).

**Step 4: Synthesis of (1-(((tert-butoxycarbonyl)amino)methyl)cyclopropyl)methyl methanesulfonate**

Tert-butyl ((1-(hydroxymethyl)cyclopropyl)methyl)carbamate (1.81 g, 8.99 mmol) was dissolved in DCM (40 mL), then triethylamine (2.5 mL, 17.99 mmol) was added, and methanesulfonyl chloride (0.8 mL, 10.79 mmol) was added slowly at 0 °C, and the reaction was continued to be stirred for 2 h. Stopped and quenched with water (50 mL), extracted with dichloromethane (30 mL×3). The combined organic phases were dried with anhydrous sodium sulfate, filtered and concentrated to give a yellowish viscous material (2.0 g, 79.6%).

**Step 5: Synthesis of tert-butyl ((1-(cyanomethyl)cyclopropyl)methyl)carbamate**

(1-(((tert-butoxycarbonyl)amino)methyl)cyclopropyl)methyl methanesulfonate (2.0 g, 7.16 mmol) and trimethylsilanecarbonitrile (1.9 mL, 14.32 mmol) were dissolved in acetonitrile (30 mL), then tetrabutylammonium fluoride (4.2 mL, 14.32 mmol) was added and the reaction was heated to 80 °C for 2 h. Stopped and cooled to room temperature.

Concentrated the solvent, diluted with water (30 mL), extracted with ethyl acetate (15 mL×3), the combined organic phases were dried with anhydrous sodium sulfate. Filtrated, concentrated the filtrate. The residue was purificated by column chromatography eluted with PE:EtOAc (v/v)=10:1 to give a light yellow solid (850 mg, 56.5%).

$^1$H NMR (500 MHz, $CDCl_3$) δ 4.75 (s, 1H), 3.13 (d, *J* = 6.4 Hz, 2H), 2.48 (s, 2H), 1.44 (s, 9H), 0.61 (d, *J* = 3.7 Hz, 4H).

**Step 6: Synthesis of 2-(1-(aminomethyl)cyclopropyl)acetonitrile hydrochloride**

Tert-butyl ((1-(cyanomethyl)cyclopropyl)methyl)carbamate (700 mg, 3.33 mmol) was dissolved in DCM (10 mL), then 1,4-dioxane hydrochloride solution (1 mL) was added, and the reaction was stirred at room temperature for 4 h. Stopped and the solvent was concentrated to give a white solid (400 mg, 81.9%).

LC-MS (ESI): [M-HCl+ H]$^+$=111.2.

**Step 7: Synthesis of methyl 6-(((1-(cyanomethyl)cyclopropyl)methyl)amino)-5-nitropicolinate**

methyl 6-chloro-5-nitropicolinate (500 mg, 2.31 mmol) and 2-(1-(atninomethyl)cyclopropyl)acetonitrile hydrochloride (1.02 g, 6.93 mmol) were dissolved in tetrahydrofuran (5 mL), then *N,N*-diisopropylethylamine (1.49 g, 11.55 mmol) was added, stirred at room temperature overnight. The reaction mixture was concentrated and the residue was purified by column chromatography eluted with PE:EtOAc (v/v)=2:1 to give a yellow solid (560 mg, 83.6%).

LC-MS (ESI): [M+ H]$^+$=291.3.

**Step 8: Synthesis of methyl 5-amino-6-(((1-(cyanomethyl)cyclopropyl)methyl)amino)picolinate**

Methyl 6-(((1-(cyanomethyl)cyclopropyl)methyl)amino)-5-nitropicolinate (160 mg, 0.55 mmol) was dissolved in ethyl acetate (20 mL), then palladium-carbon (80 mg, 10% Pd, containing 40-60% water) was added under the protection of hydrogen. The reaction was stirred at room temperature for 2 h. The reaction mixture was filtered with diatomaceous earth, and the filtrate was concentrated and the residue was purified by column chromatography eluted with DCM:MeOH (v/v)=20:1 to give a oily-yellow mucilage (120 mg, 83.6%).

LC-MS (ESI): [M+ H]$^+$=261.13.

**Step 9: Synthesis of methyl 3-((1-(cyanomethyl)cyclopropyl)methyl)-2-((4-(5-fluoro-4-(methoxymethoxy)pyrimidin-2-yl)cyclohex-3-en-1-yl)methyl)-3*H*-imidazo[4,5-*b*]pyridine-5-carboxylate**

2-(4-(5-fluoro-4-(methoxymethoxy)pyrimidin-2-yl)cyclohex-3-en-1-yl)acetaldehyde (130 mg, 0.46 mmol), methyl 5-amino-6-(((1-(cyanomethyl)cyclopropyl)methyl)amino)picolinate (120 mg, 0.46 mmol) were added to acetic acid (3 mL) and stirred for 2 h at 60 °C. The reaction mixture was concentrated under reduced pressure and the residue was purified by column chromatography eluted with DCM:MeOH (v/v)=20:1 to give a yellow solid (200 mg, 82.8%).

LC-MS (ESI): [M+H]$^+$=521.1.

**Step 10: Synthesis of methyl 3-((1-(cyanomethyl)cyclopropyl)methyl)-2-((4-(5-fluoro-4-hydroxypyrimidin-2-yl)cyclohex-3-en-1-yl)methyl)-3*H*-imidazo[4,5-*b*]pyridine-5-carboxylate**

methyl 3-((1-(cyanomethyl)cyclopropyl)methyl)-2-((4-(5 -fluoro-4-(methoxymethoxy)pyrimidin-2-yl)cyclohex-3-en-1-yl)methyl)-3*H*-imidazo[4,5-*b*]pyridine-5-carboxylate (200 mg, 0.38 mmol) was added to dichloromethane (10 mL), and the mixture was stirred for 5 min at -10 °C. Trifluoroacetic acid (5 mL, 67.31 mmol) was added dropwise and the mixture was stirred at -10 °C overnight. The mixture was concentrated under reduced pressure and the residue was purified by column chromatography eluted with DCM:MeOH (v/v)=10:1 to give a light yellow solid (95 mg, 51.9%).

LC-MS (ESI): [M+1]$^+$=477.2.

**Step 11: Synthesis of methyl 2-((4-(4-((4-chloro-2-fluorobenzofuran-7-yl)methoxy)-5-fluoropyrimidin-2-yl)cyclohex-3-en-1-yl)methyl)-3-((1-(cyanomethyl)cyclopropyl)methyl)-3*H*-imidazo[4,5-*b*]pyridine-5-carboxylate**

methyl 3-((1-(cyanomethyl)cyclopropyl)methyl)-2-((4-(5 -fluoro-4-hydroxypyrimidin-2-yl)cyclohex-3 -en-1-yl)methyl)-3*H*-imidazo[4,5-*b*]pyridine-5-carboxylate (95 mg, 0.20 mmol), 7-(bromomethyl)-4-chloro-2-fluoro-1-benzofuran (105 mg, 0.40 mmol), potassium carbonate (82 mg, 0.59 mmol) were added to *N,N*-dimethylformamide (5 mL) and stirred at 60 °C for 3 h. It was concentrated under reduced pressure and the residue was purified by column chromatography eluted with PE: EtOAc (v/v)=2:3 to give a light yellow solid (75 mg, 57.1%).

LC-MS (ESI): [M+H]$^+$=659.2.

**Step 12: Synthesis of 2-((4-(4-((4-chloro-2-fluorobenzofuran-7-yl)methoxy)-5-fluoropyrimidin-2-yl)cyclohex-3-en-1-yl)methyl)-3-((1-(cyanomethyl)cyclopropyl)methyl)-3*H*-imidazo[4,5-*b*]pyridine-5-carboxylic acid**

methyl 2-((4-(4-((4-chloro-2-fluorobenzofuran-7-yl)methoxy)-5-fluoropyrimidin-2-yl)cyclohex-3-en-1-yl) methyl)-3-((1-(cyanomethyl)cyclopropyl)methyl)-3*H*-imidazo[4,5-*b*]pyridine-5-carboxylate (75 mg, 0.11 mmol) and lithium hydroxide (14 mg, 0.58 mmol) were added to the mixture of 1,4-dioxane (2 mL) and water (0.5 mL), and the reaction was carried out at 40 °C for 2 hours. The pH was adjusted to 5 with acetic acid dropwise, concentrated under reduced pressure and the residue was purified by Prep-HPLC eluted with acetonitrile:water (v/v)=0%~60% to give a white solid (20.5 mg 27.9%).

LC-MS (ESI): $[M+H]^+$=645.2;

$^1$HNMR (400 MHz, DMSO-$d_6$) δ 12.99 (s, 1H), 8.60 - 8.55 (m, 1H), 8.09 (d, *J* = 8.0 Hz, 1H), 7.97 (d, *J* = 8.0 Hz, 1H), 7.51 - 7.41 (m, 2H), 7.12 (s, 1H), 6.55 (d, *J* = 8.0 Hz, 1H), 5.77 (s, 2H), 4.40 (s, 2H), 3.08 - 2.97 (m, 2H), 2.76 - 2.63 (m, 3H), 2.60 - 2.53 (m, 1H), 2.46 - 2.30 (m, 2H), 2.20 - 1.95 (m, 2H), 1.59 - 1.44 (m, 1H), 1.03 - 0.94 (m, 2H), 0.67 - 0.60 (m, 2H).

**Example 11 Synthesis of 2-((4-(4-((4-chlorobenzofuran-7-yl)methoxy)-5-fluoropyrimidin-2-yl)cyclohex-3-en-1-yl)methyl)-1-(((*S*)-oxetan-2-yl)methyl)-1*H*-thieno[2,3-*d*]imidazole-5-carboxylic acid (compound 11)**

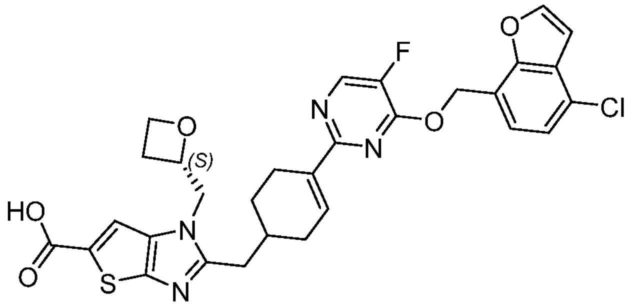

**Step 1: Synthesis of methyl 2-((4-(5-fluoro-4-(methoxymethoxy)pyrimidin-2-yl)cyclohex-3-en-1-yl) methyl)-1-(((*S*)-oxetan-2-yl)methyl)-1*H*-thieno[2,3-*d*]imidazole-5-carboxylate**

2-(4-(5-fluoro-4-(methoxymethoxy)pyrimidin-2-yl)cyclohex-3-en-1-yl)acetaldehyde (550 mg, 1.96 mmol) (synthesis method refer to step 8 of example 5) and methyl (*S*)-5-amino-4-((oxetan-2-ylmethyl)amino)thiophene-2-carboxylate (571 mg, 2.36 mmol) (synthesis method refer to step 3 of example 8) were added to acetic acid (20 mL) and stirred at 60°C for 2 hours. Concentrated under reduced pressure and the residue was purified by column chromatography eluted with DCM:MeOH (v/v)=20:1 to give a light brown solid (300 mg, 30.4%).

LC-MS (ESI): $[M+H]^+$=503.4.

**Step 2: Synthesis of methyl 2-((4-(5-fluoro-4-hydroxypyrimidin-2-yl)cyclohex-3-en-1-yl)methyl)-1-(((*S*)-oxetan-2-yl)methyl)-1*H*-thieno[2,3-*d*]imidazole-5-carboxylate**

methyl 2-((4-(5 -fluoro-4-(methoxymethoxy)pyrimidin-2-yl)cyclohex-3-en-1-yl)methyl)-1-(((*S*)-oxetan-2-yl) methyl)-1*H*-thieno[2,3-*d*]imidazole-5-carboxylate (300 mg, 0.6 mmol) was added to anhydrous dichloromethane (16 mL), cooled to -10 °C, and trifluoroacetic acid (8 mL) was added dropwise, keep the reaction at -10°C overnight. The reaction mixture was concentrated under reduced pressure and the residue was purified by column chromatography eluted with DCM:MeOH (v/v)=10:1 to give a white solid (200 mg, 73.3%).

LC-MS (ESI): $[M+H]^+$=459.4.

**Step 3: Synthesis of methyl 2-((4-(4-((4-chlorobenzofuran-7-yl)methoxy)-5-fluoropyrimidin-2-yl)cyclohex-3-en-1-yl)methyl)-1-(((*S*)-oxetan-2-yl)methyl)-1*H*-thieno[2,3-*d*]imidazole-5-carboxylate**

Methyl 2-((4-(5-fluoro-4-hydroxypyrimidin-2-yl)cyclohex-3-en-1-yl)methyl)-1-(((*S*)-oxetan-2-yl)methyl)-1*H*-thieno[2,3-*d*]imidazole-5-carboxylate (100 mg, 0.22 mmol), 7-(bromomethyl)-4-chlorobenzofuran (80 mg, 0.33 mmol) were added to *N*,*N*-dimethylformamide (5 mL), then potassium carbonate (91 mg, 0.66 mmol) was added and stir at 60°C for 1 hour. It was diluted by adding water (5 mL), extracted with ethyl acetate (15 mL×3), the combined organic phases

were washed with saturated brine, dried with anhydrous sodium sulfate, and the residue was purified by column chromatography eluted with DCM:MeOH (v/v)=10:1 to give a white solid (103 mg, 75.7%).

LC-MS (ESI): $[M+H]^+$=623.4.

**Step 4: Synthesis of 2-((4-(4-((4-chlorobenzofuran-7-yl)methoxy)-5-fluoropyrimidin-2-yl)cyclohex-3-en-1-yl) methyl)-1-(((*S*)-oxetan-2-yl)methyl)-1*H*-thieno[2,3-*d*]imidazole-5-carboxylic acid**

Methyl 2-((4-(4-((4-chlorobenzofuran-7-yl)methoxy)-5-fluoropyrimidin-2-yl)cyclohex-3-en-1-yl) methyl)-1-(((*S*)-oxetan-2-yl)methyl)-1*H*-thieno[2,3-*d*]imidazole-5-carboxylate (103 mg, 0.17 mmol) was dissolved in the mixture of dioxane (12 mL) and water (3 mL). Lithium hydroxide (16 mg, 0.68 mmol) was added and stirred for 2 h at room temperature. The pH was adjusted to 6-7 with acetic acid, extracted with ethyl acetate (15 mL × 3), and the combined organic phases were washed with saturated brine and dried with anhydrous sodium sulfate. It was concentrated under reduced pressure and the residue was purified by prep-HPLC eluted with acetonitrile:water (v/v)=60%) to give a white solid (40.7 mg, 40.5%)

LC-MS (ESI): $[M+H]^+$=609.6;
$^1$H NMR (400 MHz, DMSO-$d_6$) δ 12.85 (s, 1H), 8.56 (d, *J* = 2.8 Hz, 1H), 8.20 (d, *J* = 2.1 Hz, 1H), 7.83 (s, 1H), 7.48 (d, *J* = 8.0 Hz, 1H), 7.39 (d, *J* = 8.0 Hz, 1H), 7.15 - 7.04 (m, 2H), 5.82 (s, 2H), 5.01 (d, *J* = 6.9 Hz, 1H), 4.52 - 4.44 (m, 3H), 4.36 - 4.30 (m, 1H), 2.95 - 2.81 (m, 2H), 2.73 - 2.66 (m, 2H), 2.47 - 2.27 (m, 3H), 2.29 - 2.18 (m, 1H), 2.12 - 2.06 (m, 1H), 2.03 - 1.91 (m, 1H), 1.47 - 1.39 (m, 1H).

**Example 12 Synthesis of 2-((4-(4-((4-chloro-2-fluorobenzofuran-7-yl)methoxy)-5-fluoropyrimidin-2-yl)cyclohex-3-en-1-yl)methyl)-1-(((*S*)-oxetan-2-yl)methyl)-1*H*-thieno[2,3-*d*]imidazole-5-carboxylic acid (compound 12)**

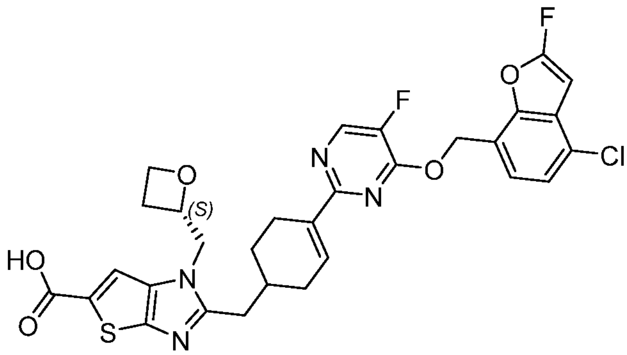

**Step 1: Synthesis of methyl 2-((4-(4-((4-chloro-2-fluorobenzofuran-7-yl)methoxy)-5-fluoropyrimidin-2-yl)cyclohex-3-en-1-yl)methyl)-1-(((*S*)-oxetan-2-yl)methyl)-1H-thieno[2,3-*d*]imidazole-5-carboxylate**

Methyl 2-((4-(5 -fluoro-4-hydroxypyrimidin-2-yl)cyclohex-3-en-1 -yl)methyl)-1 -(((*S*)-oxetan-2-yl)methyl)-1*H*-thieno[2,3-*d*]imidazole-5-carboxylate (100 mg, 0.22 mmol) (synthesis method refer to step 2 of example 11), 7-(bromomethyl)-4-chloro-2-fluoro benzofuran (86 mg, 0.33 mmol) was added to *N*,*N*-dimethylformamide (5 mL), potassium carbonate (91 mg, 0.66 mmol) was added, and stirred at 60 °C for 1 hour. It was diluted with water (5 mL), extracted with ethyl acetate (15 mL×3), the combined organic phases were washed with saturated brine, dried with anhydrous sodium sulfate, and the residue was purified by column chromatography eluted with DCM:MeOH (v/v)=10:1 to give a white solid (111 mg, 67.5%).

LC-MS (ESI): $[M+H]^+$=641.4.

**Step 2: Synthesis of 2-((4-(4-((4-chloro-2-fluorobenzofuran-7-yl)methoxy)-5-fluoropyrimidin-2-yl)cyclohex-3-en-1-yl)methyl)-1-(((*S*)-oxetan-2-yl)methyl)-1*H*-thieno[2,3-*d*]imidazole-5-carboxylic acid**

Methyl 2-((4-(4-((4-chloro-2-fluorobenzofuran-7-yl)methoxy)-5-fluoropyrimidin-2-yl)cyclohex-3-en-1-yl) methyl)-1-(((*S*)-oxetan-2-yl)methyl)-1*H*-thieno[2,3-*d*]imidazole-5-carboxylate (111 mg, 0.17 mmol) was dissolved in dioxane (12 mL ) and water (3 mL). Lithium hydroxide (16 mg, 0.68 mmol) was added and stirred for 2 h at room temperature. The pH was adjusted to 6-7 with acetic acid, extracted with ethyl acetate (15 mL × 3), and the combined

organic phases were washed with saturated brine and dried with anhydrous sodium sulfate. It was concentrated under reduced pressure and the residue was purified by prep-HPLC eluted with acetonitrile: water (v/v)=50% to give a white solid (21.7 mg, 23.5%).

LC-MS (ESI): $[M+H]^+$=627.5;
$^1$HNMR (400 MHz, DMSO-$d_6$) δ 12.85 (s, 1H), 8.57 (s, 1H), 7.82 (s, 1H), 7.46 (dd, *J* = 16.9, 8.1 Hz, 2H), 7.11 (s, 1H), 6.55 (d, *J* = 6.3 Hz, 1H), 5.76 (s, 2H), 5.01 (d, *J* = 4.7 Hz, 1H), 4.63 - 4.26 (m, 4H), 2.89 (s, 2H), 2.75 - 2.66 (m, 2H), 2.40 - 2.36 (m, 3H), 2.24 - 1.90 (m, 3H), 1.49 - 1.38 (m, 1H).

**Example 13 Synthesis of 2-((4-(4-((4-chloro-2-fluorobenzofuran-7-yl)methoxy)-5-fluoropyrimidin-2-yl)cyclohex-3-en-1-yl)methyl)-3-(2-methoxyethyl)-3*H*-imidazo[4,5-*b*]pyridine-5-carboxylic acid (compound 13)**

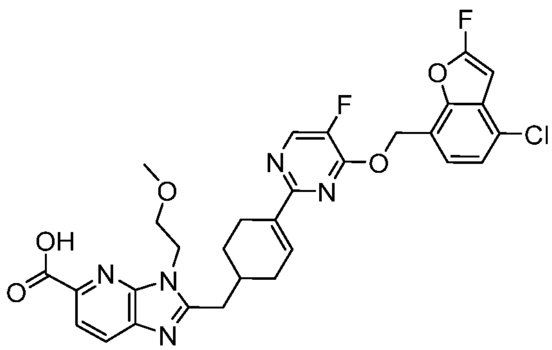

**Step 1: Synthesis of 6-chloro-5-nitropicolinic acid**

2-Chloro-6-methyl-3-nitropyridine (20 g, 115.89 mmol) was dissolved in concentrated sulfuric acid (100 mL), cooled down to 0 °C under ice bath condition, slowly added chromium trioxide (45 g, 173.84 mmol), and heated to room temperature for 12 h. The reaction mixture was slowly added to ice water (500 mL) and stirred for half an hour. Filtrated, the filter cake was washed with ice water (50 mL × 2), the filter cake was collected, concentrated and dried under reduced pressure to give an off-white solid (12.5 g, 53.2%).

**Step 2: Synthesis of methyl 6-chloro-5-nitropicolinate**

6-chloro-5-nitropicolinic acid (6 g, 23.81 mmol) was added to dichlorosulfoxide (30 mL), DMF (0.2 mL, 2.05 mmol) was added, and the reaction was heated to 60 °C for 2 hours. The reaction mixture was reduced to room temperature, concentrated under reduced pressure, methanol (50 mL) was added under an ice bath and a large amount of white solid precipitated. Filtrated to give a white solid (2.25 g, 35%).
$^1$H NMR (400 MHz, $CDCl_3$) δ 8.32 (d, *J* = 8.2 Hz, 1H), 8.24 (d, *J* = 8.2 Hz, 1H), 4.06 (s, 3H).

**Step 3: Synthesis of methyl 6-((2-methoxyethyl)amino)-5-nitropicolinate**

Methyl 6-chloro-5-nitropyridine-2-carboxylate (500 mg, 2.31 mmol) and 2-methoxyethan-1-atnine (0.2 mL, 2.31 mmol) were dissolved in acetonitrile (10 mL), potassium carbonate (957.73 mg, 6.93 mmol) was added, and stirred at room temperature under the protection of $N_2$ overnight. It was concentrated under reduced pressure and the residue was purified by column chromatography eluted with MeOH:DCM (v/v)=1:20 to give a yellow solid (583 mg, 98.8%).
LC-MS (ESI): $[M+H]^+$=256.2.

**Step 4: Synthesis of methyl 5-amino-6-((2-methoxyethyl)amino)picolinate**

Methyl 6-((2-methoxyethyl)amino)-5-nitropyridine-2-carboxylate (583 mg, 2.28 mmol) and 10% Pd/C (58 mg, 0.55 mmol) were added to ethyl acetate (10 mL), and the reaction was carried out for 16 h at room temperature under the protection of hydrogen. The palladium carbon was removed by filtration with diatomaceous earth, the filter cake was washed with ethyl acetate (10 mL), the filtrates were combined and concentrated under reduced pressure to give a colorless oil (452 mg, 87.8%).
$^1$H NMR (400 MHz, DMSO-$d_6$) δ 7.23 (d, *J* = 7.8 Hz, 1H), 6.67 (d, *J* = 7.8 Hz, 1H), 5.97 (t, *J* = 5.4 Hz, 1H), 5.64 (s,

2H), 4.56 - 4.51 (m, 2H), 3.72 (s, 3H), 3.32 (s, 3H), 2.47 - 2.34 (m, 2H).

**Step 5: Synthesis of methyl 2-((4-(5-fluoro-4-(methoxymethoxy)pyrimidin-2-yl)cyclohex-3-en-1-yl)methyl)-3-(2-methoxyethyl)-3*H*-imidazo[4,5-*b*]pyridine-5-carboxylate**

2-(4-(5-fluoro-4-(methoxymethoxy)pyrimidin-2-yl)cyclohex-3-en-1-yl)acetaldehyde (260.5 mg, 0.93 mmol) (synthesis method refer to step 8 of example 5) and methyl 5-amino-6-((2-methoxyethyl)amino)picolinate (210 mg, 0.93 mmol) were added to acetic acid (5 mL). The reaction was heated up to 60 °C for 1 h under nitrogen protection. The reaction mixture was cooled down to room temperature, concentrated under reduced pressure and the residue was purified by column chromatography eluted with MeOH:DCM (v/v)=1:20 to give a brown oil (286.6 mg, 63.5%).

$^1$H NMR (400 MHz, DMSO-$d_6$) δ 8.10 (dd, *J* = 10.2, 5.2 Hz, 2H), 8.00 (d, *J* = 8.2 Hz, 1H), 6.08 (s, 1H), 5.28 (d, *J* = 10.7 Hz, 2H), 4.49 (t, *J* = 5.2 Hz, 2H), 3.91 (s, 3H), 3.73 (t, *J* = 5.2 Hz, 3H), 3.58 - 3.48 (m, 1H), 3.33 - 3.24 (m, 2H), 3.21 (s, 3H), 3.02 (d, *J* = 6.4 Hz, 2H), 2.40 (d, *J* = 30.0 Hz, 5H), 2.07 - 1.85 (m, 2H), 1.51 (s, 1H).

**Step 6: Synthesis of methyl 2-((4-(5-fluoro-4-hydroxypyrimidin-2-yl)cyclohex-3-en-1-yl)methyl)-3-(2-methoxyethyl)-3*H*-imidazo[4,5-*b*]pyridine-5-carboxylate**

methyl 2-((4-(5-fluoro-4-(methoxymethoxy)pyrimidin-2-yl)cyclohex-3-en-1-yl)methyl)-3-(2-methoxyethyl)-3*H*-imidazo[4,5-*b*]pyridine-5-carboxylate (286 mg, 0.59 mmol) was added to dichloromethane (29 mL) and cooled down to -10 °C under nitrogen protection, then trifluoroacetic acid ( 5.5 mL, 74.04 mmol) was slowly added dropwise and the reaction was kept at -10 °C overnight. The reaction was concentrated under reduced pressure and the residue was purified by column chromatography eluted with MeOH:DCM (v/v)=1:10 to give a yellow oil (160 mg, 61.5%).

LC-MS (ESI): $[M+H]^+$=442.7.

**Step 7: Synthesis of methyl 2-((4-(4-((4-chloro-2-fluorobenzofuran-7-yl)methoxy)-5-fluoropyrimidin-2-yl)cyclohex-3-en-1-yl)methyl)-3-(2-methoxyethyl)-3*H*-imidazo[4,5-*b*]pyridine-5-carboxylate**

Methyl 2-((4-(5-fluoro-4-hydroxypyrimidin-2-yl)cyclohex-3-en-1-yl)methyl)-3-(2-methoxyethyl)-3*H*-imidazo [4,5-*b*]pyridine-5-carboxylate (160 mg, 0.36 mmol), 7-(bromomethyl)-4-chloro-2-fluoro-1-benzofuran (143 mg, 0.54 mmol) were added to DMF (12 mL). Potassium carbonate (250 mg, 1.81 mmol) was added and the reaction was warmed to 60 °C for 1 h under nitrogen protection. The reaction mixture was cooled down to room temperature, concentrated under reduced pressure and the residue was purified by column chromatography eluted with MeOH:DCM (v/v)=1:20 to give a light yellow solid (113 mg, 50.0%).

LC-MS (ESI): $[M+H]^+$=624.6.

**Step 8: Synthesis of 2-((4-(4-((4-chloro-2-fluorobenzofuran-7-yl)methoxy)-5-fluoropyrimidin-2-yl)cyclohex-3-en-1-yl)methyl)-3-(2-methoxyethyl)-3*H*-imidazo[4,5-*b*]pyridine-5-carboxylic acid**

Methyl 2-((4-(4-((4-chloro-2-fluorobenzofuran-7-yl)methoxy)-5-fluoropyrimidin-2-yl)cyclohex-3-en-1-yl) methyl)-3-(2-methoxyethyl)-3*H*-imidazo[4,5-*b*]pyridine-5-carboxylate (113 mg, 0.18 mmol) was added to dioxane (1.6 mL), lithium hydroxide ( 22 mg, 0.52 mmol) dissolved in water (0.4 mL) was added to the reaction mixture. The reaction mixture was heated to 40 °C for two hours under nitrogen protection. The pH of the reaction mixxture was adjusted to 4 with acetic acid. The reaction mixture was concentrated under reduced pressure and the residue was purified by Prep-HPLC eluted with acetonitrile:water (v/v)=50% to give a white solid (64.8 mg, 56.8%).

LC-MS (ESI): $[M+H]^+$=610.6;
$^1$H NMR (400 MHz, DMSO-$d_6$) δ 12.96 (s, 1H), 8.57 (d, *J* = 4.0 Hz, 1H), 8.10 (d, *J* = 8.0 Hz, 1H), 7.98 (d, *J* = 8.0 Hz, 1H), 7.49 - 7.43 (m, 2H), 7.12 (s, 1H), 6.55 (d, *J* = 4.0 Hz, 1H), 5.77 (s, 2H), 4.51 - 4.49 (m, 2H), 3.74 - 3.71 (m, 2H), 3.20 (s, 3H), 3.03 - 3.01 (m, 2H), 2.71 - 2.66 (m, 1H), 2.53 - 2.50 (m, 1H), 2.37 - 2.32 (m, 2H), 2.14 - 2.08 (m, 1H), 1.99 - 1.95 (m, 1H), 1.52 - 1.47 (m, 1H).

**Example 14 Synthesis of 2-((4-(2-((4-chloro-2-fluorobenzofuran-7-yl)methoxy)thiazol-4-yl)cyclohex-3-en-1-yl) methyl)-1-(((*S*)-oxetan-2-yl)methyl)-1*H*-benzo[*d*]imidazole-6-carboxylic acid (compound 14)**

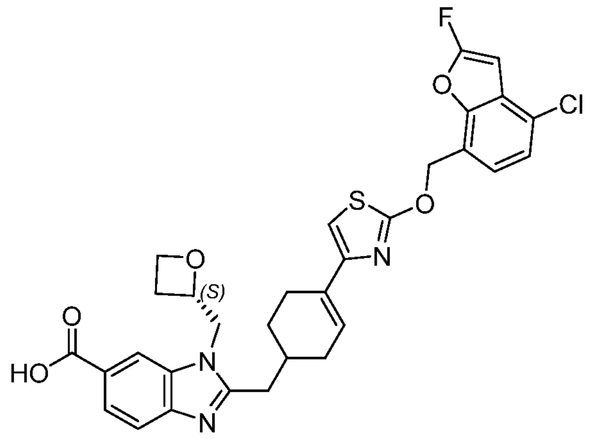

**Step 1: Synthesis of methyl 2-(4-oxocyclohexylidene)acetate**

Cyclohexane-1,4-dione (20 g, 178.36 mmol), methyl 2-(triphenyl-$\lambda^5$-phosphaneylidene)acetate (29.8 g, 89.22 mmol) were added to toluene (300 mL) and the reaction was carried out at 110 °C overnight. It was concentrated under reduced pressure and the residue was purified by column chromatography eluted with EA:PE (v/v)=25% to give a white solid (12 g, 40.0%).

$^1$HNMR (400 MHz, $CDCl_3$) $\delta$ 5.86 (s, 1H), 3.72 (s, 3H), 3.21 (t, *J* = 7.2 Hz, 2H), 2.67 (t, *J* = 6.8 Hz, 2H), 2.52 - 2.47 (m, 4H).

**Step 2: Synthesis of methyl 2-(4,4-dimethoxycyclohexyl)acetate**

Methyl 2-(4-oxocyclohexylidene)acetate (12.0 g, 71.43 mmol) was added to methanol (700 ml), replaced with nitrogen for three times, palladium carbon (10% palladium, containing 40-60% water) (1.2 g) was added, replaced with hydrogen for three times, and the reaction was carried out at room temperature overnight. The reaction mixture was filtered with diatomaceous earth, the filtrate was taken and concentrated under reduced pressure to give a colorless oil (13.57 g, 88.5%).

$^1$HNMR (400 MHz, $CDCl_3$) $\delta$ 3.67 (s, 3H), 3.19 (s, 3H), 3.14 (s, 3H), 2.23 (d, *J* = 7.2 Hz, 2H), 2.01 - 1.96 (m, 2H), 1.90 - 1.78 (m, 1H), 1.70 - 1.59 (m, 2H), 1.41 - 1.30 (m, 2H), 1.22 - 1.16 (m, 2H).

**Step 3: Synthesis of methyl 2-(4-oxocyclohexyl)acetate**

Methyl 2-(4,4-dimethoxycyclohexyl)acetate (10.5 g, 48.55 mmol), 2,3-dichloro-5,6-dicyano-1,4-benzoquinone (1.1 g, 4.85 mmol) were added to the mixture of acetonitrile (218 mL) and water (25 mL), stirred at room temperature overnight. Concentrated under reduced pressure, add water (50 mL) was added, extracted with ethyl acetate (100 mL$\times$3), the combined organic phases were dried with anhydrous sodium sulfate, concentrated under reduced pressure, and the residue was purified by column chromatography eluent with PE:MTBE (v/v)=10:3 to give a colorless oil (7.5 g, 90.8%).

$^1$HNMR (400 MHz, DMSO-$d_6$) $\delta$ 3.70 (s, 3H), 2.41 - 2.21 (m, 7H), 2.18 - 2.02 (m, 2H), 1.48 - 1.40 (m, 2H).

**Step 4: Synthesis of methyl 2-(4-(((trifluoromethyl)sulfonyl)oxy)cyclohex-3-en-1-yl)acetate**

Methyl 2-(4-oxocyclohexyl)acetate (6.4 g, 37.60 mmol) was added to tetrahydrofuran (120 mL), lithium bis(trimethylsilyl)amide (1 mol / L, 45 mL) was added dropwise at -40 °C with stirring for 30 min, and 1,1,1-trifluoro-N-phenyl-*N*-((trifluoromethyl)sulfonyl)methane sulfonamide (14.77 g, 41.36 mmol) was added dropwise and stirred overnight. Quenched with saturated ammonium chloride solution (50 mL) dropwise under an ice bath, extracted with ethyl acetate (50 mL$\times$3), the combined organic phases were dried with anhydrous sodium sulfate, concentrated under reduced pressure, and the residue was purified by column chromatography eluted with PE:MTBE (v/v)=5:1 to give a light yellow solid (8.5 g ,74.8%).

$^1$HNMR (400 MHz, DMSO-$d_6$) $\delta$ 5.86 - 5.85 (m, 1H), 3.60 (s, 3H), 2.43 - 2.21 (m, 5H), 2.02 - 1.79 (m, 3H), 1.45 - 1.40 (m, 1H)。

**Step 5: Synthesis of methyl 2-(4-(4,4,5,5-tetramethyl-1,3,2-dioxaborolan-2-yl)cyclohex-3-en-1-yl)acetate**

Methyl 2-(4-(((trifluoromethyl)sulfonyl)oxy)cyclohex-3-en-1-yl)acetate (8.5 g, 28.12 mmol), pinacol ester of

bis(boronic acid) (7.86 g, 30.93 mmol), palladium tetrakis(triphenylphosphine) (3.25 g, 2.81 mmol), and potassium acetate (5.52 g, 56.24 mmol) were added to dioxane ( 150 mL) and stirred at 90 °C for 30 min. The reaction mixture was filtered, concentrated under reduced pressure and the residue was purified by column chromatography eluted with PE:MTBE (v/v) =10:1 to give a colorless oil (5.6 g, 49.8%).

LC-MS (ESI): $[M+H]^+$=281.2;
$^1$HNMR (400 MHz, DMSO-$d_6$) $\delta$ 6.39 (s, 1H), 3.58 (s, 3H), 2.45 - 1.55 (m, 9H), 1.18 (s, 12H).

**Step 6: Synthesis of 4-bromo-2-((4-methoxybenzyl)oxy)thiazole**

2,4-Dibromothiazole (5.0 g, 20.58 mmol) was added to tetrahydrofuran (50 mL) and sodium hydride (1.23 g, 30.87 mmol) was added slowly at room temperature and stirred for 30 minutes. 4-Methoxybenzyl alcohol (2.84 g, 20.58 mmol) was added and stirred at room temperature for 2 hours. Concentrated under reduced pressure and the residue was purified by column chromatography eluted with PE:EA (v/v)=10:1 to give a white solid (4.5 g, 72.8%).

LC-MS (ESI): $[M+H]^+$=300.2;
$^1$HNMR (400 MHz, $CDCl_3$) $\delta$ 7.37 (d, *J* = 8.4 Hz, 2H), 6.91 (d, *J* = 8.8 Hz, 2H), 6.58 (s, 1H), 5.38 (s, 2H), 3.82 (s, 3H).

**Step 7: Synthesis of methyl 2-(4-(2-((4-methoxybenzyl)oxy)thiazol-4-yl)cyclohex-3-en-1-yl)acetate**

Methyl 4-bromo-2-((4-methoxybenzyl)oxy)thiazole (4.5 g, 14.99 mmol), 2-(4,4,5,5-tetramethyl-1,3,2-dioxobenzoic acid-2-yl)cyclohex-3-en-1-yl)acetate (6.6 g, 22.43 mmol), bis(tri-tert-butylphosphine)palladium (0.76 g, 1.50 mmol) and potassium carbonate (4.2 g, 30.39 mmol) were added to the mixture of 1,4-dioxane (60 mL) and water (15 mL) and stirred overnight at 95 °C under nitrogen protection. Cooled to room temperature, water (100 mL) was added, extracted with ethyl acetate (100 mL×3), the combined organic phases were dried with anhydrous sodium sulfate, concentrated under reduced pressure, and the residue was purified by column chromatography eluted with PE:EA (v/v)=90% to give a light yellow solid (2.5 g, 43.0%).
LC-MS (ESI): $[M+H]^+$=374.2.

**Step 8: Synthesis of 2-(4-(2-((4-methoxybenzyl)oxy)thiazol-4-yl)cyclohex-3-en-1-yl)ethan-1-ol**

Methyl 2-(4-(2-((4-methoxybenzyl)oxy)thiazol-4-yl)cyclohex-3-en-1-yl)acetate (2.5 g, 6.45 mmol), was added to tetrahydrofuran (70 mL), and lithium aluminum hydroxide powder (0.61 g, 16.13 mmol) was slowly added under an ice bath, and the reaction was carried out for 3 h under ice bath. Water (10 mL) was added slowly dropwise under ice bath, filtered with diatomaceous earth, extracted with ethyl acetate (20 mL×3), the combined organic phases were dried with anhydrous sodium sulfate, concentrated under reduced pressure, and purified the residue was by column chromatography eluted with PE:EA (v/v)=3:2 to give a yellow solid (2.0 g, 89.7%).
LC-MS (ESI): $[M+H]^+$=346.2.

**Step 9: Synthesis of 2-(4-(2-((4-methoxybenzyl)oxy)thiazol-4-yl)cyclohex-3-en-1-yl)acetaldehyde**

2-(4-(2-((4-methoxybenzyl)oxy)thiazol-4-yl)cyclohex-3-en-1-yl)ethan-1-ol (2.0 g, 5.80 mmol) was added to dimethylsulfoxide (60 mL), and 2-iodoacylbenzoic acid (3.25 g, 11.59 mmol) was added in an ice bath, and the mixture was stirred for 2 hours at room temperature. Diluted with water (180 ml), extracted with ethyl acetate (100 mL×3), the combined organic phases were dried with anhydrous sodium sulfate, concentrated under reduced pressure, and the residue was purified by column chromatography eluted with PE:EA (v/v)=90% to give a light yellow solid (942 mg, 47.4%).
LC-MS (ESI): $[M+H]^+$ =344.2.

**Step 10: Synthesis of methyl 2-((4-(2-hydroxythiazol-4-yl)cyclohex-3-en-1-yl)methyl)-1-(((S)-oxetan-2-yl) methyl)-1*H*-benzo[*d*]imidazole-6-carboxylate**

2-(4-(2-((4-methoxybenzyl)oxy)thiazol-4-yl)cyclohex-3-en-1-yl)acetaldehyde (942 mg, 2.75 mmol), methyl (S)-4-amino-3-((oxetan-2-ylmethyl)amino)benzoate (652 mg, 2.75 mmol) were added to acetic acid (8 mL) and reacted for 2 h at 60 °C. The solvent was removed under reduced pressure and the residue was purified by column chromatography eluted with DCM:MeOH (v/v)=20:1 to give a light yellow solid (417 mg, 34.5%).
LC-MS (ESI): $[M+H]^+$=440.1.

**Step 11: Synthesis of methyl 2-((4-(2-((4-chloro-2-fluorobenzofuran-7-yl)methoxy)thiazol-4-yl)cyclohex-3-en-1-yl)methyl)-1-(((*S*)-oxetan-2-yl)methyl)-1*H*-benzo[*d*]imidazole-6-carboxylate**

Methyl 2-((4-(2-hydroxythiazol-4-yl)cyclohex-3-en-1-yl)methyl)-1-(((S)-oxetan-2-yl)methyl)-1*H*-benzo[*d*]imidazole-6-carboxylate (417 mg, 0.95 mmol), 7-(bromomethyl)-4-chloro-2-fluorobenzofuran (250 mg, 0.95 mmol), potassium carbonate (262 mg, 1.9 mmol) were added to *N*,*N*-dimethylformamide (5 mL) and the reaction was carried out at 60 °C for 2 hours. Concentrated under reduced pressure, water (10 mL) was added, extracted with ethyl acetate (15 mL×2), the combined organic phases were dried with anhydrous sodium sulfate, concentrated under reduced pressure, and the residue was purified by column chromatography eluted with EtOAc:PE (v/v)=1:1 to give a light yellow solid (120 mg, 20.3%).

LC-MS (ESI): $[M+1]^+$=622.1.

**Step 12: Synthesis of 2-((4-(2-((4-chloro-2-fluorobenzofuran-7-yl)methoxy)thiazol-4-yl)cyclohex-3-en-1-yl) methyl)-1-(((*S*)-oxetan-2-yl)methyl)-1*H*-benzo[*d*]imidazole-6-carboxylic acid**

Methyl 2-((4-(2-((4-chloro-2-fluorobenzofuran-7-yl)methoxy)thiazol-4-yl)cyclohex-3-en-1-yl) methyl)-1-(((*S*)-oxetan-2-yl)methyl)-1*H*-benzo[*d*]imidazole-6-carboxylate (120 mg, 0.19 mmol) and lithium hydroxide (22.8 mg, 0.95 mmol) were were added to the mixture of 1,4-dioxane (8 mL) and water (2 mL) and reacted at 40 °C for 1 hour. It was cooled in an ice bath, the pH was adjusted to 6 with acetic acid, concentrated under reduced pressure and the residue was purified by pre-HPLC eluted with acetonitrile:water (v/v)=80% to give a white solid (25 mg, 26.1%).

LC-MS (ESI): $[M+H]^+$=608.1;
$^1$HNMR (400 MHz, DMSO-$d_6$) δ 12.70 (s, 1H), 8.23 (s, 1H), 7.81 (d, *J* = 8.4 Hz, 1H), 7.64 (d, *J* = 8.8 Hz, 1H), 7.47 - 7.43 (m, 2H), 6.80 (s, 1H), 6.56 - 6.51 (m, 2H), 5.66 (s, 2H), 5.03 - 5.01 (m, 1H), 4.69 - 4.63 (m, 1H), 4.55 - 4.45 (m, 2H), 4.32 - 4.30 (m, 1H), 2.99 - 2.98 (m, 2H), 2.73 - 2.67 (m, 1H), 2.50 - 2.25 (m, 5H), 2.05 - 1.92 (m, 2H), 1.48 - 1.45 (m, 1H).

**Example 15 Synthesis of 2-((4-(6-((4-chloro-2-fluorobenzofuran-7-yl)methoxy)pyridin-2-yl)cyclohex-3-en-1-yl) methyl)-3-((1-(cyanomethyl)cyclopropyl)methyl)-3*H*-imidazo[4,5-*b*]pyridine-5-carboxylic acid (compound 15)**

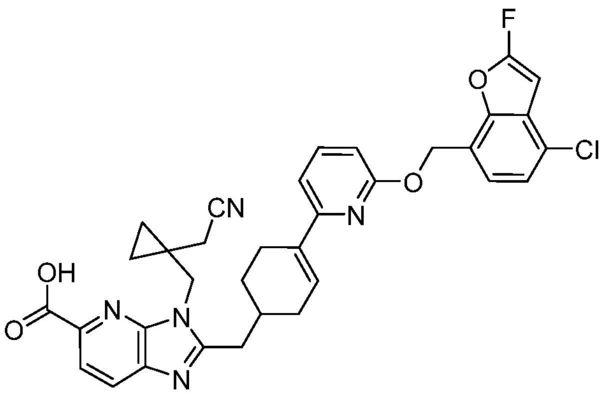

**Step 1: Synthesis of methyl 3-((1-(cyanomethyl)cyclopropyl)methyl)-2-((4-(6-hydroxypyridin-2-yl)cyclohex-3-en-1-yl)methyl)-3*H*-imidazo[4,5-*b*]pyridine-5-carboxylate**

2-(4-(6-(methoxymethoxy)pyridin-2-yl)cyclohex-3-en-1-yl)acetaldehyde (170 mg, 0.65 mmol), methyl 5-amino-6-(((1-(cyanomethyl)cyclopropyl)methyl)amino)picolinate (169 mg, 0.65 mmol) (synthesis method refer to step 8 of example 10) was added to acetic acid (5 mL), and the reaction was carried at 60 °C for 2 hours. Concentrated under reduced pressure and the residue was purified by column chromatography eluted with DCM:MeOH (v/v)=20:1 to give a light yellow solid (175 mg, 58.8%).

LC-MS (ESI): $[M+H]^+$=458.5;
$^1$HNMR (400 MHz, DMSO-$d_6$) δ 11.21 (s, 1H), 8.13 (d, *J* = 8.0 Hz, 1H), 8.00 (d, *J* = 8.0 Hz, 1H), 7.42 - 7.36 (m, 1H), 6.47 (s, 1H), 6.25 - 6.17 (m, 2H), 4.38 (s, 2H), 3.92 (s, 3H), 3.06 - 2.99 (m, 2H), 2.67 (m, 2H), 2.49 - 2.27 (m, 4H), 2.11 -1.92 (m, 2H), 1.57 - 1.45 (m, 1H), 1.04 - 0.96 (m, 2H), 0.70 - 0.61 (m, 2H).

**Step 2: Synthesis of methyl 2-((4-(6-((4-chloro-2-fluorobenzofuran-7-yl)methoxy)pyridin-2-yl)cyclohex-3-en-1-yl)methyl)-3-((1-(cyanomethyl)cyclopropyl)methyl)-3*H*-imidazo[4,5-*b*]pyridine-5-carboxylate**

Methyl 3-((1-(cyanomethyl)cyclopropyl)methyl)-2-((4-(6-hydroxypyridin-2-yl)cyclohex-3-en-1-yl)methyl)-3*H*-imidazo[4,5-*b*]pyridine-5-carboxylate (175 mg, 0.39 mmol), 7-(bromomethyl)-4-chloro-2-fluorobenzofuran (208 mg, 0.79 mmol), potassium carbonate ( 163 mg, 1.2 mmol) were added to *N,N*-dimethylformamide (5 mL) and the reaction was carried out at 60 °C for 3 hours. Concentrated under reduced pressure, water (10 mL) was add, extracted with ethyl acetate (15 mL×2), the combined organic phases were dried with anhydrous sodium sulfate. Concentrated under reduced pressure and the residue was purified by column chromatography eluted with EtOAc:PE (v/v)=1:1 to give a light yellow solid (100 mg, 35.6%).
LC-MS (ESI): $[M+H]^+$=640.5.

**Step 3: Synthesis of 2-((4-(6-((4-chloro-2-fluorobenzofuran-7-yl)methoxy)pyridin-2-yl)cyclohex-3-en-1-yl)methyl)-3-((1-(cyanomethyl)cyclopropyl)methyl)-3*H*-imidazo[4,5-*b*]pyridine-5-carboxylic acid**

Methyl 2-((4-(6-((4-chloro-2-fluorobenzofuran-7-yl)methoxy)pyridin-2-yl)cyclohex-3-en-1-yl) methyl)-3-((1-(cyanomethyl)cyclopropyl)methyl)-3*H*-imidazo[4,5-*b*]pyridine-5-carboxylate (100 mg, 0.16 mmol) and lithium hydroxide (18 mg, 0.75 mmol ) were added to the mixture of 1,4-dioxane (2 mL) and water (0.5 mL), and the reaction was carried out at 40 °C for 1 hour. It was cooled in an ice bath and the pH was adjusted to 6-7 with acetic acid. concentrated under reduced pressure and the residue was purified by Pre-HPLC eluted with acetonitrile:water (v/v)=80% to give a white solid (18.3 mg, 18.7%).

LC-MS (ESI): $[M+H]^+$=626.2;
$^1$HNMR (400 MHz, DMSO-$d_6$) δ 12.91 (s, 1H), 8.15 - 8.08 (m, 1H), 7.99 (d, *J* = 4.0 Hz, 1H), 7.67 (t, *J* = 8.0 Hz, 1H), 7.45 - 7.36 (m, 2H), 7.08 (d, *J* = 4.0 Hz, 1H), 6.77 - 6.69 (m, 2H), 6.54 - 6.48 (m, 1H), 5.60 (s, 2H), 4.40 (s, 2H), 3.08 - 2.98 (m, 2H), 2.70 (s, 2H), 2.65 - 2.53 (m, 1H), 2.48 - 2.31 (m, 3H), 2.17 - 1.96 (m, 2H), 1.59 - 1.46 (m, 1H), 1.03 - 0.94 (m, 2H), 0.69 - 0.60 (m, 2H).

**Example 16 Synthesis of 2-((4-(4-((4-chloro-2-fluorobenzofuran-7-yl)methoxy)-5-fluoropyrimidin-2-yl)cyclohex-3-en-1-yl)methyl)-1-((1-ethyl-1*H*-imidazol-5-yl)methyl)-1*H*-benzo[*d*]imidazole-6-carboxylic acid (compound 16)**

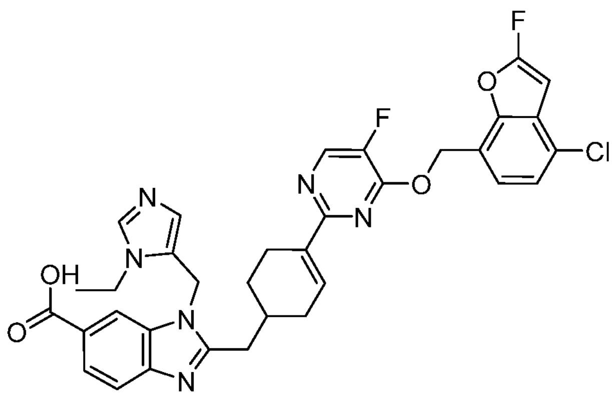

**Step 1: Synthesis of methyl 3-(((1-ethyl-1*H*-imidazol-5-yl)methyl)amino)-4-nitrobenzoate**

Methyl 3-fluoro-4-nitrobenzoate (500 mg, 2.51 mmol), (1-ethyl-1*H*-imidazol-5-yl)methanamine (500 mg, 3.99 mmol) were added to tetrahydrofuran (10 mL), then *N,N*-diisopropylethylamine (2.9 mL, 17.59 mmol) was added, and the reaction mixture was heated to 60°C for 3 hours. The reaction mixture was cooled down to room temperature and concentrated under reduced pressure, and the residue was purified by column chromatography eluted with EA:PE (v/v) =1:15 to give a yellow solid (638 mg, 83.4%).
LC-MS (ESI): $[M+H]^+$=305.5.

**Step 2: Synthesis of methyl 4-amino-3-(((1-ethyl-1*H*-imidazol-5-yl)methyl)amino)benzoate**

Methyl 3-(((1-ethyl-1*H*-imidazol-5-yl)methyl)amino)-4-nitrobenzoate (638 mg, 2.10 mmol) was added to ethyl acetate (16 mL) and stirred to dissolve, then 10% Pd/C (65 mg, 0.61 mmol) was added, and the reaction was heated to 40 °C under hydrogen atmosphere overnight. The palladium carbon was removed by filtration with diatomaceous earth and the filtrate was concentrated under reduced pressure to give a colorless oil (558 mg, 96.9%).

$^1$H NMR (400 MHz, DMSO-$d_6$) δ 7.78 (s, 1H), 7.18 (dd, *J* = 8.1, 1.8 Hz, 1H), 7.12 (d, *J* = 1.8 Hz, 1H), 6.93 (s, 1H), 6.55 (d, *J* = 8.1 Hz, 1H), 5.50 (s, 2H), 4.94 (s, 1H), 4.26 (d, *J* = 4.3 Hz, 2H), 4.03 (q, *J* = 7.3 Hz, 2H), 3.73 (s, 3H), 1.35 (t, *J* = 7.3 Hz, 3H).

**Step 3: Synthesis of methyl 1-((1-ethyl-1*H*-imidazol-5-yl)methyl)-2-((4-(5-fluoro-4-(methoxymethoxy)pyrimidin-2-yl)cyclohex-3-en-1-yl)methyl)-1*H*-benzo[*d*]imidazole-6-carboxylate**

2-(4-(5-fluoro-4-(methoxymethoxy)pyrimidin-2-yl)cyclohex-3-en-1-yl)acetaldehyde (170 mg, 0.61 mmol), methyl 4-amino-3-(((1-ethyl-1*H*-imidazol-5-yl)methyl)amino)benzoate (166.4 mg, 0.61 mmol) were added into acetic acid (11 mL) and stirred at 60 °C for 1.5 h, concentrated under reduced pressure and the residue was purified by column chromatography eluted with DCM:MeOH (v/v)=20:1) to give a yellow oil (270 mg, 83.3%).

LC-MS (ESI): [M+H]$^+$=536.0.

**Step 4: Synthesis of methyl 1-((1-ethyl-1*H*-imidazol-5-yl)methyl)-2-((4-(5-fluoro-4-hydroxypyrimidin-2-yl)cyclohex-3-en-1-yl)methyl)-1*H*-benzo[*d*]imidazole-6-carboxylate**

Methyl 1-((1-ethyl-1*H*-im idazol-5 -yl)methyl)-2-((4-(5 -fluoro-4-(methoxymethoxy)pyrimidin-2-yl)cyclohex-3-en-1-yl)methyl)-1*H*-benzo[d]imidazole-6-carboxylate (270 mg, 0.51 mmol) was added to dichloromethane (10 mL), then trifluoroacetic acid (5 mL) was added and the mixture was stirred at -10 °C overnight. Concentrated under reduced pressure and the residue was purified by column chromatography eluted with DCM:MeOH (v/v)=15:1 to give a yellow solid (191 mg, 77.1%).

LC-MS (ESI): [M+H]$^+$=491.5;
$^1$HNMR (400 MHz, DMSO-$d_6$) δ 12.58 (s, 1H), 8.16 (s, 1H), 7.99 (d, *J* = 3.3 Hz, 1H), 7.82 (dd, *J* = 8.4, 1.5 Hz, 1H), 7.72-7.62 (m, 2H), 6.73 (s, 1H), 6.42 (s, 1H), 5.65 (s, 2H), 3.94 (q, *J* = 7.3 Hz, 2H), 3.85 (s, 3H), 3.12 - 2.86 (m, 2H), 2.50 - 2.33 (m, 6H), 1.39 - 1.21 (m, 1H), 1.12 (t, *J* = 7.2 Hz, 3H).

**Step 5: Synthesis of methyl 2-((4-(4-((4-chloro-2-fluorobenzofuran-7-yl)methoxy)-5-fluoropyrimidin-2-yl)cyclohex-3-en-1-yl)methyl)-1-((1-ethyl-1*H*-imidazol-5-yl)methyl)-1*H*-benzo[*d*]imidazole-6-carboxylate**

Methyl 1-((1-ethyl-1*H*-imidazol-5-yl)methyl)-2-((4-(5-fluoro-4-hydroxypyrimidin-2-yl)cyclohex-3-en-1-yl)methyl)-1*H*-benzo[*d*]imidazole-6-carboxylate (190 mg, 0.39 mmol), 7-(bromomethyl)-4-chloro-2-fluoro-1-benzofuran (153.1 mg, 0.58 mmol) were added to *N*,*N*-dimethylformamide (5 mL), then potassium carbonate (161 mg, 1.16 mmol) was added, and the mixture was stirred at 60 °C for two hours. It was concentrated under reduced pressure and the residue was purified by column chromatography eluted with EtOAc:PE (v/v)=1:1 to give a yellow solid (170 mg, 65.2%).

LC-MS (ESI): [M+H]$^+$=673.5;
$^1$HNMR (400 MHz, DMSO-$d_6$) δ 8.56 (d, *J* = 2.8 Hz, 1H), 8.16 (d, *J* = 0.9 Hz, 1H), 7.83 (dd, *J* = 8.5, 1.5 Hz, 1H), 7.70 (d, *J* = 8.7 Hz, 2H), 7.51-7.39 (m, 2H), 7.08 (s, 1H), 6.55 (d, *J* = 6.4 Hz, 1H), 6.43 (s, 1H), 5.77 (d, *J* = 12.1 Hz, 2H), 5.66 (s, 2H), 3.95 (q, *J* = 7.2 Hz, 2H), 3.85 (s, 3H), 2.96 - 2.92 (m, 2H), 2.64 (d, *J* = 19.2 Hz, 1H), 2.50 - 1.88 (m, 6H), 1.12 (t, *J* = 7.2 Hz, 3H).

**Step 6: Synthesis of 2-((4-(4-((4-chloro-2-fluorobenzofuran-7-yl)methoxy)-5-fluoropyrimidin-2-yl)cyclohex-3-en-1-yl)methyl)-1-((1-ethyl-1*H*-imidazol-5-yl)methyl)-1*H*-benzo[*d*]imidazole-6-carboxylic acid**

Methyl 2-((4-(4-((4-chloro-2-fluorobenzofuran-7-yl)methoxy)-5-fluoropyrimidin-2-yl)cyclohex-3-en-1-yl)methyl)-1-((1-ethyl-1*H*-imidazol-5-yl)methyl)-1*H*-benzo[*d*]imidazole-6-carboxylate (100 mg, 0.15 mmol) was added to dioxane (2 mL), and the Lithium hydroxide (19 mg, 0.45 mmol) dissolved in water (0.5 mL) was added to the reaction mixture and stirred at 40 °C for 3 hours. The pH was adjusted to 6 with acetic acid. The reaction mixture was concentrated under reduced pressure and the residue was purified by Prep-HPLC eluted with $CH_3CN$: $H_2O$ (v/v) = 50% to give a white solid (44.8 mg, 45.8%).

LC-MS (ESI): $[M+H]^+$=659.3;

$^1$HNMR (400 MHz, DMSO-$d_6$) δ 12.58(s, 1H), 8.56 (d, *J* = 2.9 Hz, 1H), 8.13 (s, 1H), 7.81 (dd, *J* = 8.4, 1.4 Hz, 1H), 7.67 (d, *J* = 8.3 Hz, 2H), 7.46 (q, *J* = 8.2 Hz, 2H), 7.08 (s, 1H), 6.55 (d, *J* = 6.4 Hz, 1H), 6.44 (s, 1H), 5.76 (s, 2H), 5.64 (s, 2H), 3.94 (q, *J* = 7.2 Hz, 2H), 2.94 (t, *J* = 6.4 Hz, 2H), 2.65 (d, *J* = 19.1 Hz, 1H), 2.49 - 2.43 (m, 2H), 2.39 - 1.88 (m, 3H), 1.46 - 1.39 (m, 1H), 1.12 (t, *J* = 7.2 Hz, 3H).

**Example 17 Synthesis of 2-((4-(6-((4-chloro-2-fluorobenzofuran-7-yl)methoxy)pyridin-2-yl)cyclohex-3-en-1-yl) methyl)-1-((1-ethyl-1*H*-imidazol-5-yl)methyl)-1*H*-thieno[2,3-*d*]imidazole-5-carboxylic acid (compound 17)**

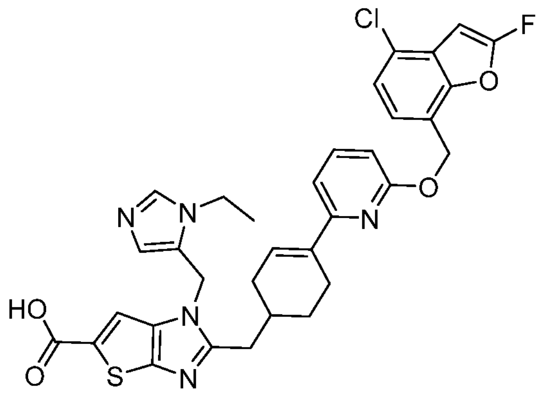

**Step 1: Synthesis of methyl 5-((tert-butoxycarbonyl)amino)-4-(((1-ethyl-1*H*-imidazol-5-yl)methyl)amino)thiophene-2-carboxylate**

Methyl 4-amino-5-((tert-butoxycarbonyl)amino)thiophene-2-carboxylate (1.0 g, 3.67 mmol) and 2-(1-ethyl-1*H*-imidazol-5-yl)acetaldehyde (608 mg, 4.4 mmol) were added to ethanol (10 mL), then acetic acid (0.1 mL) was added, and the reaction was stirred at room temperature for ten minutes. Sodium cyanoborohydride (461.2 mg, 7.34 mmol) was added and the reaction was carried out at room temperature for 2 hours. Diluted with Water (20 mL), extracted with dichloromethane (20 mL×2), the combined organic phases were dried with anhydrous sodium sulfate, concentrated under reduced pressure, and the residue was purified by column chromatography eluted with EtOAc:PE (v/v)=50% to give a light yellow solid (1.2 g, 86%).

LC-MS (ESI): $[M+H]^+$=381.1;

$^1$HNMR (400 MHz, DMSO-$d_6$) δ 8.5 (s, 1H), 7.58 (s, 1H), 7.37 (s, 1H), 6.79 (s, 1H), 4.11 (s, 2H), 3.99 - 3.94 (m, 2H), 3.77 (s, 3H), 1,49 - 1.38 (m, 12H).

**Step 2: Synthesis of methyl 5-amino-4-(((1-ethyl-1*H*-imidazol-5-yl)methyl)amino)thiophene-2-carboxylate**

Methyl 5-((tert-butoxycarbonyl)amino)-4-((1-ethyl-1*H*-imidazol-5-yl)methyl)amino)thiophene-2-carboxylate (1.2 g, 3.15 mmol) was dissolved in dichloromethane (5 mL). Methanol solution of hydrochloric acid (4 N) was added and stirred at room temperature for 4 hours. Concentrated under reduced pressure to give a pale yellow solid (440 mg) and was used directly in the next step of the reaction.

LC-MS (ESI): $[M+H]^+$=281.1.

**Step 3: Synthesis of methyl 1-((1-ethyl-1*H*-imidazol-5-yl)methyl)-2-((4-(6-hydroxypyridin-2-yl)cyclohex-3-en-1-yl)methyl)-1*H*-thieno[2,3-*d*]imidazole-5-carboxylate**

Methyl 5-amino-4-(((1-ethyl-1*H*-imidazol-5-yl)methyl)amino)thiophene-2-carboxylate (220 mg, 0.786 mmol) and 2-(4-(6-(methoxymethoxy)pyridin-2-yl)cyclohex-3-en-1-yl)acetaldehyde (205.4 mg, 0.786 mmol) were added to acetic acid (2 mL), it was displaced with oxygen for 3 times and the reaction was carried out at 60 °C for 6 hours. The reaction was concentrated under reduced pressure and the residue was purified by column chromatography eluted with DCM:MeOH (v/v)=85% to give a light yellow solid (55 mg, 14.7%).

LC-MS (ESI): $[M+H]^+$=478.3;

$^1$HNMR (400 MHz, DMSO-$d_6$) δ 11.3 (s, 1H), 7.73 (s, 1H), 7.4 - 7.36 (m, 2H), 6.92 (s, 1H), 6.44 (s, 1H), 6.22 (d, *J* = 9.2 Hz, 2H), 5.52 (s, 2H), 3.82 - 3.79 (m, 5H), 2.9 - 2.87 (m, 2H), 2.35 - 2.3 (m, 2H), 2.03 - 1.97 (m, 4H), 1.2 - 1.18 (m, 1H), 1.05 - 0.99 (m, 3H).

**Step 4: Synthesis of methyl 2-((4-(6-((4-chloro-2-fluorobenzofuran-7-yl)methoxy)pyridin-2-yl)cyclohex-3-en-1-yl)methyl)-1-((1-ethyl-1*H*-imidazol-5-yl)methyl)-1*H*-thieno[2,3-*d*]imidazole-5-carboxylate**

Methyl 1-((1-ethyl-1*H*-imidazol-5-yl)methyl)-2-((4-(6-hydroxypyridin-2-yl)cyclohex-3-en-1-yl)methyl)-1*H*-thieno[2,3-*d*]imidazole-5-carboxylate (55 mg, 0.12 mmol) and potassium carbonate (49.7 mg, 0.36 mmol) were added to *N*,*N*-dimethylformamide (3 mL), 7-(bromomethyl)-4-chloro-2-fluorobenzofuran (31.6 mg, 0.12 mmol) was added, and the reaction was carried out at 60 °C for 1.5 hours. Concentrated under reduced pressure and the residue was purified by column chromatography eluted with DCM:MeOH (v/v)=95% to give a yellow solid (28 mg, 36.9%).
LC-MS (ESI): $[M+H]^+$=660.1.

**Step 5: Synthesis of 2-((4-(6-((4-chloro-2-fluorobenzofuran-7-yl)methoxy)pyridin-2-yl)cyclohex-3-en-1-yl) methyl)-1-((1-ethyl-1*H*-imidazol-5-yl)methyl)-1*H*-thieno[2,3-*d*]imidazole-5-carboxylic acid**

Compound methyl 2-((4-(6-((4-chloro-2-fluorobenzofuran-7-yl)methoxy)pyridin-2-yl)cyclohex-3-en-1-yl) methyl)-1-((1-ethyl-1*H*-imidazol-5-yl)methyl)-1*H*-thieno[2,3-*d*]imidazole-5-carboxylate (28 mg, 0.0424 mmol) was added to the mixture of dioxane (0.2 mL) and water (0.1 mL). Lithium hydroxide (5.1 mg, 0.212 mmol) was added and reacted at 40 °C for 4 hours. The pH was adjusted to 5-6 with acetic acid (0.1 mL), concentrated under reduced pressure, and the residue was purified by Pre-HPLC eluted with $CH_3CN$: $H_2O$ (v/v) = 60%) to give a white solid (8.5 mg, 31%).

LC-MS (ESI): $[M+H]^+$=646.3;
$^1$HNMR (400 MHz, DMSO-$d_6$) δ 7.73 (s, 1H), 7.66 (t, *J* = 7.8 Hz, 1H), 7.41 (t, *J* = 2 Hz, 2H), 7.24 (s, 1H), 7.06 (d, *J* = 7.6 Hz, 1H), 6.92 (s, 1H), 6.72 (d, *J* = 8 Hz, 2H), 6.52 (d, *J* = 6.4 Hz, 1H), 5.59 (s, 2H), 5.50 (s, 2H), 3.83 - 3.81 (m, 2H), 2.92 - 2.89 (m, 2H), 2.56 (s, 1H), 2.38 - 2.27 (m, 2H), 1.99 - 1.91 (m, 3H), 1,45 - 1.01 (m, 1H), 0.99 (t, *J* = 7.2 Hz, 3H).

**Example 18 Synthesis of 2-((4-(4-((4-chloro-2-fluorobenzofuran-7-yl)methoxy)-5-fluoropyrimidin-2-yl)cyclohex-3-en-1-yl)methyl)-1-((1-ethyl-1*H*-imidazol-5-yl)methyl)-1*H*-thieno[2,3-*d*]imidazole-5-carboxylic acid (compound 18)**

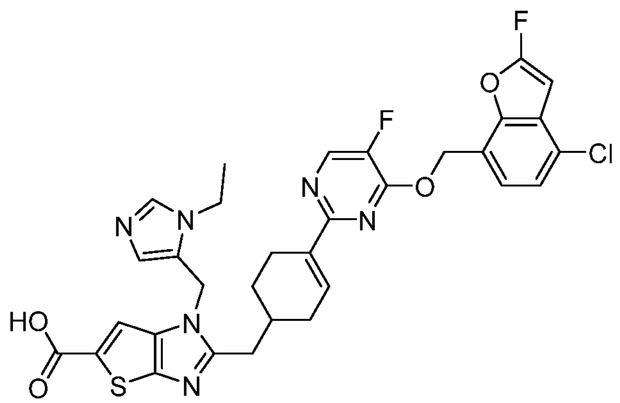

**Step 1: Synthesis of methyl 4-(((1-ethyl-1*H*-imidazol-5-yl)methyl)amino)-5-nitrothiophene-2-carboxylate**

(1-ethyl-1*H*-imidazol-5-yl)methanamine (800 mg, 6.4 mmol), methyl 4-bromo-5-nitrothiophene-2-carboxylate (2.04 g, 7.68 mmol) were added to *N*,*N*-dimethylformamide (15 mL), potassium carbonate (2.65 g, 19.2 mmol) was added, and stirred at 80 °C overnight. Water (30 mL) was added, extracted with ethyl acetate (30 mL×3), the combined organic phases were washed with saturated brine, dried with anhydrous sodium sulfate, and the residue was purified by column chromatography eluted with EA:PE (v/v)=1:1 to give a yellow solid (550 mg, 27.7%).
LC-MS (ESI): $[M+H]^+$=311.2.

**Step 2: Synthesis of methyl 5-amino-4-(((1-ethyl-1H imidazol-5-yl)methyl)amino)thiophene-2-carboxylate**

Methyl 4-(((1-ethyl-1*H*-imidazol-5-yl)methyl)amino)-5-nitrothiophene-2-carboxylate (550 mg, 1.77 mmol) was dissolved in acetic acid (10 mL). Zinc powder (1.15 g, 17.7 mmol) was added and stirred at room temperature for 5 min. Filtrated and concentrated to give the crude product (250 mg, 50.3%), and the crude product was used directly in the next step.

LC-MS (ESI): $[M+H]^+$=281.1.

**Step 3: Synthesis of methyl 1-((1-ethyl-1*H*-imidazol-5-yl)methyl)-2-((4-(5-fluoro-4-(methoxymethoxy)pyrimidin-2-yl)cyclohex-3-en-1-yl)methyl)-1*H*-thieno[2,3-*d*]imidazole-5-carboxylate**

Methyl 5-amino-4-(((1-ethyl-1*H*-imidazol-5-yl)methyl)amino)thiophene-2-carboxylate (250 mg, 0.89 mmol), 2-(4-(5-fluoro-4-(methoxymethoxy)pyrimidin-2-yl)cyclohex-3-en-1-yl)acetaldehyde (250 mg, 0.89 mmol) were added to acetic acid (10 mL). The reaction was stirred at 60 °C for 1 h under the protection of oxygen. Concentrated and the residue was purified by column chromatography eluted with DCM:MeOH (v/v)=10:1 to give a yellow solid (100 mg, 20.7%).

LC-MS (ESI): $[M+H]^+$=541.2.

**Step 4: Synthesis of methyl 1-((1-ethyl-1*H*-imidazol-5-yl)methyl)-2-((4-(5-fluoro-4-hydroxypyrimidin-2-yl)cyclohex-3-en-1-yl)methyl)-1*H*-thieno[2,3-*d*]imidazole-5-carboxylate**

Methyl 1-((1-ethyl-1*H*-im idazol-5 -yl)methyl)-2-((4-(5 -fluoro-4-(methoxymethoxy)pyrimidin-2-yl)cyclohex-3-en-1-yl)methyl)-1*H*-thieno[2,3-*d*]imidazole-5-carboxylate (100 mg, 0.19 mmol) was added to dichloromethane (10 mL), stirred at -10°C and trifluoroacetic acid (5 mL) was added, the reaction was carried out overnight. Concentrated under reduced pressure and the residue was purificated by column chromatography eluted with DCM:MeOH (v/v)=8:1 to give a yellow solid (50 mg, 54.5%).

LC-MS (ESI): $[M+H]^+$=497.3.

**Step 5: Synthesis of methyl 2-((4-(4-((4-chloro-2-fluorobenzofuran-7-yl)methoxy)-5-fluoropyrimidin-2-yl)cyclohex-3-en-1-yl)methyl)-1-((1-ethyl-1*H*-imidazol-5-yl)methyl)-1*H*-thieno[2,3-d]imidazole-5-carboxylate**

Methyl 1-((1-ethyl-1*H*-imidazol-5-yl)methyl)-2-((4-(5-fluoro-4-hydroxypyrimidin-2-yl)cyclohex-3-en-1-yl)methyl)-1*H*-thieno[2,3-*d*]imidazole-5-carboxylate (50 mg, 0.1 mmol), 7-(bromomethyl)-4-chloro-2-fluorobenzofuran (86 mg, 0.15 mmol) were added to *N*,*N*-dimethylformamide (2 mL), potassium carbonate (40 mg, 0.66 mmol) was added, and the reaction was stirred at 60 °C for 1 hour. Stopped and diluted with water (5 mL), extracted with ethyl acetate (10 mL×3), the combined organic phases were washed with saturated brine, dried with anhydrous sodium sulfate, and the residue was purified by column chromatography eluted with DCM:MeOH (v/v)=10:1 to give a yellow solid (20 mg, 29.4%).

LC-MS (ESI): $[M+H]^+$=679.5.

**Step 6: Synthesis of 2-((4-(4-((4-chloro-2-fluorobenzofuran-7-yl)methoxy)-5-fluoropyrimidin-2-yl)cyclohex-3-en-1-yl)methyl)-1-((1-ethyl-1*H*-imidazol-5-yl)methyl)-1*H*-thieno[2,3-*d*]imidazole-5-carboxylic acid**

Methyl 2-((4-(4-((4-chloro-2-fluorobenzofuran-7-yl)methoxy)-5-fluoropyrimidin-2-yl)cyclohex-3-en-1-yl)methyl)-1-((1-ethyl-1*H*-imidazol-5-yl)methyl)-1*H*-thieno[2,3-*d*]imidazole-5-carboxylate (20 mg, 0.03 mmol) was dissolved in the mixture of dioxane (2 mL) and water (0.5 mL). Lithium hydroxide (4 mg, 0.17 mmol) was added and stirred for 2 h at room temperature. The pH was adjusted to 6-7 with acetic acid, extracted with ethyl acetate (10 mL × 3), and the combined organic phases were washed with saturated saline and dried with anhydrous sodium sulfate. It was concentrated under reduced pressure and the residue was purified by prep-HPLC eluted with $CH_3CN$: $H_2O$ (v/v)=50% to give a white solid (12.2 mg, 62.2%).

LC-MS (ESI): $[M+H]^+$=665.3;
$^1H$ NMR (400 MHz, DMSO-$d_6$) δ 8.57 (d, *J* = 2.8 Hz, 1H), 7.72 (s, 1H), 7.46 (q, *J* = 8.2 Hz, 2H), 7.20 (s, 1H), 7.09 (s, 1H), 6.92 (s, 1H), 6.55 (d, *J* = 6.4 Hz, 1H), 5.76 (s, 2H), 5.49 (s, 2H), 3.82 (q, *J* = 7.2 Hz, 2H), 2.95 - 2.82 (m, 2H), 2.66 (d, *J* = 17.2 Hz, 1H), 2.36 (dd, *J* = 36.0, 15.1 Hz, 2H), 2.03 (d, *J* = 13.0 Hz, 2H), 1.90 (d, *J* = 11.2 Hz, 1H), 1.39 (d, *J* = 6.9 Hz, 1H), 0.99 (t, *J* = 7.2 Hz, 3H).

**Example 19 Synthesis of 2-((4-(6-((4-chloro-2-fluorobenzofuran-7-yl)methoxy)pyridin-2-yl)cyclohex-3-en-1-yl)methyl)-1-(2-methoxyethyl)-1*H*-thieno[2,3-*d*]imidazole-5-carboxylic acid (compound 19)**

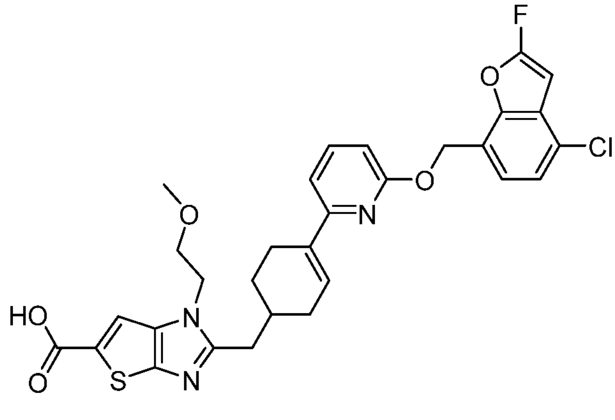

**Step 1: Synthesis of methyl 2-((4-(6-hydroxypyridin-2-yl)cyclohex-3-en-1-yl)methyl)-1-(2-methoxyethyl)-1*H*-thieno[2,3-*d*]imidazole-5-carboxylate**

Methyl 5-amino-4-(2-methoxyethyl)amino)thiophene-2-carboxylate (106 mg, 0.46 mmol) and 2-(4-(6-(methoxymethoxy)pyridin-2-yl)cyclohex-3-en-1-yl)acetaldehyde (100 mg, 0.38 mmol) were added to acetic acid (1.5 mL) and the reaction was carried out at 60 °C for 2 hours. Concentrated under reduced pressure and the residue was purified by column chromatography eluted with DCM:MeOH (v/v)=85% to give a pale yellow solid (28 mg, 17.1%).
LC-MS (ESI): $[M+H]^+$=428.3.

**Step 2: Synthesis of methyl 2-((4-(6-((4-chloro-2-fluorobenzofuran-7-yl)methoxy)pyridin-2-yl)cyclohex-3-en-1-yl)methyl)-1-(2-methoxyethyl)-1*H*-thieno[2,3-*d*]imidazole-5-carboxylate**

Methyl 2-((4-(6-hydroxypyridin-2-yl)cyclohex-3-en-1-yl)methyl)-1-(2-methoxyethyl)-1*H*-thieno[2,3-*d*]imidazole-5-carboxylate (28 mg, 0.066 mmol) and potassium carbonate (27.1 mg, 0.196 mmol) were added to *N*,*N*-dimethylformamide (2 mL), 7-(bromomethyl)-4-chloro-2-fluorobenzofuran (18.3 mg, 0.069 mmol) was added, and the reaction was carried out at 60 °C for 2 hours. Concentrated under reduced pressure and the residue was purified by column chromatography eluted with DCM:MeOH (v/v)=95% to give a yellow solid (14.5 mg, 36%).
LC-MS (ESI): $[M+H]^+$=610.3.

**Step 3: Synthesis of 2-((4-(6-((4-chloro-2-fluorobenzofuran-7-yl)methoxy)pyridin-2-yl)cyclohex-3-en-1-yl)methyl)-1-(2-methoxyethyl)-1*H*-thieno[2,3-*d*]imidazole-5-carboxylic acid**

Methyl 2-((4-(6-((4-chloro-2-fluorobenzofuran-7-yl)methoxy)pyridin-2-yl)cyclohex-3-en-1-yl)methyl)-1-(2-methoxyethyl)-1*H*-thieno[2,3-*d*]imidazole-5-carboxylate (14.5 mg, 0.0238 mmol) was added to the mixture of dioxyhexane (0.1 mL) and water (0.05 mL). Lithium hydroxide (2.9 mg, 0.119 mmol) was added and the reaction was carried ou at 40 °C for 4 hours. The pH was adjusted to 5-6 with acetic acid (0.1 mL), concentrated under reduced pressure, and the residue was purified by Pre-HPLC eluted with $CH_3CN$: $H_2O$ (v/v)=60% to give a white solid (5.7 mg, 40.2%).

LC-MS (ESI): $[M+H]^+$=596.2;
$^1$HNMR (400 MHz, DMSO-$d_6$) δ 12.88 (s, 1H), 7.79 (s, 1H), 7.66 (t, *J* = 7.8 Hz, 1H), 7.43 - 7.38 (m, 2H), 7.06 (d, *J* = 7.6 Hz, 1H), 6.72 (d, *J* = 8.4 Hz, 2H), 6.52 (d, *J* = 6.4 Hz, 1H), 5.59 (s, 2H), 4.54 - 4.38 (m, 2H), 3.65 - 3.63 (m, 2H), 3.21 (s, 3H), 2.86 - 2.83 (m, 2H), 2.57 (s, 1H), 2.03 - 2.02 (m, 2H), 2.05 - 2.03 (m, 1H), 2.01 - 1.9 (m, 2H), 1.44 - 1.42 (m, 1H).

**Example 20 Synthesis of 2-((4-(4-((4-chloro-2-fluorobenzofuran-7-yl)methoxy)-5-fluoropyrimidin-2-yl)cyclohex-3-en-1-yl)methyl)-1-(2-methoxyethyl)-1*H*-thieno[2,3-*d*]imidazole-5-carboxylic acid (compound 20)**

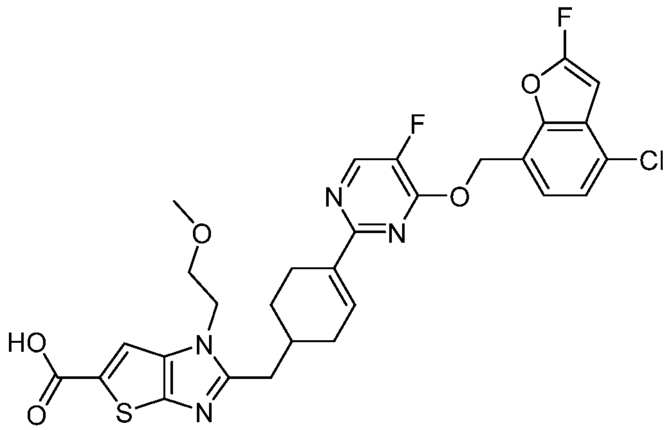

**Step 1: Synthesis of methyl 4-((2-methoxyethyl)amino)-5-nitrothiophene-2-carboxylate**

Methyl 4-bromo-5-nitrothiophene-2-carboxylate (1 g, 3.76 mmol) and triethylamine (1.6 mL, 11.28 mmol) were added to dioxane (40 mL), 2-methoxyethan-1-amine (0.4 mL, 4.51 mmol) was added, and the reaction mixture was heated to 110 °C under nitrogen protection and stirred overnight. The reaction mixture was cooled down to room temperature, concentrated under reduced pressure and the residue was purified by column chromatography eluted with MeOH:DCM (v/v)=1:20 to give a yellow solid (800 mg, 81.8%).
LC-MS (ESI): $[M+H]^+$=261.2.

**Step 2: Synthesis of methyl 5-amino-4-((2-methoxyethyl)amino)thiophene-2-carboxylate**

Methyl 4-(2-methoxyethyl)amino-5-nitrothiophene-2-carboxylate (500 mg, 1.92 mmol) was added to acetic acid (5 mL), zinc powder (628 mg, 9.61 mmol) was added, and stirred at room temperature for 0.5 hour. The reaction mixture was filtered with diatomaceous earth, the filter cake was washed with dichloromethane (50 mL) and concentrated under reduced pressure to give a gray oil (440 mg, 98.2%).
LC-MS (ESI): $[M+H]^+$=231.2.

**Step 3: Synthesis of 4-chloro-2-(2,2-diethoxyethoxy)-1-methylbenzene**

5-Chloro-2-methylphenol (100 g, 701.36 mmol) was added to *N,N*-dimethylformamide (1 L), stirred to dissolve, then 2-bromo-1,1-diethoxyethane (129.6 mL, 841.63 mmol) and potassium carbonate (193.86 g, 1402.72 mmol) were added, and the reaction was heated up to 120 °C under nitrogen protection overnight. The reaction mixture was cooled down to room temperature, added to ice water (5 L), extracted with ethyl acetate (500 mL×3), the organic phases were combined and washed with saturated saline (500 mL), the organic phase was dried with anhydrous sodium sulfate, concentrated under reduced pressure to give a light yellow oil (147 g, 81.0%).

**Step 4: Synthesis of 4-chloro-7-methylbenzofuran**

Polyphosphoric acid (121.4 mL, 3049.15 mmol) was dissolved in 1,2-dichloroethane (1 L), 4-chloro-2-(2,2-diethoxyethoxy)-1-methylbenzene (147 g, 568.14 mmol) was added, and the reaction was carried out at 85 °C overnight. The reaction mixture was cooled down to room temperature, the reaction mixture was added to ice water (2 L), extracted with dichloromethane (500 mL×2), the combined organic layers were dried with anhydrous sodium sulfate, concentrated under reduced pressure, and the residue was purified by column chromatography eluted with EA:PE (v/v)=1:20 to give a colorless oil (51 g, 53.9%).
$^1$H NMR (400 MHz, DMSO-$d_6$) δ 8.12 (d, *J* = 2.0 Hz, 1H), 7.24 (d, *J* = 8.0 Hz, 1H), 7.16 (d, *J* = 8.0 Hz, 1H), 6.99 (d, *J* = 2.0 Hz, 1H), 2.47 (s, 3H).

**Step 5: Synthesis of 7-(bromomethyl)-4-chlorobenzofuran**

4-Chloro-7-methylbenzofuran (31 g, 186.07 mmol) was added to 1,2-dichloroethane (300 mL), then *N*-bromosuccinimide (34.78 g, 195.38 mmol), and azobisisobutyronitrile (6.1 g, 37.21 mmol), it was displaced with nitrogen, and the reaction was carried out at 75 °C overnight. The reaction mixture was cooled down to room temperature, a white solid was precipitated and filtered. The filter cake was washed with dichloromethane (50 mL×3), the filtrate was collected and

concentrated under reduced pressure, and the residue was purified by column chromatography eluted with n-hexane to give a white solid (27.8 g, 60.9%).

$^1H$ NMR (400 MHz, DMSO-$d_6$) δ 8.23 (d, *J* = 2.0 Hz, 1H), 7.45 (d, *J* = 8.0 Hz, 1H), 7.36 (d, *J* = 8.0 Hz, 1H), 7.07 (d, *J* = 2.0 Hz, 1H), 4.95 (s, 2H)。

**Step 6: Synthesis of 7-(bromomethyl)-4-chloro-2-fluorobenzofuran**

7-(bromomethyl)-4-chlorobenzofuran (5.8 g, 23.63 mmol), *N*-fluoro-*N*-(phenylsulfonyl)benzenesulfonamide (9.0 g, 28.54 mmol) were added to tetrahydrofuran (100 mL), cooled down to -78 °C under nitrogen protection, and lithium diisopropylammonium (2.0 N, 20 mL, 151.22 mmol) was slowly added, and the reaction was carried out at -78 °C overnight. Quenched with water (20 mL) at -78 °C, extracted with ethyl acetate (20 mL×3), the combined organic layers were washed with saturated brine, concentrated under reduced pressure, the residue was purified by column chromatography eluted with n-hexane to give a white solid (9.2 g, 14.8%).

$^1H$ NMR (400 MHz, $CDCl_3$) δ 7.22 (d, *J* = 2.2 Hz, 2H), 6.01 (d, *J* = 6.7 Hz, 1H), 4.65 (s, 2H).

**Step 7: Synthesis of methyl 2-((4-(5-fluoro-4-(methoxymethoxy)pyrimidin-2-yl)cyclohex-3-en-1-yl)methyl)-1-(2-methoxyethyl)-1*H*-thieno[2,3-*d*]imidazole-5-carboxylate**

2-(4-(5-fluoro-4-(methoxymethoxy)pyrimidin-2-yl)cyclohex-3-en-1-yl)acetaldehyde (100 mg, 0.36 mmol), methyl 5-amino-4-(2-methoxyethyl)amino)thiophene-2-carboxylate (100 mg, 0.43 mmol) were added to acetic acid (5 mL), the reaction mixture was heated to 60°C for two hours. Concentrated under reduced pressure and the residue was purified by column chromatography eluted with MeOH:DCM (v/v)=1:20 to give a brown oil (66 mg, 37.7%).

LC-MS (ESI): $[M+H]^+$=491.4.

**Step 8: Synthesis of methyl 2-((4-(5-fluoro-4-hydroxypyrimidin-2-yl)cyclohex-3-en-1-yl)methyl)-1-(2-methoxyethyl)-1*H*-thieno[2,3-*d*]imidazole-5-carboxylate**

Methyl 2-((4-(5-fluoro-4-(methoxymethoxy)pyrimidin-2-yl)cyclohex-3-en-1-yl)methyl)-1-(2-methoxyethyl)-1*H*-thieno[2,3-*d*]imidazole-5-carboxylate (66 mg, 0.16 mmol) was added to dichloromethane (2 mL), and the reaction mixture was cooled to -10 °C under nitrogen protection. Trifluoroacetic acid (1 mL, 13.46 mmol) was added and the reaction mixture was kept at -10°C overnight. Concentrated under reduced pressure and the residue was purified by column chromatography eluted with MeOH:DCM (v/v)=1:20 to give a yellow oil (48 mg, 75%).

LC-MS (ESI): $[M+H]^+$=447.3.

**Step 9: Synthesis of methyl 2-((4-(4-((4-chloro-2-fluorobenzofuran-7-yl)methoxy)-5-fluoropyrimidin-2-yl)cyclohex-3-en-1-yl)methyl)-1-(2-methoxyethyl)-1*H*-thieno[2,3-*d*]imidazole-5-carboxylate**

Methyl 2-((4-(5-fluoro-4-hydroxypyrimidin-2-yl)cyclohex-3-en-1-yl)methyl)-1-(2-methoxyethyl)-1*H*-thieno[2,3-*d*]imidazole-5-carboxylate (48 mg, 0.13 mmol), 7-(bromomethyl)-4-chloro-2-fluorobenzofuran (30 mg, 0.13 mmol) were added to the *N*,*N*-dimethylformamide (5 mL), potassium carbonate (38 mg, 0.27 mmol) was added and the reaction was heated to 60 °C for 2 h under nitrogen protection. It was concentrated under reduced pressure and the residue was purified by column chromatography eluted with MeOH:DCM (v/v)=1:20 to give a light yellow solid (54 mg, 82.2%).

LC-MS (ESI): $[M+H]^+$=629.4.

**Step 10: Synthesis of 2-((4-(4-((4-chloro-2-fluorobenzofuran-7-yl)methoxy)-5-fluoropyrimidin-2-yl)cyclohex-3-en-1-yl)methyl)-1-(2-methoxyethyl)-1*H*-thieno[2,3-*d*]imidazole-5-carboxylic acid**

Methyl 2-((4-(4-((4-chloro-2-fluorobenzofuran-7-yl)methoxy)-5-fluoropyrimidin-2-yl)cyclohex-3-en-1-yl)methyl)-1-(2-methoxyethyl)-1*H*-thieno[2,3-*d*]imidazole-5-carboxylate (54 mg, 0.09 mmol) was added to dioxane (2 mL). Lithium hydroxide (18.0 mg, 0.43 mmol) dissolved in water (0.5 mL) was added to the reaction mixture, and the reaction was heated to 40 °C for 3 h under nitrogen protection. The pH was adjusted to about 4 with acetic acid, concentrated under reduced pressure, and the residue was purified by Prep-HPLC eluted with $CH_3CN$: $H_2O$ (v/v) = 50% to give a white solid (31 mg, 56.0%).

LC-MS (ESI): $[M+H]^+$=615.2;

$^1H$ NMR (400 MHz, DMSO-$d_6$) δ 12.83 (s, 1H), 8.57 (d, *J* = 2.8 Hz, 1H), 7.76 (s, 1H), 7.46 (q, *J* = 8.0 Hz, 2H), 7.11 (s, 1H), 6.55 (d, *J* = 6.4 Hz, 1H), 5.76 (s, 2H), 4.36 (t, *J* = 4.8 Hz, 2H), 3.64 (t, *J* = 5.2 Hz, 2H), 3.21 (s, 3H), 2.89 - 2.79 (m, 2H), 2.72 - 2.62 (m, 1H), 2.48 - 2.40 (m, 1H), 2.38 - 2.27 (m, 1H), 2.26 - 2.16 (m, 1H), 2.11 - 1.99 (m, 1H), 1.96 - 1.89 (m,

1H), 1.48 - 1.38 (m, 1H).

**Example 21 Synthesis of 2-((4-(2-((4-chloro-2-fluorobenzofuran-7-yl)methoxy)-5-fluoropyrimidin-4-yl)cyclohex-3-en-1-yl)methyl)-1-(((*S*)-oxetan-2-yl)methyl)-1*H*-benzo[*d*]imidazole-6-carboxylic acid (compound 21)**

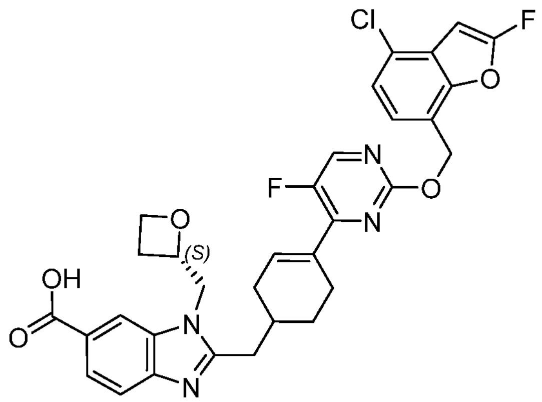

**Step 1: Synthesis of 5-fluoro-4-thioxo-3,4-dihydropyrimidin-2(1*H*)-one**

5-Fluoropyrimidine-2,4(1*H*,3*H*)-dione (10 g, 76.9 mmol) was dissolved in pyridine (100 mL), then Lawson's reagent (17.1 g, 42.3 mmol) was added, it was displaced with argon for three times and the reaction was heated up to 140 °C overnight. It was concentrated under reduced pressure and the residue was purified by column chromatography eluted with EtOAc:DCM (v/v)=1:10 to give a yellow solid (4.8 g, 42.8%).

LC-MS (ESI): $[M+H]^+$=147.2.

**Step 2: Synthesis of 4-chloro-5-fluoropyrimidin-2-ol**

5-fluoro-4-thioxo-3,4-dihydropyrimidin-2(1*H*)-one (4.0 g, 27.37 mmol) was dissolved in acetonitrile (40 mL). Sulfoxide chloride (20 mL) was added and stirred overnight at room temperature. Concentrated under reduced pressure, dichloromethane (20 mL) was added and stirred, filtered to give a yellow solid (4.0 g, 88.6%).

LC-MS (ESI): $[M+H]^+$=149.1.

**Step 3: Synthesis of 4-chloro-5-fluoro-2-(methoxymethoxy)pyrimidine**

4-Chloro-5-fluoropyrimidin-2-ol (4.0 g, 26.9 mmol) was dissolved in *N*,*N*-dimethylformamide (40 mL), triethylamine (8.15 g, 80.7 mmol) was added, and cooled to 0 °C. Bromo(methoxy)methane (4.4 g, 35.0 mmol) was added and stirred at 0 °C for 1 hour. Concentrated under reduced pressure and the residue was purified by column chromatography eluted with EA:PE (v/v)=1:4 to give a colorless oil (800 mg, 9.84%).

$^1$HNMR (400 MHz, $CDCl_3$) $\delta$ 8.30 (s, 1H), 5.45 (s, 2H), 3.50 (s, 3H).

**Step 4: Synthesis of ethyl 2-(4-(5-fluoro-2-(methoxymethoxy)pyrimidin-4-yl)cyclohex-3-en-1-yl)acetate**

4-Chloro-5-fluoro-2-(methoxymethoxy)pyrimidine (0.8 g, 4.15 mmol), ethyl 2-(4-(4,4,5,5-tetramethyl-1,3,2-dioxaborolan-2-yl)cyclohex-3-en-1-yl)acetate (1.59 g, 5.4 mmol) was added to the mixture of 1,4-dioxane (10 mL) and water (2.5 mL). Potassium carbonate (1.72 g, 12.46 mmol) was added, it was replaced with nitrogen for three times, $Pd(dppf)Cl_2$ (0.3 g, 0.42 mmol) was added, it was replaced with nitrogen for three times again, and stirred at 85 °C overnight. It was concentrated under reduced pressure and the residue was purified by column chromatography eluted with EtOAc:PE (v/v) =1:8 to give a colorless oil (900 mg, 66.8%).

LC-MS (ESI): $[M+H]^+$=325.3.

**StepS: Synthesis of 2-(4-(5-fluoro-2-(methoxymethoxy)pyrimidin-4-yl)cyclohex-3-en-1-yl)ethan-1-ol**

Ethyl 2-(4-(5-fluoro-2-(methoxymethoxy)pyrimidin-4-yl)cyclohex-3-en-1-yl)acetate (900 mg, 2.77 mmol) was added to tetrahydrofuran (40 mL) and stirred at -78 °C for five minutes. DIBAL-H (10 ml, 8.31 mmol) was added and stirred

at -78 °C for one hour. Quenched with saturated ammonium chloride (10 mL), extracted with ethyl acetate (30 mL × 3), the combined organic phases were washed with saturated brine, dried with anhydrous sodium sulfate, concentrated under reduced pressure, and the residue was purified by column chromatography eluted with EtOAc:PE (v/v)=1:3 to give a colorless oil (513 mg, 65.5%).

LC-MS (ESI): $[M+H]^+$=283.3.

**Step 6: Synthesis of 2-(4-(5-fluoro-2-(methoxymethoxy)pyrimidin-4-yl)cyclohex-3-en-1-yl)acetaldehyde**

2-(4-(5-fluoro-2-(methoxymethoxy)pyrimidin-4-yl)cyclohex-3-en-1-yl)ethan-1-ol (513 mg, 1.8 mmol) was added to dichloromethane (10 mL), and Desmartin's Reagent (1.15 g, 2.7 mmol) was added and stirred in an ice bath for 1 h. A large amound of solid precipitated. Saturated sodium bicarbonate (10 mL) was added, filtered with diatomaceous earth, concentrated under reduced pressure, and the residue was purified by column chromatography eluted with EtOAc:PE (v/v)=1:6 to give a colorless oil (200 mg, 40.0%).

LC-MS (ESI): $[M+H]^+$=281.3.

**Step 7: Synthesis of methyl 2-((4-(5-fluoro-2-hydroxypyrimidin-4-yl)cyclohex-3-en-1-yl)methyl)-1-(((*S*)-oxetan-2-yl)methyl)-1*H*-benzo[*d*]imidazole-6-carboxylate**

2-(4-(5-fluoro-2-(methoxymethoxy)pyrimidin-4-yl)cyclohex-3-en-1-yl)acetaldehyde (200 mg, 0.71 mmol), methyl (S)-4-amino-3-((oxetan-2-ylmethyl)amino)benzoate (169 mg, 0.71 mmol) were added to acetic acid (5 mL) and stirred at 60°C for 2 hours. Concentrated under reduced pressure and the residue was purified by column chromatography eluted with DCM:MeOH (v/v)=20:1 to give a light brown solid (198 mg, 61.3%).

LC-MS (ESI): $[M+H]^+$=453.5.

**Step 8: Synthesis of methyl 2-((4-(2-((4-chloro-2-fluorobenzofuran-7-yl)methoxy)-5-fluoropyrimidin-4-yl)cyclohex-3-en-1-yl)methyl)-1-(((*S*)-oxetan-2-yl)methyl)-1*H*-benzo[*d*]imidazole-6-carboxylate**

Methyl 2-((4-(5 -fluoro-2-hydroxypyrimidin-4-yl)cyclohex-3-en-1 -yl)methyl)-1 -(((S)-oxetan-2-yl)methyl)-1*H*-benzo[*d*]imidazole-6-carboxylate (110 mg, 0.24 mmol), 7-(bromomethyl)-4-chloro-2-fluoro-1-benzofuran (77 mg, 0.29 mmol) was added to *N*,*N*-dimethylformamide (5 mL). DIPEA (126 mg, 0.97 mmol) was added and stirred at 60°C for 1 hour. Water (5 mL) was added and extracted with ethyl acetate (15 mL × 3), the combined organic phases were washed once with saturated brine, dried with anhydrous sodium sulfate, and the residue was purified by column chromatography eluted with DCM:MeOH (v/v)=20:1 to give a white solid (15 mg, 9.7%).

LC-MS (ESI): $[M+H]^+$=635.4.

**Step 9: Synthesis of 2-((4-(2-((4-chloro-2-fluorobenzofuran-7-yl)methoxy)-5-fluoropyrimidin-4-yl)cyclohex-3-en-1-yl)methyl)-1-(((*S*)-oxetan-2-yl)methyl)-1*H*-benzo[*d*]imidazole-6-carboxylic acid**

Methyl 2-((4-(2-((4-chloro-2-fluorobenzofuran-7-yl)methoxy)-5-fluoropyrimidin-4-yl)cyclohex-3-en-1-yl) methyl)-1-(((*S*)-oxetan-2-yl)methyl)-1*H*-benzo[*d*]imidazole-6-carboxylate (15 mg, 0.02 mmol) was dissolved in the mixture of dioxane (1 mL) and water ( 0.25 mL). Lithium hydroxide (0.5 mg, 0.12 mmol) was added and stirred for 2 h at room temperature. The pH was adjusted to 6-7 with acetic acid, concentrated under reduced pressure, and the residue was purified by Prep-HPLC eluted with $CH_3CN$: $H_2O$ (v/v)=50% to give a white solid (2.5 mg, 16.9%).

LC-MS (ESI): $[M+H]^+$=621.3;
$^1$HNMR (400 MHz, DMSO-$d_6$) δ 8.60 (d, *J* = 3.2 Hz, 1H), 8.20 (s, 1H), 7.79 (d, *J* = 8.3 Hz, 1H), 7.62 (d, *J* = 8.3 Hz, 1H), 7.45 - 7.40 (m, 2H), 6.84 (s, 1H), 6.54 (d, *J* = 6.3 Hz, 1H), 5.58 (s, 2H), 5.07 - 4.97 (m, 1H), 4.67 - 4.27 (m, 4H), 3.04 - 2.92 (m, 2H), 2.75 - 2.53 (m, 3H), 2.45 - 2.31 (m, 3H), 2.20 - 1.92 (m, 2H), 1.55 - 4.13 (m, 1H).

**Example 22 Synthesis of 2-((4-(2-((4-chloro-2-fluorobenzofuran-7-yl)methoxy)thiazol-4-yl)cyclohex-3-en-1-yl) methyl)-3-(((*S*)-oxetan-2-yl)methyl)-3*H*-imidazo[4,5-*b*]pyridine-5-carboxylic acid (compound 22)**

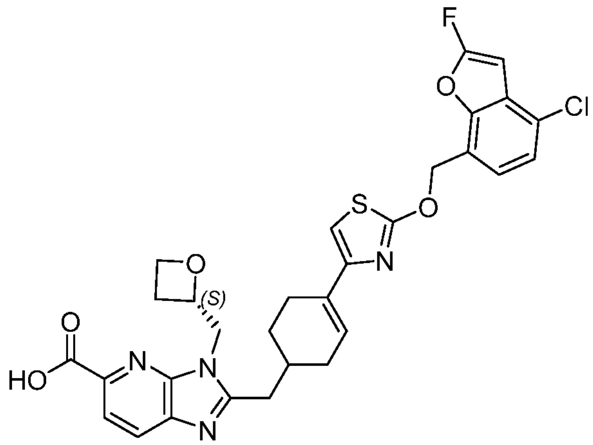

**Step 1: Synthesis of methyl 2-((4-(2-hydroxythiazol-4-yl)cyclohex-3-en-1-yl)methyl)-3-(((*S*)-oxetan-2-yl)methyl)-3*H*-imidazo[4,5-*b*]pyridine-5-carboxylate**

2-(4-(2-((4-methoxybenzyl)oxy)thiazol-4-yl)cyclohex-3-en-1-yl)acetaldehyde (942 mg, 2.75 mmol) (synthesis method refer to step 9 of example 14), methyl (S)-5-amino-6-((oxetan-2-ylmethyl)amino)picolinate (652 mg, 2.75 mmol) were added to acetic acid (8 mL) and reacted at 60 °C for 2 hours. The solvent was removed under reduced pressure and the residue was purified by column chromatography eluted with DCM:MeOH (v/v)=20:1 to give a light yellow solid (417 mg, 34.5%).

LC-MS (ESI): $[M+H]^+$=441.1.

**Step 2: Synthesis of methyl 2-((4-(2-((4-chloro-2-fluorobenzofuran-7-yl)methoxy)thiazol-4-yl)cyclohex-3-en-1-yl)methyl)-3-(((*S*)-oxetan-2-yl)methyl)-3*H*-imidazo[4,5-*b*]pyridine-5-carboxylate**

Methyl 2-((4-(2-hydroxythiazol-4-yl)cyclohex-3-en-1-yl)methyl)-3-(((*S*)-oxetan-2-yl)methyl)-3*H*-imidazo[4,5-*b*]pyridine-5-carboxylate (417 mg, 0.95 mmol), 7-(bromomethyl)-4-chloro-2-fluorobenzofuran (250 mg, 0.95 mmol), potassium carbonate ( 262 mg, 1.9 mmol) were added to *N*,*N*-dimethylformamide (5 mL) and the reaction was carried out at 60 °C for 2 hours. Concentrated under reduced pressure, water (10 mL) was added, extracted with ethyl acetate (15 mL×2), the combined organic phases were dried with anhydrous sodium sulfate, concentrated under reduced pressure, and the residue was purified by column chromatography eluted with EtOAc:PE (v/v)=1:1 to give a light yellow solid (120 mg, 20.3%).

LC-MS (ESI): $[M+1]^+$=623.1;
$^1$HNMR (400 MHz, DMSO-$d_6$) δ 8.14 (d, *J* = 4.0 Hz, 1H), 8.00 (d, *J* = 4.0 Hz, 1H), 7.52 - 7.42 (m, 2H), 6.80 (s, 1H), 6.57 - 6.50 (m, 2H), 5.67 (s, 2H), 5.15 - 5.06 (m, 1H), 4.71 - 4.60 (m, 1H), 4.57 - 4.42 (m, 2H), 4.36 - 4.27 (m, 1H), 3.91 (s, 3H), 3.14 - 3.03 (m, 2H), 2.75 - 2.64 (m, 1H), 2.47 - 1.90 (m, 8H).

**Step 3: Synthesis of 2-((4-(2-((4-chloro-2-fluorobenzofuran-7-yl)methoxy)thiazol-4-yl)cyclohex-3-en-1-yl)methyl)-3-(((*S*)-oxetan-2-yl)methyl)-3*H*-imidazo[4,5-*b*]pyridine-5-carboxylic acid**

Methyl 2-((4-(2-((4-chloro-2-fluorobenzofuran-7-yl)methoxy)thiazol-4-yl)cyclohex-3-en-1-yl)methyl)-3-(((*S*)-oxetan-2-yl)methyl)-3*H*-imidazo[4,5-*b*]pyridine-5-carboxylate (120 mg, 0.19 mmol) and lithium hydroxide (22.8 mg, 0.95 mmol) were added to the mixture of 1,4-dioxane (8 mL) and water (2 mL), and the reaction was carried out at 40 °C for 1 hour. It was cooled in an ice bath, the pH was adjusted to 6 with acetic acid, concentrated under reduced pressure and the residue was purified by pre-HPLC eluted with acetonitrile:water (v/v)=80% to give a white solid (25 mg, 26.1%).

LC-MS (ESI): $[M+H]^+$=609.1;
$^1$HNMR (400 MHz, DMSO-$d_6$) δ 12.96 (s, 1H), 8.11 (d, *J* = 4.0 Hz, 1H), 7.98 (d, *J* = 4.0 Hz, 1H), 7.50 - 7.43 (m, 2H), 6.80 (s, 1H), 6.57 - 6.50 (m, 2H), 5.67 (s, 2H), 5.16 - 5.06 (m, 1H), 4.71 - 4.61 (m, 1H), 4.58 - 4.42 (m, 2H), 4.36 - 4.28 (m, 1H), 3.14 - 3.10 (m, 2H), 2.75 - 2.64 (m, 1H), 2.48 - 2.20 (m, 5H), 2.10 - 1.90 (m, 2H), 1.57 - 1.41 (m, 1H).

**Example 23 Synthesis of 2-((3'-((4-chloro-2-fluorobenzofuran-7-yl)methoxy)-2,3,4,5-tetrahydro-[1,1'-biphenyl]-4-yl)methyl)-1-(((*S*)-oxetan-2-yl)methyl)-1*H*-benzo[*d*]imidazole-6-carboxylic acid (compound 23)**

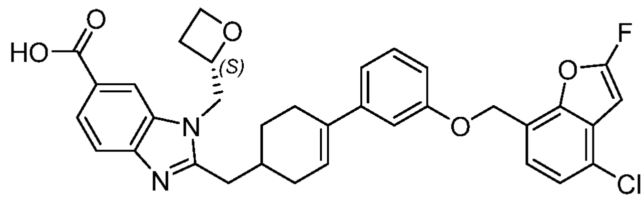

**Step 1: Synthesis of 1-bromo-3-(methoxymethoxy)benzene**

3-Bromophenol (1.0 g, 5.78 mmol) was added to *N*,*N*-dimethylformamide (10 mL) and cooled in an ice bath, sodium hydride (60%, 350 mg, 14.45 mmol) was added and stirred in an ice bath for 10 min. Bromo(methoxy)methane (1.08 g, 8.67 mmol) was added and stirred at room temperature for 1 hour. Water (10 mL) was add, extracted with ethyl acetate (20 mL×3), the combined organic phaseswere washed with saturated brine, dried with anhydrous sodium sulfate, concentrated under reduced pressure, and the residue was purified by column chromatography eluted with EtOAc:PE (v/v)=1:20 to give a colorless oil (1.2 g, 96.0%).

$^1$H NMR (400 MHz, $CDCl_3$) δ 7.14 (dd, *J* = 2.5, 1.3 Hz, 1H), 7.08 - 7.04 (m, 2H), 6.89 (m, 1H), 5.08 (s, 2H), 3.40 (s, 3H).

**Step 2: Synthesis of ethyl 2-(3'-(methoxymethoxy)-2,3,4,5-tetrahydro-[1,1'-biphenyl]-4-yl)acetate**

1-Bromo-3-(methoxymethoxy)benzene (1.2 g, 5.53 mmol), ethyl 2-(4-(4,4,5,5-tetramethyl-1,3,2-dioxaborolan-2-yl)cyclohex-3-en-1-yl)acetate (2.44 g, 8.29 mmol) were added to the mixture of 1,4-dioxane (20 mL) and water (5 mL). Potassium carbonate (2.29 g, 16.59 mmol) was added, it was replaced with nitrogen for three times, $Pd(PPh_3)_4$ (192 mg, 0.0166 mmol) was added, it was replaced with nitrogen for three times again, and stirred at 85 °C overnight. Concentrated under reduced pressure and the residue was purified by column chromatography eluted with EtOAc:PE (v/v)=1:15 to give a colorless oil (1.03 g, 61.4%).

$^1$HNMR (400 MHz, $CDCl_3$) δ 7.15 (t, *J* = 7.9 Hz, 1H), 6.97 (dt, *J* = 8.8, 4.7 Hz, 2H), 6.84 (dd, *J* = 8.1, 1.6 Hz, 1H), 6.02 - 5.98 (m, 1H), 5.11 (s, 2H), 4.09 (q, *J* = 7.1 Hz, 2H), 3.42 (s, 3H), 2.43 - 2.36 (m, 2H), 2.36 - 2.24 (m, 3H), 2.16 - 2.04 (m, 1H), 1.92 - 1.81 (m, 2H), 1.43 - 1.36 (m, 1H), 1.21 (t, *J* = 7.1 Hz, 3H).

**Step 3: Synthesis of 2-(3'-(methoxymethoxy)-2,3,4,5-tetrahydro-[1,1'-biphenyl]-4-yl)ethan-1-ol**

Ethyl 2-(3'-(methoxymethoxy)-2,3,4,5-tetrahydro-[1,1'-biphenylyl]-4-yl)acetate (1.03 g, 3.39 mmol) was added to tetrahydrofuran (20 mL) and stirred at 0 °C for five minutes. Lithium tetrahydroaluminum (0.64 g, 16.95 mmol) was added and stirred at 0 °C for two hours. Quenched with water (10 mL), extracted with ethyl acetate (20 mL×3), the combined organic phases were washed with saturated brine, dried with anhydrous sodium sulfate, concentrated under reduced pressure, and the residue was purified by column chromatography eluted with EtOAc:PE (v/v)=1:5 to give a colorless oil (0.57 g, 64.2%).

LC-MS (ESI): $[M+H]^+$=263.4.

**Step 4: Synthesis of 2-(3'-(methoxymethoxy)-2,3,4,5-tetrahydro-[1,1'-biphenyl]-4-yl)acetaldehyde**

2-(3'-(Methoxymethoxy)-2,3,4,5-tetrahydro-[1,1'-biphenylyl]-4-yl)ethan-1-ol (570 mg, 2.2 mmol) was added to dichloromethane (20 mL), Desmartin's reagent (1.40 g, 3.3 mmol) was added and stirred in an ice bath with for 1 h. A large amount of solid precipitated. Water (5 mL) was added, filtered and concentrated under reduced pressure, and the residue was purified by column chromatography eluted with EtOAc:PE (v/v)=1:5 to give a clorless oil (250 mg,44.2).

LC-MS (ESI): $[M+H]^+$=261.3.

**Step 5: Synthesis of methyl 2-((3'-(methoxymethoxy)-2,3,4,5-tetrahydro-[1,1'-biphenyl]-4-yl)methyl)-1-(((*S*)-oxetan-2-yl)methyl)-1*H*-benzo[*d*]imidazole-6-carboxylate**

2-(3'-(methoxymethoxy)-2,3,4,5-tetrahydro-[1,1'-biphenyl]-4-yl)acetaldehyde (250 mg, 0.96 mmol), methyl

(S)-4-amino-3-((oxetan-2-ylmethyl)amino)benzoate (250 mg, 1.05 mmol) were added to acetic acid (10 mL), and the mixture was stirred at 60 °C for 2 hours. It was concentrated under reduced pressure and the residue was purified by column chromatography eluted with (v/v)=1:1 to give a light brown solid (200 mg, 43.7%).

LC-MS (ESI): $[M+H]^+$=477.6.

**Step 6: Synthesis of methyl 2-((3'-hydroxy-2,3,4,5-tetrahydro-[1,1'-biphenyl]-4-yl)methyl)-1-(((*S*)-oxetan-2-yl)methyl)-1*H*-benzo[*d*]imidazole-6-carboxylate**

Methyl 2-((3'-(methoxymethoxy)-2,3,4,5-tetrahydro-[1,1'-biphenyl|-4-yl)methyl)-1-(((*S*)-oxetan-2-yl)methyl)-1*H*-benzo[*d*]imidazole-6-carboxylate (200 mg, 0.42 mmol) was added to dichloromethane (10 mL), and cooled to -10 °C. Trifluoroacetic acid (10 mL) was added and stirred at -10°C for 2 hours. Concentrated under reduced pressure and the residue was purified by column chromatography eluted with DCM:MeOH (v/v)=20:1 to give a light brown solid (50 mg, 27.6%).

LC-MS (ESI): $[M+H]^+$=433.4;

$^1$HNMR (400 MHz, DMSO-$d_6$) δ 9.28 (s, 1H), 8.24 (s, 1H), 7.81 (d, *J* = 8.4 Hz, 1H), 7.65 (d, *J* = 8.4 Hz, 1H), 7.09 (t, *J* = 7.9 Hz, 1H), 6.83 (d, *J* = 7.8 Hz, 1H), 6.77 (s, 1H), 6.62 (d, *J* = 8.0 Hz, 1H), 6.06 (s, 1H), 5.02 (d, *J* = 7.1 Hz, 1H), 4.66 (dd, *J* = 15.5, 7.4 Hz, 1H), 4.53 (d, *J* = 13.3 Hz, 1H), 4.46 (dd, *J* = 13.8, 7.4 Hz, 1H), 4.34 - 4.26 (m, 1H), 3.87 (s, 3H), 3.17 (s, 1H), 2.98 (dd, *J* = 12.6, 6.6 Hz, 2H), 2.70 (dd, *J* = 16.6, 7.4 Hz, 1H), 2.38 (d, *J* = 9.8 Hz, 4H), 1.99 (s, 2H), 1.50 (s, 1H).

**Step 7: Synthesis of methyl 2-((3'-((4-chloro-2-fluorobenzofuran-7-yl)methoxy)-2,3,4,5-tetrahydro-[1,1'-biphenyl]-4-yl)methyl)-1-(((*S*)-oxetan-2-yl)methyl)-1*H*-benzo[*d*]imidazole-6-carboxylate**

Methyl 2-((3 '-hydroxy-2,3,4,5 -tetrahydro- [1,1'-biphenyl] -4-yl)methyl)-1-(((*S*)-oxetan-2-yl)methyl)-1*H*-benzo[*d*]imidazole-6-carboxylate (50 mg, 0.12 mmol), 7-(bromomethyl)-4-chloro-2-fluoro-1-benzofuran (46 mg, 0.17 mmol) were added to *N*,*N*-dimethylformamide (3 mL), potassium carbonate (50 mg, 0.36 mmol) was added and stirred at 60 °C for one hour. Water (5 mL) was added, extracted with ethyl acetate (10 mL×3), the combined organic phases were washed with saturated brine, dried with anhydrous sodium sulfate, and the residue was purified by column chromatography eluted with DCM:MeOH (v/v)=10:1 to give an off-white solid (55 mg, 77.5%).

LC-MS (ESI): $[M+H]^+$=615.3.

**Step 8: Synthesis of 2-((3'-((4-chloro-2-fluorobenzofuran-7-yl)methoxy)-2,3,4,5-tetrahydro-[1,1'-biphenyl]-4-yl)methyl)-1-(((*S*)-oxetan-2-yl)methyl)-1*H*-benzo[*d*]imidazole-6-carboxylic acid**

Methyl 2-((3 '-((4-chloro-2-fluorobenzofuran-7-yl)methoxy)-2,3,4,5-tetrahydro-[1,1'-biphenyl]-4-yl)methyl)-1-(((*S*)-oxetan-2-yl)methyl)-1*H*-benzo[*d*]imidazole-6-carboxylate (55 mg, 0.09 mmol) was added to the mixture of dioxane (2 mL) and water (0.5 mL). Lithium hydroxide (11 mg, 0.45 mmol) was added and stirred at room temperature for 4 hours. The pH was adjusted to 6-7 with hydrochloric acid (0.5 N), extracted with ethyl acetate (5 mL×3), and combined the organic phases were washed with saturated saline and dried with anhydrous sodium sulfate. Concentrated under reduced pressure and the residue was purified by Prep-HPLC eluted with $CH_3CN$: $H_2O$ (v/v) = 50% to give a white solid (19.2 mg, 35.8%).

LC-MS (ESI): $[M+H]^+$=601.3;

$^1$HNMR (400 MHz, DMSO-$d_6$) δ 12.75 (s, 1H), 8.21 (s, 1H), 7.80 (dd, *J* = 8.4, 1.3 Hz, 1H), 7.62 (d, *J* = 8.4 Hz, 1H), 7.45 (q, *J* = 8.2 Hz, 2H), 7.24 (t, *J* = 7.9 Hz, 1H), 7.10 - 6.99 (m, 2H), 6.92 (dd, *J* = 8.1, 1.9 Hz, 1H), 6.55 (d, *J* = 6.4 Hz, 1H), 6.15 (s, 1H), 5.35 (s, 2H), 5.03 (d, *J* = 6.9 Hz, 1H), 4.65 (dd, *J*= 15.5, 7.1 Hz, 1H), 4.56 - 4.40 (m, 2H), 4.31 (dt, *J* = 12.0, 5.9 Hz, 1H), 3.07 - 2.91 (m, 2H), 2.70 (dt, *J* = 16.3, 8.0 Hz, 1H), 2.47 - 2.24 (m, 5H), 2.10 - 1.93 (m, 2H), 1.52 (s, 1H).

**Example 24 Synthesis of 2-((4-(6-((4-chloro-2-fluorobenzofuran-7-yl)methoxy)-5-fluoropyridin-2-yl)cyclohex-3-en-1-yl)methyl)-1-(((*S*)-oxetan-2-yl)methyl)-1*H*-benzo[*d*]imidazole-6-carboxylic acid (compound 24)**

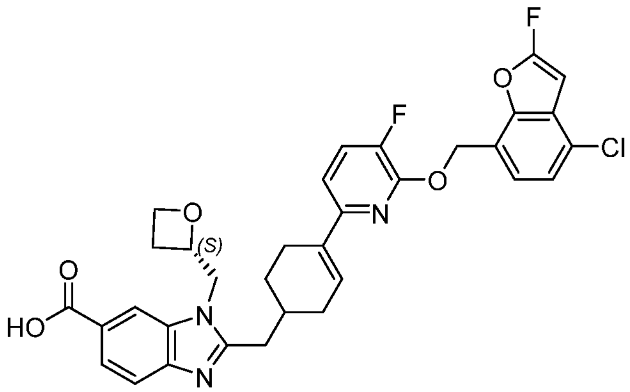

**Step 1: Synthesis of 2-chloro-5-fluoropyridine 1-oxide**

2-Chloro-5-fluoropyridine (10.0 g, 76.03 mmol) was added to trifluoroacetic acid (75 mL), hydrogen peroxide (43 mL, 1428.70 mmol) was added dropwise under an ice bath, and the reaction was carried out at 70 °C overnight. The reaction mixture was cooled to room temperature, concentrated under reduced pressure, the pH was adjusted to 6-7 with saturated sodium bicarbonate solution, extracted with dichloromethane (100 mL×3), the combined organic phases ware dried with anhydrous sodium sulfate, filtered, concentrated under reduced pressure to give a light yellow product (7.9 g, 70.4%).

LC-MS (ESI): $[M+H]^+$ =148.2;
$^1$HNMR (400 MHz, $CDCl_3$) δ 8.26 - 8.24 (m, 1H), 7.43 - 7.39 (m, 1H), 7.01 - 6.97 (m, 1H).

**Step 2: Synthesis of 6-chloro-3-fluoropyridin-2-ol**

2-Chloro-5-fluoropyridine 1-oxide (7.9 g, 53.74 mmol), triethylamine (6.79 mL, 53.53 mmol) were added to tetrahydrofuran (80 mL) and stirred in an ice bath for 0.5 h. Trifluoroacetic anhydride (20.54 mL, 150.42 mmol) was slowly added dropwise and stirred at room temperature overnight. Quenched with saturated sodium hydroxide solution, extracted with ethyl acetate (100 mL×3), the combined organic phases were were dried with anhydrous sodium sulfate, concentrated under reduced pressure, and the residue was purified by column chromatography eluted with PE:EA (v/v) =50% to give a light yellow solid (3.2 g, 40.0%).

LC-MS (ESI): $[M+H]^+$=148.2;
$^1$HNMR (400 MHz, DMSO-$d_6$) δ 12.43 (s, 1H), 7.65 (t, *J* = 8.0 Hz, 1H), 6.94 - 6.86 (m, 1H).

**Step 3: Synthesis of ethyl 2-(4-(5-fluoro-6-hydroxypyridin-2-yl)cyclohex-3-en-1-yl)acetate**

6-Chloro-3-fluoropyridin-2-ol (1.2 g, 8.13 mmol), ethyl 2-(4-(4,4,5,5-tetramethyl-1,3,2-dioxaborolan-2-yl)cyclohex-3-en-1-yl)acetate (2.87 g, 9.76 mmol), palladium bis(tri-tert-butylphosphine) (4 mg, 0.01 mmol), potassium carbonate (2.25 g, 16.26 mmol) were added to the mixture of 1,4-dioxane (35 mL) and water (9 mL), it was displaced with nitrogen for three times, and the reaction solution was stirred at 85 °C overnight. The reaction mixture was filtered with diatomaceous earth, concentrated under reduced pressure, and the residue was purified by column chromatography eluted with PE:EA (v/v)=1:1 to give a light yellow solid (250 mg, 11.0%).

LC-MS (ESI): $[M+H]^+$ =280.2;
$^1$HNMR (400 MHz, DMSO-$d_6$) δ 11.77 (s, 1H), 7.37 - 7.29 (m, 1H), 6.39 - 6.32 (m, 1H), 6.17 - 6.07 (m, 1H), 4.13 - 4.03 (m, 2H), 2.38 - 2.23 (m, 5H), 2.03 - 1.76 (m, 3H), 1.40 - 1.27 (m, 1H), 1.19 (t, *J* = 8.0 Hz, 3H).

**Step 4: Synthesis of 3-fluoro-6-(4-(2-hydroxyethyl)cyclohex-1-en-1-yl)pyridin-2-ol**

Ethyl 2-(4-(5-fluoro-6-hydroxypyridin-2-yl)cyclohex-3-en-1-yl)acetate (220 mg, 0.79 mmol) was added to tetrahydrofuran (20 mL), and lithium aluminum hydride (150 mg, 3.95 mmol) was added in an ice bath and stirred for 2 hours. Quenched with water dropwise slowly and filtered with diatomaceous earth, concentrated under reduced pressure, and the residue was purified by column chromatography eluted with DCM:MeOH (v/v)=20:1 to give a light yellow solid (140 mg, 74.9%).

LC-MS (ESI): $[M+H]^+$=238.1;
$^1$HNMR (400 MHz, DMSO-$d_6$) δ 11.74 (s, 1H), 7.39 - 7.28 (m, 1H), 6.43 - 6.34 (m, 1H), 6.17 - 6.04 (m, 1H), 4.40 - 4.33 (m, 1H), 3.54 - 3.42 (m, 2H), 2.38 - 2.19 (m, 3H), 1.90 - 1.57 (m, 3H), 1.51 - 1.35 (m, 2H), 1.28 - 1.23 (m, 1H).

**Step 5: Synthesis of 2-(4-(5-fluoro-6-hydroxypyridin-2-yl)cyclohex-3-en-1-yl)acetaldehyde**

3-fluoro-6-(4-(2-hydroxyethyl)cyclohex-1-en-1-yl)pyridin-2-ol (120 mg, 0.51 mmol) was added to dimethylsulfoxide (4 mL), and Dess-Martin oxidizer (643 mg, 1.52 mmol) was added in an ice bath and stirred for 2 h at room temperature. Water (20 ml) was added, extracted with ethyl acetate (10 mL × 3), the combined organic phases were dried with anhydrous sodium sulfate, and concentrated under reduced pressure to give a light yellow solid (350 mg).
LC-MS (ESI): $[M+H]^+$ =236.1.

**Step 6: Synthesis of methyl 2-((4-(5-fluoro-6-hydroxypyridin-2-yl)cyclohex-3-en-1-yl)methyl)-1-(((*S*)-oxetan-2-yl)methyl)-1*H*-benzo[*d*]imidazole-6-carboxylate**

2-(4-(5-fluoro-6-hydroxypyridin-2-yl)cyclohex-3-en-1-yl)acetaldehyde (350 mg, crude), methyl (*S*)-4-amino-3-((oxetan-2-ylmethyl)amino)benzoate (70 mg, 0.30 mmol) was added to acetic acid (3 mL), and the reaction mixture was stirred at 60 °C for 2 hours. The reaction mixture was concentrated under reduced pressure and the residue was purified by column chromatography eluted with DCM:MeOH (v/v)=20:1 to give a brown solid (40 mg, 29.8%).

LC-MS (ESI): $[M+1]^+$=452.2;
$^1$HNMR (400 MHz, DMSO-$d_6$) δ 11.75 (s, 1H), 7.91 - 7.84 (m, 2H), 7.83 - 7.78 (m, 1H), 7.14 - 7.06 (m, 2H), 6.15 - 6.09 (m, 1H), 5.06 - 4.97 (m, 1H), 4.72 - 4.60 (m, 1H), 4.59 - 4.40 (m, 2H), 4.35 - 4.25 (m, 1H), 3.87 (s, 3H), 3.01 - 2.92 (m, 2H), 2.75 - 2.63 (m, 1H), 2.46 - 2.20 (m, 5H), 2.08 - 1.88 (m, 2H), 1.54 - 1.39 (m, 1H).

**Step 7: Synthesis of methyl 2-((4-(6-((4-chloro-2-fluorobenzofuran-7-yl)methoxy)-5-fluoropyridin-2-yl)cyclohex-3-en-1-yl)methyl)-1-(((*S*)-oxetan-2-yl)methyl)-1*H*-benzo[*d*]imidazole-6-carboxylate**

Methyl 2-((4-(5-fhioro-6-hydroxypyridin-2-yl)cyclohex-3-en-1-yl)methyl)-1-(((*S*)-oxetan-2-yl)methyl)-1*H*-benzo[*d*]imidazole-6-carboxylate (35 mg, 0.08 mmol), 7-(bromomethyl)-4-chloro-2-fluoro-1-benzofuran (41 mg, 0.16 mmol), potassium carbonate (21 mg, 0.15 mmol) were added to *N*,*N*-dimethylformamide (3 mL) and stirred at 60 °C for 2 hours. Concentrated under reduced pressure, water (10 mL) was added, extracted with ethyl acetate (10 mL×3), the combined organic phases were dried with anhydrous sodium sulfate, purified the residue was by column chromatography eluted with DCM:MeOH (v/v)=20:1 to give a brown solid (15 mg, 30.5%).
LC-MS (ESI): $[M+H]^+$=634.2.

**Step 8: Synthesis of 2-((4-(6-((4-chloro-2-fluorobenzofuran-7-yl)methoxy)-5-fluoropyridin-2-yl)cyclohex-3-en-1-yl)methyl)-1-(((*S*)-oxetan-2-yl)methyl)-1*H*-benzo[*d*]imidazole-6-carboxylic acid**

Methyl 2-((4-(6-((4-chloro-2-fluorobenzofuran-7-yl)methoxy)-5-fluoropyridin-2-yl)cyclohex-3-en-1-yl)methyl)-1-(((*S*)-oxetan-2-yl)methyl)-1*H*-benzo[*d*]imidazole-6-carboxylate (15 mg, 0.02 mmol), lithium hydroxide (3 mg, 0.13 mmol) were added to the mixture of 1,4-dioxane (2 mL) and water (0.5 mL), and stirred at 40 °C for 2 hours. Acetic acid was added dropwise until the mixture was weakly acidic. Concentrated under reduced pressure and the residue was purified by Prep-HPLC eluted with acetonitrile:water (v/v) = 90% to give a white solid (5 mg, 34.1%).

LC-MS (ESI): $[M+H]^+$=620.2;
$^1$HNMR (400 MHz, DMSO-$d_6$) δ 12.62 (s, 1H), 8.21 (s, 1H), 7.82 - 7.76 (m, 1H), 7.67 - 7.59 (m, 2H), 7.47 - 7.38 (m, 2H), 7.11- 7.05 (m, 1H), 6.66 (s, 1H), 6.53 (d, *J* = 8.0 Hz, 1H), 5.68 (s, 2H), 5.08- 4.98 (m, 1H), 4.70-4.60 (m, 1H), 4.57- 4.40 (m, 2H), 4.35- 4.25 (m, 1H), 3.05- 2.92 (m, 2H), 2.75- 2.62 (m, 1H), 2.44- 2.24 (m, 5H), 2.11- 1.91 (m, 2H), 1.56- 1.40 (m, 1H).

**Example 25 Synthesis of 2-((4-(6-((2,4-dichlorobenzofuran-7-yl)methoxy)pyridin-2-yl)cyclohex-3-en-1-yl)methyl)-1-(((*S*)-oxetan-2-yl)methyl)-1*H*-benzo[*d*]imidazole-6-carboxylic acid (compound 25)**

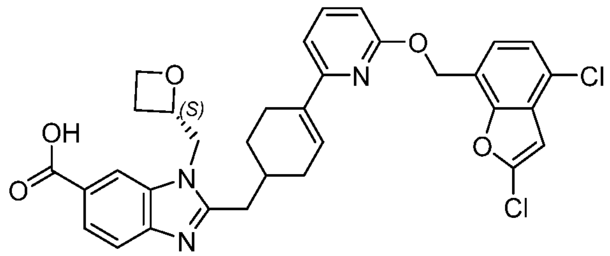

**Step 1: Synthesis of 4-chloro-2-(2,2-diethoxyethoxy)-1-methylbenzene**

5-Chloro-2-methylphenol (15 g, 105.2 mmol), 2-bromo-1,1-diethoxyethane (25 g, 126.9 mmol) and potassium carbonate (29.1 g, 210.4 mmol) were added to *N*,*N*-dimethylformamide (50 mL), and the reaction was carried out under nitrogen protection at 120 °C for 11 h. The reaction was cooled down to room temperature, and then water (100 mL) was added. Extracted with ethyl acetate (35 mL×3), the combined organic phases were washed with saturated saline (30 mL), the organic phase was dried with anhydrous sodium sulfate, concentrated under reduced pressure, and the residue was purified by column chromatography eluted with EtOAc:PE (v/v)=1% to give a light brown liquid (21.1 g, 77.5%).

$^1$HNMR (500 MHz, $CDCl_3$) δ 7.03 (d, *J* = 7.9 Hz, 1H), 6.84 (dd, *J* = 7.9, 2.0 Hz, 1H), 6.81 (d, *J* = 2.0 Hz, 1H), 4.84 (t, *J* = 5.2 Hz, 1H), 3.98 (d, *J* = 5.2 Hz, 2H), 3.81 - 3.74 (m, 2H), 3.68 - 3.62 (m, 2H), 2.18 (s, 3H), 1.25 (t, *J* = 7.0 Hz, 6H).

**Step 2: Synthesis of 4-chloro-7-methylbenzofuran**

4-Chloro-2-(2,2-diethoxyethoxy)-1-methylbenzene (21 g, 81.2 mmol) and polyphosphoric acid (16.6 g, 202.9 mmol) were added to 1,2-dichloroethane (50 mL), and the reaction was carried out at 85 °C under nitrogen protection overnight. Cooled to room temperature, the solvent was removed under reduced pressure, water (80 mL) was added, extracted with ethyl acetate (50 mL×2), the combined organic phases were dried with anhydrous sodium sulfate, concentrated under reduced pressure, and the residue was purified by column chromatography eluted with PE=100% to give a colorless liquid (9.2 g, 68.0%).

$^1$HNMR (500 MHz, $CDCl_3$) δ 7.63 (d, *J* = 2.3 Hz, 1H), 7.12 (d, *J* = 7.9 Hz, 1H), 7.00 (d, *J* = 7.9 Hz, 1H), 6.84 (d, *J* = 2.3 Hz, 1H), 2.48 (s, 3H).

**Step 3: Synthesis of 7-(bromomethyl)-4-chlorobenzofuran**

4-Chloro-7-methylbenzofuran (4.1 g, 24.61 mmol), azodiisobutyronitrile (810 mg, 4.92 mmol) and *N*-bromosuccinimide (5.26 g, 29.53 mmol) were added to 1,2-dichloroethane (30 mL), and reacted at 75 °C under nitrogen protection overnight. Cooled to room temperature, water (50 mL) was added, extracted with dichloromethane (15 mL×3), the combined organic phases were dried with anhydrous sodium sulfate, concentrated under reduced pressure, and the residue was purified by column chromatography eluted with PE=100% to give a colorless liquid (4.3 g, 71.2%).

$^1$HNMR (500 MHz, $CDCl_3$) δ 7.71 (s, 1H), 7.24 - 7.20 (m, 2H), 6.89 (s, 1H), 4.75 (s, 2H).

**Step 4: Synthesis of 7-(bromomethyl)-2,4-dichlorobenzofuran**

7-(Bromomethyl)-4-chlorobenzofuran (5.8 g, 23.63 mmol) and hexachloroethane (5.59 g, 23.63 mmol) were added to anhydrous tetrahydrofuran (100 mL), cooled under nitrogen protection at -50 °C, then tetrahydrofuran solution of lithium diisopropylammonium (2.0 mol/L, 20 mL, 40 mmol) was added and the reaction was carried out at this temperature overnight, water (300 mL) was added, extracted with ethyl acetate (50 mL×3), the combined organic phases were dried with anhydrous sodium sulfate, concentrated under reduced pressure, and the residue was purified by column chromatography eluted with PE(v)=100% to give a white solid (2.0 g, 30.2%).

$^1$HNMR (500 MHz, $CDCl_3$) δ 7.31 - 7.25 (m, 2H), 6.76 (s, 1H), 4.74 (s, 2H).

**Step 5: Synthesis of 2-bromo-6-(methoxymethoxy)pyridine**

6-Bromopyridin-2-ol (16 g, 92.0 mmol) was added to dichloromethane (160 mL), triethylamine (63.9 mL, 459.5 mmol) was added, and the mixture was stirred in an ice bath for 10 minutes. Bromo(methoxy)methane (23 g, 184.0 mmol) was added and stirred at room temperature for 2 hours. Diluted with water (30 mL), extracted with dichloromethane (30 mL×3), the combined organic phases were washed with saturated brine, dried with anhydrous sodium sulfate, concentrated under reduced pressure, and the residue was purified by column chromatography eluted with EtOAc:PE (v/v)=1:20 to give a colorless oil (5.2 g, 25.9%).

$^1$HNMR (400 MHz, DMSO-$d_6$) δ 7.72 (d, *J* = 7.8 Hz, 1H), 7.28 (d, *J* = 7.3 Hz, 1H), 6.93 (d, *J* = 7.9 Hz, 1H), 5.42 (s, 2H), 3.42 (s, 3H).

**Step 6: Synthesis of methyl 2-(4-(6-(methoxymethoxy)pyridin-2-yl)cyclohex-3-en-1-yl)acetate**

2-bromo-6-(methoxymethoxy)pyridine (5.2 g, 23.8 mmol), methyl 2-(4-(4,4,5,5-tetramethyl-1,3,2-dioxaborolan-2-yl)cyclohex-3-en-1-yl)acetate (7.68 g, 27.4 mmol) were added to the mixture of 1,4-dioxane (60 mL) and water (15 mL). Potassium carbonate (6.59 g, 47.7 mmol) was added, it was replaced with nitrogen for three times, Pd(dppf)$Cl_2$ (0.52 g, 0.72 mmol) was added, it was replaced with nitrogen for three times again, and stirred at 85 °C overnight. It was concentrated under reduced pressure and the residue was purified by column chromatography eluted with EtOAc:PE (v/v) =1:15 to give a colorless oil (4.0 g, 57.6%).

$^1$HNMR (400 MHz, $CDCl_3$) δ 7.48 (t, *J* = 7.8 Hz, 1H), 6.90 (d, *J* = 7.5 Hz, 1H), 6.68 (dd, *J* = 3.1, 1.9 Hz, 1H), 6.57 (d, *J* = 8.1 Hz, 1H), 5.49 (s, 2H), 3.62 (s, 3H), 3.45 (d, *J* = 3.2 Hz, 3H), 2.53 (dd, *J* = 16.5, 3.0 Hz, 1H), 2.46 - 2.31 (m, 2H), 2.28 (d, *J* = 7.2 Hz, 2H), 2.17 - 2.03 (m, 1H), 1.96 - 1.84 (m, 2H), 1.38 (m, 1H).

**Step 7: Synthesis of 2-(4-(6-(methoxymethoxy)pyridin-2-yl)cyclohex-3-en-1-yl)ethan-1-ol**

Methyl 2-(4-(6-(methoxymethoxy)pyridin-2-yl)cyclohex-3-en-1-yl)acetate (1.5 g, 5.2 mmol) was added to tetrahydrofuran (30 mL) and stirred at -20 °C for five minutes. Lithium tetrahydroaluminum (0.39 g, 10.3 mmol) was added and stirred at 0 °C for two hours. Quenched with water (10 mL), extracted with ethyl acetate (30 mL × 3), the combined organic phases were washed with saturated brine, dried with anhydrous sodium sulfate, concentrated under reduced pressure, and the residue was purified by column chromatography eluted with EtOAc:PE (v/v)=1:5 to give a colorless oil (1.3 g, 95.9%).

LC-MS (ESI): $[M+H]^+$=264.3.

**Step 8: Synthesis of 2-(4-(6-(methoxymethoxy)pyridin-2-yl)cyclohex-3-en-1-yl)acetaldehyde**

2-(4-(6-(methoxymethoxy)pyridin-2-yl)cyclohex-3-en-1-yl)ethan-1-ol (600 mg, 2.2 mmol) was added to dichloromethane (20 mL), Desmartin's Reagent (1.45 g, 3.4 mmol) was added and stirred in an ice bath for 3 h. A large amount of solid precipitated. Water (4 mL) was added, filtered and concentrated under reduced pressure, the residue was purified by column chromatography eluted with EtOAc:PE (v/v)=1:5 to give a colorless oil (260 mg, 43.7%).

LC-MS (ESI): $[M+H]^+$=262.1.

**Step 9: Synthesis of methyl 2-((4-(6-hydroxypyridin-2-yl)cyclohex-3-en-1-yl)methyl)-1-(((*S*)-oxetan-2-yl)methyl)-1*H*-benzo[*d*]imidazole-6-carboxylate**

2-(4-(6-(methoxymethoxy)pyridin-2-yl)cyclohex-3-en-1-yl)acetaldehyde (700 mg, 2.68 mmol) and methyl (S)-4-amino-3-((oxetan-2-ylmethyl)amino)benzoate (633 mg, 2.68 mmol) were added to acetic acid (10 mL), stirred at 60°C for 1 h. Concentrated under reduced pressure and the residue was purified by column chromatography eluted with DCM:MeOH (v/v)=20:1 to give a light green solid (420 mg, 36.2%).

LC-MS (ESI): $[M+H]^+$=434.6.

**Step 10: Synthesis of methyl 2-((4-(6-((2,4-dichlorobenzofuran-7-yl)methoxy)pyridin-2-yl)cyclohex-3-en-1-yl) methyl)-1-(((*S*)-oxetan-2-yl)methyl)-1*H*-benzo[*d*]imidazole-6-carboxylate**

Methyl 2-((4-(6-hydroxypyridin-2-yl)cyclohex-3-en-1-yl)methyl)-1-(((*S*)-oxetan-2-yl)methyl)-1*H*-benzo[*d*]imidazole-6-carboxylate (100 mg, 0.23 mmol), 7-(bromomethyl)-2,4-dichlorobenzofuran (98 mg, 0.35 mmol) were added to *N,N*- dimethylformamide (5 mL), potassium carbonate (159 mg, 1.15 mmol) was added and stirred at 60 °C for 1 hour. Diluted with water (15 mL), extracted with ethyl acetate (20 mL×3), the combined organic phases were washed with saturated brine, dried with anhydrous sodium sulfate, and the residue was purified by column chromatography eluted with EA:PE (v/v)=1:1 to give a light yellow solid (95 mg, 65.1%).

LC-MS (ESI): $[M+H]^+$=632.5.

**Step 11: Synthesis of 2-((4-(6-((2,4-dichlorobenzofuran-7-yl)methoxy)pyridin-2-yl)cyclohex-3-en-1-yl) methyl)-1-(((*S*)-oxetan-2-yl)methyl)-1*H*-benzo[*d*]imidazole-6-carboxylic acid**

Methyl 2-((4-(6-((2,4-dichlorobenzofuran-7-yl)methoxy)pyridin-2-yl)cyclohex-3-en-1-yl)methyl)-1-(((*S*)-oxetan-2-yl)methyl)-1*H*-benzo[*d*]imidazole-6-carboxylate (95 mg, 0.15 mmol) was added to the mixture of dioxane (5 mL) and water (1.25 mL). Lithium hydroxide (22 mg, 0.9 mmol) was added and stirred at room temperature for 4 hours. The pH was adjusted to 6-7 with acetic acid (0.2 mL), extracted with ethyl acetate (10 mL×3), and the combined organic phases were washed with saturated saline, and dried with anhydrous sodium sulfate. It was concentrated under reduced pressure and the residue was purified by Prep-HPLC eluted with $CH_3CN$: $H_2O$ (v/v)=50% to give a white solid (33.8 mg, 36.4%).

LC-MS (ESI): $[M+H]^+$=618.3;
$^1$HNMR (400 MHz, DMSO-$d_6$) δ 12.74 (s, 1H), 8.21 (d, *J* = 1.6 Hz, 1H), 7.79 (dd, *J* = 8.5, 1.6 Hz, 1H), 7.66 (t, *J* = 7.8 Hz, 1H), 7.62 (d, *J* = 8.4 Hz, 1H), 7.47 - 7.36 (m, 2H), 7.18 (s, 1H), 7.07 (d, *J* = 7.5 Hz, 1H), 6.72 (d, *J* = 8.2 Hz, 1H), 6.70 (s, 1H), 5.61 (s, 2H), 5.05 - 4.99 (m, 1H), 4.68 - 4.60 (m, 1H), 4.55 - 4.42 (m, 2H), 4.32 - 4.27 (m, 1H), 3.04 - 2.90 (m, 2H), 2.73 - 2.64 (m, 1H), 2.58 - 2.51 (m, 1H), 2.42 - 2.27 (m, 4H), 2.10 - 1.93 (m, 2H), 1.54 - 1.42 (m, 1H).

**Example 26 Synthesis of 2-((4-(6-((2-chloro-4-cyanobenzofuran-7-yl)methoxy)pyridin-2-yl)cyclohex-3-en-1-yl)methyl)-1-(((*S*)-oxetan-2-yl)methyl)-1*H*-benzo[*d*]imidazole-6-carboxylic acid (compound 26)**

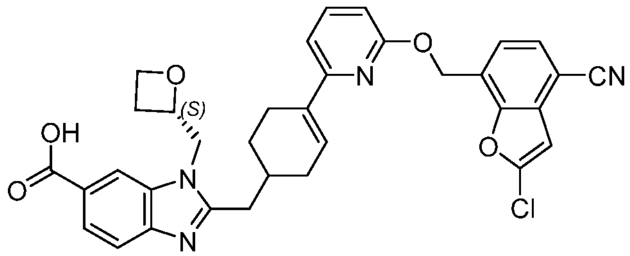

**Step 1: Synthesis of 4-bromo-2-(2,2-diethoxyethoxy)-1-methylbenzene**

5-Bromo-2-methylphenol (25 g, 134.4 mmol), 2-bromo-1,1-diethoxyethane (31.8 g, 162.3 mmol) and potassium carbonate (37.1 g, 268.8 mmol) were added to *N*,*N*-dimethylformamide (250 mL) and stirred overnight at 120 °C under nitrogen protection. The reaction was completed, diluted with water (300 mL) and extracted with ethyl acetate (100 mL × 3). The combined organic phases were washed with saturated saline (30 mL), the organic phase was dried with anhydrous sodium sulfate, concentrated under reduced pressure, and the residue was purified by column chromatography eluted with EtOAc:PE (v/v)=1% to give a light brown liquid (40 g, 98.5%).

**Step 2: Synthesis of 4-bromo-7-methylbenzofuran**

4-bromo-2-(2,2-diethoxyethoxy)-1-methylbenzene (40 g, 132.2 mmol) and polyphosphoric acid (68 g, 829.4 mmol) were added to 1,2-dichloroethane (400 mL), and the reaction was carried out at 85 °C overnight under nitrogen protection. The solvent was removed under reduced pressure, water (100 mL) was added, extracted with dichloromethane (100 mL×2), the combined organic phases were dried with anhydrous sodium sulfate, concentrated under reduced pressure, the residue was purified by column chromatography eluted with PE=100% to give a colorless liquid (17 g, 60.8%).

$^1$HNMR (400 MHz, $CDCl_3$) δ 7.68 (d, *J* = 4 Hz, 1H), 7.30 - 7.26 (m, 1H), 6.97 (d, *J* = 8.0 Hz, 1H), 6.80 (d, *J* = 4 Hz, 1H), 2.48 (s, 3H).

**Step 3: Synthesis of 7-methylbenzofuran-4-carbonitrile**

4-Bromo-7-methylbenzofuran (16 g, 75.8 mmol) was added to *N*,*N*-dimethylformamide (160 mL), cuprous cyanide (33.95 g, 378.5 mmol) was added, and the reaction was carried out at 120 °C under nitrogen protection overnight. It was diluted with water (500 mL), extracted with ethyl acetate (150 mL×3), the combined organic phases were dried with anhydrous sodium sulfate, concentrated under reduced pressure, and the residue was purified by column chromatography eluted with PE=100% to give a colorless liquid (8.3 g, 69.7%).

$^1$HNMR (400 MHz, $CDCl_3$) δ 7.78 (d, *J* = 4 Hz, 1H), 7.49 (d, *J* = 8 Hz, 1H), 7.16 (d, *J* = 8 Hz, 1H), 6.98 (s, 1H), 2.59 (s, 3H).

**Step 4: Synthesis of 2-chloro-7-methylbenzofuran-4-carbonitrile**

7-methylbenzofuran-4-carbonitrile (1 g, 6.4 mmol) was added to tetrahydrofuran (15 mL). It was cooled to -78 °C under nitrogen protection. At this temperature, lithium diisopropylammonium (2.6 mL) was added and stirred for 1 hour. Hexachloroethane (1.6 g, 6.8 mmol) was added, the cryopump was withdrawn, and the mixture was stirred overnight. Diluted with water (45 mL) at 0 °C, extracted with ethyl acetate (50 mL × 3), the combined organic phases were dried with anhydrous sodium sulfate, concentrated under reduced pressure, and the residue was purified by column chromatography eluted with EtOAc:PE (v/v)=5% to give a colorless liquid (800 mg, 65.6%).

$^1$HNMR (400 MHz, $CDCl_3$) δ 7.48 (d, *J* = 8 Hz, 1H), 7.15(d, *J* = 8 Hz, 1H), 6.81 (s, 1H), 2.56 (s, 3H).

**Step 5: Synthesis of 7-(bromomethyl)-2-chlorobenzofuran-4-carbonitrile**

2-Chloro-7-methylbenzofuran-4-carbonitrile (800 mg, 4.2 mmol), azobisisobutyronitrile (137 mg, 0.8 mmol) and N-bromosuccinimide (1.1 g, 6.2 mmol) were added to 1,2-dichloroethane (36 mL), and reacted at 85 °C under nitrogen protection overnight. Diluted with water (50 mL), extracted with dichloromethane (30 mL×3), the combined organic phases were dried with anhydrous sodium sulfate, concentrated under reduced pressure, and the residue was purified by column chromatography eluted with EtOAc:PE (v/v)=1% to give a colorless liquid (700 mg, 61.9%).

$^1$HNMR (400 MHz, $CDCl_3$) δ 7.56 (d, *J* =8.0 Hz, 1H), 7.39 (d, *J* = 8.0 Hz, 1H), 6.86 (s, 1H), 4.71 (s, 2H).

**Step 6: Synthesis of methyl 2-((4-(6-((2-chloro-4-cyanobenzofuran-7-yl)methoxy)pyridin-2-yl)cyclohex-3-en-1-yl)methyl)-1-(((*S*)-oxetan-2-yl)methyl)-1*H*-benzo[*d*]imidazole-6-carboxylate**

methyl 2-((4-(6-hydroxypyridin-2-yl)cyclohex-3-en-1-yl)methyl)-1-(((*S*)-oxetan-2-yl)methyl)-1*H*-benzo[*d*]imidazole-6-carboxylate (100 mg, 0.23 mmol) (synthesis refer to step 9 of example 25), 7-(bromomethyl)-2-chlorobenzofuran-4-carbonitrile (74 mg, 0.27 mmol) and potassium carbonate (95.6 mg, 0.68 mmol) were added to *N*,*N*-dimethylformamide (5 mL), and reacted at 60 °C for 1.5 hours. Water (20 mL) was added, extracted with dichloromethane (15 mL×3), the combined organic phase was washed with saturated saline, dried with anhydrous sodium sulfate, concentrated under reduced pressure, and the residue was purified by column chromatography eluted with EtOAc:PE (v/v)=50% to give a colorless oil (79 mg, 48.7%).

LC-MS: $[M+H]^+$=623.3.

**Step 7: Synthesis of 2-((4-(6-((2-chloro-4-cyanobenzofuran-7-yl)methoxy)pyridin-2-yl)cyclohex-3-en-1-yl) methyl)-1-(((*S*)-oxetan-2-yl)methyl)-1*H*-benzo[*d*]imidazole-6-carboxylic acid**

Methyl 2-((4-(6-((2-chloro-4-cyanobenzofuran-7-yl)methoxy)pyridin-2-yl)cyclohex-3-en-1-yl) methyl)-1-(((*S*)-oxetan-2-yl)methyl)-1*H*-benzo[*d*]imidazole-6-carboxylate (79 mg, 0.13 mmol), lithium hydroxide (15.2 mg, 0.63 mmol) were added to the mixture of 1,4-dioxane (1.6 mL) and water (0.4 mL), and the reactin is carried out at 40 °C for three hours. the pH was adjusted to 6-7 with acetic acid (0.2 mL). Concentrated under reduced pressure and the residue was purified by Prep-HPLC eluted with acetonitrile:water (v/v)=40% to give a white solid (34 mg, 44%).

LC-MS: $[M+H]^+$=609.2;
$^1$HNMR (400 MHz, DMSO-$d_6$) δ 12.69 (s, 1H), 8.20 (d, *J* = 1.6 Hz, 1H), 7.85 - 7.76 (m, 2H), 7.68 (t, *J* = 7.8 Hz, 1H), 7.62 (d, *J* = 8.4 Hz, 1H), 7.56 (d, *J* = 7.9 Hz, 1H), 7.39 (s, 1H), 7.07 (d, *J* = 7.5 Hz, 1H), 6.77 (d, *J* = 8.1 Hz, 1H), 6.63 (s, 1H), 5.70 (s, 2H), 5.03 - 5.01 (m, 1H), 4.67 - 4.28 (m, 4H), 3.04 - 2.89 (m, 2H), 2.73 - 2.64 (m, 1H), 2.45 - 2.28 (m, 5H), 2.08 - 1.92 (m, 2H), 1.51 -1.40 (m, 1H).

**Example 27 Synthesis of 2-(((1*s*,4*R*)-4-(6-((4-chloro-2-fluorobenzofuran-7-yl)methoxy)pyridin-2-yl)cyclohexyl) methyl)-1-(((*S*)-oxetan-2-yl)methyl)-1*H*-benzo[*d*]imidazole-6-carboxylic acid (compound 27A) and 2-(((1*r*,4*S*)-4-(6-((4-chloro-2-fluorobenzofuran-7-yl)methoxy)pyridin-2-yl)cyclohexyl)methyl)-1-(((*S*)-oxetan-2-yl) methyl)-1*H*-benzo[*d*]imidazole-6-carboxylic acid (compound 27B)**

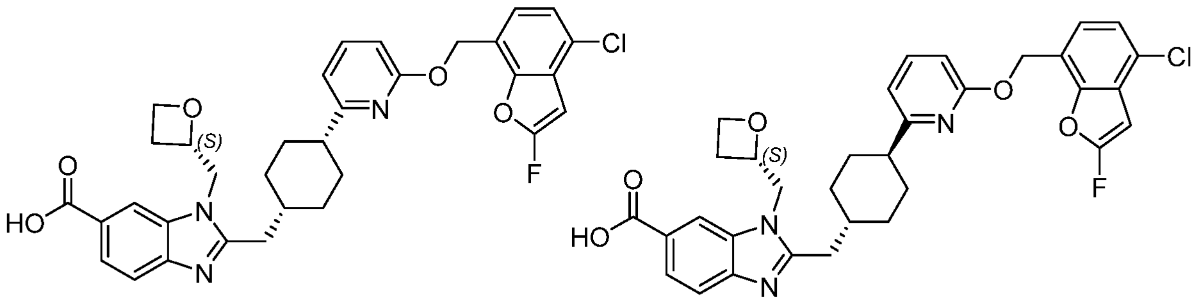

**Step 1: Synthesis of 4-chloro-2-fluoro-7-methylbenzofuran**

4-Chloro-7-methylbenzofuran (20 g, 120 mmol) was added to anhydrous tetrahydrofuran (500 mL), cooled at -78°C under nitrogen protection, n-butyllithium (2.5 mol/L tetrahydrofuran/cyclohexane solution) (58 mL, 145 mmol), *N*-fluoro-*N*-(phenylsulfonyl)benzenesulfonamide (41.6 g, 132 mmol) were added slowly, the reaction was carried out for 4 h at this temperature. Aqueous ammonium chloride solution (100 mL) was added, extracted with ethyl acetate (100 mL×3), the combined organic phases were dried with anhydrous sodium sulfate, concentrated under reduced pressure, and the residue was purified by column chromatography eluted with PE(v)=100% to give a colorless liquid (9.45 g, 42.6%).

$^1$HNMR (500 MHz, $CDCl_3$) δ 7.13 (d, *J* = 8.1 Hz, 1H), 6.97 (d, *J* = 8.0 Hz, 1H), 5.94 (d, *J* = 6.6 Hz, 1H), 2.42 (s, 3H).

**Step 2: Synthesis of 7-(bromomethyl)-4-chloro-2-fluorobenzofuran**

4-Chloro-2-fluoro-7-methylbenzofuran (11.4 g, 61.76 mmol) and azodiisobutyronitrile (2.03 g, 12.35 mmol) were added to 1,2-dichloroethane (120 mL), and then *N*-bromosuccinimide (16.49 g, 92.64 mmol) was slowly added, and the reaction was carried out at 85 °C under nitrogen protection overnight. Cooled to room temperature, added water (50 mL), extracted with dichloromethane (15 mL×3), the combined organic phases were dried with anhydrous sodium sulfate, concentrated under reduced pressure, and the residue was purified by column chromatography eluted with PE=100% to give a white solid (9.8 g, 60.2%).

$^1$HNMR (500 MHz, $CDCl_3$) δ 7.24 - 7.20 (m, 2H), 6.01 (d, *J* = 6.6 Hz, 1H), 4.65 (s, 2H).

**Step 3: Synthesis of ethyl 2-(4-(((trifluoromethyl)sulfonyl)oxy)cyclohex-3-en-1-yl)acetate**

Ethyl 2-(4-oxocyclohexyl)acetate (20 g, 108.55 mmol) and 2,6-di-tert-butylpyridine (24.92 g, 130.3 mmol) were added to dichloromethane (160 mL), cooled in an ice bath, trifluoromethanesulfonic anhydride (35.22 g, 124.84 mmol) was added dropwise and the reaction was carried out for 2.5 h at room temperature, and the organic phase was washed with water (40 mL × 2), dried with anhydrous sodium sulfate, concentrated under reduced pressure, the residue was purified by column chromatography eluted with EtOAc:PE (v/v)=5% to give a colorless liquid (24.56 g, 71.5%).

$^1$HNMR (500 MHz, $CDCl_3$) δ 5.72 (s, 1H), 4.15 (q, *J* = 7.1 Hz, 2H), 2.49 - 2.39 (m, 1H), 2.38 - 2.31 (m, 2H), 2.30 (d, *J* = 7.1 Hz, 2H), 2.18 - 2.09 (m, 1H), 1.97 - 1.89 (m, 2H), 1.57 - 1.48 (m, 1H), 1.26 (t, *J* = 7.1 Hz, 3H).

**Step 4: Synthesis of ethyl 2-(4-(4,4,5,5-tetramethyl-1,3,2-dioxaborolan-2-yl)cyclohex-3-en-1-yl)acetate**

Ethyl 2-(4-(((trifluoromethyl)sulfonyl)oxy)cyclohex-3-en-1-yl)acetate (26 g, 82.1 mmol), pinacol ester of bis(boronic acid) (25 g, 98.5 mmol), [1,1'-bis(diphenylphosphino)ferrocene]palladium dichloride dichloromethane complex (3.35 g, 4.1 mmol) and potassium acetate (16.1 g, 164.2 mmol) were added to 1,4-dioxacyclo (300 mL), the reaction was carried out at 100 °C for 2 h under nitrogen protection, cooled to room temperature, the reaction was quenched with water (300 mL), the reaction was extracted with ethyl acetate (50 mL×3), the combined organic phases were dried with anhydrous sodium sulfate, concentrated under reduced pressure, and the residue was purified by column chromatography eluted with EtOAc:PE (v/v) = 5% to give a light yellow liquid (19.5 g, 81%).

$^1$HNMR (500 MHz, $CDCl_3$) δ 6.50 (s, 1H), 4.15 - 4.10 (m, 2H), 2.30 - 2.19 (m, 4H), 2.15 - 2.03 (m, 2H), 1.84 - 1.73 (m, 2H), 1.40 - 1.28 (m, 1H), 1.25 (s, 12H), 1.28 - 1.23 (m, 3H).

**Step 5: Synthesis of ethyl 2-(4-(6-(benzyloxy)pyridin-2-yl)cyclohex-3-en-1-yl)acetate**

Ethyl 2-(4-(4-(4,4,5,5-tetramethyl-1,3,2-dioxaborolan-2-yl)cyclohex-3-en-1-yl)acetate (29 g, 98.5 mmol), 2-benzyloxy-6-bromopyridine (26 g, 98.5 mmol), [1,1'-bis(diphenylphosphino)ferrocene] palladium dichloride dichloromethane complex (4.02 mg. 4.93 mmol) and potassium carbonate (273 g, 197.2 mmol) were added to the mixture of 1,4-dioxane (240 mL) and water (60 mL), and the reaction was carried out at 100 °C for 2 h under nitrogen protection. The reaction was cooled down to room temperature, and then filtered, and the filtrate was added to water (300 mL), extracted with ethyl acetate (50 mL×3), the combined organic phases were dried with anhydrous sodium sulfate, concentrated under reduced pressure. The residue was purificated by column chromatography eluted with EtOAc:PE (v/v)=7% to give a light yellow liquid (23.43 g, 67.6%).

$^1$HNMR (500 MHz, $CDCl_3$) δ 7.51 (t, *J* = 7.8 Hz, 1H), 7.49 - 7.44 (m, 2H), 7.36 (t, *J* = 7.4 Hz, 2H), 7.29 (t, *J* = 7.4 Hz, 1H), 6.93 (d, *J* = 7.4 Hz, 1H), 6.78 (tt, *J* = 3.8, 1.7 Hz, 1H), 6.63 (d, *J* = 8.2 Hz, 1H), 5.41 (s, 2H), 4.16 (q, *J* = 7.1 Hz, 2H), 2.66 - 2.59 (m, 1H), 2.52 - 2.40 (m, 2H), 2.34 (d, *J* = 7.2 Hz, 2H), 2.23 - 2.13 (m, 1H), 2.04 - 1.93 (m, 2H), 1.52 - 1.42 (m, 1H), 1.27 (t, *J* = 7.2 Hz, 3H).

**Step 6: Synthesis of ethyl 2-((1*s*,4*s*)-4-(6-hydroxypyridin-2-yl)cyclohexyl)acetate (27A-1) and ethyl 2-((1*r*,4*r*)-4-(6-hydroxypyridin-2-yl)cyclohexyl)acetate (27B-1)**

Ethyl 2-(4-(6-(benzyloxy)pyridin-2-yl)cyclohex-3-en-1-1-yl)acetate (23.43 g, 66.67 mmol) was dissolved in ethanol (180 mL), 10% palladium carbon (60% water content, 2.3 g) was added, and it was replaced with hydrogen for three times, and the hydrogen reaction was carried out at room temperature and pressure overnight. The reaction mixture was filtered with diatomaceous earth, and the filtrate was concentrated under reduced pressure, pulped with methyl tert-Butyl ether (50 mL) to give a white solid (13.18 g, 75.1%). Chiral separation (SFC-80, CHIRALCEL OD (30*250 mm, 5 μm) Daircel, 35 °C, mobile phases: A=$CO_2$, 20%B=IPA, flow rate: 45 mL/min) to give the first peak of 27A-1 at 11.5 min and the second peak of 27B-1 at 14.3 min, with a ratio of 1:1 between 27A-1 and 27B-1.

27A-1:

$^1$HNMR (400 MHz, $CDCl_3$) δ 12.45 (brs, 1H), 7.39 (dd, *J* = 9.0, 6.9 Hz, 1H), 6.47 (d, *J* = 9.0 Hz, 1H), 6.11 (d, *J* = 6.9 Hz, 1H), 4.14 (q, *J* = 7.1 Hz, 2H), 2.56 (d, *J* = 7.8 Hz, 2H), 2.05 - 1.80 (m, 5H), 1.56 - 1.43 (m, 2H), 1.26 (t, *J* = 7.1 Hz, 3H), 1.25 - 1.08 (m, 2H).

LC-MS (ESI): $[M+H]^+$ =264.2.

27B-1:

$^1$HNMR (400 MHz, $CDCl_3$) δ 12.05 (brs, 1H), 7.37 (dd, *J* = 9.1, 6.9 Hz, 1H), 6.40 (d, *J* = 9.0 Hz, 1H), 6.04 (d, *J* = 6.9 Hz, 1H), 4.13 (q, *J* = 7.2 Hz, 2H), 2.52 - 2.41 (m, 1H), 2.23 (d, *J* = 6.7 Hz, 2H), 2.05 - 1.80 (m, 5H), 1.56 - 1.43 (m, 2H), 1.26 (t, *J* = 7.1 Hz, 3H), 1.25 - 1.08 (m, 2H).

LC-MS (ESI): $[M+H]^+$ =264.2.

**Step 7: Synthesis of ethyl 2-((1s,4s)-4-(6-((4-chloro-2-fluorobenzofuran-7-yl)methoxy)pyridin-2-yl)cyclohexyl) acetate (27A-2) and ethyl 2-((1r,4r)-4-(6-((4-chloro-2-fluorobenzofuran-7-yl)methoxy)pyridin-2-yl)cyclohexyl) acetate (27B-2)**

Synthesis of 27A-2

Compound 27A-1 (1.0 g, 38.0 mmol), 7-(bromomethyl)-4-chloro-2-fluorobenzofuran (1.05 g, 3.99 mmol) and cesium carbonate (2.48 g, 7.5.9 mmol) were added to *N*,*N*-dimethylformamide (10 mL) and the reaction was carried out at room temperature for 2 h. Water (50 mL) was added and extracted with ethyl acetate (10 mL×4), the combined organic phases were dried with anhydrous sodium sulfate, concentrated under reduced pressure, and the residue was purified by column chromatography eluted with EtOAc:PE (v/v)=10% to give a colorless liquid (1.52 g, 89.8%).

LC-MS (ESI): $[M+H]^+$ =446.2.

Synthesis of 27B-2

Compound 27B-1 (10 g, 37.97 mmol), 7-(bromomethyl)-4-chloro-2-fluorobenzofuran (10.5 g, 39.85 mmol) and cesium carbonate (24.75 g, 75.95 mmol) were added to *N*,*N*-dimethylformamide (100 mL) and the reaction was carried out at room temperature for 2 h. Water (200 mL) was added and extracted with ethyl acetate (50 mL×2), the combined organic phases were dried with anhydrous sodium sulfate, concentrated under reduced pressure, and the residue was purified by column chromatography eluted with EtOAc:PE (v/v)=8 ~ 30% to give a colorless liquid (15.2 g, 89.8%).

$^1$HNMR (500 MHz, $CDCl_3$) δ 7.48 (t, *J* = 7.8 Hz, 1H), 7.32 (d, *J* = 8.2 Hz, 1H), 7.22 (d, *J* = 8.2 Hz, 1H), 6.71 (d, *J* = 7.3 Hz, 1H), 6.59 (d, *J* = 8.2 Hz, 1H), 6.00 (d, *J* = 6.6 Hz, 1H), 5.59 (s, 2H), 4.15 (q, *J* = 7.2 Hz, 2H), 2.55 - 2.50 (m, 1H), 2.24 (d, *J* = 6.7 Hz, 2H), 1.95 - 1.83 (m, 5H), 1.61 - 1.52 (m, 2H), 1.28 (t, *J* = 7.2 Hz, 3H), 1.20 - 1.09 (m, 2H).

LC-MS (ESI): $[M+H]^+$ =446.3.

**Step 8: Synthesis of 2-((1s,4s)-4-(6-((4-chloro-2-fluorobenzofuran-7-yl)methoxy)pyridin-2-yl)cyclohexyl)acetic acid (27A-3) and 2-((1r,4r)-4-(6-((4-chloro-2-fluorobenzofuran-7-yl)methoxy)pyridin-2-yl)cyclohexyl)acetic acid (27B-3)**

Synthesis of 27A-3

27A-2 (1.32 g, 2.96 mmol) was dissolved in the mixture of 1,4-dioxane (10 mL) and water (5 mL), lithium hydroxide monohydrate (1.2 g, 29.6 mmol) was added, and the reaction was carried out at 60 °C for 1.5 h. The reaction was cooled in an ice bath, and the pH was adjusted to 4 with dilute hydrochloric acid, and the organic phases were extracted with ethyl acetate (15 mL×3), the combined organic phases were dried with anhydrous sodium sulfate, concentrated under reduced pressure to give a light yellow solid (1.3 g, 91.1%).

$^1$H NMR (500 MHz, DMSO-$d_6$) δ 11.96 (s, 1H), 7.62 (t, *J* = 7.8 Hz, 1H), 7.39 (q, *J* = 8.2 Hz, 2H), 6.88 (d, *J* = 7.3 Hz, 1H), 6.68 (d, *J* = 8.2 Hz, 1H), 6.49 (d, *J* = 6.3 Hz, 1H), 5.58 (s, 2H), 2.70 - 2.63 (m, 1H), 2.21 (d, *J* = 7.5 Hz, 2H), 2.10 - 1.95 (m, 1H), 1.83 - 1.71 (m, 2H), 1.63 - 1.49 (m, 4H), 1.48 - 1.39 (m, 2H).

LC-MS (ESI): [M+H] =418.2.

Synthesis of 27B-3

Compound 27B-2 (15 g, 33.64 mmol) was dissolved in the mixture of 1,4-dioxane (100 mL) and water (50 mL), lithium hydroxide monohydrate (7.06 g, 168.2 mmol) was added, and the reaction was carried out at 60 °C for 2 h. The reaction was cooled in an ice bath, and the pH was adjusted to 4 with dilute hydrochloric acid, and the organic phases were extracted with ethyl acetate (15 mL×3), the combined organic phases were dried with anhydrous sodium sulfate and concentrated under reduced pressure to give a light yellow solid (13.2 g, 93.9%).

$^1$H NMR (500 MHz, DMSO-$d_6$) δ 11.99 (s, 1H), 7.60 (t, *J* = 7.8 Hz, 1H), 7.40 (q, *J* = 8.2 Hz, 2H), 6.82 (d, *J* = 7.3 Hz, 1H), 6.66 (d, *J* = 8.2 Hz, 1H), 6.49 (d, *J* = 6.4 Hz, 1H), 5.56 (s, 2H), 2.50 - 2.45 (m, 1H), 2.13 (d, *J* = 6.9 Hz, 2H), 1.85 - 1.75 (m, 4H), 1.73 - 1.64 (m, 1H), 1.51- 1.39 (m, 2H), 1.15 - 1.03 (m, 2H).

LC-MS (ESI): [M+H] =418.2.

**Step 9: Synthesis of methyl 4-(2-((1*s*,4*R*)-4-(6-((4-chloro-2-fluorobenzofuran-7-yl)methoxy)pyridin-2-yl)cyclohexyl)acetamido)-3-((((*S*)-oxetan-2-yl)methyl)amino)benzoate (27A-4) and methyl 4-(2-((1*r*,4*S*)-4-(6-((4-chloro-2-flnorobenzofuran-7-yl)methoxy)pyridin-2-yl)cyclohexyl)acetamido)-3-((((*S*)-oxetan-2-yl)methyl)amino)benzoate (27B-4)**

Synthesis of 27A-4

Compound 27A-3 (1.13 g, 2.43 mmol), methyl (S)-4-amino-3-((oxetan-2-ylmethyl)amino)benzoate (580 mg, 2.43 mmol) and HOAT (500 mg, 3.65 mmol) were added to *N*,*N*-dimethylformamide (10 mL), EDCI (940 mg, 4.84 mmol) and *N*,*N*-diisopropylethylamine (790 mg, 6.08 mmol) were added, and the reaction was carried out at 40 °C overnight. Water (50 mL) was added, extracted with ethyl acetate (10 mL × 4), the combined organic phases were dried with anhydrous sodium sulfate, concentrated under reduced pressure, and the residue was purified by column chromatography eluted with MeOH:DCM (v/v) = 0 ~ 5% to give a white solid (1.04 g, 67.1%).

$^1$HNMR (500 MHz, $CDCl_3$) δ 7.77 (d, *J* = 7.8 Hz, 1H), 7.66 (s, 1H), 7.55 (d, *J* = 8.5 Hz, 1H), 7.52 (d, *J* = 8.1 Hz, 2H), 7.31 (d, *J* = 8.1 Hz, 1H), 7.22 (d, *J* = 8.3 Hz, 1H), 6.79 (d, *J* = 7.4 Hz, 1H), 6.61 (d, *J* = 8.3 Hz, 1H), 5.99 (d, *J* = 6.5 Hz, 1H), 5.60 (s, 2H), 5.04 - 4.96 (m, 1H), 4.70 - 4.66 (m, 1H), 4.58 - 4.51 (m, 1H), 3.89 (s, 3H), 3.42 - 3.21 (m, 2H), 2.80 - 2.65 (m, 2H), 2.55 - 2.46 (m, 1H), 2.43 - 2.35 (m, 2H), 2.31 - 2.24 (m, 1H), 2.02 - 1.87 (m, 3H), 1.81 - 1.65 (m, 5H).

LC-MS (ESI): $[M+H]^+$ =636.3.

Synthesis of 27B-4

Compound 27B-3 (13.2 g, 31.59 mmol), methyl (S)-4-amino-3-((oxetan-2-ylmethyl)amino)benzoate (7.46 g, 31.59 mmol) and HOAT (5.59 g, 41.07 mmol) were added to *N*,*N*-dimethylformamide (130 mL), cooled in an ice bath, EDCI (9.08 g, 47.38 mmol) and *N*,*N*-diisopropylethylamine (9.17 g, 63.18 mmol) were added and the reaction was carried out at 40 °C overnight, ice water (1.1 L) was added slowly, filtered, the filter cake was washed with water (30 mL × 2), and the filter cake was pulped with EtOAc:PE (v/v)=1:1 to give a white solid (13.6 g, 71.2%).

$^1$HNMR (500 MHz, $CDCl_3$) δ 7.81 (d, *J* = 8.3 Hz, 1H), 7.70 (s, 1H), 7.58 (d, *J* = 8.3 Hz, 1H), 7.53 (s, 1H), 7.48 (t, *J* = 7.7 Hz, 1H), 7.32 (d, *J* = 8.2 Hz, 1H), 7.22 (d, *J* = 8.2 Hz, 1H), 6.71 (d, *J* = 7.4 Hz, 1H), 6.58 (s, 1H), 6.00 (d, *J* = 6.6 Hz, 1H),

5.59 (s, 2H), 5.09 - 5.02 (m, 1H), 4.78 - 4.71 (m, 1H), 4.64 - 4.57 (m, 1H), 3.89 (s, 3H), 3.45 - 3.29 (m, 2H), 2.78 - 2.71 (m, 1H), 2.59 - 2.50 (m, 2H), 2.37 - 2.30 (m, 2H), 2.01 - 1.91 (m, 5H), 1.66 - 1.56 (m, 2H), 1.25 - 1.15 (m, 2H).

LC-MS (ESI): $[M+H]^+$ =636.4.

**Step 10: Synthesis of methyl 2-(((1*s*,4*R*)-4-(6-((4-chloro-2-fluorobenzofuran-7-yl)methoxy)pyridin-2-yl)cyclohexyl)methyl)-1-(((*S*)-oxetan-2-yl)methyl)-1*H*-benzo[*d*]imidazole-6-carboxylate (27A-5) and methyl 2-(((1*r*,4*S*-4-(6-((4-chloro-2-fluorobenzofuran-7-yl)methoxy)pyridin-2-yl)cyclohexyl)methyl)-1-(((*S*)-oxetan-2-yl)methyl)-1*H*-benzo[*d*]imidazole-6-carboxylate (27B-5)**

Synthesis of 27A-5

Compound 27A-4 (940 mg, 1.48 mmol) was added to glacial acetic acid (10 mL), and the reaction was carried out at 80 °C for 4 h. The solvent was removed under reduced pressure, the pH was adjusted to 8 with saturated aqueous sodium bicarbonate, extracted with ethyl acetate (10 mL×4), and the combined organic phases were dried with anhydrous sodium sulfate, concentrated under reduced pressure, and the residue was purified by column chromatography eluted with EtOAc:PE (v/v ) = 15 ~ 75% to give a light yellow solid (670 mg, 73.6%).

$^1$HNMR (500 MHz, $CDCl_3$) δ 8.07 (s, 1H), 7.95 (d, *J* = 10.0 Hz, 1H), 7.73 (d, *J* = 8.5 Hz, 1H), 7.52 (t, *J* = 7.7 Hz, 1H), 7.33 (d, *J* = 8.3 Hz, 1H), 7.24 (d, *J* = 8.4 Hz, 1H), 6.81 (d, *J* = 7.4 Hz, 1H), 6.61 (d, *J* = 8.2 Hz, 1H), 6.00 (d, *J* = 6.6 Hz, 1H), 5.61 (s, 2H), 5.19 - 5.11 (m, 1H), 4.59 - 4.55 (m, 1H), 4.46 - 4.28 (m, 3H), 3.94 (s, 3H), 3.00 (d, *J* = 7.3 Hz, 2H), 2.83 - 2.75 (m, 1H), 2.74 - 2.64 (m, 1H), 2.48 - 2.34 (m, 2H), 2.10 - 1.99 (m, 2H), 1.83 - 1.53 (m, 6H).

LC-MS (ESI): $[M+H]^+$ =618.4.

Synthesis of 27B-5

Compound 27B-4 (13.5 g, 21.22 mmol) was added to glacial acetic acid (150 mL), and the reaction was carried out at 80 °C for 2 h. The solvent was removed under reduced pressure, the pH was adjusted to 8 with saturated aqueous sodium bicarbonate, extracted with dichloromethane (20 mL×2), and the combined organic phases were dried with anhydrous sodium sulfate, concentrated under reduced pressure, and the residue was purified by column chromatography eluted with EtOAc:PE (v /v)=40 ~ 60% to give a light brown solid (12 g, 91.5%).

$^1$HNMR (500 MHz, $CDCl_3$) δ 8.09 (s, 1H), 7.96 (dd, *J* = 8.5, 1.6 Hz, 1H), 7.74 (d, *J* = 8.4 Hz, 1H), 7.47 (t, *J* = 7.7 Hz, 1H), 7.31 (d, *J* = 8.3 Hz, 1H), 7.22 (d, *J* = 8.2 Hz, 1H), 6.71 (d, *J* = 7.3 Hz, 1H), 6.58 (d, *J* = 8.2 Hz, 1H), 5.99 (d, *J* = 6.6 Hz, 1H), 5.58 (s, 2H), 5.21 - 5.17 (m, 1H), 4.64 - 4.60 (m, 1H), 4.48 - 4.35 (m, 3H), 3.94 (s, 3H), 2.94 (d, *J* = 7.3 Hz, 2H), 2.77 - 2.70 (m, 1H), 2.61 - 2.52 (m, 1H), 2.47 - 2.40 (m, 1H), 2.18 - 2.07 (m, 1H), 2.00 - 1.91 (m, 4H), 1.67 - 1.53 (m, 4H).

LC-MS (ESI): $[M+H]^+$ =618.5.

**Step 11: Synthesis of 2-(((1*s*,4*R*)-4-(6-((4-chloro-2-fluorobenzofuran-7-yl)methoxy)pyridin-2-yl)cyclohexyl)methyl)-1-(((*S*)-oxetan-2-yl)methyl)-1*H*-benzo[*d*]imidazole-6-carboxylic acid (27A) and 2-(((1*r*,4*S*)-4-(6-((4-chloro-2-fluorobenzofuran-7-yl)methoxy)pyridin-2-yl)cyclohexyl)methyl)-1-(((*S*)-oxetan-2-yl)methyl)-1*H*-benzo[*d*]imidazole-6-carboxylic acid (27B)**

Synthesis of 27A

Compound 27A-5 (530 mg, 0.86 mmol) and lithium hydroxide monohydrate (181 mg, 4.29 mmol) were added to the mixture of 1,4-dioxane (10 mL) and water (5 mL), and the reaction was carried out at 40 °C for 2 h. Water (30 mL) was added and the pH was adjusted to 6 with dilute hydrochloric acid (2 M) in an ice bath, extracted with ethyl acetate (15 mL×3), the combined organic phases were dried with anhydrous sodium sulfate, concentrated under reduced pressure, and the residue was purified by column chromatography eluted with MeOH: DCM (v/v)=3% to give a white solid (470 mg, 90.7%).

$^1$HNMR (500 MHz, DMSO-$d_6$) δ 12.64 (s, 1H), 8.18 (s, 1H),7.79 (d, *J* = 8.4 Hz, 1H), 7.68 - 7.59 (m, 2H), 7.42 (q, *J* = 8.2 Hz, 2H), 6.93 (d, *J* = 7.3 Hz, 1H), 6.69 (d, *J* = 8.1 Hz, 1H), 6.48 (d, *J* = 6.3 Hz, 1H), 5.59 (s, 2H), 5.04 - 4.96 (m, 1H), 4.64 - 4.55 (m, 1H), 4.52 - 4.38 (m, 2H), 4.30 - 4.22 (m, 1H), 2.97 (d, *J* = 7.3 Hz, 2H), 2.75 - 2.59 (m, 2H), 2.42 - 2.28 (m, 2H), 2.01 - 1.87 (m, 2H), 1.72 - 1.51 (m, 6H).

LC-MS (ESI): $[M+H]^+$ =604.5.

Synthesis of 27B

Compound 27B-5 (11.5 g, 18.61 mmol) and lithium hydroxide monohydrate (2.34 g, 55.82 mmol) were added to the mixture of 1,4-dioxane (80 mL) and water (40 mL), and the reaction was carried out at 40 °C for 2 h. Water (200 mL) was

added, the pH was adjusted to 6 with trifluoroacetic acid in an ice bath, filtrated and the filter cake was washed with water (50 mL), the filter cake was dissolved with dichloromethane, the organic phase was separated, dried with anhydrous sodium sulfate, concentrated under reduced pressure, and the residue was purified by column chromatography eluted with MeOH: DCM (v/v) = 5%) to give a white solid (8.2 g, 71%). Compound 27B was cultured as a single crystal and compound 27B was shown to be trans by single crystal diffraction.

$^1$HNMR (500 MHz, DMSO-$d_6$) δ 12.66 (s, 1H), 8.21 (s, 1H), 7.79 (dd, *J* = 8.3, 1.5 Hz, 1H), 7.61 (dd, *J* = 15.6, 8.0 Hz, 2H), 7.43 - 7.37 (m, 2H), 6.84 (d, *J* = 7.3 Hz, 1H), 6.66 (d, *J* = 8.2 Hz, 1H), 6.49 (d, *J* = 6.4 Hz, 1H), 5.56 (s, 2H), 5.07 - 5.00 (m, 1H), 4.67 - 4.60 (m, 1H), 4.55 - 4.45 (m, 2H), 4.35 - 4.29 (m, 1H), 2.95 - 2.84 (m, 2H), 2.74 - 2.67 (m, 1H), 2.58 - 2.51 (m, 1H), 2.42 - 2.35 (m, 1H), 2.08 - 1.97 (m, 1H), 1.92 - 1.77 (m, 4H), 1.54 - 1.44 (m, 2H), 1.27 - 1.18 (m, 2H).

LC-MS (ESI): $[M+H]^+$ =604.3.

**Example 28 Synthesis of 2-((4-(6-((4-chloro-2-fluorobenzofuran-7-yl)methoxy)pyridin-2-yl)cyclohex-3-en-1-yl) methyl)-1-((1-(cyanomethyl)cyclopropyl)methyl)-1*H*-benzo[*d*]imidazole-6-carboxylic acid (compound 28)**

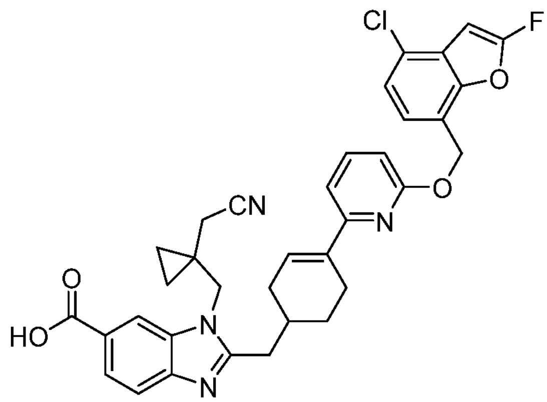

**Step** 1: **Synthesis** of **tert-Butyl ((1-(hydroxymethyl)cyclopropyl)methyl)carbamate**

(1-(Aminomethyl)cyclopropyl)methanol (2.5 g, 24.72 mmol) and di-tert-butyl dicarbonate (2.5 g, 24.72 mmol) were dissolved in DCM (50 mL), and then di-isopropylethylamine (8.2 mL, 49.43 mmol) was added, and the reaction was stirred at room temperature for 3 h. Stopped and water (40 mL) was added, extracted by methylene chloride (15 mL×3). The combined organic phase was washed twice with dilute hydrochloric acid (1M) and dried with anhydrous sodium sulfate, filtered and concentrated, the residue was purified by column chromatography eluted with PE:EtOAc (v/v)=3:2 to give a light yellow viscous material (4.24 g, 85.2%).

$^1$H NMR (500 MHz, $CDCl_3$) δ 4.98 (s, 1H), 3.37 (d, *J* = 6.5 Hz, 2H), 3.25 (s, 1H), 3.10 (d, *J* = 6.4 Hz, 2H), 1.44 (d, *J* = 5.8 Hz, 9H), 0.45 - 0.40 (m, 4H).

**Step 2: Synthesis of (1-(((tert-butoxycarbonyl)amino)methyl)cyclopropyl)methyl methanesulfonate**

Tert-butyl ((1-(hydroxymethyl)cyclopropyl)methyl)carbamate (1.81 g, 8.99 mmol) was dissolved in DCM (40 mL), then triethylamine (2.5 mL, 17.99 mmol) was added, and methanesulfonyl chloride (0.8 mL, 10.79 mmol) was added slowly at 0 °C, and the reaction was stirred for 2 h. Stopped and the reaction was quenched with water (50 mL). Extracted with dichloromethane for three times (30 mL×3), the combined organic phases were dried with anhydrous sodium sulfate and dried with anhydrous sodium sulfate. The filtrate was concentrated to give a yellowish viscous material (2.0 g, 79.6%).

**Step 3: Synthesis of tert-Butyl ((1-(cyanomethyl)cyclopropyl)methyl)carbamate**

(1-(((tert-butoxycarbonyl)amino)methyl)cyclopropyl)methyl methanesulfonate (2.0 g, 7.16 mmol) and trimethylsilanecarbonitrile (1.9 mL, 14.32 mmol) were dissolved in acetonitrile (30 mL), and then tetrabutylammonium fluoride (4.2 mL, 14.32 mmol) was added and the reaction was heated to 80 °C for 2 h. Stopped and the reaction was cooled to room temperature. Concentrated the solvent, diluted with water (30 mL), extracted with ethyl acetate (15 mL×3), the combined organic phases were dried with anhydrous sodium sulfate, filtered, concentrated the filtrate, and the residue was purified by column chromatography eluted with PE:EtOAc (v/v)=10:1 to give a light yellow solid (850 mg, 56.5%).

$^1$H NMR (500 MHz, $CDCl_3$) δ 4.75 (s, 1H), 3.13 (d, *J* = 6.4 Hz, 2H), 2.48 (s, 2H), 1.44 (s, 9H), 0.61 (d, *J* = 3.7 Hz,

4H).

**Step 4: Synthesis of 2-(1-(aminomethyl)cyclopropyl)acetonitrile** hydrochloride

Tert-butyl (1-(cyanomethyl)cyclopropyl)methyl)carbamate (700 mg, 3.33 mmol) was dissolved in DCM (10 mL), then 1,4-dioxane hydrochloride solution (1 mL) was added, and the reaction was stirred at room temperature for 4 h. Stopped and concentrated to give a white solid (400 mg, 81.9%).

LC-MS (ESI): $[M\text{-}HCl+H]^+$ =111.2.

**Step 5: Synthesis of methyl 3-(((1-(cyanomethyl)cyclopropyl)methyl)amino)-4-nitrobenzoate**

Methyl 3-fluoro-4-nitrobenzoate (500 mg, 2.51 mmol) and 2-(1-(aminomethyl)cyclopropyl)acetonitrile hydrochloride (368 mg, 2.51 mmol) were dissolved in a mixture of tetrahydrofuran (12 mL) and methanol (8 mL), and then triethylamine (2.1 mL, 15.06 mmol) was added, and the reaction was heated to 60 °C for 6 h. Stopped and the solvent was concentrated, diluted with water (30 mL) and extracted with ethyl acetate (15 mL × 3). The combined organic phases were dried over anhydrous sodium sulfate, filtered, and the filtrate was concentrated. The residue was purified by column chromatography eluted with PE:EtOAc (v/v)=5:1 to give a yellow solid (240 mg, 33.0%).

LC-MS (ESI): $[M+H]^+$ =290.0.

**Step 6: Synthesis of methyl 4-amino-3-(((1-(cyanomethyl)cyclopropyl)methyl)amino)benzoate**

Methyl 3-(((1-(cyanomethyl)cyclopropyl)methyl)amino)-4-nitrobenzoate (230 mg, 0.80 mmol) was dissolved in the mixture of ethyl acetate (8 mL) and ethanol (8 mL), and then palladium-carbon (23 mg, 10% Pd in 40-60% water) was added, protected by hydrogen, and the reaction was carried out at room temperature overnight. The reaction mixture was filtered with diatomaceous earth, concentrated under reduced pressure and the residue was purified by column chromatography eluted with PE:EtOAc (v/v)=2:1 to give a white solid (130 mg, 63.1%).

$^1H$ NMR (500 MHz, $CDCl_3$) δ 7.49 (dd, *J* = 8.1, 1.9 Hz, 1H), 7.30 (d, *J* = 1.8 Hz, 1H), 6.69 (d, *J* = 8.1 Hz, 1H), 4.05 - 3.88 (m, 2H), 3.85 (s, 3H), 3.13 (s, 3H), 2.56 (s, 2H), 0.71 (dt, *J* = 6.2, 2.0 Hz, 4H)

LC-MS (ESI): $[M+H]^+$ =260.2.

**Step 7: Synthesis of methyl 4-(2-(4-(4-(6-((4-chloro-2-fluorobenzofuran-7-yl)methoxy)pyridin-2-yl)cyclohex-3-en-1-yl)acetamido)-3-(((1-(cyanomethyl)cyclopropyl)methyl)amino)benzoate**

2-(4-(6-((4-chloro-2-fluorobenzofuran-7-yl)methoxy)pyridin-2-yl)cyclohex-3-en-1-yl)acetic acid (150 mg, 0.36 mmol) and methyl 4-amino-3-(((1-(cyanomethyl)cyclopropyl)methyl)amino)benzoate (112 mg, 0.43 mmol) were added to *N*,*N*-dimethylformamide (4 mL), HATU (247 mg, 0.65 mmol) and DIPEA (140 mg, 1.08 mmol) were added and the reaction was carried out at 40 °C overnight. The solvent was removed under reduced pressure, water (50 mL) was added, extracted with ethyl acetate (25 mL×3), the combined organic phases were dried with anhydrous sodium sulfate, concentrated under reduced pressure, and the residue was purified by column chromatography eluted with EtOAc:PE (v/v)=1:2 to give a light brown solid (215 mg, 90.7%).

$^1H$NMR (500 MHz, $CDCl_3$) δ 7.71 - 7.66 (m, 1H), 7.57 - 7.51 (m, 2H), 7.41 (d, *J* = 1.9 Hz, 1H), 7.32 (d, *J* = 8.2 Hz, 1H), 7.22 (d, *J* = 8.2 Hz, 1H), 6.95 (d, *J* = 7.4 Hz, 1H), 6.76 (s, 1H), 6.64 (d, *J* = 8.1 Hz, 1H), 5.99 (d, *J* = 6.6 Hz, 1H), 5.62 (s, 2H), 3.89 (s, 3H), 3.16 (s, 2H), 2.80 (s, 2H), 2.67 - 2.59 (m, 1H), 2.52 - 2.42 (m, 4H), 2.34 - 2.26 (m, 1H), 2.10 - 2.00 (m, 2H), 1.59 - 1.49 (m, 1H), 0.74 - 0.71 (m, 4H).

LC-MS (ESI): $[M+H]^+$ =657.4.

**Step 8: Synthesis of methyl 2-((4-(6-((4-chloro-2-fluorobenzofuran-7-yl)methoxy)pyridin-2-yl)cyclohex-3-en-1-yl)methyl)-1-((1-(cyanomethyl)cyclopropyl)methyl)-1*H*-benzo[*d*]imidazole-6-carboxylate**

Methyl 4-(2-(4-(4-(6-((4-chloro-2-fluorobenzofuran-7-yl)methoxy)pyridin-2-yl)cyclohex-3-en-1-yl)acetamido)-3-(((1-(cyanomethyl)cyclopropyl)methyl)amino)benzoate (210 mg, 0.32 mmol) was added to glacial acetic acid (5 mL), and the reaction was carried out at 70 °C for 9 h. The solvent was removed under reduced pressure, the residue was dissolved in ethyl acetate and purified by column chromatography eluted with EtOAc:PE (v/v)=45% to give a light brown solid (135 mg, 66.1%).

$^1H$NMR (500 MHz, $CDCl_3$) δ 8.15 (s, 1H), 7.98 (d, *J* = 8.5 Hz, 1H), 7.75 (d, *J* = 8.5 Hz, 1H), 7.52 (t, *J* = 7.9 Hz, 1H), 7.31 (d, *J* = 8.2 Hz, 1H), 7.22 (*d, J* = 8.2 Hz, 1H), 6.94 (*d, J* = 7.5 Hz, 1H), 6.74 (*d, J* = 4.8 Hz, 1H), 6.64 (d, *J* = 8.2 Hz, 1H), 5.99 (d, *J* = 6.5 Hz, 1H), 5.61 (s, 2H), 4.36 (s, 2H), 3.96 (s, 3H), 3.04 - 2.94 (m, 2H), 2.69 - 2.60 (m, 1H), 2.56 - 2.40 (m, 3H), 2.32 (s, 2H), 2.15 - 1.96 (m, 3H), 0.79 (d, *J* = 3.6 Hz, 2H), 0.77 (d, *J* = 3.6 Hz, 2H).

LC-MS (ESI): $[M+H]^+$ =639.4.

**Step 9: Synthesis of 2-((4-(6-((4-chloro-2-fluorobenzofuran-7-yl)methoxy)pyridin-2-yl)cyclohex-3-en-1-yl) methyl)-1-((1-(cyanomethyl)cyclopropyl)methyl)-1*H*-benzo[*d*]imidazole-6-carboxylic acid**

Methyl 2-((4-(6-((4-chloro-2-fluorobenzofuran-7-yl)methoxy)pyridin-2-yl)cyclohex-3-en-1-yl) methyl)-1-((1-(cyanomethyl)cyclopropyl)methyl)-1*H*-benzo[*d*]imidazole-6-carboxylate (135 mg, 0.21 mmol) and lithium hydroxide monohydrate (62 mg, 1.48 mmol) were add to the mixture of 1,4-dioxane (3 mL) and water (3 mL), the reaction was carried out at 40 °C for 1 h, cooled in an ice bath, adjust the pH to 4 with dilute hydrochloric acid (2M), water (40 mL) was added, extracted with ethyl acetate (15 mL×3), the combined organic phases were dried with anhydrous sodium sulfate, concentrated under reduced pressure, and the residue was purified by column chromatography eluted with MeOH: DCM (v/v)=10% to give a white solid (96 mg, 69.6%).

$^1$HNMR (500 MHz, DMSO-$d_6$) $\delta$ 12.76 (s, 1H), 8.22 (s, 1H), 7.81 (d, *J* = 8.3 Hz, 1H), 7.66 (t, *J* = 8.4 Hz, 2H), 7.47 - 7.34 (m, 2H), 7.07 (d, *J* = 7.4 Hz, 1H), 6.80 - 6.68 (m, 2H), 6.49 (d, *J* = 6.4 Hz, 1H), 5.59 (s, 2H), 4.45 (s, 2H), 3.03 - 2.91 (m, 2H), 2.60 (s, 2H), 2.55 - 2.42 (m, 2H), 2.41 - 2.29 (m, 2H), 2.16 - 1.91 (m, 2H), 1.57 - 1.44 (m, 1H), 0.66 (d, *J* = 15.5 Hz, 4H).

LC-MS (ESI): $[M+H-HCl]^+$ =625.4.

**Example 29 Synthesis of 2-((4-(6-((4-chloro-2-fluorobenzofuran-7-yl)methoxy)pyridin-2-yl)cyclohex-3-en-1-yl) methyl)-1-((1-ethyl-1*H*-imidazol-5-yl)methyl)-1*H*-benzo[*d*]imidazole-6-carboxylic acid hydrochloride (compound 29)**

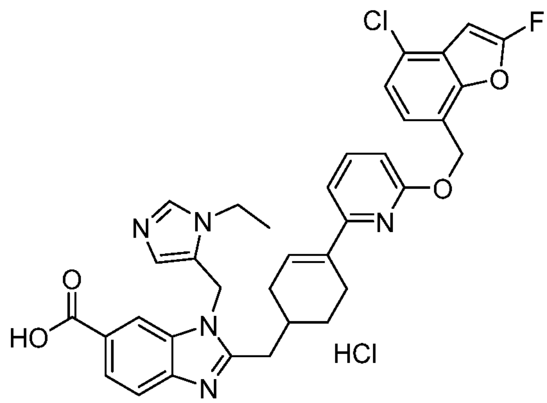

**Step 1: Synthesis of 1-ethyl-1-hydro-imidazole-5-carboxamide**

1-Ethyl-l-hydro-imidazole-5-carboxylic acid (485 mg, 3.46 mmol) was dissolved in sulfoxide chloride (6 mL), then DMF (0.05 mL) was added, heated to 80 °C and the reaction was carried out for 5 h. The reaction was cooled to room temperature. The solvent was concentrated, then tetrahydrofuran (10 mL) was added, ammonia (3 mL, 19.47 mmol) was added slowly, and the reaction was stirred at room temperature overnight. Stopped and filtered, and the filtrate was concentrated and the residue was purified by column chromatography eluted with DCM:MeOH (v/v)=10:1 to give a white solid (266 mg, 55.2%).

LC-MS (ESI): $[M+H]^+$=140.2.

**Step 2: Synthesis of (1-ethyl-1-hydro-imidazol-5-yl)methylamine hydrochloride**

1-Ethyl-1-hydro-imidazole-5-carboxatnide (1.0 g, 7.19 mmol) was dissolved in THF (35 mL), then borane dimethyl sulfide (7.2 mL, 14.37 mmol) was added, heated to 70 °C, and the reaction was stirred for 8 h. Methanol (10 mL) was slowly added, and the reaction was stirred for 10 min at room temperature, then 1,4-dioxane hydrochloride solution (1mL), the reaction was stirred for 2 hours, a white solid precipitated. The mixture was concentrated to give a white solid (842 mg, 85.1%).

LC-MS (ESI): $[M-HCl+ H]^+$ =126.3.

**Step 3: Synthesis of methyl 3-(((1-ethyl-1-hydro-imidazol-5-yl)methyl)amino)-4-nitrobenzoate**

Methyl 3-fluoro-4-nitrobenzoate (600 mg, 3.01 mmol) and (1-ethyl-1-hydro-imidazol-5-yl)methylamine hydrochloride (487 mg, 3.01 mmol) were dissolved in the mixture of THF (15 mL) and methanol (10 mL). Triethylamine (2.5 mL, 18.08 mmol) was then added and the reaction was heated to 60 °C overnight. Stopped and the solvent was concentrated, diluted with water (30 mL) and extracted with ethyl acetate (15 mL × 3). The combined organic phases were dried with anhydrous sodium sulfate, filtered, and the filtrate concentrated. The residue was purification by column chromatography eluted with DCM:MeOH (v/v)=15:1 to give a yellow solid (482 mg, 52.6%).

$^1$H NMR (500 MHz, $CDCl_3$) δ 8.22 (d, *J* = 8.8 Hz, 1H), 7.92 (t, *J* = 5.1 Hz, 1H), 7.67 (d, *J* = 1.9 Hz, 1H), 7.54 (s, 1H), 7.31 (dd, *J* = 8.9, 1.7 Hz, 1H), 7.09 (s, 1H), 4.52 (d, *J* = 4.9 Hz, 2H), 3.99 (q, *J* = 7.3 Hz, 2H), 3.94 (s, 3H), 1.46 (t, *J* = 7.4 Hz, 3H).

LC-MS (ESI): $[M+ H]^+$ =305.3.

**Step 4: Synthesis of methyl 4-amino-3-(((1-ethyl-1-hydro-imidazol-5-yl)methyl)amino)benzoate**

Methyl 3-(((1-ethyl-1-hydro-imidazol-5-yl)methyl)amino)-4-nitrobenzoate (400 mg, 1.31 mmol) was dissolved in methanol (15 mL), then palladium-carbon (210 mg, 10% Pd in 40-60% water) was added, protected by hydrogen and the reaction was stirred at room temperature overnight. Stopped and filtered with diatomaceous earth, the solvent was concentrated and the residue was purified by column chromatography eluted with DCM:MeOH (v/v)=20:1 to give a white solid (303 mg, 84.0%).

$^1$H NMR (500 MHz, $CDCl_3$) δ 7.49 (d, *J* = 8.3 Hz, 1H), 7.46 (s, 1H), 7.42 (s, 1H), 6.89 (s, 1H), 6.68 (d, *J* = 8.1 Hz, 1H), 4.24 (s, 2H), 3.99 (q, *J* = 7.3 Hz, 2H), 3.86 (s, 3H), 1.43 (t, *J* = 7.4 Hz, 3H).

LC-MS (ESI): $[M+ H]^+$ =275.2.

**Step 5: Synthesis of methyl 4-(2-(4-(4-(6-((4-chloro-2-fluorobenzofuran-7-yl)methoxy)pyridin-2-yl)cyclohex-3-en-1-yl)acetamido)-3-(((1-ethyl-1*H*-imidazol-5-yl)methyl)amino)benzoate**

2-(4-(6-((4-chloro-2-fluorobenzofuran-7-yl)methoxy)pyridin-2-yl)cyclohex-3-en-1-yl)acetic acid (200 mg, 0.48 mmol) and methyl 4-atnino-3-(((1-ethyl-1-hydro-imidazol-5-yl)methyl)amino)benzoate (158 mg, 0.58 mmol) were added to *N*,*N*-dimethylformamide (4 mL), HATU (329 mg, 0.87 mmol) and DIPEA (155 mg, 1.20 mmol) were added and the reaction was carried out at 40 °C overnight. The solvent was removed under reduced pressure, water (50 mL) was added, extracted with ethyl acetate (25 mL×3), the combined organic phases were dried with anhydrous sodium sulfate, concentrated under reduced pressure, and the residue was purified by column chromatography eluted with MeOH:DCM (v/v) = 5% to give a light brown solid (209 mg, 64.7%).

$^1$HNMR (500 MHz, $CDCl_3$) δ 7.81 (s, 1H), 7.79 (br.s, 1H), 7.54 - 7.46 (m, 3H), 7.43 (s, 1H), 7.30 (d, *J* = 8.1 Hz, 1H), 7.21 (d, *J* = 8.2 Hz, 1H), 7.03 (s, 1H), 6.91 (d, *J* = 7.5 Hz, 1H), 6.71 (s, 1H), 6.63 (d, *J* = 8.2 Hz, 1H), 5.98 (d, *J* = 6.6 Hz, 1H), 5.59 (s, 2H), 4.30 (s, 2H), 4.07 - 4.00 (m, 2H), 3.88 (s, 3H), 2.59 - 2.51 (m, 1H), 2.47 - 2.36 (m, 5H), 2.03 - 1.92 (m, 3H), 1.45 (t, *J* = 7.3 Hz, 3H).

LC-MS (ESI): $[M+H]^+$ =672.4.

**Step 6: Synthesis of methyl 2-((4-(6-((4-chloro-2-fluorobenzofuran-7-yl)methoxy)pyridin-2-yl)cyclohex-3-en-1-yl)methyl)-1-((1-ethyl-1*H*-imidazol-5-yl)methyl)-1*H*-benzo[*d*]imidazole-6-carboxylate**

Methyl 4-(2-(4-(4-(6-((4-chloro-2-fluorobenzofuran-7-yl)methoxy)pyridin-2-yl)cyclohex-3-en-1-yl)acetamido)-3-(((1-ethyl-1*H*-imidazol-5-yl)methyl)amino)benzoate (200 mg, 0.30 mmol) was added to glacial acetic acid (5 mL), and the reaction was carried out at 70 °C for 20 h. The solvent was removed, the residue was dissolved in ethyl acetate and concentrated under reduced pressure (20 mL×2), and the residue was purified by column chromatography eluted with MeOH:DCM (v/v)=10% to give a light yellow solid (106 mg, 54.5%).

$^1$HNMR (500 MHz, $CDCl_3$) δ 8.07 - 7.94 (m, 2H), 7.77 (d, *J*= 8.2 Hz, 1H), 7.56 - 7.47 (m, 2H), 7.31 (d, *J* = 7.9 Hz, 1H), 7.29 - 7.20 (m, 1H), 6.93 (d, *J* = 7.4 Hz, 1H), 6.76 (d, *J* = 6.9 Hz, 1H), 6.72 (s, 1H), 6.64 (d,*J* = 8.0 Hz, 1H), 5.99 (d, *J* = 6.6 Hz, 1H), 5.61 (s, 2H), 5.36 (s, 2H), 3.93 (s, 3H), 3.82 (q, *J* = 7.2 Hz, 2H), 2.94 - 2.84 (m, 2H), 2.64 - 2.54 (m, 1H), 2.50 - 2.37 (m, 2H), 2.38 - 2.27 (m, 1H), 2.06 - 1.94 (m, 2H), 1.59 - 1.50 (m, 1H), 1.25 (d, *J* = 5.5 Hz, 3H).

LC-MS (ESI): $[M+H]^+$ =654.4.

**Step 7: Synthesis of 2-((4-(6-((4-chloro-2-fluorobenzofuran-7-yl)methoxy)pyridin-2-yl)cyclohex-3-en-1-yl)methyl)-1-((1-ethyl-1*H*-imidazol-5-yl)methyl)-1*H*-benzo[*d*]imidazole-6-carboxylic acid hydrochloride**

Methyl 2-((4-(6-((4-chloro-2-fluorobenzofuran-7-yl)methoxy)pyridin-2-yl)cyclohex-3-en-1-yl)methyl)-1-((1-ethyl-1*H*-imidazol-5-yl)methyl)-1*H*-benzo[*d*]imidazole-6-carboxylate (100 mg, 0.15 mmol) and lithium hydroxide monohydrate (52 mg, 1.24 mmol) were added to the mixture of 1,4-dioxane (3 mL) and water (3 mL), and the reaction was carried out at 40 °C for 1.5 h. The reaction was cooled in an ice bath, the pH was adjusted to 6 with dilute hydrochloric acid (2 M), concentrated under reduced pressure, and the residue was purified by column chromatography eluted with MeOH: DCM (v/v)=18% to give a white solid (73 mg, 74.6%).

$^1$HNMR (500 MHz, DMSO-$d_6$) δ 8.13 (s, 1H), 7.82 (d, *J* = 8.4 Hz, 1H), 7.72 - 7.61 (m, 3H), 7.45 - 7.36 (m, 2H), 7.04 (d, *J* = 7.4 Hz, 1H), 6.75 - 6.67 (m, 2H), 6.49 (d, *J* = 6.3 Hz, 1H), 6.45 (s, 1H), 5.64 (s, 2H), 5.59 (s, 2H), 3.96 (q, *J* = 7.7 Hz, 2H), 2.99 - 2.86 (m, 2H), 2.56 - 2.49 (m, 1H),1.42 - 2.24 (m, 2H), 2.20 - 2.10 (m, 1H), 2.04 - 1.87 (m, 2H), 1.49 - 1.37 (m, 1H),1.13 (t, *J* = 7.8 Hz, 3H).

LC-MS (ESI): $[M+H-HCl]^+$ =640.4.

**Example 30 Synthesis of (*S*)-2-((4-(6-((4-chloro-2-fluorobenzofuran-7-yl)methoxy)pyridin-2-yl)cyclohexyl) methyl)-3-(oxetan-2-ylmethyl)-3*H*-imidazo[4,5-*b*]pyridine-5-carboxylic acid (compound 30)**

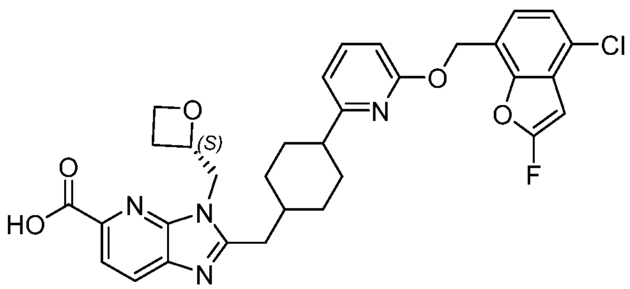

**Step 1: Synthesis of 6-chloro-5-nitropicolinic acid**

2-Chloro-6-methyl-3-nitropyridine (8.0 g, 46.36 mmol) was added to concentrated sulfuric acid (40 mL), and potassium dichromate (20.46 g, 69.54 mmol) was added slowly at room temperature, and the reaction was stirred at room temperature overnight. Under the condition of ice bath, ice water (500 mL) was added slowly, extracted with ethyl acetate (150 mL×3), the combined organic phases were dried with anhydrous sodium sulfate, and concentrated under reduced pressure to give a light yellow solid (8.26 g, 88.0%).

LC-MS (ESI): $[M+H]^+$ =203.0.

**Step 2: Synthesis of methyl 6-chloro-5-nitropicolinate**

6-chloro-5-nitropicolinic acid (200 mg, 0.99 mmol) was dissolved in methanol (15 mL), concentrated sulfuric acid (0.5 mL, 0.99 mmol) was added, and the reaction was heated to 68 °C overnight. The solvent was concentrated, diluted with water (30 mL), extracted with ethyl acetate (15 mL×3), the combined organic phases were dried with anhydrous sodium sulfate, filtered, and the filtrate was concentrated. The residue was purified by column chromatography eluted with PE:EtOAc (v/v)=2:1 to give a white solid (185 mg, 86.5%).

LC-MS (ESI): $[M+H]^+$ =217.1;
$^1$H NMR (500 MHz, $CDCl_3$) δ 8.31 (dd, *J* = 8.3, 0.8 Hz, 1H), 8.22 (dd, *J* = 8.2, 0.8 Hz, 1H), 4.04 (d, *J* = 0.9 Hz, 3H).

**Step 3: Synthesis of methyl (5)-5-nitro-6-((oxetan-2-ylmethyl)amino)picolinate**

methyl 6-chloro-5-nitropicolinate (1.3 g, 6.0 mmol) and (S)-oxetane-2-methanamine (0.58 g, 6.6 mmol) were dissolved in tetrahydrofuran (20 mL), and potassium carbonate (1.24 g, 9.00 mmol) was added and stirred at room temperature overnight. Filtered with diatomaceous earth, concentrated and the residue was purified by column chromatography eluted with PE:EtOAc (v/v)=2:1 to give a yellow solid (1.09 g, 68.0%).

LC-MS (ESI): $[M+ H]^+$ =268.2;
$^1$H NMR (500 MHz, DMSO-$d_6$) δ 8.58 (dd, *J*= 8.4, 1.0 Hz, 1H), 8.51 (t, *J* = 5.8 Hz, 1H), 7.31 (dd, *J* = 8.4, 1.0 Hz, 1H), 5.01 - 4.93 (m, 1H), 4.56 -4.52 (m, 1H), 4.48 - 4.45 (m, 1H), 3.96 - 3.87 (m, 4H), 3.82 (dt, *J* = 13.8, 5.2 Hz, 1H), 2.68 - 2.58 (m, 1H), 2.53 - 2.49 (m, 1H).

**Step 4: Synthesis of methyl (5)-5-amino-6-((oxetan-2-ylmethyl)amino)picolinate**

methyl (*S*)-5-nitro-6-((oxetan-2-ylmethyl)amino)picolinate (1.09 g, 4.08 mmol) was dissolved in the mixture of tetrahydrofuran (10 mL) and methanol (10 mL), then palladium-carbon (10% Pd in 40-60% water, 185 mg, 1.74 mmol) was added, protected by hydrogen. The reaction was stirred at room temperature for 8 hours. Filtered with diatomaceous earth and concentrated. The residue was purified by column chromatography eluted with DCM:MeOH (v/v)=20:1 to give an oily yellow viscous (885 mg, 91.5%).

LC-MS (ESI): $[M+H]^+$ =238.1;
1H NMR (500 MHz, DMSO-$d_6$) δ 7.23 (d, *J* = 7.8 Hz, 1H), 6.68 (d, *J* = 7.8 Hz, 1H), 5.95 (t, *J* = 5.6 Hz, 1H), 5.61 (s, 2H), 4.92 - 4.87 (m, 1H), 4.54 - 4.50 (m, 1H), 4.45 (dt, *J* = 9.0, 5.9 Hz, 1H), 3.72 (s, 4H), 3.62 (dt, *J* = 13.8, 5.2 Hz, 1H), 2.65 - 2.57 (m, 1H), 2.44 - 2.39 (m, 1H).

**Step 5: Synthesis of 2-(benzyloxy)-6-bromopyridine**

6-bromopyridin-2-ol (2.0 g, 11.49 mmol) and benzyl bromide (2.16 g, 12.64 mmol) were dissolved in DMF (15 mL), and then potassium carbonate (3.18 g, 22.99 mmol) was added, and the reaction was heated to 60 °C for 2 h, and then cooled to room temperature. Water (80 mL) was added, extracted with ethyl acetate (25 mL×2), the combined organic phases were dried with anhydrous sodium sulfate, concentrated under reduced pressure, and the residue was purified by column chromatography eluted with EtOAc:PE (v/v)=5% to give a colorless liquid (2.1 g, 69.2 %).

**Step 6: Synthesis of ethyl 2-(4-(6-(benzyloxy)pyridin-2-yl)cyclohex-3-en-1-yl)acetate**

2-(Benzyloxy)-6-bromopyridine (500 mg, 1.97 mmol), 2-(4-(4,4,5,5-tetramethyl-1,3,2-dioxaborolan-2-yl)cyclohex-3-en-1-yl)ethyl acetate (608 mg, 2.07 mmol), potassium carbonate (408 mg, 2.95 mmol), and 1,1'-bis(diphenylphosphinyl ) ferrocene palladium(II) dichloride dichloromethane complex (80 mg, 0.10 mmol) were added to the mixture of 1,4-dioxane (8 mL) and water (2 mL), and the reaction was heated to 90 °C under nitrogen protection for 4.5 h. The reaction was cooled to room temperature, quenched by the addition of water (30 mL), and extracted with ethyl acetate (20 mL × 3). the combined organic phases were dried with anhydrous sodium sulfate, filtered with diatomaceous earth, concentrated under reduced pressure, and the residue was purified by column chromatography eluted with EtOAc:PE (v/v)=10% to give a colorless liquid (673 mg, 97.3%).

$^1$H NMR (500 MHz, $CDCl_3$) δ 7.51 (t, *J* = 7.8 Hz, 1H), 7.48 - 7.44 (m, 2H), 7.36 (t, *J* = 7.4 Hz, 2H), 7.29 (t, *J* = 7.4 Hz, 1H), 6.93 (d, *J* = 7.4 Hz, 1H), 6.78 (tt, *J* = 3.8, 1.7 Hz, 1H), 6.63 (d, *J* = 8.2 Hz, 1H), 5.41 (s, 2H), 4.16 (q, *J* = 7.1 Hz, 2H), 2.66 - 2.59 (m, 1H), 2.51 -2.40 (m, 2H), 2.34 (d, *J* = 7.2 Hz, 2H), 2.22 - 2.15 (m, 1H), 2.02 - 1.94 (m, 2H), 1.52 - 1.44 (m, 1H), 1.27 (t, *J* = 7.2 Hz, 3H).

**Step 7: Synthesis of 2-(4-(6-(benzyloxy)pyridin-2-yl)cyclohex-3-en-1-yl)acetic acid**

Ethyl 2-(4-(6-(benzyloxy)pyridin-2-yl)cyclohex-3-en-1-yl)acetate (490 mg, 1.39 mmol) and lithium hydroxide monohydrate (293 mg, 6.98 mmol) were added to the mixture of 1,4-dioxane (5 mL) and water (4 mL), and the reaction was carried out at 50 °C for 2 h. The pH was adjusted to 4 with dilute hydrochloric acid to the mixture in an ice bath. Extracted with ethyl acetate (20 mL×3). The combined organic phases were dried with anhydrous sodium sulfate, and the solvent was removed under reduced pressure to give a light yellow solid (386 mg, 85.6%).

LC-MS (ESI): $[M+H]^+$ =324.2;
$^1$H NMR (500 MHz, $CDCl_3$) δ 7.51 (t, *J* = 7.8 Hz, 1H), 7.46 (d, *J* = 7.6 Hz, 2H), 7.36 (t, *J* = 7.5 Hz, 2H), 7.33 - 7.26 (m, 1H), 6.93 (d, *J* = 7.4 Hz, 1H), 6.77 (d, *J* = 4.2 Hz, 1H), 6.64 (d, *J* = 8.2 Hz, 1H), 5.41 (s, 2H), 2.68 - 2.60 (m, 1H), 2.54 - 2.44 (m, 2H), 2.40 (d, *J* = 7.2 Hz, 2H), 2.26 - 2.14 (m, 1H), 2.05 - 1.96 (m, 2H), 1.55 - 1.46 (m, 1H).

**Step 8: Synthesis of methyl 5-(2-(4-(6-(benzyloxy)pyridin-2-yl)cyclohex-3-en-1-yl)acetamido)-6-((((S)-oxetan-2-yl)methyl)amino)picolinate**

2-(4-(6-(benzyloxy)pyridin-2-yl)cyclohex-3-en-1-yl)acetic acid (375 mg, 1.16 mmol) and methyl (*S*)-5-amino-6-((oxetan-2-ylmethyl)amino)pyridinecarboxylate (250 mg, 1.05 mmol) were added to *N,N*-dimethylformamide (5 mL), HATU ( 601 mg, 1.58 mmol) and DIPEA (341 mg, 2.64 mmol) were added, the reaction was carried out at 50 °C overnight, water (60 mL) was added, and the organic phases were extracted with ethyl acetate (20 mL×3), the combined organic phases were dried with anhydrous sodium sulfate, concentrated under reduced pressure, and the residue was purified by column chromatography eluted with EtOAc:PE (v/v) = 70% to give a light brown oil (301 mg, 52.6%).

LC-MS (ESI): $[M+H]^+$ =543.4;

$^1$H NMR (500 MHz, $CDCl_3$) δ 8.06 - 7.98 (m, 2H), 7.53 - 7.44 (m, 4H), 7.37 - 7.25 (m, 3H), 6.92 (d, *J* = 7.4 Hz, 1H), 6.75 (s, 1H), 6.64 (d, *J* = 8.0 Hz, 1H), 5.40 (s, 2H), 5.30 - 5.21 (m, 1H), 5.08 - 5.00 (m, 1H), 4.70 - 4.61 (m, 1H), 4.59 - 4.50 (m, 1H), 3.91 (s, 3H), 3.82 - 3.73 (m, 1H), 2.69 - 2.56 (m, 2H), 2.54 - 2.35 (m, 4H), 2.32 - 2.21 (m, 1H), 2.08 - 1.84 (m, 3H), 1.54 - 1.44 (m, 1H).

**Step 9: Synthesis of 2-((4-(6-(benzyloxy)pyridin-2-yl)cyclohex-3-en-1-yl)methyl)-3-(((*S*)-oxetan-2-yl)methyl)-3*H*-imidazo[4,5-*b*]pyridine-5-carboxylic acid**

Methyl 5-(2-(4-(6-(benzyloxy)pyridin-2-yl)cyclohex-3-en-1-yl)acetamido)-6-((((S)-oxetan-2-yl)methyl)amino) picolinate (500 mg, 0.92 mmol) was dissolved in tetrahydrofuran (15 mL), and potassium tert-butanate (259 mg, 2.31 mmol) was added and counteracted at 85 °C for 2 h. The solvent was removed under reduced pressure, water (50 mL) was added, the pH was adjusted to 5 with dilute hydrochloric acid, extracted with ethyl acetate (15 mL×3), the combined organic phases were dried with anhydrous sodium sulfate, and concentrated under reduced pressure to give a light brown solid (463 mg, 98.4%).

LC-MS (ESI): $[M+H]^+$ =511.4.

**Step 10: Synthesis of methyl 2-((4-(6-(benzyloxy)pyridin-2-yl)cyclohex-3-en-1-yl)methyl)-3-(((*S*)-oxetan-2-yl) methyl)-3*H*-imidazo[4,5-*b*]pyridine-5-carboxylate**

2-((4-(6-(benzyloxy)pyridin-2-yl)cyclohex-3-en-1-yl)methyl)-3-(((*S*)-oxetan-2-yl)methyl)-3*H*-imidazo[4,5-*b*]pyridine-5-carboxylic acid (0.7 g, 1.37 mmol), methanol (439 mg, 13.7 mmol), and 1-hydroxy-7-azobenzotriazole ( 280 mg, 2.06 mmol) were dissolved in the mixture of dichloromethane (10 mL) and DMF (5 mL), 1-ethyl-(3-dimethylaminopropyl) carbodiimide hydrochloride (657 mg, 3.43 mmol) was added, cooled in an ice bath, and DIPEA (532 mg, 4.12 mmol) was added, and the reaction was carried out at room temperature overnight, the solvent was removed under reduced pressure, water (50 mL) was added, extracted with dichloromethane (15 mL×3), the combined organic phases were dried with anhydrous sodium sulfate, concentrated under reduced pressure, and the residue was purified by column chromatography eluted with EtOAc:PE (v/v) = 60% to give a white solid (208 mg, 28.9%).

LC-MS (ESI): $[M+H]^+$ =525.5.

**Step 11: Synthesis of methyl (*S*)-2-((4-(6-hydroxypyridin-2-yl)cyclohexyl)methyl)-3-(oxetan-2-ylmethyl)-3*H*-imidazo[4,5-*b*]pyridine-5-carboxylate**

methyl 2-((4-(6-(benzyloxy)pyridin-2-yl)cyclohex-3-en-1-yl)methyl)-3-(((*S*)-oxetan-2-yl)methyl)-3*H*-imidazo [4,5-*b*]pyridine-5-carboxylate (190 mg, 0.36 mmol) was dissolved in a solution of methanol (5 mL), then palladium-carbon (118 mg, 10% Pd in 40-60% water) was added, and the reaction was carried out at room temperature under the protection of hydrogen at 1 atm overnight, the reaction was filtered with diatomaceous earth, and the filtrate was concentrated to give a pale yellow viscous (127 mg, 80.3%).

LC-MS (ESI): $[M+ H]^+$ =437.4.

**Step 12: Synthesis of methyl (*S*)-2-((4-(6-((4-chloro-2-fluorobenzofuran-7-yl)methoxy)pyridin-2-yl)cyclohexyl) methyl)-3-(oxetan-2-ylmethyl)-3*H*-imidazo[4,5-*b*]pyridine-5-carboxylate**

Methyl (*S*)-2-((4-(6-hydroxypyridin-2-yl)cyclohexyl)methyl)-3-(oxetan-2-ylmethyl)-3*H*-imidazo[4,5-*b*]pyridine-5-carboxylate (100 mg, 0.23 mmol) was dissolved in DMF (5 mL), 7-(bromomethyl)-4-chloro-2-fluorobenzofuran (61 mg, 0.23 mmol) and potassium carbonate (143 mg, 1.03 mmol) were added, and the reaction was carried out at 60 °C for 3 h. The reaction mixture was concentrated under reduced pressure and the residue was purified by column chromatography eluted with EtOAc:PE (v/v)=40% to give a yellow oil (35 mg, 24.6%).

LC-MS (ESI): $[M+H]^+$ =619.5;

$^1$HNMR (500 MHz, $CDCl_3$) δ 8.08 (d, *J* = 8.2 Hz, 1H), 8.03 (d, *J* = 8.3 Hz, 1H), 7.52 (t, *J* = 7.9 Hz, 1H), 7.37 - 7.29 (m, 1H), 7.24 (d, *J* = 8.3 Hz, 1H), 6.81 (d, *J* = 7.3 Hz, 1H), 6.61 (d, *J* = 8.2 Hz, 1H), 5.99 (d, *J* = 6.6 Hz, 1H), 5.61 (s, 2H), 5.21 - 5.17 (m, 1H), 4.64 - 4.53 (m, 3H), 4.35 - 4.30 (m, 1H), 3.99 (s, 3H), 3.16 - 3.05 (m, 2H), 2.83 - 2.68 (m, 2H), 2.52 - 2.42 (m, 2H), 2.07 - 1.99 (m, 2H), 1.83 - 1.62 (m, 6H).

**Step 13: Synthesis of (*S*)-2-((4-(6-((4-chloro-2-fluorobenzofuran-7-yl)methoxy)pyridin-2-yl)cyclohexyl)methyl)-3-(oxetan-2-ylmethyl)-3*H*-imidazo[4,5-*b*]pyridine-5-carboxylic acid**

Methyl (*S*)-2-((4-(6-((4-chloro-2-fluorobenzofuran-7-yl)methoxy)pyridin-2-yl)cyclohexyl)methyl)-3-(oxetan-2-ylmethyl)-3*H*-imidazo[4,5-*b*]pyridine-5-carboxylate (35 mg, 0.06 mmol) and lithium hydroxide monohydrate (13 mg, 0.31 mmol) were add to the mixture of 1,4-dioxane (3 mL) and water (1.5 mL), the reaction was carried out at 40 °C for 1h, the pH was adjusted to 6 with dilute hydrochloric acid (2M) in an ice bath, extracted with ethyl acetate (10 mL×3), the combined organic phases were dried with anhydrous sodium sulfate, concentrated under reduced pressure, and the residue was purified by column chromatography eluted with MeOH: DCM (v/v)=5% to give a light yellow solid (25 mg, 71.4%).

LC-MS (ESI): $[M+H]^+$ =605.4;

$^1$HNMR (500 MHz, $CDCl_3$) δ 8.24 - 8.13 (m, 2H), 7.52 (t, *J* = 8.0 Hz, 1H), 7.36 - 7.32 (m, 1H), 7.25 (d, *J* = 8.3 Hz, 1H), 6.82 (d, *J* = 7.4 Hz, 1H), 6.62 (d, *J* = 8.3 Hz, 1H), 6.00 (d, *J* = 6.6 Hz, 1H), 5.62 (s, 2H), 5.24 - 5.15 (m, 1H), 4.65 - 4.46 (m, 3H), 4.39 - 4.29 (m, 1H), 3.81 - 3.63 (m, 2H), 3.16 - 3.04 (m, 2H), 2.83 - 2.70 (m, 2H), 2.52 - 2.39 (m, 2H), 2.09 - 1.95 (m, 2H), 1.86 - 1.67 (m, 4H).

**Example 31 Synthesis of 2-((4-(4-((4-chloro-2-fluorobenzofuran-7-yl)methoxy)-5-fluoropyrimidin-2-yl)cyclohex-3-en-1-yl)methyl)-1-(((*S*-oxetan-2-yl)methyl)-1*H*-benzo[*d*]imidazole-6-carboxylic acid (compound 31)**

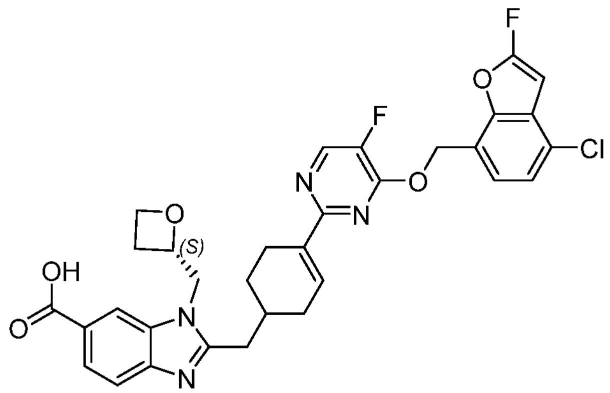

**Step 1: Synthesis of (4-chloro-2-fluorobenzofuran-7-yl)methyl acetate**

7-(Bromomethyl)-4-chloro-2-fluorobenzofuran (0.60 g, 2.28 mmol) was added to DMF (12 mL), then potassium acetate (1.10 g, 11.21 mmol) was added. The reaction was carried out at 50 °C for 1.5 h. The reaction was quenched by the addition of water (100 mL), extracted with ethyl acetate (15 mL × 3). The combined organic phase was washed with saturated saline (15 mL×3), dried with anhydrous sodium sulfate, filtered, concentrated under reduced pressure, and the residue was purified by column chromatography eluted with EtOAc:PE (v/v)=5% to give a light yellow liquid (340 mg, 61.5%).

**Step 2: Synthesis of (4-chloro-2-fluorobenzofuran-7-yl)methanol**

(4-chloro-2-fluorobenzofuran-7-yl)methyl acetate (340 mg, 1.40 mmol) was added to the mixture of 1,4-dioxane (9 mL) and water (3 mL), followed by lithium hydroxide monohydrate (177 mg, 4.22 mmol). The reaction was carried out at room temperature for 2 h. Diluted with water (6 ml), extracted with ethyl acetate (3 mL × 3), and the combined organic phases were dried with anhydrous sodium sulfate. Filtered, concentrated and the residue was purified by column chromatography eluted with EtOAc:PE (v/v)=20% to give a white solid (222 mg, 79.0%).

$^1$H NMR (500 MHz, $CDCl_3$) δ 7.24 (s, 2H), 6.00 (d, *J* = 6.6 Hz, 1H), 4.92 (d, *J* = 6.1 Hz, 2H).

**Step 3: Synthesis of 2-chloro-4-((4-chloro-2-fluorobenzofuran-7-yl)methoxy)-5-fluoropyrimidine**

(4-Chloro-2-fluorobenzofuran-7-yl)methanol (222 mg, 1.11 mmol) and 2,4-dichloro-5-fluoropyrimidine (194 mg, 1.16 mmol) were dissolved in acetonitrile (10 mL), and cesium carbonate (545 mg, 1.67 mmol) was added in an ice bath

condition. The reaction was brought to room temperature for 14 h. Filtered with diatomaceous earth, the filtrate was concentrated and the residue was purified by column chromatography eluted with EtOAc:PE (v/v)=10% to give a white solid (318 mg, 86.8%).

LC-MS (ESI): $[M+H]^+$= 331.1;
$^1$H NMR (500 MHz, DMSO-$d_6$) δ 8.64 (d, $J$ = 2.6 Hz, 1H), 7.50 (d, $J$ = 8.2 Hz, 1H), 7.46 (d, $J$ = 8.3 Hz, 1H), 6.55 (d, $J$ = 6.4 Hz, 1H), 5.73 (s, 2H).

**Step 4: Synthesis of ethyl 2-(4-(4-((4-chloro-2-fluorobenzofuran-7-yl)methoxy)-5-fluoropyrimidin-2-yl)cyclohex-3-en-1-yl)acetate**

2-Chloro-4-((4-chloro-2-fluorobenzofuran-7-yl)methoxy)-5-fluoropyrimidine (200 mg, 0.60 mmol), 2-(4-(4-(4,4,5,5-tetramethyl-1,3,2-dioxaborolan-2-yl)cyclohex-3-en-1-yl)ethyl acetate (213 mg, 0.72 mmol), potassium carbonate (167 mg, 1.21 mmol) and 1,1'-bis(diphenylphosphinyl)ferrocene dichloropalladium(II) dichloromethane complex (49 mg, 0.06 mmol) were added to the mixture of 1,4-dioxane (4 mL) and water (1 mL). The reaction was heated to 90 °C under nitrogen protection for 5 h. Quenched with water (100 mL), extracted with ethyl acetate (30 mL × 3), and the combined organic phases were dried with anhydrous sodium sulfate, filtered with diatomaceous earth, concentrated under reduced pressure, and the residue was purified by column chromatography eluted with EtOAc:PE (v/v)=10% to give a colorless liquid (183 mg, 65.5%).

LC-MS (ESI): $[M+H]^+$= 463.3;
$^1$H NMR (500 MHz, $CDCl_3$) δ 8.27 (d, $J$ = 2.6 Hz, 1H), 7.32 (d, $J$ = 8.2 Hz, 1H), 7.26 (d, $J$ = 8.2 Hz, 1H), 7.11 (s, 1H), 6.02 (d, $J$ = 6.6 Hz, 1H), 5.73 (s, 2H), 4.16 (q, $J$ = 7.1 Hz, 2H), 2.76 - 2.67 (m, 1H), 2.50 - 2.42 (m, 2H), 2.35 - 2.31 (m, 2H), 2.22 - 2.12 (m, 1H), 2.06 - 1.90 (m, 2H), 1.47 - 1.39 (m, 1H), 1.27 (t, $J$ = 8.8 Hz, 3H).

**Step 5: Synthesis of 2-(4-(4-((4-chloro-2-fluorobenzofuran-7-yl)methoxy)-5-fluoropyrimidin-2-yl)cyclohex-3-en-1-yl)acetic acid**

Ethyl 2-(4-(4-((4-chloro-2-fluorobenzofuran-7-yl)methoxy)-5-fluoropyrimidin-2-yl)cyclohex-3-en-1-yl)acetate (170 mg, 0.37 mmol) and lithium hydroxide monohydrate (78 mg, 1.86 mmol) were added to the mixture of 1,4-dioxane (4 mL) and water (2 mL), and the reaction was carried out at 40 °C for 1 h. The pH was adjusted to 4 with dilute hydrochloric acid in an ice bath. Extracted with ethyl acetate (15 mL × 3). The combined organic phases were dried with anhydrous sodium sulfate, and the solvent was removed under reduced pressure to give a light yellow solid (156 mg, 97.7%).

LC-MS (ESI): $[M+H]^+$ =435.2;
$^1$H NMR (500 MHz, $CDCl_3$) δ 8.28 (d, $J$ = 2.6 Hz, 1H), 7.32 (d, $J$ = 8.2 Hz, 1H), 7.26 (d, $J$ = 8.2 Hz, 1H), 7.13 - 7.10 (m, 1H), 6.02 (d, $J$ = 6.6 Hz, 1H), 5.73 (s, 2H), 2.76 - 2.68 (m, 1H), 2.54 - 2.43 (m, 2H), 2.40 (d, $J$ = 7.1 Hz, 2H), 2.23 - 2.15 (m, 1H), 2.06 - 1.96 (m, 2H), 1.52 - 1.42 (m, 1H).

**Step 6: Synthesis of methyl 4-(2-(4-(4-((4-chloro-2-fluorobenzofuran-7-yl)methoxy)-5-fluoropyrimidin-2-yl)cyclohex-3-en-1-yl)acetamido)-3-((((*S*)-oxetan-2-yl)methyl)amino)benzoate**

2-(4-(4-((4-chloro-2-fluorobenzofuran-7-yl)methoxy)-5-fluoropyrimidin-2-yl)cyclohex-3-en-1-yl)acetic acid (155 mg, 0.36 mmol) and methyl (S)-4-amino-3-((oxetan-2-ylmethyl)amino)benzoate (93 mg, 0.39 mmol) were added to the mixture of methylene chloride (4 mL) and *N*,*N*-dimethylformamide (2 mL). HATU (203 mg, 0.53 mmol) and DIPEA (115 mg, 0.89 mmol) were added, and the reaction was carried out at 50 °C overnight. The solvent was removed under reduced pressure, water (30 mL) was added, extracted with ethyl acetate (15 mL×3), the combined organic phases were dried with anhydrous sodium sulfate, concentrated under reduced pressure, and the residue was purified by column chromatography eluted with EtOAc:DCM (v/v)=65% to give a light yellow solid (206 mg, 88.5%).

LC-MS (ESI): $[M+H]^+$ =653.4;
$^1$H NMR (500 MHz, $CDCl_3$) δ 8.27 (d, $J$ = 2.5 Hz, 1H), 7.82 (s, 2H), 7.58 (d, $J$ = 8.3 Hz, 1H), 7.53 (s, 1H), 7.33 (d, $J$ = 8.1 Hz, 1H), 7.26 (d, $J$ = 2.3 Hz, 1H), 7.13 (s, 1H), 6.02 (d, $J$ = 6.6 Hz, 1H), 5.73 (s, 2H), 5.07 - 5.02 (m, 1H), 4.76 - 4.69 (m, 1H), 4.65 - 4.55 (m, 1H), 3.90 (s, 3H), 3.45 - 3.28 (m, 3H), 2.77 - 2.68 (m, 2H), 2.57 - 2.40 (m, 4H), 2.35 - 2.26 (m, 1H), 2.12 - 1.98 (m, 2H), 1.54 - 1.46 (m, 1H).

**Step 7: Synthesis of methyl 2-((4-(4-((4-chloro-2-fluorobenzofuran-7-yl)methoxy)-5-fluoropyrimidin-2-yl)cyclohex-3-en-l-yl)methyl)-1-((*S*)-oxetan-2-yl)methyl)-1*H*-benzo[*d*]imidazole-6-carboxylate**

Methyl 4-(2-(4-(4-((4-chloro-2-fluorobenzofuran-7-yl)methoxy)-5-fluoropyrimidin-2-yl)cyclohex-3-en-1-yl)acetamido)-3-((((*S*)-oxetan-2-yl)methyl)amino)benzoate (96 mg, 0.15 mmol), p-toluenesulfonic acid monohydrate (28 mg, 0.15 (0.15 mmol) were added to tetrahydrofuran (4 mL), and the reaction was carried out at 75 °C for 4 h. The solvent was removed under reduced pressure, water (10 mL) was added, extracted with ethyl acetate (10 mL×2), the combined organic phases were dried with anhydrous sodium sulfate, concentrated under reduced pressure, and the residue was purified by column chromatography eluted with EtOAc:PE (v/v)=70% to give a light brown solid (46 mg, 49.3%).

LC-MS (ESI): $[M+H]^+$ =635.4.

**Step 8: Synthesis of 2-((4-(4-((4-chloro-2-fluorobenzofuran-7-yl)methoxy)-5-fluoropyrimidin-2-yl)cyclohex-3-en-1-yl)methyl)-1-(((*S*-oxetan-2-yl)methyl)-1*H*-benzo[*d*]imidazole-6-carboxylic acid**

methyl 2-((4-(4-((4-chloro-2-fluorobenzofuran-7-yl)methoxy)-5-fluoropyrimidin-2-yl)cyclohex-3-en-1-yl) methyl)-1-(((*S*)-oxetan-2-yl)methyl)-1*H*-benzo[*d*]imidazole-6-carboxylate (40 mg, 0.06 mmol) and lithium hydroxide monohydrate (27 mg, 0.64 mmol) were added to the mixture of 1,4-dioxane (3 mL) and water (1.5 mL). and the reaction was carried out at 40 °C for 1 h. The reaction was cooled in an ice bath, the pH was adjusted to 6 with aqueous acetic acid (2 M), extracted with ethyl acetate (15 mL×3), the combined organic phases were dried with anhydrous sodium sulfate, concentrated under reduced pressure, and the residue was purified by column chromatography eluted with MeOH: DCM (v/v)=5% to give a white solid (9 mg, 21.6%).

LC-MS (ESI): $[M+H]^+$ =621.3;
$^1$HNMR (500 MHz, $CDCl_3$) δ 8.27 (d, *J* = 2.6 Hz, 1H), 8.16 (s, 1H), 8.04 (d, *J* = 8.4 Hz, 1H), 7.79 (d, *J* = 8.5 Hz, 1H), 7.32 (d, *J* = 8.3 Hz, 1H), 7.26 (d, *J* = 2.3 Hz, 1H), 7.13 (s, 1H), 6.01 (d, *J* = 6.6 Hz, 1H), 5.71 (s, 2H), 5.22 - 5.15 (m, 1H), 4.64 - 4.60 (m, 1H), 4.50 - 4.32 (m, 3H), 3.05 (d, *J* = 6.8 Hz, 2H), 2.79 - 2.70 (m, 2H), 2.56 - 2.41 (m, 4H), 2.18 - 2.11 (m, 1H), 2.08 - 1.99 (m, 2H).

**Example 32 Synthesis of 2-((4-(6-((4-chlorobenzofuran-7-yl)methoxy-*d*$_2$)pyridin-2-yl)cyclohex-3-en-1-yl) methyl)-1-(((*S*)-oxetan-2-yl)methyl)-1*H*-benzo[*d*]imidazole-6-carboxylic acid (compound 32)**

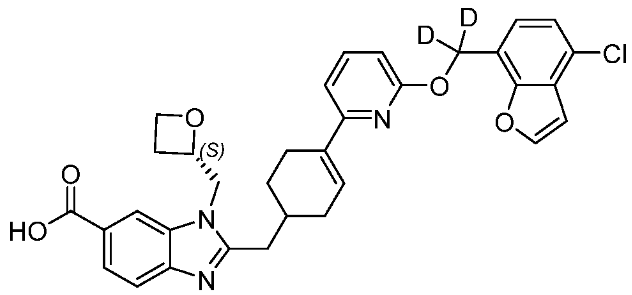

**Step 1: Synthesis of (4-chlorobenzofuran-7-yl)methyl acetate**

7-(Bromomethyl)-4-chlorobenzofuran (2.50 g, 10.18 mmol) was added to DMF (20 mL), then potassium acetate (7.00 g, 71.28 mmol) was added. The reaction was carried out at room temperature for 3 h. The reaction was quenched by the addition of water (80 mL), extracted with ethyl acetate (30 mL × 3), and the combined organic phases were washed with saturated saline (30 mL×3), dried with anhydrous sodium sulfate, and concentrated under reduced pressure to give a brown oil (2.0 g, 87.4%).

**Step 2: Synthesis of (4-chlorobenzofuran-7-yl)methanol**

(4-chlorobenzofuran-7-yl)methyl acetate (2.0 g, 8.90 mmol) was added to the mixture of 1,4-dioxane (30 mL) and water (8 mL), then lithium hydroxide monohydrate (1.12 g, 26.71 mmol) was added. The reaction was heated to 42 °C for 2 h. Cooled to room temperature, diluted with water (60 mL), extracted with ethyl acetate (30 mL×3), the combined organic phases were dried with anhydrous sodium sulfate. Filtered, concentrated and the residue was purified by column

chromatography eluted with PE:EtOAc (v/v)=3:1 to give a white solid (1.6 g, 98.4%).

LC-MS (ESI): $[M-H_2O+H]^+$ =165.0;
$^1$H NMR (500 MHz, DMSO-$d_6$) δ 8.10 (d, *J* = 2.2 Hz, 1H), 7.33 (q, *J* = 8.0 Hz, 2H), 6.99 (d, *J* = 2.2 Hz, 1H), 5.36 (d, *J* = 5.3 Hz, 1H), 4.79 (d, *J* = 2.8 Hz, 2H).

**Step 3: Synthesis of 4-chlorobenzofuran-7-carboxylic acid**

(4-Chlorobenzofuran-7-yl)methanol (390 mg, 2.14 mmol) was dissolved in 1,4-dioxane (12 mL), potassium bromide (26 mg, 0.22 mmol), sodium bicarbonate (269 mg, 3.20 mmol), and water (4 mL) were added, and cooled in an ice bath with stirring, then 2,2,6,6-tetramethylpiperidinium oxide (17 mg, 0.11 mmol) and add 10% sodium hypochlorite solution (6.4 mL) were added, The reaction was carried out at room temperature for 30 min, water (60 mL) was added, the pH was adjusted to 5 with dilute hydrochloric acid, extracted with ethyl acetate for (15 mL × 3), the combined organic phase were dried with anhydrous sodium sulfate, concentrated under reduced pressure, the residue was purificated by column chromatography eluted with EtOAc: DCM (v/v) = 60% to give a white solid (146 mg, 34.8%).
$^1$HNMR (500 MHz, DMSO-$d_6$) δ 8.24 (s, 1H), 7.86 (d, *J* = 8.2 Hz, 1H), 7.47 (d, *J* = 8.2 Hz, 1H), 7.12 (s, 1H).

**Step 4: Synthesis of (4-chlorobenzofuran-7-yl)methan-$d_2$-ol**

4-Chlorobenzofuran-7-carboxylic acid (275 mg, 1.40 mmol) was dissolved in dichloromethane (20 mL), CDI (272 mg, 1.68 mmol) was added, and the reaction was carried out at room temperature for 30 min, then water (30 mL) was added, extracted with dichloromethane (20 mL×2), and the combined organic phases were washed with saturated aqueous ammonium chloride (20 mL×2). The organic phase was dried with anhydrous sodium sulfate, concentrated under reduced pressure, deuterated methanol-$d_4$ (8 mL) was added, cooled in an ice bath, sodium boron deuteride (70 mg, 1.67 mmol) was added with stirring, and the reaction was carried out at room temperature for 30 min, quenched with deuterated water (0.5 mL), and then the solvent was removed under reduced pressure, and then extracted with ethyl acetate (15 mL×3), and the combined organic phases were dried with anhydrous sodium sulfate, concentrated under reduced pressure, and the residue was purified by column chromatography eluted with EtOAc:PE (v/v)=20% to give a white solid (103 mg, 39.9%).
$^1$HNMR (500 MHz, DMSO-$d_6$) δ 8.11 (d, *J* = 2.2 Hz, 1H), 7.37 - 7.30 (m, 2H), 7.00 (d, *J* = 2.2 Hz, 1H), 5.30 (s, 1H).

**Step 5: Synthesis of 2-bromo-6-((4-chlorobenzofuran-7-yl)methoxy-$d_2$)pyridine**

(4-chlorobenzofuran-7-yl)methan-$d_2$-ol (138 mg, 0.75 mmol) and 2-bromo-6-fluoropyridine (157 mg, 0.89 mmol) were dissolved in DMF (5 mL), and cesium carbonate (484 mg, 1.49 mmol) was added. The reaction was carried out at room temperature overnight. The reaction was quenched with water (15 mL), extracted with ethyl acetate (10 mL×3), the combined organic phases were dried with anhydrous sodium sulfate, concentrated under reduced pressure, and the residue was purified by column chromatography eluted with EtOAc:PE (v/v)=5% to give a white solid (212 mg, 83.3%).
$^1$H NMR (500 MHz, $CDCl_3$) δ 7.68 (d, *J* = 2.2 Hz, 1H), 7.42 (t, *J* = 7.8 Hz, 1H), 7.37 (d, *J* = 7.9 Hz, 1H), 7.24 (d, *J* = 7.9 Hz, 1H), 7.08 (d, *J* = 7.5 Hz, 1H), 6.89 (d, *J* = 2.3 Hz, 1H), 6.73 (d, *J* = 8.2 Hz, 1H).

**Step 6: Synthesis of ethyl 2-(4-(6-((4-chlorobenzofuran-7-yl)methoxy-$d_2$)pyridin-2-yl)cyclohex-3-en-1-yl)acetate**

2-bromo-6-((4-chlorobenzofuran-7-yl)methoxy-$d_2$)pyridine (210 mg, 0.62 mmol), ethyl 2-(4-(4,4,5,5-tetramethyl-1,3,2-dioxaborolan-2-yl)cyclohex-3-en-1-yl)acetate (201 mg, 0.68 mmol), potassium carbonate (172 mg, 1.24 mmol) and 1,1'-bis(diphenylphosphino)ferrocene palladium(II) dichloride dichloromethane complex (25 mg, 0.03 mmol) were added to the mixture of 1,4-dioxane (8 mL) and water (2 mL). The reaction was heated to 90 °C under nitrogen protection for 2.5 h. The reaction was cooled to room temperature, quenched with water (50 mL), extracted with ethyl acetate (15 mL × 3), and the combined organic phases were dried with anhydrous sodium sulfate, filtered with diatomaceous earth, concentrated under reduced pressure, and the residue was purified by column chromatography eluted with EtOAc:PE (v/v)=5% to give a colorless liquid (252 mg, 95.0%).

LC-MS (ESI): $[M+H]^+$ = 428.2;
$^1$H NMR (500 MHz, $CDCl_3$) δ 7.68 (d, *J* = 2.2 Hz, 1H), 7.52 (t, *J* = 7.8 Hz, 1H), 7.36 (d, *J* = 8.0 Hz, 1H), 7.22 (d, *J* = 8.0 Hz, 1H), 6.93 (d, *J* = 7.5 Hz, 1H), 6.89 (d, *J* = 2.2 Hz, 1H), 6.76 - 6.72 (m, 1H), 6.64 (d, *J* = 8.1 Hz, 1H), 4.16 (q, *J* = 7.1 Hz, 2H), 2.64 - 2.56 (m, 1H), 2.50 - 2.38 (m, 2H), 2.33 (d, *J* = 7.2 Hz, 2H), 2.22 - 2.12 (m, 1H), 2.03 - 1.91 (m, 2H), 1.51 - 1.42 (m, 1H), 1.28 (t, *J* = 7.2 Hz, 3H).

**Step 7: Synthesis of 2-(4-(6-((4-chlorobenzofuran-7-yl)methoxy-*d*$_2$)pyridin-2-yl)cyclohex-3-en-1-yl)acetic acid**

Ethyl 2-(4-(6-((4-chlorobenzofuran-7-yl)methoxy-*d*$_2$)pyridin-2-yl)cyclohex-3-en-1-yl)acetate (250 mg, 0.60 mmol) and lithium hydroxide monohydrate (127 mg, 3.03 mmol) were added to the mixture of 1,4-dioxane (6 mL) and water (3 mL), and the reaction was carried out at 50 °C for 2 h. The pH was adjusted to 4 with dilute hydrochloric acid in an ice bath, and extracted with ethyl acetate (15 mL×3). The combined organic phases were dried with anhydrous sodium sulfate, and the solvent was removed under reduced pressure to give a colorless liquid (233 mg, 96.5%).

LC-MS (ESI): $[M+H]^+$ =400.2;
$^1$H NMR (500 MHz, $CDCl_3$) δ 7.68 (d, *J* = 2.2 Hz, 1H), 7.52 (t, *J* = 7.8 Hz, 1H), 7.36 (d, *J* = 8.0 Hz, 1H), 7.22 (d, *J* = 8.0 Hz, 1H), 6.94 (d, *J* = 7.5 Hz, 1H), 6.89 (d, *J* = 2.2 Hz, 1H), 6.75 - 6.71 (m, 1H), 6.64 (d, *J* = 8.1 Hz, 1H), 2.64 - 2.57 (m, 1H), 2.50 - 2.43 (m, 2H), 2.40 (d, *J* = 7.2 Hz, 2H), 2.23 - 2.15 (m, 1H), 2.04 - 1.95 (m, 2H), 1.54 - 1.46 (m, 1H).

**Step 8: Synthesis of methyl 4-(2-(4-(6-((4-chlorobenzofuran-7-yl)methoxy-*d*$_2$)pyridin-2-yl)cyclohex-3-en-1-yl)acetamido)-3-((((*S*)-oxetan-2-yl)methyl)amino)benzoate**

2-(4-(6-((4-chlorobenzofuran-7-yl)methoxy-*d*$_2$)pyridin-2-yl)cyclohex-3-en-1-yl)acetic acid (220 mg, 0.55 mmol) and methyl (*S*)-4-amino-3-((oxetan-2-ylmethyl)amino)benzoate (143 mg, 0.61 mmol) were added to the mixture of dichloromethane (3 mL) and *N*,*N*-dimethylformamide (3 mL), HATU (314 mg, 0.83 mmol) and DIPEA (178 mg, 1.38 mmol) were added, and the reaction was carried out at 40 °C overnight. The solvent was removed under reduced pressure, water (30 mL) was added, extracted with ethyl acetate (15 mL×3), the organic combined phases were dried with anhydrous sodium sulfate, and concentrated under reduced pressure to give a light brown solid (286 mg, 84.1%).
LC-MS (ESI): $[M+H]^+$ =618.3.

**Step 9: Synthesis of methyl 2-((4-(6-((4-chlorobenzofuran-7-yl)methoxy-*d*)pyridin-2-yl)cyclohex-3-en-1-yl)methyl)-1-(((*S*)-oxetan-2-yl)methyl)-1*H*-benzo[*d*]imidazole-6-carboxylate**

Methyl **4-(2-(4-(6-((4-chlorobenzofuran-7-yl)methoxy-*d*$_2$)pyridin-2-yl)cyclohex-3-en-1-yl)acetamido)-3-**((((*S*)-oxetan-2-yl)methyl)amino)benzoate (280 mg, 0.45 mmol) was dissolved in acetic acid (6 mL), the reaction was carried out at 70 °C for 3 h. The solvent was removed under reduced pressure. Water (30 mL) was added, the pH was adjusted to 8 with saturated aqueous sodium bicarbonate, extracted with ethyl acetate (15 mL×3), the combined organic phases were dried with anhydrous sodium sulfate, concentrated under reduced pressure, and the residue was purified by column chromatography eluted with EtOAc:PE (v/v)=60% to give a light-brown solid (203 mg, 74.7%).
LC-MS (ESI): [M+H]+ =600.3.

**Step 10: Synthesis of 2-((4-(6-((4-chlorobenzofuran-7-yl)methoxy-*d*$_2$)pyridin-2-yl)cyclohex-3-en-1-yl)methyl)-1-(((*S*)-oxetan-2-yl)methyl)-1*H*-benzo[*d*]imidazole-6-carboxylic acid**

Methyl 2-((4-(6-((4-chlorobenzofuran-7-yl)methoxy-*d*$_2$)pyridin-2-yl)cyclohex-3-en-1-yl)methyl)-1-(((*S*)-oxetan-2-yl)methyl)-1*H*-benzo[*d*]imidazole-6-carboxylate (200 mg, 0.33 mmol) and lithium hydroxide monohydrate (70 mg, 1.67 mmol) was added to the mixture of 1,4-dioxane (6 mL) and water (3 mL), and the reaction was carried out at 40 °C for 1 h. The reaction was cooled in an ice bath, the pH was adjusted to 4 with dilute hydrochloric acid aqueous solution (1 M), extracted by ethyl acetate (15 mL×3), and the combined organic phases were dried with anhydrous sodium sulfate, concentrated under reduced pressure, and the residue was purified by column chromatography eluted with MeOH: DCM (v/v)=1:30 to give a white solid (79 mg, 39.9%).

LC-MS (ESI): $[M+H]^+$ =586.4;
$^1$HNMR (500 MHz, DMSO-*d*$_6$) δ 12.67 (br.s, 1H), 8.21 (s, 1H), 8.16 (s, 1H), 7.79 (d, *J* = 8.3 Hz, 1H), 7.69 - 7.60 (m, 2H), 7.42 (d, *J* = 8.0 Hz, 1H), 7.34 (d, *J* = 8.0 Hz, 1H), 7.06 (d, *J* = 7.5 Hz, 1H), 7.04 (s, 1H), 6.73 (s, 1H), 6.70 (d, *J* = 8.3 Hz, 1H), 5.06 - 4.99 (m, 1H), 4.68 - 4.60 (m, 1H), 4.55 - 4.42 (m, 2H), 4.33 - 4.27 (m, 1H), 3.05 - 2.92 (m, 2H), 2.73 - 2.65 (m, 1H), 2.61 - 2.53 (m, 1H), 2.45 - 2.29 (m, 4H), 2.11 - 1.94 (m, 2H), 1.55 - 1.44 (m, 1H).

**Example 33 Synthesis of 2-((4-(6-((4-chloro-2-fluorobenzofuran-7-yl)methoxy-*d*$_2$)pyridin-2-yl)cyclohex-3-en-1-yl)methyl)-1-(((*S*-oxetan-2-yl)methyl)-1*H*-benzo[*d*]imidazole-6-carboxylic acid (compound 33)**

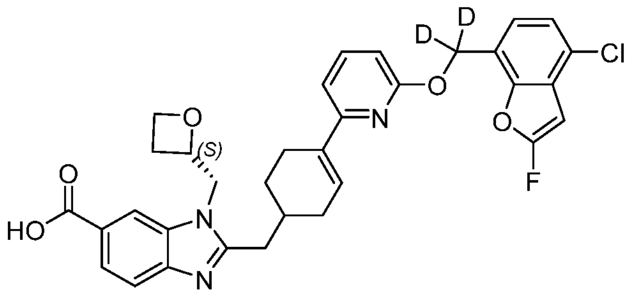

**Step 1: Synthesis of (4-chloro-2-fluorobenzofuran-7-yl)methyl acetate**

7-(Bromomethyl)-4-chloro-2-fluorobenzofuran (0.60 g, 2.28 mmol) was added to DMF (12 mL), then potassium acetate (1.10 g, 11.21 mmol) was added. The reaction was carried out at 50 °C for 1.5 h. quenched with water (100 mL), extracted with ethyl acetate (15 mL × 3), the combined organic phase was washed with saturated saline (15 mL×3), dried with anhydrous sodium sulfate, filtered, concentrated under reduced pressure, and the residue was purified by column chromatography eluted with EtOAc:PE (v/v)=5% to give a light yellow liquid (340 mg, 61.5%).

**Step 2: Synthesis of (4-chloro-2-fluorobenzofuran-7-yl)methanol**

(4-chloro-2-fluorobenzofuran-7-yl)methyl acetate (340 mg, 1.40 mmol) was added to the mixture of 1,4-dioxane (6 mL) and water (3 mL), then lithium hydroxide monohydrate (177 mg, 4.22 mmol) was added.The reaction was carried out at room temperature for 2 h. It was diluted with water (6 mL), extracted with ethyl acetate (3 mL×3), the combined organic phases were dried with anhydrous sodium sulfate. Filtered, concentrated and the residue was purified by column chromatography eluted with EtOAc:PE (v/v)=20% to give a white solid (222 mg, 79.0%).

$^1$H NMR (500 MHz, $CDCl_3$) δ 7.24 (s, 2H), 6.00 (d, *J* = 6.6 Hz, 1H), 4.92 (d, *J* = 6.1 Hz, 2H).

**Step 3: Synthesis of 4-chloro-2-fluorobenzofuran-7-carboxylic acid**

Potassium bromide (50 mg, 0.42 mmol), sodium bicarbonate (1.05 g, 12.5 mmol) and water (12 mL) were dissolved, then added to the mixture of (4-chloro-2-fluorobenzofuran-7-yl)methanol (832 mg, 4.15 mmol) and 1,4-dioxane (30 mL), cooled in an ice bath with stirring, and 2,2,6,6-tetramethylpiperidine oxide (65 mg, 0.42 mmol) was added, then 10% sodium hypochlorite aqueous solution (9.9 mL) was added, the reaction was carried out at room temperature for 4 h, water (100 mL) was added, and the pH was adjusted to 4 with dilute hydrochloric acid, extracted with ethyl acetate (25 mL × 3), the combined organic phases were dried with anhydrous sodium sulfate, concentrated under reduced pressure, the residue was purificated by column chromatography eluted with EtOAc:DCM (v/v)=60% to give a white solid (186 mg, 20.9%).

LC-MS (ESI): $[M+H]^+$= 215.0.

**Step 4: Synthesis of (4-chloro-2-fluorobenzofuran-7-yl)methan-$d_2$-ol**

4-Chloro-2-fluorobenzofuran-7-carboxylic acid (180 mg, 0.84 mmol) was dissolved in tetrahydrofuran (8 mL), CDI (163 mg, 1.01 mmol) was added, the reaction was carried out at room temperature for 30 min, water (30 mL) was added, and the organic phases were combined and washed with saturated aqueous ammonium chloride (20 mL×2). The organic phase was dried with anhydrous sodium sulfate, concentrated under reduced pressure, added deuterated methanol-$d_4$ (8 mL), cooled in an ice bath, sodium borodeuteride (43 mg, 1.03 mmol) was added under stirring, the reaction was carried out for 30 min at room temperature, Quenched with deuterated water (0.5 mL), and the solvent was removed under reduced pressure, extracted with ethyl acetate (15 mL×3), the combined organic phases were dried with anhydrous sodium sulfate, concentrated under reduced pressure and the residue was purified by column chromatography eluted with EtOAc:PE (v/v)=20% to give a white solid (81 mg, 47.7%).

$^1$HNMR (500 MHz, DMSO-$d_6$) δ 7.40 - 7.31 (m, 2H), 6.46 (d, *J* = 6.4 Hz, 1H), 5.35 (s, 1H).

**Step 5: Synthesis of 2-bromo-6-((4-chloro-2-fluorobenzofuran-7-yl)methoxy-$d_2$)pyridine**

(4-chloro-2-fluorobenzofuran-7-yl)methan-$d_2$-ol (57 mg, 0.28 mmol), 2-bromo-6-hydroxypyridine (59 mg, 0.34 mmol) and triphenylphosphine (104 mg, 0.40 mmol) were dissolved in tetrahydrofuran (4 mL), cooled in an ice bath, and DIAD (86 mg, 0.43 mmol) was added. The reaction was carried out at room temperature for 1 h. Quenched with water (30

mL), extracted with ethyl acetate (15 mL×3), and the combined organic phases were dried with anhydrous sodium sulfate, concentrated under reduced pressure, and the residue was purified by column chromatography eluted with EtOAc:PE (v/v)=5%) to give a colorless liquid (63 mg, 62.6%).

$^1$H NMR (500 MHz, $CDCl_3$) δ 7.43 (t, *J* = 7.8 Hz, 1H), 7.32 (s, 1H), 7.25 (d, *J* = 8.9 Hz, 2H), 7.09 (d, *J* = 7.5 Hz, 1H), 6.73 (d, *J* = 8.2 Hz, 1H), 6.01 (d, *J* = 6.6 Hz, 1H).

**Step 6: Synthesis of ethyl 2-(4-(6-((4-chloro-2-fluorobenzofuran-7-yl)methoxy-*d*$_2$)pyridin-2-yl)cyclohex-3-en-1-yl)acetate**

2-bromo-6-((4-chloro-2-fluorobenzofuran-7-yl)methoxy-*d*$_2$)pyridine (57 mg, 0.16 mmol), ethyl 2-(4-(4-(4,4,5,5-tetramethyl-1,3,2-dioxaborolan-2-yl)cyclohex-3-en-1-yl)acetate (47 mg, 0.16 mmol), potassium carbonate (44 mg, 0.32 mmol), potassium carbonate (44 mg, 0.32 mmol) and 1,1'-bis(diphenylphosphino)ferrocene palladium(II) dichloride dichloromethane complex (7 mg, 0.01 mmol) were added to the mixture of 1,4-dioxane (4 mL) and water (1 mL). The reaction was heated to 90 °C under nitrogen protection for 1.5 h. The reaction was cooled to room temperature, Quenched with water (30 mL), extracted with ethyl acetate (15 mL × 3), and the combined organic phases were dried with anhydrous sodium sulfate, filtered with diatomaceous earth, concentrated under reduced pressure, and the residue was purified by column chromatography eluted with EtOAc:PE (v/v)=5% to give a colorless liquid (57 mg, 80.4%).

LC-MS (ESI): $[M+H]^+$ = 446.2;
$^1$H NMR (500 MHz, $CDCl_3$) δ 7.52 (t, *J* = 7.9 Hz, 1H), 7.32 (d, *J* = 8.2 Hz, 1H), 7.23 (d, *J* = 8.2 Hz, 1H), 6.94 (d, *J* = 7.4 Hz, 1H), 6.76 - 6.73 (m, 1H), 6.63 (d, *J* = 8.1 Hz, 1H), 6.00 (d, *J* = 6.6 Hz, 1H), 4.16 (q, *J* = 7.1 Hz, 2H), 2.63 - 2.56 (d, *J* = 17.2 Hz, 1H), 2.49 - 2.40 (m, 2H), 2.34 (d, *J* = 7.2 Hz, 2H), 2.21 - 2.12 (m, 1H), 2.03 - 1.92 (m, 2H), 1.51 - 1.44 (m, 1H), 1.28 (t, *J* = 7.2 Hz, 3H).

**Step 7: Synthesis of 2-(4-(6-((4-chloro-2-fluorobenzofuran-7-yl)methoxy-*d*$_2$)pyridin-2-yl)cyclohex-3-en-1-yl) acetic acid**

ethyl 2-(4-(6-((4-chloro-2-fluorobenzofuran-7-yl)methoxy-*d*$_2$)pyridin-2-yl)cyclohex-3-en-1-yl)acetate (55 mg, 0.13 mmol) and lithium hydroxide monohydrate (17 mg, 0.41 mmol) were added to the mixture of 1,4-dioxane (3 mL) and water (1.5 mL), and the reaction was carried out at 40 °C for 1 h. The pH was adjusted to 4 with dilute hydrochloric acid in an ice bath, extracted with ethyl acetate (15 mL × 3). The combined organic phases were dried with anhydrous sodium sulfate, and the solvent was removed under reduced pressure to give a colorless liquid (52 mg, 97.7%).

LC-MS (ESI): $[M+H]^+$ =418.2.

**Step 8: Synthesis of methyl 4-(2-(4-(6-((4-chloro-2-fluorobenzofuran-7-yl)methoxy-*d*$_2$)pyridin-2-yl)cyclohex-3-en-1-yl)acetamido)-3-((((*S*)-oxetan-2-yl)methyl)amino)benzoate**

2-(4-(6-((4-chloro-2-fluorobenzofuran-7-yl)methoxy-*d*$_2$)pyridin-2-yl)cyclohex-3-en-1-yl)acetic acid (50 mg, 0.12 mmol) and methyl (S)-4-amino-3-((oxetan-2-ylmethyl)amino)benzoate (34 mg, 0.14 mmol) were added to the mixture of dichloromethane (3 mL) and *N*,*N*-dimethylformamide (1.5 mL), HATU (38 mg, 0.18 mmol) and DIPEA (47 mg, 0.36 mmol) were added, and the reaction was carried out at 40 °C overnight. The solvent was removed under reduced pressure, water (30 mL) was added, extracted with ethyl acetate (15 mL×3), the combined organic phases were dried with anhydrous sodium sulfate, concentrated under reduced pressure, and the residue was purified by column chromatography eluted with EtOAc:PE (v/v)=55% to give a white solid (53 mg, 69.6%).

LC-MS (ESI): $[M+H]^+$ =636.4.

**Step 9: Synthesis of methyl 2-((4-(6-((4-chloro-2-fhiorobenzofuran-7-yl)methoxy-*d*$_2$)pyridin-2-yl)cyclohex-3-en-1-yl)methyl)-1-(((*S*)-oxetan-2-yl)methyl)-1*H*-benzo[*d*]imidazole-6-carboxylate**

methyl 4-(2-(4-(6-((4-chloro-2-fluorobenzofuran-7-yl)methoxy-*d*$_2$)pyridin-2-yl)cyclohex-3-en-1-yl)acetamido)-3-((((*S*)-oxetan-2-yl)methyl)amino)benzoate (50 mg, 0.45 mmol) was dissolved in acetic acid (5 mL), the reaction was carried out at 80 °C for 4 h. The solvent was removed under reduced pressure. Water (30 mL) was added, and the pH was adjusted to 8 with saturated aqueous sodium bicarbonate, extracted with ethyl acetate (15 mL×3), the combined organic phases were dried with anhydrous sodium sulfate, concentrated under reduced pressure, and the residue was purified by column chromatography eluted with EtOAc:PE (v/v)=60% to give a light brown solid (35 mg, 72.0%).

LC-MS (ESI): $[M+H]^+$ =618.4.

**Step 10: Synthesis of 2-((4-(6-((4-chloro-2-fhiorobenzofuran-7-yl)methoxy-*d*$_2$)pyridin-2-yl)cyclohex-3-en-1-yl) methyl)-1-(((*S*)-oxetan-2-yl)methyl)-1*H*-benzo[*d*]imidazole-6-carboxylic acid**

methyl 2-((4-(6-((4-chloro-2-fluorobenzofuran-7-yl)methoxy-*d*$_2$)pyridin-2-yl)cyclohex-3-en-1-yl) methyl)-1-(((*S*)-oxetan-2-yl)m ethyl)-1*H*-benzo[*d*]imidazole-6-carboxylate (35 mg, 0.06 mmol) and Lithium hydroxide monohydrate (24 mg, 0.57 mmol) was added to the mixture of 1,4-dioxane (4 mL) and water (2 mL), and the reaction was carried out at 40 °C for 1 h. The reaction was cooled in an ice bath, the pH was adjusted to 6 with acetic acid, and the organic phases were extracted with ethyl acetate (15 mL×3), and the combined organic phases were dried with anhydrous sodium sulfate, concentrated under reduced pressure, and the residue was purified by column chromatography eluted with MeOH: DCM (v/v)=1:30 to give white solid (26 mg, 76.0%).

LC-MS (ESI): $[M+H]^+$ =604.5;

$^1$HNMR (500 MHz, DMSO-*d*$_6$) δ 12.66 (br.s, 1H), 8.21 (s, 1H), 7.79 (d, *J* = 8.4 Hz, 1H), 7.69 - 7.60 (m, 2H), 7.43 - 7.38 (m, 2H), 7.06 (d, *J* = 7.5 Hz, 1H), 6.74 - 6.69 (m, 2H), 6.50 (d, *J* = 6.3 Hz, 1H), 5.06 - 4.99 (m, 1H), 4.67 - 4.60 (m, 1H), 4.56 - 4.42 (m, 2H), 4.32 - 4.27 (m, 1H), 3.04 - 2.92 (m, 2H), 2.73 - 2.64 (m, 1H), 2.59 - 2.50 (m, 1H), 2.40 - 2.27 (m, 4H), 2.10 - 1.94 (m, 2H), 1.56 - 1.44 (m, 1H).

**Example 34 Synthesis of 2-((4-(6-((4-chlorobenzofuran-7-yl)methoxy)pyridin-2-yl)cyclohex-3-en-1-yl) methyl)-3-(((*S*)-oxetan-2-yl)methyl)-3*H*-imidazo[4,5-*b*]pyridine-5-carboxylic acid (compound 34)**

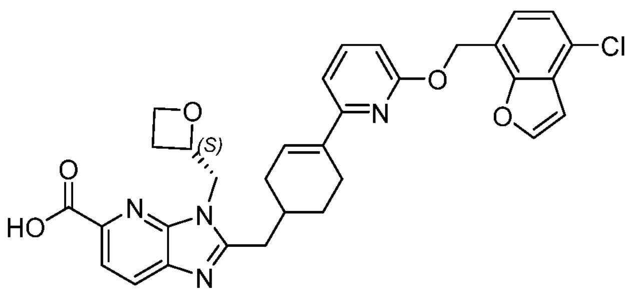

**Step 1: Synthesis of methyl 5-(2-(4-(6-((4-chlorobenzofuran-7-yl)methoxy)pyridin-2-yl)cyclohex-3-en-1-yl)acetamido)-6-((((S)-oxetan-2-yl)methyl)amino)picolinate**

2-(4-(6-((4-chlorobenzofuran-7-yl)methoxy)pyridin-2-yl)cyclohex-3-en-1-yl)acetic acid (250 mg, 0.63 mmol) and methyl (S)-5-amino-6-((oxetan-2-ylmethyl)amino)picolinate (149 mg, 0.63 mmol) were added to the mixture of dichloromethane (8 mL) and DMF (8 mL), HATU (358 mg, 0.94 mmol) and DIPEA (203 mg, 1.57 mmol) were added, the reaction was heated to 45 °C overnight, the solvent was removed under reduced pressure, water (30 mL) was added, and the mixture was extracted with ethyl acetate (20 mL×3), the combined organic phases were dried with anhydrous sodium sulfate, concentrated under reduced pressure, and purified by column chromatography eluted with PE:EtOAc (v/v) =1:1 to give a yellow-orange solid (158 mg, 40.7%).

$^1$H NMR (500 MHz, $CDCl_3$) δ 8.03 (d, *J* = 8.0 Hz, 1H), 7.93 (s, 1H), 7.67 (d, *J* = 2.2 Hz, 1H), 7.52 (d, *J* = 7.6 Hz, 2H), 7.35 (d, *J* = 8.0 Hz, 1H), 7.21 (d, *J* = 8.0 Hz, 1H), 6.92 (d, *J* = 7.5 Hz, 1H), 6.88 (d, *J* = 2.3 Hz, 1H), 6.73 (s, 1H), 6.64 (d, *J* = 8.2 Hz, 1H), 5.69 (s, 2H), 5.20 (s, 1H), 5.07- 5.02 (m, 1H), 4.67 (q, *J* = 7.4 Hz, 1H), 4.58-4.53(m, 1H), 3.91 (s, 3H), 3.78 (d, *J* = 17.6 Hz, 1H), 3.73 - 3.64 (m, 1H), 2.79 (s, 2H), 2.70 - 2.63 (m, 1H), 2.59 (d, *J* = 17.4 Hz, 1H), 2.47 (d, *J* = 7.7 Hz, 1H), 2.40 (d, *J* = 7.1 Hz, 1H), 2.27 (s, 1H), 1.98 (s, 2H), 1.49 (s, 1H).

LC-MS (ESI): $[M+H]^+$ =617.4.

**Step 2: Synthesis of 2-((4-(6-((4-chlorobenzofuran-7-yl)methoxy)pyridin-2-yl)cyclohex-3-en-1-yl) methyl)-3-(((*S*)-oxetan-2-yl)methyl)-3*H*-imidazo[4,5-*b*]pyridine-5-carboxylic acid**

Methyl 5-(2-(4-(6-((4-chlorobenzofuran-7-yl)methoxy)pyridin-2-yl)cyclohex-3-en-1-yl)acetamido)-6-((((S)-oxetan-2-yl)methyl)amino)picolinate (148 mg, 0.24 mmol) was dissolved in isopropanol (3 mL), then potassium tert-butanolate (54 mg, 0.48 mmol) was added. The reaction was heated to 80 °C for 30 min. The solvent was removed under reduced pressure, water (30 mL) was added, the pH was adjusted to 5 with dilute hydrochloric acid solution (2M), the organic phases were extracted with ethyl acetate (15 mL×3), the combined organic phases were dried with anhydrous

sodium sulfate, concentrated under reduced pressure, and the residue was purified by column chromatography eluted with DCM:MeOH (v/v)=10:1 to give a yellowish solid (40 mg, 28.5%).

$^1$H NMR (500 MHz, $CDCl_3$) δ 8.19 (q, *J* = 8.5 Hz, 2H), 7.67 (s, 1H), 7.52 (t, *J* = 8.1 Hz, 1H), 7.35 (d, *J* = 7.7 Hz, 1H), 7.21 (d, *J* = 8.3 Hz, 1H), 6.94 (d, *J* = 7.7 Hz, 1H), 6.88 (s, 1H), 6.75 (s, 1H), 6.65 (d, *J* = 8.3 Hz, 1H), 5.69 (s, 2H), 5.21 (s, 1H), 4.77 - 4.46 (m, 3H), 4.36 (t, *J* = 7.8 Hz, 1H), 3.12 (q, *J* = 17.3, 13.8 Hz, 2H), 2.76 (s, 1H), 2.66 (d, *J*= 17.6 Hz, 1H), 2.47 (dd, *J* = 26.3, 12.6 Hz, 4H), 2.08 (q, *J* = 16.6, 12.8 Hz, 2H), 1.60 (d, *J* = 25.3 Hz, 1H).

LC-MS (ESI): $[M+H]^+$=585.3.

**Example 35 Synthesis of 2-((4-(4-((4-chlorobenzofuran-7-yl)methoxy)-5-fluoropyrimidin-2-yl)cyclohex-3-en-1-yl)methyl)-3-(((*S*)-oxetan-2-yl)methyl)-3*H*-imidazo[4,5-*b*]pyridine-5-carboxylic acid (compound 35)**

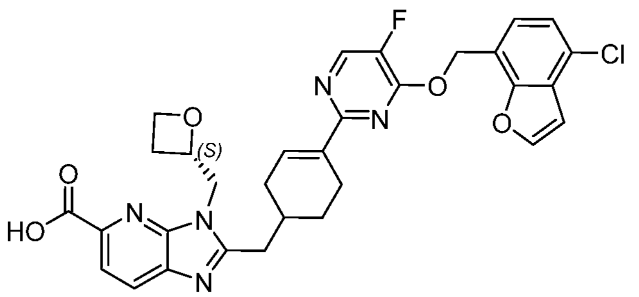

**Step 1: Synthesis of (4-chlorobenzofuran-7-yl)methyl acetate**

7-(bromomethyl)-4-chlorobenzofuran (2.50 g, 10.18 mmol) was added to DMF (20 mL), then potassium acetate (7.00 g, 71.28 mmol) was added, and the reaction was carried out at room temperature for 3 h. Stopped and quenched with water (80 mL), and the reaction was extracted with ethyl acetate (30 mL×3), the combined organic phases were washed with saturated brine (30 mL×3), the organic phase was dried with anhydrous sodium sulfate, and concentrated under reduced pressure to give a brown oil (2.0 g, 87.4%).

**Step 2: Synthesis of (4-chlorobenzofuran-7-yl)methanol**

(4-chlorobenzofuran-7-yl)methyl acetate (2.0 g, 8.90 mmol) was added to the mixture of 1,4-dioxane (30 mL) and water (8 mL), then lithium hydroxide monohydrate (1.12 g, 26.71 mmol) was added, and the reaction was heated to 42 ° C for 2 h. Stopped and the reaction was cooled down to room temperature, diluted with water (60 ml), extracted with ethyl acetate (30 mL×3), the combined organic phases were dried with anhydrous sodium sulfate, filtered, concentrated the filtrate, the residue was purified by column chromatography eluted with PE:EtOAc (v/v)=3:1 to give a white solid (1.6 g, 98.4%).

$^1$H NMR (500 MHz, DMSO-$d_6$) δ 8.10 (d, *J* = 2.2 Hz, 1H), 7.33 (q, *J* = 8.0 Hz, 2H), 6.99 (d, *J* = 2.2 Hz, 1H), 5.36 (d, *J* = 5.3 Hz, 1H), 4.79 (d, *J* = 2.8 Hz, 2H).

LC-MS (ESI): [M-17] =165.0.

**Step 3: Synthesis of 2-chloro-4-((4-chlorobenzofuran-7-yl)methoxy)-5-fluoropyrimidine**

(4-Chlorobenzofuran-7-yl)methanol (850 mg, 4.65 mmol) and 2,4-dichloro-5-fluoropyrimidine (816 mg, 4.89 mmol) were dissolved in acetonitrile (20 mL), and under the condition of ice-bath, cesium carbonate (2.275 g, 6.98 mmol) was added, and the reaction was brought to room temperature for 17 h. Stopped and filtered with diatomaceous earth, the filtrate was concentrated, and the residue was purified by column chromatography eluted with PE:EtOAc (v/v)=10:1 to give a white solid (1.31 g, 89.9%).

$^1$H NMR (500 MHz, DMSO-$d_6$) δ 8.63 (d, *J* = 2.6 Hz, 1H), 8.18 (d, *J* = 2.3 Hz, 1H), 7.49 (d, *J* = 8.0 Hz, 1H), 7.40 (d, *J* = 8.0 Hz, 1H), 7.07 (d, *J* = 2.2 Hz, 1H), 5.77 (s, 2H).

**Step 4: Synthesis of ethyl 2-(4-(4-((4-chlorobenzofuran-7-yl)methoxy)-5-fluoropyrimidin-2-yl)cyclohex-3-en-1-yl)acetate**

Ethyl 2-(4-(4-(4,4,5,5-tetramethyl-1,3,2-dioxaborolan-2-yl)cyclohex-3-en-1-yl)acetate (1.35 g, 4.60 mmol), 2-chloro-4-((4-chlorobenzofuran-7-yl)methoxy)-5-fluoropyrimidine (1.31 g, 4.18 mmol), [1,1'-bis(diphenylphosphine) ferrocene]palladium dichloride (310 mg, 0.42 mmol) and potassium carbonate (1.16 g, 8.37 mmol) were added to the mixture of 1,4-dioxane (32 mL) and water (8 mL), the reaction was carried out under nitrogen protection at 85 °C for 11 h. The reaction was cooled down to room temperature, and then water (100 mL) was added and extracted with ethyl acetate (30 mL×3), the combined organic phases were dried with anhydrous sodium sulfate, concentrated under reduced pressure and the residue was purified by column chromatography eluted with PE:EtOAc (v/v)=10:1 to give a yellow viscous material (1.24 g, 66.6%).

$^1$H NMR (500 MHz, DMSO-$d_6$) δ 8.53 (d, *J* = 2.8 Hz, 1H), 8.17 (d, *J* = 2.2 Hz, 1H), 7.46 (d, *J* = 8.0 Hz, 1H), 7.36 (d, *J* = 8.0 Hz, 1H), 7.06 (dd, *J* = 5.8, 2.4 Hz, 2H), 5.80 (s, 2H), 4.08 (q, *J* = 7.1 Hz, 2H), 2.67 - 2.57 (m, 1H), 2.41 - 2.28 (m, 4H), 2.02 - 1.90 (m, 2H), 1.86- 1.81 (m, 1H), 1.36- 1.29 (m, 1H), 1.19 (t, *J* = 7.1 Hz, 3H).

LC-MS (ESI): $[M+H]^+$ =445.2.

**Step 5: Synthesis of 2-(4-(4-((4-chlorobenzofuran-7-yl)methoxy)-5-fluoropyrimidin-2-yl)cyclohex-3-en-1-yl) acetic acid**

Ethyl 2-(4-(4-((4-chlorobenzofuran-7-yl)methoxy)-5 -fluoropyrimidin-2-yl)cyclohex-3-en-1-yl)acetate (1.24 g, 2.79 mmol) was dissolved in the mixture of 1,4-dioxane (30 mL) and water (10 mL), and lithium hydroxide monohydrate (350 mg, 8.36 mmol) was added, and the reaction was carried out at 40 °C for 2 h. The reaction was cooled to room temperature, diluted with water (60 mL), the pH was adjusted to 5 with dilute hydrochloric acid, extracted with ethyl acetate (30 mL×3), and the combined organic phases were dried with anhydrous sodium sulfate, and concentrated under reduced pressure to give an oily-yellow viscous substance (1.1 g, 94.7%).

LC-MS (ESI): $[M- H]^-$ =417.2.

**Step 6: Synthesis of methyl 5-(2-(4-(4-((4-chlorobenzofuran-7-yl)methoxy)-5-fluoropyrimidin-2-yl)cyclohex-3-en-1-yl)acetamido)-6-((((*S*)-oxetan-2-yl)methyl)amino)picolinate**

2-(4-(4-((4-chlorobenzofuran-7-yl)methoxy)-5-fluoropyrimidin-2-yl)cyclohex-3-en-1-yl)acetic acid (424 mg, 1.02 mmol) and methyl (S)-5-amino-6-((oxetan-2-ylmethyl)amino)picolinate (283 mg, 1.20 mmol) were dissolved in the mixture of DCM (12 mL) and DMF (3 mL), then HATU (580 mg, 1.53 mmol) and *N,N*-diisopropylethylamine (0.3 mL, 2.03 mmol) were added, and the reaction was heated to 50 °C with stirring overnight. Stopped and the reaction of the ingredients was monitored by TLC to be complete. The solvent was concentrated, water (30 mL) was added, extracted with ethyl acetate (15 mL × 3). The combined organic phases were dried with anhydrous sodium sulfate, filtered with diatomaceous earth, the filtrate was concentrated and the residue was purificated by column chromatography eluted with PE:EtOAc (v/v)=2:1 to give a light yellow viscous material (135 mg, 20.9%).

$^1$H NMR (500 MHz, DMSO-$d_6$) δ 9.38 (s, 1H), 8.55 (d, *J* = 2.8 Hz, 1H), 8.18 (d, *J* = 2.2 Hz, 1H), 7.95 (s, 1H), 7.47 (d, *J* = 8.0 Hz, 1H), 7.38 (d, *J* = 8.0 Hz, 1H), 7.32 (d, *J* = 7.9 Hz, 1H), 7.10 (s, 1H), 7.06 (d, *J* = 2.3 Hz, 1H), 6.42 (t, *J* = 5.6 Hz, 1H), 5.82 (s, 2H), 4.92 (p, *J* = 6.3 Hz, 1H), 4.54 - 4.50 (m, 1H), 4.47 - 4.43 (m, 1H), 3.81 (s, 3H), 3.76 - 3.70 (m, 1H), 3.66 - 3.61 (m, 1H), 2.65 - 2.58 (m, 2H), 2.44 (dd, *J* = 12.8, 5.1 Hz, 4H), 2.36 (s, 1H), 2.13 (s, 1H), 2.02 (dd, *J* = 20.0, 6.2 Hz, 1H), 1.94 - 1.87 (m, 1H), 1.46 - 1.38 (m, 1H).

LC-MS (ESI): $[M+H]^+$=636.4.

**Step 7: Synthesis of 2-((4-(4-((4-chlorobenzofuran-7-yl)methoxy)-5-fluoropyrimidin-2-yl)cyclohex-3-en-1-yl) methyl)-3-(((*S*)-oxetan-2-yl)methyl)-3*H*-imidazo[4,5-*b*]pyridine-5-carboxylic acid**

Methyl 5-(2-(4-(4-((4-chlorobenzofuran-7-yl)methoxy)-5-fluoropyrimidin-2-yl)cyclohex-3-en-1-yl)acetamido)-6-((((*S*)-oxetan-2-yl)methyl)amino)picolinate (148 mg, 0.24 mmol) was dissolved in isopropanol (3 mL), and then potassium tert-butoxide (43 mg, 0.38 mmol) was added and heated to 80 °C for 30 min. Stopped and the reaction of the ingredients was monitored by TLC to be complete, diluted with water (30 mL), and the pH was adjusted to 5 with dilute hydrochloric acid solution (2 M). extracted with ethyl acetate (15 mL × 3), and the combined organic phases were dried with anhydrous sodium sulfate, filtered, and the filtrate was concentrated and the residue was purificated by column chromatography eluted with DCM:MeOH (v/v)=10:1 to give a light yellow solid (5 mg, 4.3%).

LC-MS (ESI): $[M+H]^+$ =604.4.

**Example 36 Synthesis of 2-(((*S*)-4-(6-((4-chloro-2-fluorobenzofuran-7-yl)methoxy)pyridin-2-yl)cyclohex-3-en-1-yl)methyl)-1-(((*S*-oxetan-2-yl)methyl)-1*H*-benzo[*d*]imidazole-6-carboxylic acid and 2-(((*R*)-4-(6-((4-chloro-2-fluorobenzofuran-7-yl)methoxy)pyridin-2-yl)cyclohex-3-en-1-yl)methyl)-1-(((*S*)-oxetan-2-yl)methyl)-1*H*-benzo[*d*]imidazole-6-carboxylic acid**

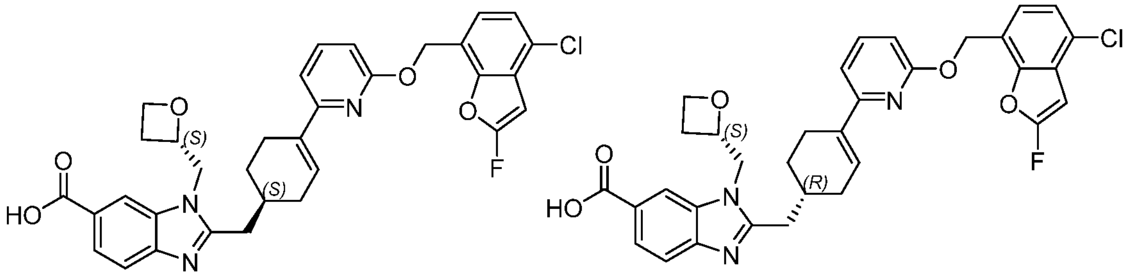

Compound 2 was separated by chiral splitting using high performance liquid phase to give a white solid Compound 36A (119 mg) and another white solid Compound 36B (110 mg).

Compound 36A:

LC-MS (ESI): $[M+H]^+$ =602.2;
$^1$HNMR (400 MHz, DMSO-$d_6$) δ 12.61 (br.s, 1H), 8.22 (d, *J* = 1.6 Hz, 1H), 7.80 (dd, *J* = 8.4, 1.6 Hz, 1H), 7.71 - 7.60 (m, 2H), 7.46 - 7.36 (m, 2H), 7.07 (d, *J* = 7.4 Hz, 1H), 6.75 (s, 1H), 6.73 (d, *J* = 8.2 Hz, 1H), 6.52 (d, *J* = 6.4 Hz, 1H), 5.60 (s, 2H), 5.06 - 5.00 (m, 1H), 4.65 (dd, *J* = 15.5, 7.2 Hz, 1H), 4.54 - 4.42 (m, 2H), 4.33 - 4.28 (m, 1H), 3.05 - 2.93 (m, 2H), 2.75 - 2.65 (m, 1H), 2.61 - 2.46 (m, 1H), 2.43 - 2.27 (m, 4H), 2.12 - 1.93 (m, 2H), 1.54 - 1.44 (m, 1H).

Compound 36B:

LC-MS (ESI): $[M+H]^+$=602.3;
$^1$HNMR (400 MHz, DMSO-$d_6$) δ 12.76 (br.s, 1H), 8.21 (d, *J*= 1.6 Hz, 1H), 7.80 (dd, *J* = 8.4, 1.6 Hz, 1H), 7.71 - 7.60 (m, 2H), 7.46 - 7.36 (m, 2H), 7.07 (d, *J* = 7.4 Hz, 1H), 6.75 (s, 1H), 6.72 (d, *J* = 8.2 Hz, 1H), 6.52 (d, *J* = 6.4 Hz, 1H), 5.60 (s, 2H), 5.06 - 5.00 (m, 1H), 4.65 (dd, *J* = 15.5, 7.2 Hz, 1H), 4.54 - 4.43 (m, 2H), 4.33 - 4.28 (m, 1H), 2.99 (d, *J* = 7.0 Hz, 2H), 2.74 - 2.65 (m, 1H), 2.61 - 2.46 (m, 1H), 2.43 - 2.27 (m, 4H), 2.12 - 1.93 (m, 2H), 1.55 - 1.45 (m, 1H).

**Example 37 Synthesis of 2-(((*S*)-4-(4-((4-chloro-2-fluorobenzofuran-7-yl)methoxy)-5-fluoropyrimidin-2-yl)cyclohex-3-en-1-yl)mcthyl)-3-(((*S*)-oxetan-2-yl)methyl)-3*H*-imidazo[4,5-*b*]pyridine-5-carboxylic acid and 2-(((*R*)-4-(4-((4-chloro-2-fluorobenzofuran-7-yl)methoxy)-5-fluoropyrimidin-2-yl)cyclohex-3-en-1-yl)methyl)-3-(((*S*)-oxetan-2-yl)methyl)-3*H*-imidazo[4,5-*b*]pyridine-5-carboxylic acid**

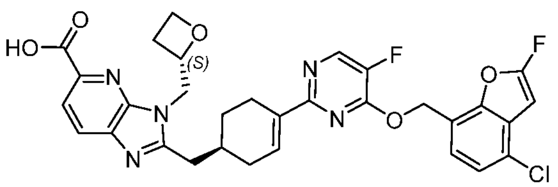 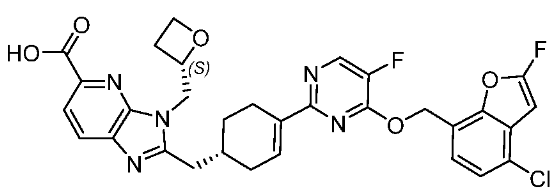

Compound 5 was separated by chiral splitting using high performance liquid phase to give a white solid Compound 37A (35 mg) and another white solid Compound 37B (20 mg).

Compound 37A:

LC-MS (ESI): $[M+H]^+$ =622.3;
$^1$HNMR (400 MHz, DMSO-$d_6$) δ 8.57 (d, *J* = 2.8 Hz, 1H), 8.11 (dd, *J* = 8.2, 2.8 Hz, 1H), 7.98 (d, *J* = 8.1 Hz, 1H), 7.46 (q, *J* = 8.2 Hz, 2H), 7.12 (s, 1H), 6.55 (dd, *J* = 6.5, 2.4 Hz, 1H), 5.77 (s, 2H), 5.14 - 5.07 (m, 1H), 4.70 - 4.63 (m, 1H),

4.57 - 4.42 (m, 2H), 4.35 - 4.28 (m, 1H), 3.08 (d, *J* = 7.0 Hz, 2H), 2.77 - 2.64 (m, 2H), 2.45 - 2.30 (m, 4H), 2.14 - 1.97 (m, 2H), 1.55 - 1.44 (m, 1H).

Compound 37B:

LC-MS (ESI): $[M+H]^+$ =622.3;
$^1$HNMR (400 MHz, DMSO-$d_6$) δ 12.91 (br.s, 1H), 8.57 (d, *J* = 2.9 Hz, 1H), 8.12 (d, *J* = 8.2 Hz, 1H), 7.98 (d, *J* = 8.2 Hz, 1H), 7.46 (q, *J* = 8.3 Hz, 2H), 7.12 (s, 1H), 6.55 (d, *J* = 6.4 Hz, 1H), 5.77 (s, 2H), 5.14 - 5.07 (m, 1H), 4.70 - 4.63 (m, 1H), 4.58 - 4.42 (m, 2H), 4.34 - 4.27 (m, 1H), 3.15 - 3.01 (m, 2H), 2.74 - 2.65 (m, 2H), 2.55 - 2.31 (m, 4H), 2.18 - 1.93 (m, 2H), 1.55 - 1.41 (m, 1H).

**Example 38 Synthesis of 2-(((1*s*,4*R*)-4-(6-((2,4-dichlorobenzofuran-7-yl)methoxy)pyridin-2-yl)cyclohexyl) methyl)-1-(((*S*)-oxctan-2-yl)mcthyl)-1*H*-benzo[*d*]imidazole-6-carboxylic acid (compound 38A) and 2-(((1*r*,4*S*)-4-(6-((2,4-dichlorobenzofuran-7-yl)methoxy)pyridin-2-yl)cyclohexyl)methyl)-1-(((S)-oxetan-2-yl) methyl)-1*H*-benzo[d]imidazole-6-carboxylic acid (compound 38B)**

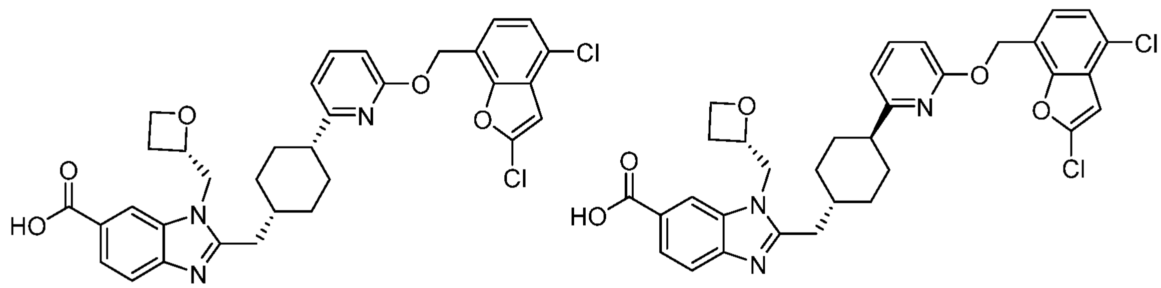

Synthesis method Refer to the reaction conditions of example 27, 27A-1 reacts with 7-(bromomethyl)-2,4-dichlorobenzofuran to give a white solid 38A (252 mg, 66.0%), 27B-1 reacts with 7-(bromomethyl)-2,4-dichlorobenzofuran to give another white solid 38B (220mg, 64.3%).

38A:

$^1$H NMR (500 MHz, DMSO-$d_6$) δ 8.17 (s, 1H), 7.78 (d, *J* = 8.4 Hz, 1H), 7.65 (t, *J* = 7.7 Hz, 1H), 7.59 (d, *J* = 8.4 Hz, 1H), 7.46 (d, *J* = 8.1 Hz, 1H), 7.40 (d, *J* = 8.2 Hz, 1H), 7.15 (s, 1H), 6.93 (d, *J* = 7.4 Hz, 1H), 6.69 (d, *J* = 8.2 Hz, 1H), 5.62 (s, 2H), 5.08 - 4.96 (m, 1H), 4.67 - 4.54 (m, 1H), 4.51 - 4.39 (m, 2H), 4.30 - 4.23 (m, 1H),3.02 - 2.91(m, 2H), 2.77 - 2.61 (m, 2H), 2.44 - 2.30 (m, 2H), 1.95 - 1.85 (m, 2H), 1.72 - 1.51 (m, 6H).
LC-MS (ESI): $[M+H]^+$ =620.4.

38B:

$^1$H NMR (500 MHz, DMSO-$d_6$) δ 12.64 (s, 1H), 8.20( s, 1H), 7.79 (dd, *J* = 8.4, 1.6 Hz, 1H), 7.66 - 7.58 (m, 2H), 7.44 (d, *J* = 8.1 Hz, 1H), 7.38 (d, *J* = 8.1 Hz, 1H), 7.16 (s, 1H), 6.83 (d, *J* = 7.3 Hz, 1H), 6.66 (d, *J* = 8.1 Hz, 1H), 5.58 (s, 2H), 5.62 - 5.56 (m, 1H), 4.69 - 4.62 (m, 1H), 4.55 - 4.45 (m, 2H),4.35 - 4.29 (m, 1H),2.94 - 2.85 (m, 2H), 2.76 - 2.63 (m, 1H),2.59 - 2.52 (m, 1H), 2.43 - 2.34 (m, 1H), 2.08 - 1.95 (m, 1H), 1.92 - 1.85 (m, 2H), 1.84 - 1.73 (m, 2H),1.49 - 1.43 (m, 2H), 1.24 - 1.16 (m, 2H).
LC-MS (ESI): $[M+H]^+$ =620.4.

**Example 39 Synthesis of 2-(((1*r*,4*S*)-4-(6-((4-chloro-2-fluorobenzofuran-7-yl)methoxy)pyridin-2-yl)cyclohexyl) methyl)-4-fluoro-1-(((*S*)-oxetan-2-yl)methyl)-1*H*-benzo[*d*]imidazole-6-carboxylic acid** (compound 39)

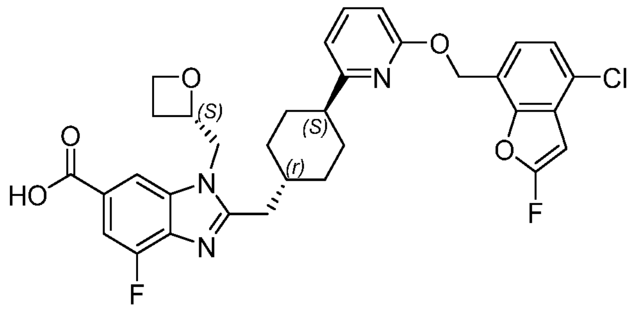

**Step** 1: **Synthesis of methyl (*S*)-3-fluoro-4-nitro-5-((oxetan-2-ylmethyl)amino)benzoate**

Methyl 3,5-difluoro-4-nitrobenzoate (200 mg, 0.92 mmol), ((2S)-oxetan-2-yl)methanamine (80 mg, 0.92 mmol), potassium carbonate (254 mg, 1.84 mmol) were added to *N*,*N*-dimethylformamide (15 mL), and the mixture was stirred at room temperature for 3 h. Concentrated under reduced pressure, the residue was purifieded by column chromatography eluted with PE/EA=1:1 to give a yellow oil (255 mg, 97.4%).

LC-MS (ESI): $[M+H]^+$ =285.1;
$^1$H NMR (400 MHz, $CDCl_3$) δ 7.34 - 7.25 (m, 2H), 6.99 (dd, *J* = 11.4, 1.8 Hz, 1H), 5.09 - 5.02 (m, 1H), 4.71 - 4.64 (m, 1H), 4.58 - 4.49 (m, 1H), 3.87 (s, 3H), 3.49 (t, *J* = 4.8 Hz, 2H), 2.74 - 2.63 (m, 1H), 2.56 - 2.46 (m, 1H).

**Step 2: Synthesis of methyl (*S*)-4-amino-3-fluoro-5-((oxetan-2-ylmethyl)amino)benzoate**

Methyl (*S*)-3-fluoro-4-nitro-5-((oxetan-2-ylmethyl)amino)benzoate (255 mg, 0.90 mmol), palladium carbon (100 mg, 5%) were added to ethyl acetate (25 mL), it was replaced with hydrogen for three times and stirred under the atmosphere of hydrogen for 2 h. It was filtered with diatomaceous earth, and concentrated under reduced pressure to give a brown solid (230 mg, 100%).

LC-MS (ESI): $[M+1]^+$ =255.1.

**Step 3: Synthesis of 2-(((1*r*,4*S*)-4-(6-((4-chloro-2-fluorobenzofuran-7-yl)methoxy)pyridin-2-yl)cyclohexyl) methyl)-4-fluoro-1-(((*S*)-oxetan-2-yl)methyl)-1*H*-benzo[d]imidazole-6-carboxylic acid**

Synthesis method Refer to the reaction conditions of example 27, methyl (*S*)-4-amino-3-fluoro-5-((oxetan-2-ylmethyl)amino)benzoate reacts with 27B-3 finally to give a white solid (27.4 mg, 93.4%).

LC-MS (ESI): $[M+H]^+$ =622.1;
$^1$H NMR (400 MHz, DMSO-$d_6$) δ 12.97 (s, 1H), 8.10 (s, 1H), 7.61 (t, *J* = 7.8 Hz, 1H), 7.50 (dd, *J* = 11.4, 1.3 Hz, 1H), 7.43 - 7.37 (m, 2H), 6.84 (d, *J* = 7.2 Hz, 1H), 6.66 (d, *J* = 8.2 Hz, 1H), 6.51 (d, *J* = 6.4 Hz, 1H), 5.55 (s, 2H), 5.07 - 4.98 (m, 1H), 4.68 (dd, *J* = 15.4, 7.2 Hz, 1H), 4.60 - 4.39 (m, 2H), 4.35 - 4.26 (m, 1H), 3.03 - 2.83 (m, 2H), 2.76 - 2.64 (m, 1H), 2.44 - 2.28 (m, 2H), 2.05 (d, *J* = 17.2 Hz, 1H), 1.95 - 1.73 (m, 4H), 1.55 - 1.41 (m, 2H), 1.28 - 1.14 (m, 2H).

**Example 40 Synthesis of 2-(((1*r*,4*r*)-4-(6-((4-chloro-2-fluorobenzofuran-7-yl)methoxy)pyridin-2-yl)cyclohexyl) methyl)-1-((1-(fluoromethyl)cyclopropyl)methyl)-1*H*-benzo[*d*]imidazole-6-carboxylic acid (compound 40)**

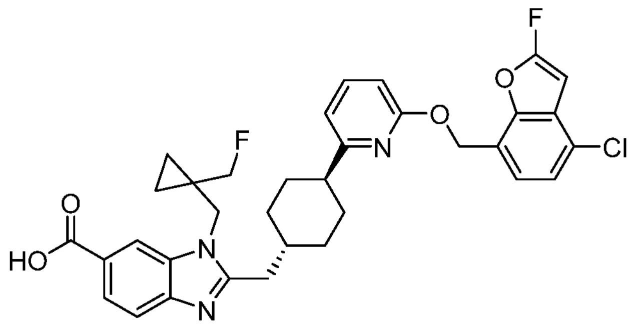

**Step 1: Synthesis of (1-(aminomethyl)cyclopropyl)methanol**

1-(Hydroxymethyl)cyclopropane-1-carbonitrile (900 mg, 9.27 mmol) was added to tetrahydrofuran (10 mL) and stirred to dissolve, then lithium tetrahydroaluminate (703 mg, 18.52 mmol) was added, and the reaction was carried out at room temperature under nitrogen protection overnight. The reaction mixture was quenched with water (1 mL) and 10% aqueous sodium hydroxide (1 mL), then water (1 mL) was added and filtered, and the filter cake was washed with tetrahydrofuran (10 mL×2), and the filtrate was collected and concentrated to give a colorless oil (1.3 g), which was used directly in the next step.

**Step 2: Synthesis of methyl 3-(((1-(hydroxymethyl)cyclopropyl)methyl)amino)-4-nitrobenzoate**

(1-(Aminomethyl)cyclopropyl)methanol (1 g, 9.89 mmol) and methyl 3-fluoro-4-nitrobenzoate (2.07 g, 10.38 mmol) were added to *N*,*N*-dimethylformamide (20 mL), then triethylamine (2.7 mL, 19.77 mmol) was added, and the reaction mixture was heated up to 60 °C for 1 h under nitrogen protection. The reaction mixture was cooled down to room temperature, quenched with water (15 mL), the reaction mixture was filtered, the filtrate was dried and concentrated, and the residue was purified by column chromatography eluted with PE:EA (v/v)=10:1 to give a yellow solid (2.7 g, 97.4%).
$^1$H NMR (400 MHz, $CDCl_3$) δ 8.14 (d, *J* = 8.8 Hz, 1H), 7.30 - 7.06 (m, 2H), 3.87 (s, 3H), 3.55 (s, 2H), 3.33 (s, 2H), 0.58 (s, 4H).

**Step 3: Synthesis of methyl 3-(((1-(fluoromethyl)cyclopropyl)methyl)amino)-4-nitrobenzoate**

Methyl 3-(((1-(hydroxymethyl)cyclopropyl)methyl)amino)-4-nitrobenzoate (2.7 g, 9.63 mmol) was added to dichloromethane (27 mL), and cooled to 0 °C under nitrogen protection, then diethylamino sulfur trifluoride (1.5 mL, 11.56 mmol) was added, and the reaction was heated to room temperature for 5 hours after addition. Quenched with water (20 mL), the organic phases were dried and concentrated, and the residue was purified by column chromatography eluted with PE:EA (v/v)=1:1 to give a yellow solid (1.7 g, 62.5%).
$^1$H NMR (400 MHz, $CDCl_3$) δ 7.43 (dd, *J* = 8.0, 1.6 Hz, 1H), 7.33 (d, *J* = 1.6 Hz, 1H), 6.63 (d, *J* = 8.0 Hz, 1H), 3.80 (d, *J* = 4.8 Hz, 3H), 3.33 (d, *J* = 23.2 Hz, 2H), 2.38 - 2.16 (m, 4H), 1.87 - 1.75 (m, 1H), 1.49 (m, 1H).

**Step 4: Synthesis of methyl 4-amino-3-(((1-(fluoromethyl)cyclopropyl)methyl)amino)benzoate**

Methyl 3-(((1-(fluoromethyl)cyclopropyl)methyl)atnino)-4-nitrobenzoate (2.1 g, 7.44 mmol) and palladium carbon (0.21 g, 1.97 mmol) were added to methanol (50 mL), it was replaced with hydrogen for three times and then kept in a hydrogen atmosphere for 5 hours at room temperature. The reaction mixture was filtered, the filtrate was concentrated and the residue was purified by column chromatography eluent with PE:EA (v/v)=1:1 to give a white solid (1.2 g, 62.9%).
$^1$H NMR (400 MHz, DMSO-$d_6$) δ 7.16 (t, *J*= 10.0 Hz, 1H), 7.11 (s, 1H), 6.56 (d, *J* = 8.0 Hz, 1H), 5.51 (s, 2H), 4.64 (t, *J* = 5.6 Hz, 1H), 3.73 (s, 3H), 3.40 (d, *J* = 5.6 Hz, 1H), 3.36 - 3.27 (m, 1H), 2.33 - 2.13 (m, 4H), 1.87 - 1.71 (m, 1H), 1.64 - 1.46 (m, 1H).

**Step 5: Synthesis of 2-(((1*r*,4*r*)-4-(6-((4-chloro-2-fluorobenzofuran-7-yl)methoxy)pyridin-2-yl(cyclohexyl(methyl)-1-((1-(fluoromethyl)cyclopropyl)methyl)-1*H*-benzo[*d*]imidazole-6-carboxylic acid**

Synthesis method refer to the reaction conditions of example 27, methyl 4-amino-3-(((1-(fluoromethyl)cyclopropyl)methyl)amino)benzoate reacts with 27B-3 finally to give a white solid (41 mg,48.8%).
$^1$H NMR (400 MHz, DMSO-$d_6$) $\delta$ 8.22 (s, 1H), 7.79 (dd, *J* = 8.4, 1.2 Hz, 1H), 7.67 - 7.54 (m, 2H), 7.41 (q, *J* = 8.4 Hz, 2H), 6.84 (d, *J* = 7.2 Hz, 1H), 6.66 (d, *J* = 8.4 Hz, 1H), 6.51 (d, *J* = 6.4 Hz, 1H), 5.56 (s, 2H), 4.72 (d, *J* = 24.4 Hz, 2H), 2.85 (d, *J* = 6.8 Hz, 2H), 2.56 (d, *J* = 12.0 Hz, 1H), 2.20 (dt, *J* = 15.2, 7.6 Hz, 4H), 2.06 (s, 1H), 1.98 - 1.71 (m, 6H), 1.58 - 1.39 (m, 2H), 1.33 - 1.14 (m, 2H).

**Example 41 Synthesis of 2-(((1*r*,4*r*)-4-(6-((4-chloro-2-fluorobenzofuran-7-yl)methoxy)pyridin-2-yl)cyclohexyl) methyl)-1-(2-methoxyethyl)-1*H*-benzo[d]imidazole-6-carboxylic acid (compound 41)**

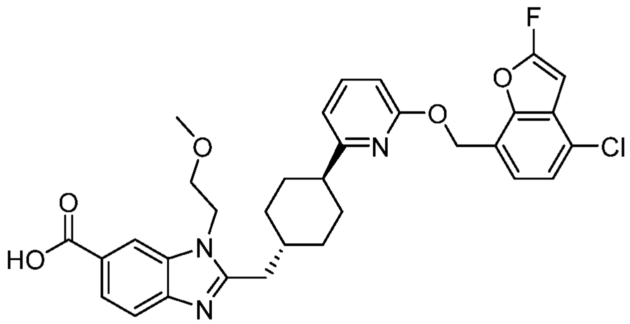

**Step 1: Synthesis of methyl 3-((2-methoxyethyl)amino)-4-nitrobenzoate**

Methyl 3-fluoro-4-nitrobenzoate (1 g, 5.02 mmol) and 2-methoxyethan-1-atnine (0.5 mL, 6.03 mmol) were dissolved in DMF (15 mL), then potassium carbonate (1.39 g, 10.04 mmol) was added and stirred at room temperature for 2.5 h. Water was added, extracted with ethyl acetate for three times, the combined organic phases were dried, concentrated, and then the residue was purified by chromatography eluted with PE/EA (V/V)=5/1 to give a yellow oil (920 mg, 72.1%).

LC-MS (ESI): $[M+H]^+$ =255.2.

**Step 2: Synthesis of methyl 4-amino-3-((2-methoxyethyl)amino)benzoate**

Methyl 3-((2-methoxyethyl)amino)-4-nitrobenzoate (920 mg, 3.62 mmol) was dissolved in the mixture of dioxane (10 mL) and water (2.00 mL), then zinc powder (1.2 g, 18.10 mmol) and ammonium chloride (968 mg, 18.10 mmol) were added, and the reaction was carried out at room temperature for 2 h. The reaction mixture was filtered with diatomaceous earth and the filtrate was concentrated to give of a purple oil (730 mg, 90.0%), which was used directly in the next step.

LC-MS (ESI): $[M+H]^+$ =225.2.

**Step 3: Synthesis of 2-(((1*r*,4*r*)-4-(6-((4-chloro-2-fluorobenzofuran-7-yl)methoxy)pyridin-2-yl)cyclohexyl)methyl)-1-(2-methoxyethyl)-1*H*-benzo[d]imidazole-6-carboxylic acid**

Synthesis method Refer to the reaction conditions of example 27, methyl 4-amino-3-((2-methoxyethyl)amino)benzoate reacts with 27B-3 finally to give a white solid (10.5 mg, 23.9%).

$^1$H NMR (400 MHz, DMSO-$d_6$) $\delta$ 8.14 (s, 1H), 7.78 (d, *J* = 8 Hz, 1H), 7.63 - 7.61 (m, 2H), 7.59 - 7.38 (m,2H), 6.83 (d, *J* = 8 Hz, 1H), 6.65 (d, *J* = 8 Hz, 1H), 6.50 (d, *J* = 8 Hz, 1H), 5.56 (s, 2H), 4.47-4.46 (m, 2H), 3.67 - 3.64 (m, 2H), 3.20 (s, 3H), 2.84 (d, *J* = 8 Hz, 2H), 2.58 - 2.50 (m, 1H), 2.08 - 2.02 (m, 1H), 1.90 - 1.80 (m, 4H), 1.53 - 1.44 (m, 2H), 1.26 - 1.17 (m, 2H).

**Example 42 Synthesis of 2-(((1*r*,4*r*)-4-(6-((4-chloro-2-fluorobenzofuran-7-yl)methoxy)pyridin-2-yl)cyclohexyl)methyl)-1-((1-ethyl-1*H*-imidazol-5-yl)methyl)-1*H*-benzo[*d*]imidazole-6-carboxylic acid (compound 42)**

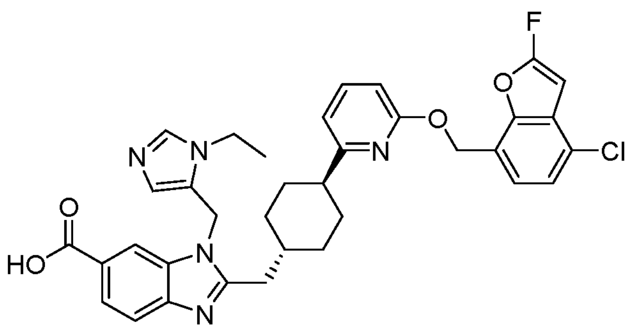

**Step 1: Synthesis of methyl 3-(((1-ethyl-1*H*-imidazol-5-yl)methyl)amino)-4-nitrobenzoate**

Methyl 3-fluoro-4-nitrobenzoate (100 mg, 0.80 mmol) and (3-ethylimidazol-4-yl)methylamine (100 mg, 0.80 mmol) were added to tetrahydrofuran (5 mL) to dissolve, and then diisopropylethylamine (195 mg, 1.51 mmol) was added,

and the reaction solution was heated to 60 °C under the protection of nitrogen for 3 hours. The reaction mixture was cooled down to room temperature and concentrated directly under reduced pressure to give the crude product. The crude product was purified by column chromatography eluted with EtOAc:PE (v/v)=1:5 to give a yellow solid (130 mg, 85.0%).

$^1$H NMR (400 MHz, $CDCl_3$) $\delta$ 8.18 (d, *J* = 8.8 Hz, 1H), 7.90 (s, 1H), 7.70 - 7.56 (m, 2H), 7.28 (dd, *J* = 8.8, 1.6 Hz, 1H), 7.06 (s, 1H), 5.23 (s, 2H), 4.50 (d, *J* = 4.8 Hz, 2H), 3.89 (s, 3H), 1.49 (s, 3H).

**Step 2: Synthesis of methyl 4-amino-3-(((1-ethyl-1*H*-imidazol-5-yl)methyl)amino)benzoate**

Methyl 3-(((1-ethyl-1*H*-imidazol-5-yl)methyl)amino)-4-nitrobenzoate (130 mg, 0.43 mmol) and palladium carbon (13 mg, 0.12 mmol) were added to methanol (5 mL), and it was replaced with hydrogen and then kept at 15 Psi at room temperature for 4 h. The reaction mixture was filtered with diatomaceous earth, the filter cake was washed with methanol (10 mL), and the filtrate was concentrated to give a colorless oil (100 mg, 85.3%).

LC-MS (ESI): $[M+H]^+$ =275.3.

**Step 3: Synthesis of 2-(((1*r*,4*r*)-4-(6-((4-chloro-2-fluorobenzofuran-7-yl)methoxy)pyridin-2-yl)cyclohexyl)methyl)-1-((1-ethyl-1*H*-imidazol-5-yl)methyl)-1*H*-benzo[*d*]imidazole-6-carboxylic acid**

Synthesis method Refer to the reaction conditions of example 27, methyl 4-amino-3-(((1-ethyl-1*H*-imidazol-5-yl)methyl)amino)benzoate reacts with 27B-3 finally to give a white solid (7.0 mg, 17.9%).

LC-MS (ESI): $[M+H]^+$=642.2;
$^1$H NMR (400 MHz, DMSO-*d*$_6$) $\delta$ 8.11 (s, 1H), 7.80 (d, *J* = 8.4 Hz, 1H), 7.70 - 7.54 (m, 3H), 7.41 (q, *J* = 8.4 Hz, 2H), 6.82 (d, *J* = 7.2 Hz, 1H), 6.66 (d, *J* = 8.4 Hz, 1H), 6.51 (d, *J* = 6.4 Hz, 1H), 6.44 (s, 1H), 5.63 (s, 2H), 5.55 (s, 2H), 3.96 (q, *J* = 7.2 Hz, 2H), 2.84 (d, *J* = 6.4 Hz, 2H), 1.88 - 1.72 (m, 5H), 1.51 - 1.04 (m, 8H).

**Example 43 Synthesis of 2-(((1*r*,4*r*)-4-(6-((4-chloro-2-fluorobenzofuran-7-yl)methoxy)pyridin-2-yl)cyclohexyl)methyl)-1-((1-(cyanomethyl)cyclopropyl)methyl)-1*H*-benzo[*d*]imidazole-6-carboxylic acid (compound 43)**

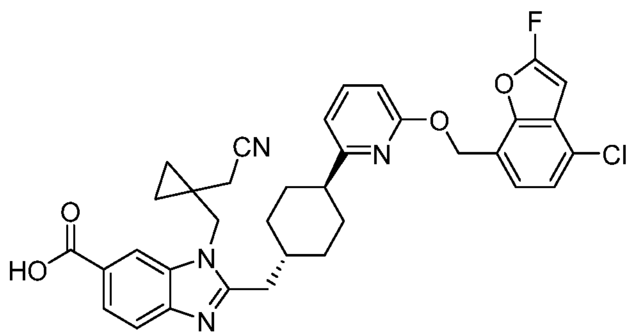

**Step 1: Synthesis of 2-(1-((1,3-dioxoisoindolin-2-yl)methyl)cyclopropyl)acetonitrile**

2-(1-(Hydroxymethyl)cyclopropyl)acetonitrile (100 mg, 0.9 mmol) was added to tetrahydrofuran (5 mL), then phthalimide (199 mg, 1.35 mmol) and triphenylphosphine (354 mg, 1.35 mmol) were added, and after stirring for 5 min DIAD (0.39 mL) was added dropwise under an ice bath and stirred at room temperature overnight. Concentrated under reduced pressure and the residue was purified by column chromatography eluted with PE:EtOAc (v/v)=1:1 to give a crude light yellow oil (660 mg).

**Step 2: Synthesis of 2-(1-(aminomethyl)cyclopropyl)acetonitrile**

2-(1-((1,3-dioxoisoindolin-2-yl)methyl)cyclopropyl)acetonitrile (300 mg, 1.33 mmol) was added to anhydrous ethanol (25 mL), and then hydrazine hydrate (80 mg, 1.59 mmol) was added, and heated to reflux at 85 °C for 5 hours. The solid was filtered, concentrated under reduced pressure, then water (20 mL) was added, and concentrated hydrochloric acid was added, the solid was filtered, and the filtrate was concentrated under reduced pressure to give a light yellow oil (580 mg).

**Step 3: Synthesis of methyl 3-(((1-(cyanomethyl)cyclopropyl)methyl)amino)-4-nitrobenzoate**

2-(1-(Aminomethyl)cyclopropyl)acetonitrile (230 mg, 2.1 mmol) was added to *N*,*N*-dimethylformamide (10 mL), then methyl 3-fluoro-4-nitrobenzoate (416 mg, 2.1 mmol) and potassium carbonate powder (1.44 g, 10.5 mmol) was added, it was replaced with nitrogen and stirred at room temperature overnight. Extracted with ethyl acetate for 3 times, the organic phases were combined, dried with anhydrous sodium sulfate, and concentrated under reduced pressure to give a brown oil (100 mg).

**Step 4: Synthesis of methyl 4-amino-3-(((1-(cyanomethyl)cyclopropyl)methyl)amino)benzoate**

Methyl 3-(((1-(cyanomethyl)cyclopropyl)methyl)amino)-4-nitrobenzoate (100 mg, 0.39 mmol) was added to anhydrous methanol (30 mL), then palladium charcoal (25 mg, 0.24 mmol) was added, it was replaced with hydrogen for three times and stirred at room temperature for 1 h. The reaction mixture was filtered with diatomaceous earth, and the mixture was concentrated under reduced pressure to give a pale yellow oil (73 mg).

**Step 5: Synthesis of 2-(((1*r*,4*r*)-4-(6-((4-chloro-2-fluorobenzofuran-7-yl)methoxy)pyridin-2-yl)cyclohexyl) methyl)-1-((1-(cyanomethyl)cyclopropyl)methyl)-1*H*-benzo[*d*]imidazole-6-carboxylic acid**

Synthesis method Refer to the reaction conditions of example 27, methyl 4-amino-3-(((1-(cyanomethyl)cyclopropyl)methyl)amino)benzoate reacts with 27B-3 finally to give a white solid (13.4 mg, 30.0%).

LC-MS: $[M+H]^+$ =627.2;
$^1H$ NMR (400 MHz, DMSO-$d_6$) $\delta$ 12.81 (s, 1H), 8.28 (s, 1H), 7.87 (d, *J* = 8.4 Hz, 1H), 7.71 (d, *J* = 8.4 Hz, 1H), 7.65 (t, *J* = 7.6 Hz, 1H), 7.46 (d, *J* = 8.0 Hz, 1H), 7.42 (d, *J* = 8.0 Hz, 1H), 6.88 (d, *J* = 7.2 Hz, 1H), 6.70 (d, *J* = 8.0 Hz, 1H), 6.54 (d, *J* = 6.4 Hz, 1H), 5.59 (s, 2H), 4.52 (s, 2H), 2.97 - 2.92 (m, 3H), 2.77 (s, 1H), 2.66 (s, 2H), 1.93 (d, *J* = 11.6 Hz, 2H), 1.85 (d, *J* = 11.6 Hz, 2H), 1.53 (d, *J* = 12.4 Hz, 2H), 1.28 (d, *J* = 10.4 Hz, 2H), 0.70 (d, *J* = 12.8 Hz, 4H).

**Example 44 Synthesis of 2-(((1*r*,4*S*)-4-(6-((2-chloro-4-cyanobenzofuran-7-yl)methoxy)pyridin-2-yl)cyclohexyl) mcthyl)-1-(((*S*)-oxetan-2-yl)mehyl)-1*H*-benzo[*d*]imidazole-6-carboxylic acid (compound 44).**

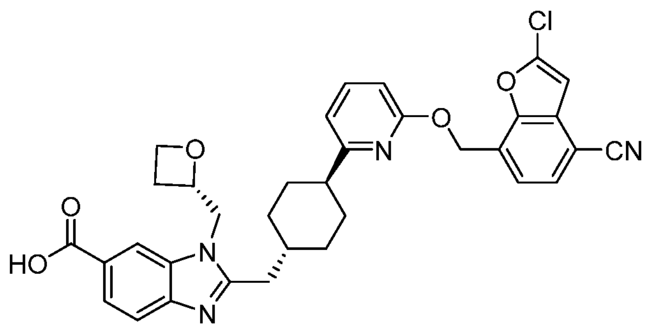

Synthesis method Refer to the reaction conditions of example 27, 27B-1 reacts with 7-(bromomethyl)-2-chlorobenzofuran-4-carbonitrile finally to give a white solid (20 mg, 51.2%).

LC-MS (ESI): $[M+H]^+$ =611.2;
$^1H$ NMR (400 MHz, DMSO) $\delta$ 12.71 (s, 1H), 8.21 (s, 1H), 7.80 (dd, *J* = 10.0, 4.4 Hz, 2H), 7.70 - 7.46 (m, 3H), 6.84 (d, *J* = 7.2 Hz, 1H), 6.71 (d, *J* = 8.4 Hz, 1H), 5.67 (s, 2H), 5.06 - 4.96 (m, 1H), 4.64 - 4.61 (m, 1H), 4.59 - 4.40 (m, 2H), 4.34 - 4.29 (m, 1H), 2.99 - 2.80 (m, 2H), 2.77 - 2.62 (m, 1H), 2.43 - 2.33 (m, 1H), 1.99 (s, 1H), 1.86 (d, *J* = 11.2 Hz, 2H), 1.78 - 1.64 (m, 2H), 1.40 - 1.34 (m, 2H), 1.30 - 1.02 (m, 3H).

**Example 45 Synthesis of 2-(((1*r*,4*S*)-4-(6-((4-chloro-2-fluorobenzofuran-7-yl)methoxy)pyridin-2-yl)cyclohexyl) methyl)-3-(((S)-oxetan-2-yl)methyl)-3*H*-imidazo[4,5-*b*]pyridine-5-carboxylic acid (compound 45)**

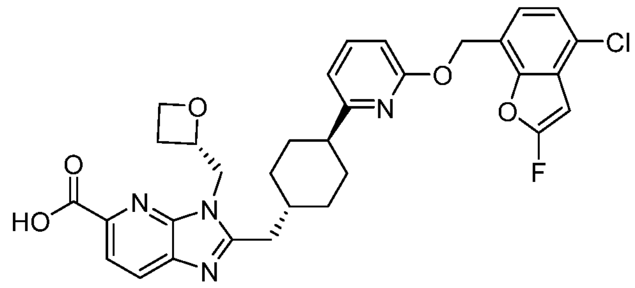

Synthesis method Refer to the reaction conditions of example 27, methyl (*S*)-5-amino-6-((oxetan-2-ylmethyl) amino)picolinate reacts with 27B-3 finally to give a white solid (24 mg, 44.4%).

LC-MS (ESI): $[M+H]^+$ =605.2;
1H NMR (400 MHz, DMSO) $\delta$ 12.98 (s, 1H), 8.11 (d, *J* = 8.2 Hz, 1H), 7.98 (d, *J* = 8.2 Hz, 1H), 7.66 - 7.56 (m, 1H), 7.45 - 7.36 (m, 2H), 6.84 (d, *J* = 7.2 Hz, 1H), 6.67 (d, *J* = 8.2 Hz, 1H), 6.51 (d, *J* = 6.4 Hz, 1H), 5.56 (s, 2H), 5.13 - 5.11 (m, 1H), 4.66 - 4.63 (m, 1H), 4.49 - 4.47 (m, 2H), 4.34 - 4.33 (m, 1H), 3.30 (s, 1H), 3.07 - 2.93 (m, 2H), 2.69 - 2.63 (m, 1H), 2.43 - 2.41 (m, 1H), 2.07 - 2.02 (m, 1H), 1.91 - 1.83 (m, 2H), 1.82-1.79 (m, 2H), 1.48 - 1.45 (m, 2H), 1.30 - 1.18 (m, 2H).

**Example 46 Synthesis of 2-(((1*s*,4*R*)-4-(4-((4-chloro-2-fluorobenzofuran-7-yl)methoxy)-5-fluoropyrimidin-2-yl) cyclohexyl)methyl)-3-(((*S*)-oxetan-2-yl)methyl)-3*H*-imidazo[4,5-*b*]pyridine-5-carboxylic acid (compound 46A) and 2-(((1*r*,4*S*)-4-(4-((4-chloro-2-fluorobcnzofuran-7-yl)methoxy)-5-fluoropyrimidin-2-yl)cyclohexyl) methyl)-3-(((*S*)-oxetan-2-yl)methyl)-3*H*-imidazo[4,5-*b*]pyridine-5-carboxylic acid (compound 46B)**

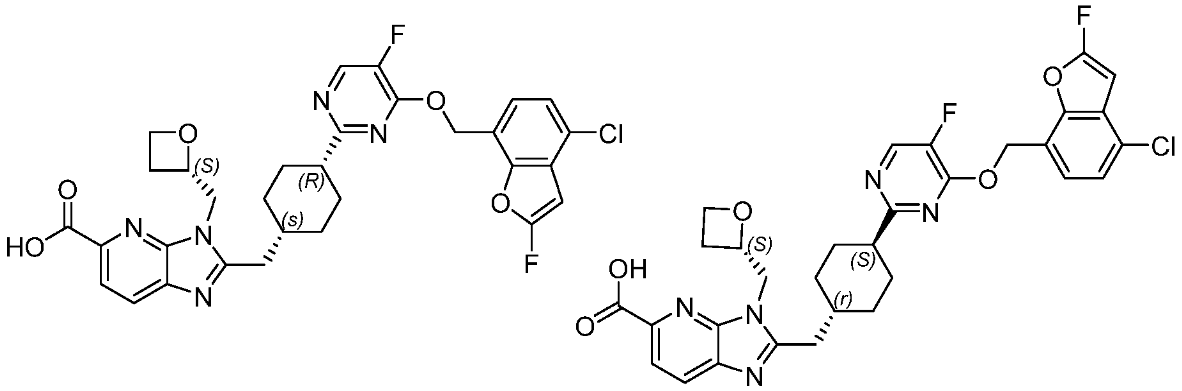

Synthesis method refer to the reaction conditions of example 27, reacts finally to give a white solid 46A (4.3 mg, 22.0%) and another white solid (10 mg, 51.1%).

46A:

LC-MS (ESI): $[M+H]^+$ =624.1;
$^1$H NMR (400 MHz, DMSO-$d_6$) $\delta$ 12.97 (s, 1H), 8.54 (d, *J* = 3.2 Hz, 1H), 8.11 (d, *J* = 8.2 Hz, 1H), 7.98 (d, *J* = 8.2 Hz, 1H), 7.51 - 7.40 (m, 2H), 6.54 (t, *J* = 6.4 Hz, 1H), 5.74 (s, 2H), 5.19 - 5.06 (m, 1H), 4.69 - 4.62 (m, 1H), 4.59 - 4.43 (m, 2H), 4.38 - 4.29 (m, 1H), 3.05 - 2.94 (m, 2H), 2.75 - 2.64 (m, 2H), 2.47 - 2.39 (m, 2H), 1.92 (d, *J* = 10.2 Hz, 4H), 1.57 - 1.45 (m, 2H), 1.32 - 1.20 (m, 2H).

46B:

LC-MS (ESI): $[M+H]^+$ =624.1;
$^1$H NMR (400 MHz, DMSO-$d_6$) $\delta$ 12.88 (s, 1H), 8.59 (d, *J* = 3.0 Hz, 1H), 8.09 (d, *J* = 8.2 Hz, 1H), 7.97 (d, *J* = 8.2 Hz, 1H), 7.50 - 7.40 (m, 2H), 6.53 (d, *J* = 6.3 Hz, 1H), 5.76 (s, 2H), 5.13 - 5.03 (m, 1H), 4.66-4.56 (m, 1H), 4.56 - 4.36 (m, 2H), 4.36 - 4.22 (m, 1H), 3.01 - 2.89 (m, 3H), 2.73 - 2.62 (m, 1H), 2.44 - 2.32 (m, 2H), 2.17 - 2.05 (m, 2H), 1.76 - 1.65 (m, 2H), 1.65 - 1.54 (m, 2H), 1.48 - 1.35 (m, 2H).

**Example 47 Synthesis of 2-(((1*r*,4*S*)-4-(6-((4-chloro-2-fluorobenzofuran-7-yl)methoxy-*d*$_2$)pyridin-2-yl)cyclohexyl)methyl)-1-(((*S*)-oxetan-2-yl)methyl)-1*H*-benzo[*d*]imidazole-6-carboxylic acid (compound 47)**

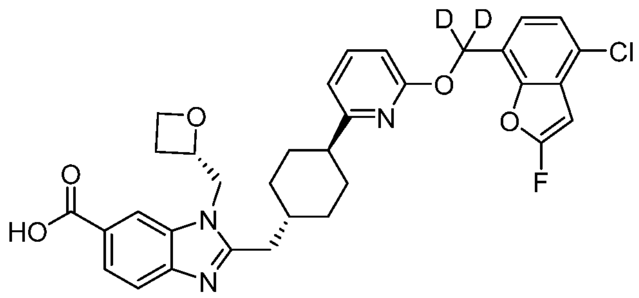

**Step 1: Synthesis of 7-(bromomethyl-*d*$_2$)-4-chloro-2-fluorobenzofuran**

(4-Chloro-2-fluorobenzofuran-7-yl)methan-*d*$_2$-ol (250 mg, 1.2 mmol) was dissolved in toluene (7 mL), and phosphorus tribromide (365 mg, 1.3 mmol) was added under an ice bath. The reaction was carried out at room temperature for 10 min, water (5 mL) was added under an ice bath, extracted with ethyl acetate (10 mL× 2), the combined organic phases were washed with saturated saline (10 mL), the organic phases were dried with anhydrous sodium sulfate, concentrated under reduced pressure, and the residue was purified by column chromatography eluted with pure PE to give a white solid (149 mg, 46.3%).

$^1$H NMR (400 MHz, DMSO-*d*$_6$) δ 7.45 (d, *J* = 8.0 Hz, 1H), 7.41 (d, *J* = 8.0 Hz, 1H), 6.56 (dd, *J* = 6.4, 2.0 Hz, 1H).

**Step 2: Synthesis of 2-(((1*r*,4*S*)-4-(6-((4-chloro-2-fluorobenzofuran-7-yl)methoxy-*d*$_2$)pyridin-2-yl)cyclohexyl)methyl)-1-(((*S*)-oxetan-2-yl)methyl)-1*H*-benzo[*d*]imidazole-6-carboxylic acid**

Synthesis method Refer to the reaction conditions of example 27, 7-(bromomethyl-*d*$_2$)-4-chloro-2-fluorobenzofuran reacts with 27B-1 finally to give a white solid (20.6 mg, 33.9%).

LC-MS: [M+H]$^+$=606.2;

$^1$H NMR (400 MHz, DMSO-*d*$_6$) δ 12.73 (s, 1H), 8.23 (s, 1H), 7.83 - 7.78 (m, 1H), 7.62 (dd, *J* = 16.0, 8.4 Hz, 2H), 7.41 (q, *J* = 8.0 Hz, 2H), 6.84 (d, *J* = 7.2 Hz, 1H), 6.66 (d, *J* = 8.0 Hz, 1H), 6.51 (d, *J* = 6.4 Hz, 1H), 5.08 - 4.99 (m, 1H), 4.66 (dd, *J* = 15.6, 7.2 Hz, 1H), 4.52 (dd, *J* = 11.2, 8.4 Hz, 1H), 4.49 - 4.43 (m, 1H), 4.32 (dt, *J* = 9.2,6.0 Hz, 1H), 2.95 - 2.83 (m, 2H), 2.73 - 2.65 (m, 1H), 2.65 - 2.61 (m, 1H), 2.42 - 2.35 (m, 1H), 1.90 - 1.87 (m, 2H), 1.82 - 1.79 (m, 2H), 1.53 - 1.44 (m, 2H), 1.27 - 1.21 (m, 3H).

**Example 48 Synthesis of (*S*)-2-((4-(6-((4-chloro-2-fluorobenzofuran-7-yl)methoxy)pyridin-2-yl)cyclohexyl)methyl)-1-(oxetan-2-ylmethyl)-1*H*-thieno[2,3-*d*]imidazole-5-carboxylic acid (compound 48)**

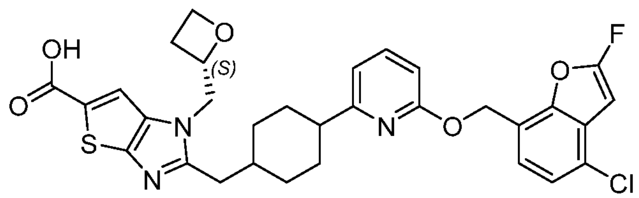

Synthesis method refer to the reaction conditions of example 27, methyl (*S*)-5-amino-4-((oxetan-2-ylmethyl)amino)thiophene-2-carboxylate reacts with ethyl 2-(4-(6-hydroxypyridin-2-yl)cyclohexyl)acetate finally to give a white solid (6.3 mg, 8.1%).

LC-MS (ESI): [M+H]$^+$ =610.1;

$^1$HNMR (400 MHz, DMSO-*d*$_6$) δ 12.78 (s, 1H), 7.79 (s, 1H), 7.61 - 7.63 (m, 1H), 7.45 - 7.40 (m, 2H), 6.93 - 6.82 (m, 1H), 6.70 - 6.65 (m, 1H), 6.52 - 6.48 (m, 1H), 5.59 - 5.55 (m, 2H), 5.06 - 4.94 (m, 1H), 4.57 - 4.24 (m, 4H), 2.91 - 2.61 (m, 4H),

2.38 - 2.19 (m, 2H), 2.00 - 1.78 (m, 3H), 1.70 - 1.41 (m, 5H).

**Experimental example 1**

1. Material

1) Cell Line

The cells were constructed by Wuxi New Drug Development Co., Ltd. Shanghai. Shown in the following table 1.

Table 1

| Target | Host cell |
|---|---|
| GLP-1 | HEK293 |

2) Reagent shown in the following table 2.

Table 2

| Name | Item No. | Manufacturer |
|---|---|---|
| cAMP Detection Kit | 62AM4PEJ | Cisbio |
| HEPES(1M) | 15630-106 | Invitrogen |
| HBSS | 14025 | Invitrogen |
| BSA | B2064-100G | Sigma |
| IBMX | I5879-5G | Sigma |
| ECHO qualified 384 well plate | PP-0200 | Labcyte |
| OptiPlate-384 | 6007299 | PerkinElmer |

3) Equipment shown in the following table 3.

Table 3

| Name | Item No. | Manufacturer |
|---|---|---|
| EnVision | envision2014 | PerkinElmer |
| Vi-cell counter | Vi-CELL™ XR Cell Viability Analyzer | Beckman |
| Bravo | Bravo V11 | Agilent |
| ECHO | ECHO 555 | Labcyte |
| Centrifuge | Allegra™ 25R Centrifuge | Beckman |

4) Compound Information

The compound was prepared with DMSO to a working concentration of 100μM or 10μM. 10 points of 4-fold dilution (maximum concentration of 1000nM or 100nM after dilution) was done with Bravo V11. The activity of compound PF-06882961 and compound 16 disclosed in CN113480534Awere also tested in this assay.

2. Method

1) Experimental Material

Experimental Buffer shown in the following table 4.

table 4

| Reagent | Final concentration |
|---|---|
| HBSS | 1x |
| HEPES | 5mM |
| 10%BSA | 0.1% |
| IBMX | 0.5mmol/L |

The final concentrations of the test reagents prepared shown in table 5 below.

Table 5

| Reagent | Final concentration |
|---|---|
| Lysis Buffer | ≈1x |
| D2-cAMP Solution | 1x |
| cAMP-antibody Solution | 1x |

2) Experimental Method

a) Preparation of Compound Plates:

The compound to be tested was diluted 4 times with Bravo to obtain 10 concentrations and the initial concentration was 100μM by Bravo V11. The initial concentration was 100μM.

b) Transfer of Compounds:

b1) 100 nL 100x of compound was transferred to OptiPlate-384 plate using Echo 555.
b2) OptiPlate-384 plate was centrifuged at 1000 rpm for 5 seconds.

c) Preparation of cell suspension:

c1) A HEK293 cell cryopreservation tube with high expression of hGLP-1R was quickly thawed in 37°C warm water.
c2) The cell suspension was transferred to a Transfer 15 mL of centrifuge tube and gently rinsed with 10 mL of HBSS.
c3) The centrifuge tube was centrifuged at 1000 rpm at room temperature for 1 minute.
c4) The supernatant was discarded.
c5) The cells at the bottom were gently dispersed and gently rinsed with 10 mL of HBSS, then the cells were centrifuged for sedimentation, and finally the cells were re-suspended in experimental buffer.
c6) Cell density and activity were measured.
c7) The concentration of GLP-1 cells was diluted to $1.0*10^5$/mL with the experimental buffer.
c8) 10μL of diluted cell suspension was transferred into OptiPlate-384 plate.
c9) The cells were incubated at room temperature for 30 minutes.

d) detection:

d1) 10 μL of 800 nM gradient diluted cAMP standard was added to the empty well of OptiPlate-384 plate.
d2) 10 μL of cAMP detection reagent was added.
d3) incubated for 60 min at room temperature and the EnVision reading was performed.

The experimental results are shown in Table 1, Table 1 shows the agonistic effects ($EC_{50}$) of the compounds provided in some examples of the present invention.

Table 1. Experimental results of the agonistic effects of the compounds provided in some examples of the present invention

| Compound | $EC_{50}$(nM) |
|---|---|
| 2 | 0.23 |
| 3 | 0.26 |
| 7 | 0.22 |
| 27B | 0.17 |
| 28 | 0.07 |
| 29 | 0.22 |
| 31 | 0.19 |
| 34 | 0.20 |
| 36B | 0.22 |
| 39 | 0.15 |
| 43 | 0.20 |
| PF-06882961 | 0.35 |
| Compound 16 in CN113480534A | 0.26 |
| Conclusion: The compound of the present invention shows a better agonistic ability to GLP-1 receptor. | |

**Experimental example 2: Pharmacokinetic study in rats**

1. Material

SD rats: male, 6-8 weeks old, weighing 200-300 g, purchased from SPF ( Beijing ) Biotechnology Co.,Ltd..
Reagents: chromatography grade acetonitrile was purchased from Thermo Fisher Scientific, chromatography grade formic acid was purchased from Beijing Dikema Technology Co., Ltd, the water used in the experiment was ultrapure water, and the rest of the reagents were commercially available analytically pure.
Equipment: AB LCMS-5500 Tandem Mass Spectrometer.

2. Experimental Method

The compounds were weighed and dissolved in 10% DMSO/5% Kolliphor-EL/85% HP-β-CD (20%) or other appropriate systems, and the preparations were all clarified solution. The rats were administered intravenously or by gavage, and the blood samples were collected at 5min, 15min, 30min, 1h, 2h, 4h, 6h, 8h, and 24h after the administration of the compounds. At each PK sampling point, 200 μL of whole blood was collected into EDTA-K2 anticoagulant blood tubes and centrifuged at 4°C for 30 min to obtain plasma. Whole blood samples were placed on wet ice before centrifugation. All collected plasma samples were stored on dry ice or frozen until analyzed.
Approximately1 mg of the compound of the present application was weighed and dissolved in DMSO, vortexed and sonicated to obtain a 1 mg/mL standard stock solution. The standard stock solution was diluted with 50% aqueous acetonitrile to obtain standard working solutions with concentrations of 5, 10, 20, 50, 100, 500, 1000, 5000 and 10000 ng/mL. QC working solutions with concentrations of 10, 20, 500 and 8000 ng/mL were prepared using the same dilution method. These QC samples were prepared on the day of analysis in the same way as the standard curve samples.5 μL of standard working solution with concentrations of (5, 10, 20, 50, 100, 500, 1000, 5000 and 10000 ng/mL) was added to 50 μL of blank SD rat plasma to obtain a total volume of 55 μL with concentrations of 0.5-1000 ng/mL (0.5, 1, 2, 5, 10, 50, 100, 500, and 1000 ng/mL) of standard curve samples. QC samples with concentrations of (1 ng/ml (low-1) and 2 ng/ml (low-2),

50 ng/ml (medium), 800 ng/ml (high)) were configured separately.

55 μL of standard samples, 55 μL of QC samples or 55 μL of unknown samples (50 μL of plasma samples added to 5 μL of 50% aqueous acetonitrile) were added to 200 μL of acetonitrile containing an internal standard (dexamethasone) to precipitate proteins. Then the samples were vortexed for 30 seconds and centrifuged at 4000 g for 15 min at 4°C. The supernatant was taken and diluted 3-fold with water, and 5 μL of the supernatant dilution was injected into the LC-MS/MS system for quantitative analysis under the following assay conditions:

Column: HALO 90A Phenly-Hexyl, 2μm 2.1×30mm.

Mobile phase: Solution A: 100% water (0.1% formic acid); Solution B: 95% acetonitrile (0.1% formic acid, 5% water), gradient elution according to the following table.

| Time (min) | Pump A (%) | Pump B (%) |
|---|---|---|
| 0.20 | 90.0 | 10.0 |
| 1.20 | 0.00 | 100 |
| 1.90 | 0.00 | 100 |
| 1.91 | 90.0 | 10.0 |
| 2.30 | 90.0 | 10.0 |

3. data processing

Pharmacokinetic parameters were calculated by the non-atrial model of Phoenix TM Winnonlin® 8.3 software.

The experimental results are shown in Table 2, Table 2 shows the pharmacokinetic parameters in rats.

Table 2. Experimental results of the pharmacokinetic parameters of the compounds provided in some examples of the present invention

| Compound | Administration | Dosages (mg/kg) | $T_{max}$ (h) | $C_{max}$ (ng/mL) | $AUC_{last}$ (h*ng/mL) | $T_{1/2}$ (h) | F (%) |
|---|---|---|---|---|---|---|---|
| 2 | iv | 1 | 0.083 | 2300 | 1244 | 1.15 | 31.8 |
| | po | 5 | 1.00 | 754 | 1955 | 2.56 | |
| 27B | iv | 1 | 0.083 | 2830 | 1675 | 0.707 | 44.4 |
| | po | 5 | 0.833 | 1161 | 3673 | 4.18 | |
| 25 | iv | 1 | 0.083 | 1081 | 1247 | 2.57 | 38.8 |
| | po | 5 | 4.00 | 460 | 2441 | 5.29 | |
| 38B | iv | 1 | 0.083 | 5283 | 3945 | 1.45 | 21.1 |
| | po | 5 | 1.17 | 1398 | 4129 | 2.62 | |
| Compound 16 in CN113480534A | iv | 1 | 0.083 | 1083 | 642 | 1.87 | 26.8 |
| | po | 5 | 0.67 | 236 | 842 | 1.23 | |
| Conclusion: In this experiment, the pharmacokinetic properties of compound 16 disclosed in CN113480534A were also determined, and the experimental results showed that the compounds of the present invention were better absorbed orally in rats, had higher exposure and were significantly higher than the compound 16 disclosed in CN113480534A. | | | | | | | |

**Experimental example 3: Human Liver Microsome Stability Test**

1. Material

| Liver microsomal species | Manufacturer | Item No. | Batch No. |
|---|---|---|---|
| Human | Corning | 452117 | 38296 |

2. Experimental Method

Preparation of compound working solutions: A high-concentration stock solution of the subject and control drug verapamil powder was prepared in DMSO and diluted to a working solution of 100 μM with DMSO before use, with a final concentration of 1 μM of the subject and verapamil.

Preparation of phosphate buffer salt (100 mM, pH 7.4): 7.098 g of disodium hydrogen phosphate was weighed and dissolved ultrasonically in 500 mL of purified water as solution A. 3.400 g of potassium dihydrogen phosphate was weighed and dissolved ultrasonically in 250 mL of purified water as solution B. Solution B was added to the inside of solution A to give a pH of 7.4.

Preparation of 10 mM NADPH: An appropriate amount of NADPH is now weighed before the experiment and a working solution of 10 mM concentration is prepared from phosphate solution.

Preparation of the incubation system: The incubation system was prepared according to the following table, and the incubation system was preheated in a water bath at 37 °C for 10 minutes before use.

| Component | Concentration stock solution | Volumes | Final concentration |
|---|---|---|---|
| Phosphate buffer salt | 100 mM | 216.25 μL | 100 mM |
| Microsome | 20 mg/mL | 6.25 μL | 0.5 mg/mL |

Transfer 25 μL of NADPH or phosphate buffer salt to the above incubation system and add 2.5 μL of 100 μM subject or verapamil. For NADPH-added samples, double-parallel preparations were performed; for NADPH-negative samples, single-parallel preparations were performed.

30 μL of the suspension was taken at 0.5, 5, 15, 30, and 60 min, respectively. The reaction was terminated by adding 150 μL of acetonitrile containing the internal standard and vortexed for 10 min.

This was followed by centrifugation at 3220 g for 40 min for protein precipitation. 100 μL of supernatant was transferred to the injection plate and mixed with 100 μL of pure water for UPLC-MS/MS analysis.

3. data processing

All data calculations were performed by Microsoft Excel software. Peak areas were detected by extracted ion mapping. The in vitro half-life ($t_{1/2}$) of the parent drug was assayed by fitting the natural logarithm of the percentage elimination of the parent drug linearly with time.

The in vitro half-life ($t_{1/2}$) was calculated by slope: $t_{1/2}$ = 0.693 / k

The in vitro clearance (in μL/min/mg) was calculated using the following formula:

$$CL_{int} = 0.693 / t_{1/2} * (\text{Incubation system volume } (\mu L) / \text{protein content } (mg))$$

The experimental results are shown in Table 3, Table 3 shows the results of human liver microsome of the compounds provided in some examples of the present invention.

Table 3 the experimental results of human liver microsome of the compounds provided in some examples of the present invention

| Compound | $T_{1/2}$(min) |
|---|---|
| 2 | 136.13 |
| 3 | 110.52 |
| 5 | > 184.78 |
| 6 | 111.5 |
| 8 | 180.25 |
| 27B | 102.29 |
| 28 | > 184.78 |
| 31 | 166.33 |

(continued)

| Compound | $T_{1/2}$(min) |
|---|---|
| Compound 16 in CN113480534A | 29.41 |
| Conclusion: In this experiment, the human liver microsomal stability of compound 16 disclosed in CN113480534A was also determined, and the experimental results showed that when the compounds of the present invention were incubated in human liver microsomes, the compounds of the present invention described herein exhibited good stability, and the stability was significantly higher than that of compound 16 disclosed in CN113480534A. | |

In the description of this specification, reference to the terms "an embodiment", "some embodiments", "examples", "specific examples", or "some examples" means that the specific features, structures, materials, or characteristics described in conjunction with the embodiment or example are included in at least one embodiment or example of the present invention. ", "some examples", "exemplary", "specific examples", etc. are used to mean that the specific features, structures, materials, or characteristics described in conjunction with the embodiment or example are included in at least one embodiment or example of the present invention. In this specification, schematic expressions of the above terms need not be directed to the same embodiment or example. Moreover, the specific features, structures, materials or characteristics described may be combined in a suitable manner in any one or more embodiments or examples. Furthermore, without contradicting each other, those skilled in the art may combine and combine different embodiments or examples and features of different embodiments or examples described in this specification.

## Claims

**1.** A compound having formula (X) or a stereoisomer, a geometric isomer, a tautomer, an N-oxide, a hydrate, a solvate, a metabolite, a pharmaceutically acceptable salt or a prodrug thereof,

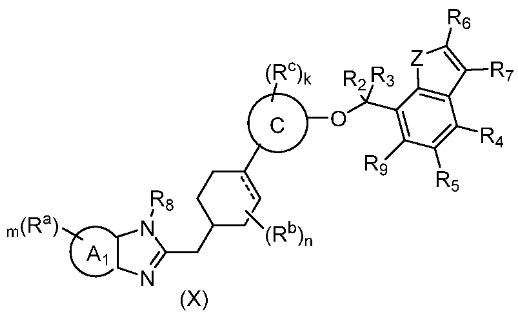

(X)

,

Wherein:

Ring $A_1$ is selected from 5-6 membered heteroaryl, phenyl, 5-6 membered heterocyclyl and $C_{5-6}$ cycloalkyl;
Ring C is 5-6 membered heteroaryl;
Z is selected from O, S, $N(R^z)$ and $C(R^z)_2$;
each of $R^c$, $R_2$ and $R_3$ is independently selected from H, D, F, Cl, Br, I, hydroxyl, cyano, nitro and $C_{1-6}$ alkyl, and wherein the $C_{1-6}$ alkyl is optionally substituted with 1, 2 or 3 substituents selected from F, Cl, Br, I, hydroxyl, cyano, amino and nitro;
each of $R_4$, $R_5$, $R_6$, $R_7$ and $R_9$ is independently selected from H, D, F, Cl, Br, I, hydroxyl, cyano, nitro and $C_{1-6}$ alkyl, and wherein the $C_{1-6}$ alkyl is optionally substituted with 1, 2 or 3 substituents selected from F, Cl, Br, I, hydroxyl, cyano, amino and nitro;
each $R^a$ is independently selected from H, F, Cl, Br, I, hydroxyl, cyano, amino, $-C(=O)OH$, $-C(=O)NHC(=O)C_{1-6}$ alkyl, $-C(=O)NHS(=O)_2C_{1-6}$ alkyl, $-C(=O)NHS(=O)_2NH_2$, $-C(=O)NHS(=O)_2NHC_{1-6}$ alkyl,$-C(=O)NHS(=O)_2C_{3-6}$ cycloalkyl, $-C(=O)NHS(=O)_2$ phenyl, 5-6 membered heteroaryl and

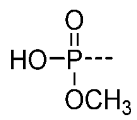

,

and wherein the -C(=O)NHC(=O)$C_{1-6}$ alkyl, -C(=O)NHS(=O)$_2C_{1-6}$ alkyl, -C(=O)NHS(=O)$_2NH_2$,-C(=O) NHS(=O)$_2$NH$C_{1-6}$ alkyl, -C(=O)NHS(=O)$_2C_{3-6}$ cycloalkyl, -C(=O)NHS(=O)$_2$ phenyl or 5-6 membered heteroaryl is optionally substituted with 1, 2, 3, 4 or 5 substituents selected from D, F, Cl, Br, I, hydroxyl, oxo, amino, nitro and cyano;

each $R^z$ is independently selected from H, F, Cl, Br, I, hydroxyl, cyano, nitro, $C_{1-6}$ alkyl, $C_{1-6}$ alkoxy, $C_{1-6}$ alkylthio, $C_{1-6}$ alkylamino, carboxyl, -C(=O)O$C_{1-6}$ alkyl, -C(=O)NH$C_{1-6}$ alkyl and -$C_{1-6}$ alkylene C(=O)O$C_{1-6}$ alkyl, and wherein the C(=O)O$C_{1-6}$ alkyl, -C(=O)NH$C_{1-6}$ alkyl or -$C_{1-6}$ alkylene C(=O)O$C_{1-6}$ alkyl is optionally substituted with 1, 2, 3, 4 or 5 substituents selected from D, F, Cl, Br, I, hydroxyl, oxo, amino, nitro and cyano;

each $R^b$ is independently selected from H, F, Cl, Br, I, hydroxyl, cyano, nitro, $C_{1-6}$ alkyl, $C_{1-6}$ alkoxy, $C_{1-6}$ alkylthio, $C_{1-6}$ alkylamino, carboxyl, -C(=O)O$C_{1-6}$ alkyl and -$C_{1-6}$ alkylene C(=O)O$C_{1-6}$ alkyl, and wherein the C(=O)O$C_{1-6}$ alkyl or -$C_{1-6}$ alkylene C(=O)O$C_{1-6}$ alkyl is optionally substituted with 1, 2, 3, 4 or 5 substituents selected from D, F, Cl, Br, I, hydroxyl, oxo, amino, nitro and cyano;

$R_8$ is selected from H, $C_{1-6}$ alkyl, $C_{2-6}$ alkenyl, $C_{2-6}$ alkynyl, $C_{3-6}$ cycloalkyl, 3-6 membered heterocyclyl, -$C_{1-6}$ alkylene -$R_{10}$ and $C_{1-6}$ alkylene C($R_SR_MR_N$), and wherein the $C_{1-6}$ alkyl, $C_{2-6}$ alkenyl, $C_{2-6}$ alkynyl, $C_{3-6}$ cycloalkyl, 3-6 membered heterocyclyl, -$C_{1-6}$ alkylene -$R_{10}$ or $C_{1-6}$ alkylene C($R_SR_MR_N$) is optionally substituted with 1, 2 or 3 $R^{8a}$;

each $R^{8a}$ is independently selected from D, F, Cl, Br, I, CN, $C_{1-6}$ alkyl, $C_{1-6}$ alkoxy, $C_{1-6}$ alkylthio, $C_{1-6}$ alkylamino, $C_{3-6}$ cycloalkyl and 3-6 membered heterocyclyl, and wherein the $C_{1-6}$ alkyl, $C_{1-6}$ alkoxy, $C_{1-6}$ alkylthio, $C_{1-6}$ alkylamino, $C_{3-6}$ cycloalkyl and 3-6 membered heterocyclyl is optionally substituted with 1, 2 or 3 $R^{8b}$;

each $R^{8b}$ is independently selected from D, F, Cl, Br, I, CN, $C_{1-6}$ alkyl, $C_{1-6}$ alkoxy, $C_{1-6}$ alkylthio, $C_{1-6}$ alkylamino, $C_{3-6}$ cycloalkyl and 3-6 membered heterocyclyl;

$R_{10}$ is selected from $C_{3-6}$ cycloalkyl, 3-8 membered heterocyclyl and 5-10 membered heteroaryl, and wherein the $C_{3-6}$ cycloalkyl, 3-8 membered heterocyclyl or 5-10 membered heteroaryl is optionally substituted with 1, 2, 3, 4 or 5 substituents selected from D, F, Cl, Br, I, oxo, hydroxyl, Amino, cyano, nitro, $C_{1-6}$ alkyl, $C_{1-6}$ alkoxy, $C_{1-6}$ alkylthio and $C_{1-6}$ alkylamino;

each of $R_S$, $R_M$ and $R_N$ is independently selected from D, F, Cl, Br, I, hydroxyl, oxo, amino, nitro and cyano; or $R_S$ and $R_M$ join together to form $C_{3-6}$ cycloalkyl or 3-6 membered heterocyclyl;

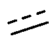

represents a single or double bond;

m is 0, 1, 2, 3 or 4;

k is 0, 1, 2, 3 or 4;

n is 0, 1, 2, 3, 4, 5, 6, 7 or 8.

**2.** The compound of claim 1 having formula (I-a), (I-b) or (I-c) or a stereoisomer, a geometric isomer, a tautomer, an N-oxide, a hydrate, a solvate, a metabolite, a pharmaceutically acceptable salt or a prodrug thereof,

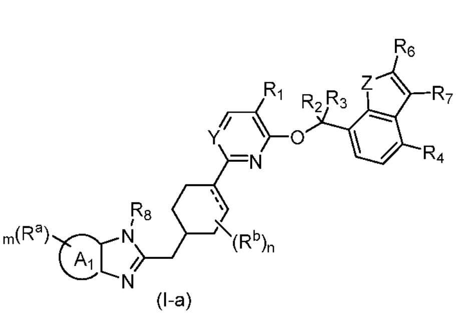

(I-a)

,

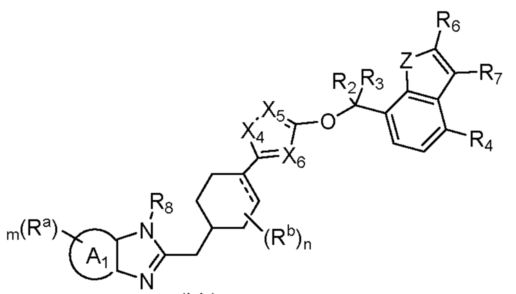

(I-b)

,

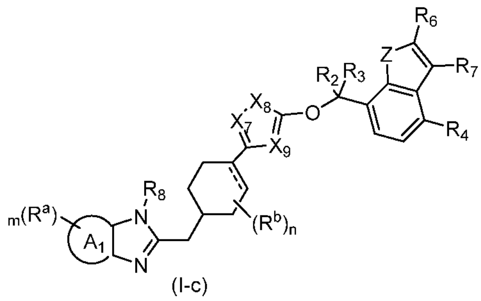

(I-c)

,

Wherein:

Ring $A_1$ is selected from 5-6 membered heteroaryl, phenyl, 5-6 membered heterocyclyl and $C_{5-6}$ cycloalkyl;
each of $X_5$, $X_6$, $X_7$ and $X_9$ is independently selected from $C(R^c)$ and N;
each of $X_4$ and $X_8$ is independently selected from O, S, $C(R^c)_2$ and $N(R^c)$;
each of Y is independently selected from $C(R^Y)$ and N;
each of Z is independently selected from O, S, $N(R^z)$ and $C(R^z)_2$;
each of $R^c$, $R^1$, $R_2$ and $R_3$ is independently selected from H, D, F, Cl, Br, I, hydroxyl, cyano, nitro and $C_{1-6}$ alkyl, and wherein the $C_{1-6}$ alkyl is optionally substituted with 1, 2 or 3 substituents selected from F, Cl, Br, I, hydroxyl, cyano, amino and nitro;
each of $R_4$, $R_6$ and $R_7$ is independently selected from H, D, F, Cl, Br, I, hydroxyl, cyano, nitro and $C_{1-6}$ alkyl, and wherein the $C_{1-6}$ alkyl is optionally substituted with 1, 2 or 3 substituents selected from F, Cl, Br, I, hydroxyl, cyano, amino and nitro;
each $R^a$ is independently selected from H, F, Cl, Br, I, hydroxyl, cyano, amino, -C(=O)OH, -C(=O)NHC(=O)$C_{1-6}$ alkyl, -C(=O)NHS(=O)$_2C_{1-6}$ alkyl, -C(=O)NHS(=O)$_2NH_2$, -C(=O)NHS(=O)$_2NHC_{1-6}$ alkyl,-C(=O)NHS(=O)$_2C_{3-6}$ cycloalkyl, -C(=O)NHS(=O)$_2$ phenyl, 5-6 membered heteroaryl and

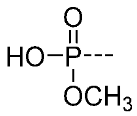

,

and wherein the -C(=O)NHC(=O)$C_{1-6}$ alkyl, -C(=O)NHS(=O)$_2C_{1-6}$ alkyl, -C(=O)NHS(=O)$_2NH_2$,-C(=O)NHS(=O)$_2NHC_{1-6}$ alkyl, -C(=O)NHS(=O)$_2C_{3-6}$ cycloalkyl, -C(=O)NHS(=O)$_2$ phenyl or 5-6 membered heteroaryl is optionally substituted with 1, 2, 3, 4 or 5 substituents selected from D, F, Cl, Br, I, hydroxyl, oxo, amino, nitro and cyano;
each of $R^Y$ and $R^z$ is independently selected from H, F, Cl, Br, I, hydroxyl, cyano, nitro, $C_{1-6}$ alkyl, $C_{1-6}$ alkoxy, $C_{1-6}$ alkylthio, $C_{1-6}$ alkylamino, carboxyl, -C(=O)O$C_{1-6}$ alkyl, -C(=O)NH$C_{1-6}$ alkyl and -$C_{1-6}$ alkylene C(=O)O$C_{1-6}$ alkyl, and wherein the C(=O)O$C_{1-6}$ alkyl, -C(=O)NH$C_{1-6}$ alkyl or -$C_{1-6}$ alkylene C(=O)O$C_{1-6}$ alkyl is optionally substituted with 1, 2, 3, 4 or 5 substituents selected from D, F, Cl, Br, I, hydroxyl, oxo, amino, nitro and cyano;
each $R^b$ is independently selected from H, F, Cl, Br, I, hydroxyl, cyano, nitro, $C_{1-6}$ alkyl, $C_{1-6}$ alkoxy, $C_{1-6}$ alkylthio, $C_{1-6}$ alkylamino, carboxyl, -C(=O)O$C_{1-6}$ alkyl and -$C_{1-6}$ alkylene C(=O)O$C_{1-6}$ alkyl, and wherein the C(=O)O$C_{1-6}$ alkyl or -$C_{1-6}$ alkylene C(=O)O$C_{1-6}$ alkyl is optionally substituted with 1, 2, 3, 4 or 5 substituents selected from D, F, Cl, Br, I, hydroxyl, oxo, amino, nitro and cyano;
$R_8$ is selected from H, $C_{1-6}$ alkyl, $C_{2-6}$ alkenyl, $C_{2-6}$ alkynyl, $C_{3-6}$ cycloalkyl, 3-6 membered heterocyclyl, -$C_{1-6}$ alkylene -$R_{10}$ and $C_{1-6}$ alkylene $C(R_SR_MR_N)$, and wherein the $C_{1-6}$ alkyl, $C_{2-6}$ alkenyl, $C_{2-6}$ alkynyl, $C_{3-6}$ cycloalkyl, 3-6 membered heterocyclyl, -$C_{1-6}$ alkylene -$R_{10}$ or $C_{1-6}$ alkylene $C(R_SR_MR_N)$ is optionally substituted with 1, 2 or 3 $R^{8a}$;
each $R^{8a}$ is independently selected from D, F, Cl, Br, I, CN, $C_{1-6}$ alkyl, $C_{1-6}$ alkoxy, $C_{1-6}$ alkylthio, $C_{1-6}$ alkylamino, $C_{3-6}$ cycloalkyl and 3-6 membered heterocyclyl, and wherein the $C_{1-6}$ alkyl, $C_{1-6}$ alkoxy, $C_{1-6}$ alkylthio, $C_{1-6}$ alkylamino, $C_{3-6}$ cycloalkyl and 3-6 membered heterocyclyl is optionally substituted with 1, 2 or 3 $R^{8b}$;
each $R^{8b}$ is independently selected from D, F, Cl, Br, I, CN, $C_{1-6}$ alkyl, $C_{1-6}$ alkoxy, $C_{1-6}$ alkylthio, $C_{1-6}$ alkylamino, $C_{3-6}$ cycloalkyl and 3-6 membered heterocyclyl;
$R_{10}$ is selected from $C_{3-6}$ cycloalkyl, 3-8 membered heterocyclyl and 5-10 membered heteroaryl, and wherein the $C_{3-6}$ cycloalkyl, 3-8 membered heterocyclyl or 5-10 membered heteroaryl is optionally substituted with 1, 2, 3, 4 or 5 substituents selected from D, F, Cl, Br, I, oxo, hydroxyl, Amino, cyano, nitro, $C_{1-6}$ alkyl, $C_{1-6}$ alkoxy, $C_{1-6}$ alkylthio and $C_{1-6}$ alkylamino;

each of $R_S$, $R_M$ and $R_N$ is independently selected from D, F, Cl, Br, I, hydroxyl, oxo, amino, nitro and cyano; or $R_S$ and $R_M$ join together to form $C_{3-6}$ cycloalkyl or 3-6 membered heterocyclyl;

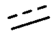

represents a single or double bond;
m is 0, 1, 2, 3 or 4;
k is 0, 1, 2, 3 or 4;
n is 0, 1, 2, 3, 4, 5, 6, 7 or 8.

**3.** The compound of claim 1 having formula (II-a), (II-b), (II-c), (II-d), (II-e), (II-f), (II-g), (II-h) or (II-i) or a stereoisomer, a geometric isomer, a tautomer, an N-oxide, a hydrate, a solvate, a metabolite, a pharmaceutically acceptable salt or a prodrug thereof,

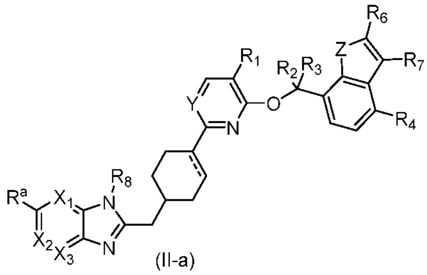
(II-a) ,

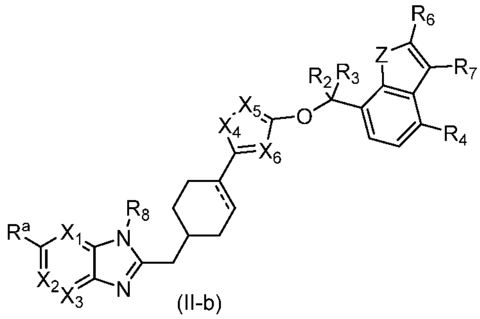
(II-b) ,

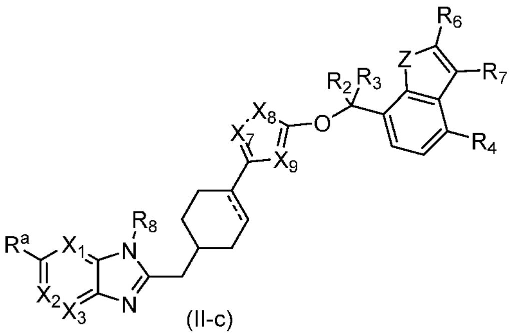
(II-c) ,

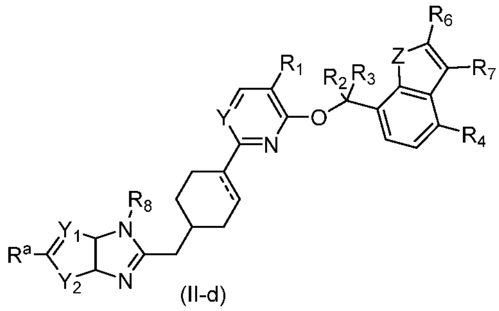
(II-d) ,

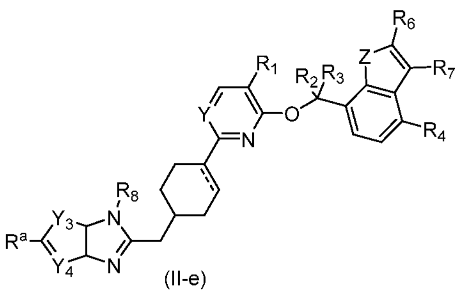
(II-e) ,

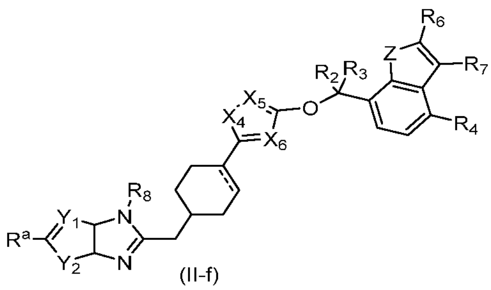
(II-f) ,

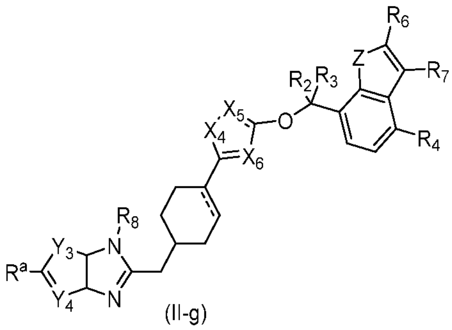

(II-g) ,

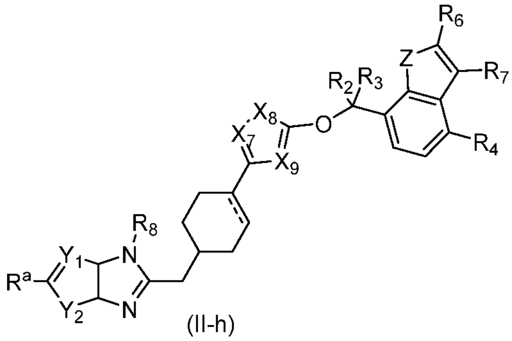

(II-h) ,

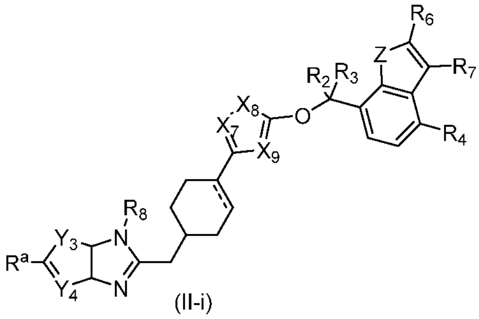

(II-i) ,

Wherein:

each of $X_1$, $X_2$ and $X_3$ is independently selected from $C(R^a)$ and N;
each of $X_5$, $X_6$, $X_7$, $X_9$, $Y_1$ and $Y_4$ is independently selected from $C(R^c)$ and N;
each of $X_4$, $X_8$, $Y_2$ and $Y_3$ is independently selected from O, S, $C(R^c)_2$ and $N(R^c)$;
Y is selected from $C(R^Y)$ and N;
Z is selected from O, S, $N(R^z)$ and $C(R^z)_2$;
each $R^a$ is independently selected from H, F, Cl, Br, I, hydroxyl, cyano, amino, -C(=O)OH, -C(=O)NHC(=O)$C_{1-6}$ alkyl, -C(=O)NHS(=O)$_2$$C_{1-6}$ alkyl, -C(=O)NHS(=O)$_2$$NH_2$, -C(=O)NHS(=O)$_2$NH$C_{1-6}$ alkyl,-C(=O)NHS(=O)$_2$$C_{3-6}$ cycloalkyl, -C(=O)NHS(=O)$_2$ phenyl, 5-6 membered heteroaryl and

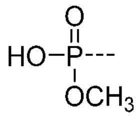
,

and wherein the -C(=O)NHC(=O)$C_{1-6}$ alkyl, -C(=O)NHS(=O)$_2$$C_{1-6}$ alkyl, -C(=O)NHS(=O)$_2$$NH_2$,-C(=O) NHS(=O)$_2$NH$C_{1-6}$ alkyl, -C(=O)NHS(=O)$_2$$C_{3-6}$ cycloalkyl, -C(=O)NHS(=O)$_2$ phenyl or 5-6 membered heteroaryl is optionally substituted with 1, 2, 3, 4 or 5 substituents selected from D, F, Cl, Br, I, hydroxyl, oxo, amino, nitro and cyano;
each of $R^c$, $R_1$, $R_2$ and $R_3$ is independently selected from H, D, F, Cl, Br, I, hydroxyl, cyano, nitro and $C_{1-6}$ alkyl, and wherein the $C_{1-6}$ alkyl is optionally substituted with 1, 2 or 3 substituents selected from F, Cl, Br, I, hydroxyl, cyano, amino and nitro;
each of $R_4$, $R_6$ and $R_7$ is independently selected from H, D, F, Cl, Br, I, hydroxyl, cyano, nitro and $C_{1-6}$ alkyl, and wherein the $C_{1-6}$ alkyl is optionally substituted with 1, 2 or 3 substituents selected from F, Cl, Br, I, hydroxyl, cyano, amino and nitro;
each of $R^Y$ and $R^z$ is independently selected from H, F, Cl, Br, I, hydroxyl, cyano, nitro, $C_{1-6}$ alkyl, $C_{1-6}$ alkoxy, $C_{1-6}$ alkylthio, $C_{1-6}$ alkylamino, carboxyl, -C(=O)O$C_{1-6}$ alkyl, -C(=O)NH$C_{1-6}$ alkyl and -$C_{1-6}$ alkylene C(=O)O$C_{1-6}$ alkyl, and wherein the C(=O)O$C_{1-6}$ alkyl, -C(=O)NH$C_{1-6}$ alkyl or -$C_{1-6}$ alkylene C(=O)O$C_{1-6}$ alkyl is optionally substituted with 1, 2, 3, 4 or 5 substituents selected from D, F, Cl, Br, I, hydroxyl, oxo, amino, nitro and cyano; each $R^b$ is independently selected from H, F, Cl, Br, I, hydroxyl, cyano, nitro, $C_{1-6}$ alkyl, $C_{1-6}$ alkoxy, $C_{1-6}$ alkylthio, $C_{1-6}$ alkylamino, carboxyl, -C(=O)O$C_{1-6}$ alkyl and -$C_{1-6}$ alkylene C(=O)O$C_{1-6}$ alkyl, and wherein the C(=O)O$C_{1-6}$ alkyl or -$C_{1-6}$ alkylene C(=O)O$C_{1-6}$ alkyl is optionally substituted with 1, 2, 3, 4 or 5 substituents selected from D, F, Cl,

Br, I, hydroxyl, oxo, amino, nitro and cyano;

$R_8$ is selected from H, $C_{1-6}$ alkyl, $C_{2-6}$ alkenyl, $C_{2-6}$ alkynyl, $C_{3-6}$ cycloalkyl, 3-6 membered heterocyclyl, -$C_{1-6}$ alkylene -$R_{10}$ and $C_{1-6}$ alkylene $C(R_SR_MR_N)$, and wherein the $C_{1-6}$ alkyl, $C_{2-6}$ alkenyl, $C_{2-6}$ alkynyl, $C_{3-6}$ cycloalkyl, 3-6 membered heterocyclyl, -$C_{1-6}$ alkylene -$R_{10}$ or $C_{1-6}$ alkylene $C(R_SR_MR_N)$ is optionally substituted with 1, 2 or 3 $R^{8a}$;

each $R^{8a}$ is independently selected from D, F, Cl, Br, I, CN, $C_{1-6}$ alkyl, $C_{1-6}$ alkoxy, $C_{1-6}$ alkylthio, $C_{1-6}$ alkylamino, $C_{3-6}$ cycloalkyl and 3-6 membered heterocyclyl, and wherein the $C_{1-6}$ alkyl, $C_{1-6}$ alkoxy, $C_{1-6}$ alkylthio, $C_{1-6}$ alkylamino, $C_{3-6}$ cycloalkyl and 3-6 membered heterocyclyl is optionally substituted with 1, 2 or 3 $R^{8b}$;

each $R^{8b}$ is independently selected from D, F, Cl, Br, I, CN, $C_{1-6}$ alkyl, $C_{1-6}$ alkoxy, $C_{1-6}$ alkylthio, $C_{1-6}$ alkylamino, $C_{3-6}$ cycloalkyl and 3-6 membered heterocyclyl;

$R_{10}$ is selected from $C_{3-6}$ cycloalkyl, 3-8 membered heterocyclyl and 5-10 membered heteroaryl, and wherein the $C_{3-6}$ cycloalkyl, 3-8 membered heterocyclyl or 5-10 membered heteroaryl is optionally substituted with 1, 2, 3, 4 or 5 substituents selected from D, F, Cl, Br, I, oxo, hydroxyl, Amino, cyano, nitro, $C_{1-6}$ alkyl, $C_{1-6}$ alkoxy, $C_{1-6}$ alkylthio and $C_{1-6}$ alkylamino;

each of $R_S$, $R_M$ and $R_N$ is independently selected from D, F, Cl, Br, I, hydroxyl, oxo, amino, nitro and cyano;

or $R_S$ and $R_M$ join together to form $C_{3-6}$ cycloalkyl or 3-6 membered heterocyclyl;

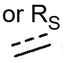 represents a single or double bond.

**4.** The compound of any one of claim 1 to 3 or a stereoisomer, a geometric isomer, a tautomer, an N-oxide, a hydrate, a solvate, a metabolite, a pharmaceutically acceptable salt or a prodrug thereof,

wherein the $R_8$ is selected from H, $C_{1-3}$ alkyl, $C_{2-3}$ alkenyl, $C_{2-3}$ alkynyl, $C_{3-6}$ cycloalkyl, 3-6 membered heterocyclyl and -$C_{1-3}$ alkylene -$R_{10}$, and wherein the $C_{1-3}$ alkyl, $C_{2-3}$ alkenyl, $C_{2-3}$ alkynyl, $C_{3-6}$ cycloalkyl, 3-6 membered heterocyclyl or -$C_{1-3}$ alkylene -$R_{10}$ is optionally substituted with 1, 2 or 3 $R^{8a}$;

each $R^{8a}$ is independently selected from D, F, Cl, Br, I, CN, $C_{1-3}$ alkyl, $C_{1-3}$ alkoxy, $C_{1-3}$ alkylthio, $C_{1-3}$ alkylamino, $C_{4-6}$ cycloalkyl and 5-6 membered heterocyclyl, and wherein the $C_{1-3}$ alkyl, $C_{1-3}$ alkoxy, $C_{1-3}$ alkylthio, $C_{1-3}$ alkylamino, $C_{4-6}$ cycloalkyl and 5-6 membered heterocyclyl is optionally substituted with 1, 2 or 3 $R^{8b}$;

each $R^{8b}$ is independently selected from D, F, Cl, Br, I, CN, $C_{1-3}$ alkyl, $C_{1-3}$ alkoxy, $C_{1-3}$ alkylthio, $C_{1-3}$ alkylamino, $C_{5-6}$ cycloalkyl and 5-6 membered heterocyclyl;

$R_{10}$ is optionally selected from $C_{5-6}$ cycloalkyl, 5-6 membered heterocyclyl and 5-10 membered heteroaryl;

preferably, $R_{10}$ is optionally selected from cyclopropyl, cyclobutyl, cyclopentyl, cyclohexyl, azetidinyl, pyrrolidinyl, tetrahydrofuranyl, piperidyl, piperazinyl, morpholinyl,

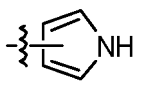, 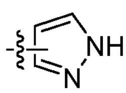, 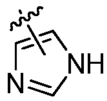,

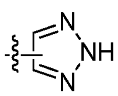, 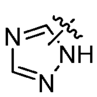, 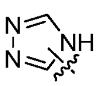, 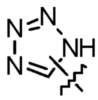, 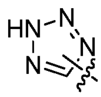, 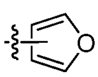, 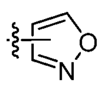, 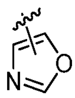, 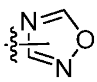,

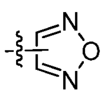, 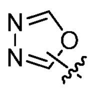, 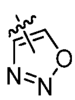, 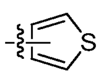, 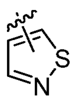, 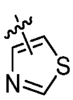, 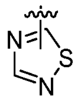, 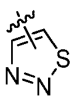, 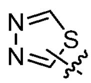, 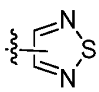, 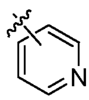,

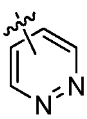, 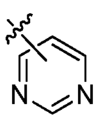, 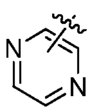, 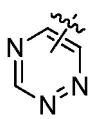, 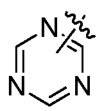 and 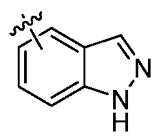;

$R_8$ is optionally selected from H,

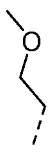, 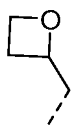, 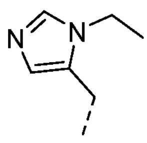, 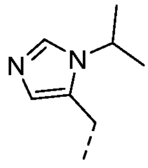, 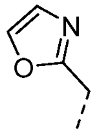, 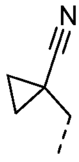, 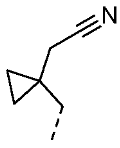

and

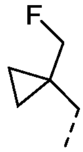;

each of $R^1$, $R_2$ and $R_3$ is optionally independently selected from H, D, F, Cl, Br and I;
each of $R_4$, $R_6$ and $R_7$ is optionally independently selected from H, D, F, Cl, Br and I;
each $R^a$ is optionally independently selected from -C(=O)OH, -C(=O)NHC(=O)$C_{1-3}$ alkyl, -C(=O)NHS(=O)$_2C_{1-3}$ alkyl, -C(=O)NHS(=O)$_2NH_2$, -C(=O)NHS(=O)$_2NHC_{1-3}$ alkyl, -C(=O)NHS(=O)$_2C_{3-6}$ cycloalkyl,-C(=O)NHS(=O)$_2$ phenyl, 5-6 membered heteroaryl and

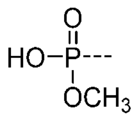,

and wherein the -C(=O)NHC(=O)$C_{1-3}$ alkyl, -C(=O)NHS(=O)$_2C_{1-3}$ alkyl, -C(=O)NHS(=O)$_2NH_2$, -C(=O)NHS(=O)$_2NHC_{1-3}$ alkyl, -C(=O)NHS(=O)$_2C_{3-6}$ cycloalkyl, -C(=O)NHS(=O)$_2$ phenyl or 5-6 membered heteroaryl is optionally substituted with 1, 2, 3, 4 or 5 substituents selected from D, F, Cl, Br, I, hydroxyl, oxo, amino, nitro and cyano;
preferably, each $R^a$ is optionally independently selected from

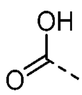, 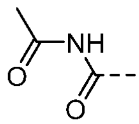, 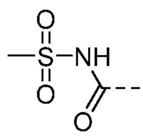,

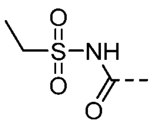, 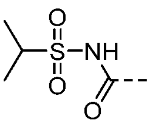, 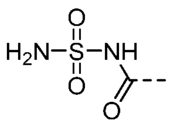, 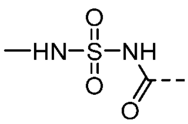, 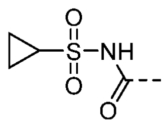, 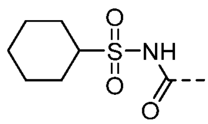,

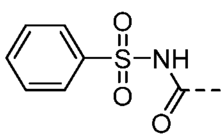, 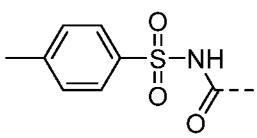, 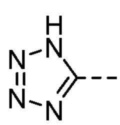 and 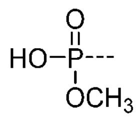.

**5.** The compound of any one of claim 1 to 3 or a stereoisomer, a geometric isomer, a tautomer, an *N*-oxide, a hydrate, a solvate, a metabolite, a pharmaceutically acceptable salt or a prodrug thereof,

wherein each $R^a$ is optionally independently selected from H, F, Cl, Br, I, hydroxyl, cyano, amino, -C(=O)OH,-C(=O)NHC(=O)$C_{1-3}$ alkyl, -C(=O)NHS(=O)$_2C_{1-3}$ alkyl, -C(=O)NHS(=O)$_2NH_2$, -C(=O)NHS(=O)$_2NHC_{1-3}$ alkyl, -C(=O)NHS(=O)$_2C_{3-6}$ cycloalkyl, -C(=O)NHS(=O)$_2$ phenyl, 5-6 membered heteroaryl and

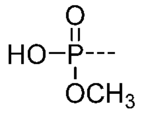,

and wherein the $-C(=O)NHC(=O)C_{1-3}$ alkyl, $-C(=O)NHS(=O)_2C_{1-3}$ alkyl, $-C(=O)NHS(=O)_2NH_2$,$-C(=O)NHS(=O)_2NHC_{1-3}$ alkyl, $-C(=O)NHS(=O)_2C_{3-6}$ cycloalkyl, $-C(=O)NHS(=O)_2$ phenyl or 5-6 membered heteroaryl is optionally substituted with 1, 2, 3, 4 or 5 substituents selected from D, F, Cl, Br, I, hydroxyl, oxo, amino, nitro and cyano;

preferably, each $R^a$ is optionally independently selected from H, F, Cl, Br, I, hydroxyl, cyano, amino,

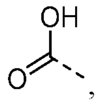,

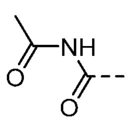, 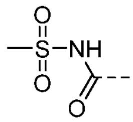, 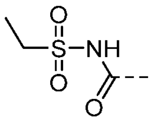, 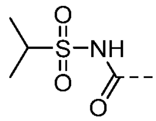, 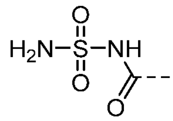, 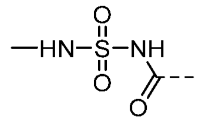,

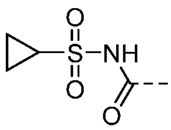, 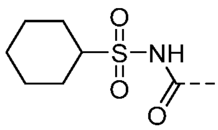, 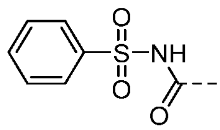, 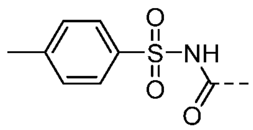, 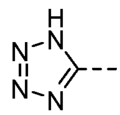

and

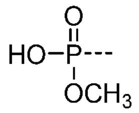.

**6.** The compound of claim 1 is selected from one of the following structures or a stereoisomer, a geometric isomer, a tautomer, an *N*-oxide, a hydrate, a solvate, a metabolite, a pharmaceutically acceptable salt or a prodrug thereof:

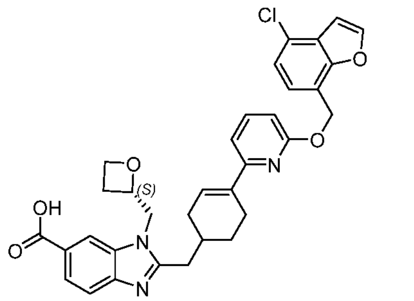, 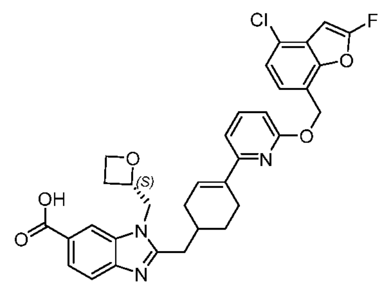,

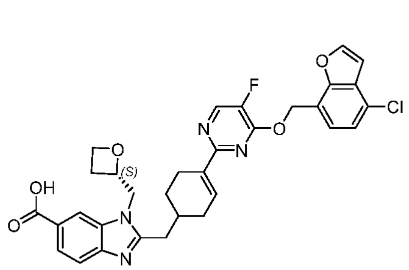, 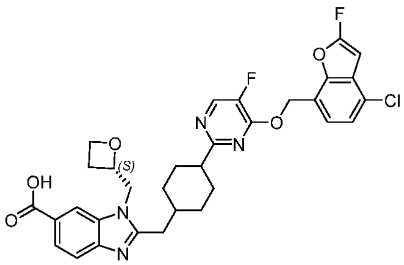,

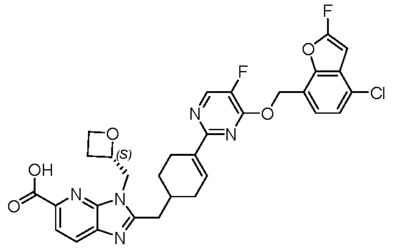,
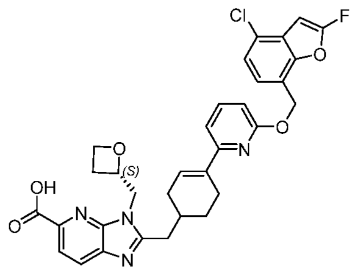,
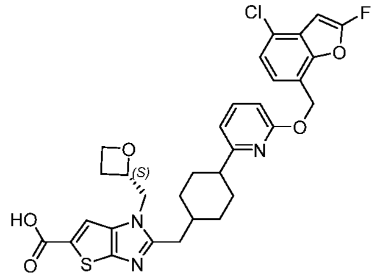,

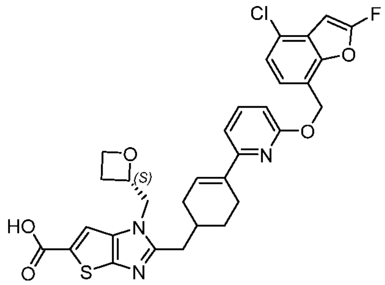,
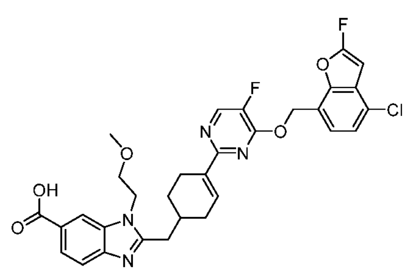,
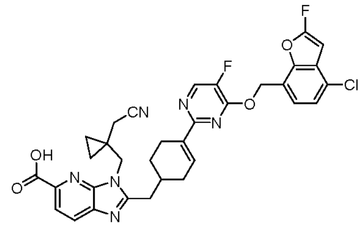,

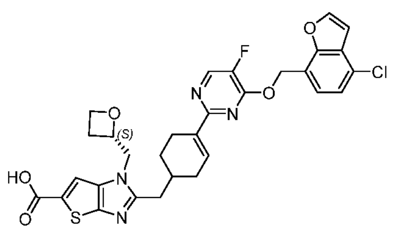,
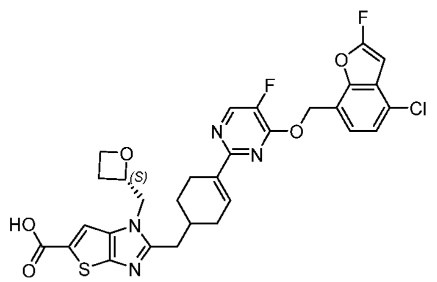,

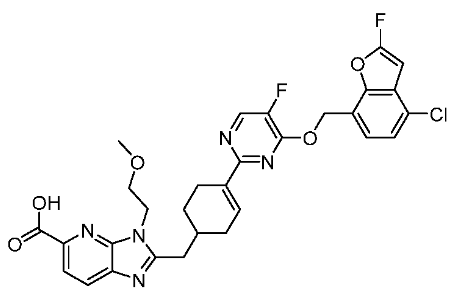,
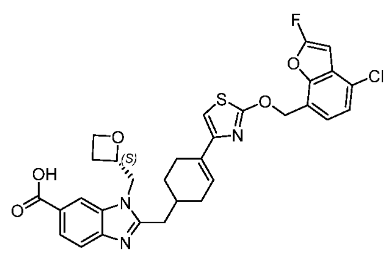,

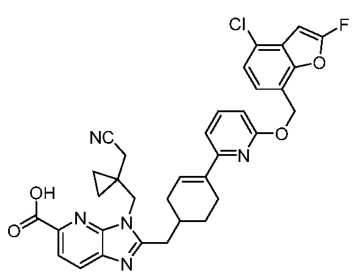,
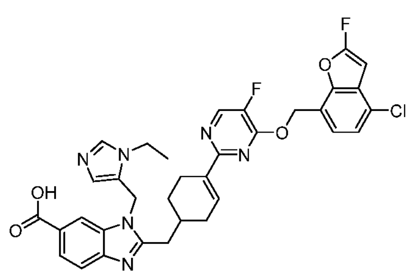,
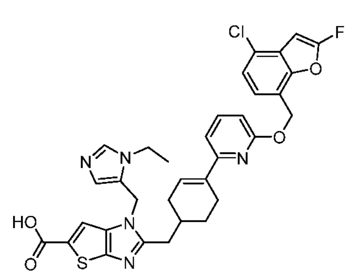,

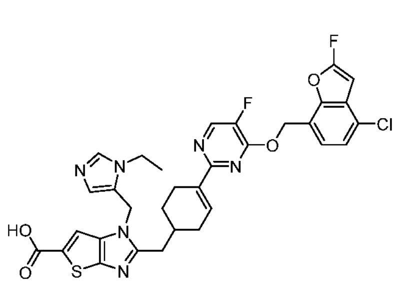
,
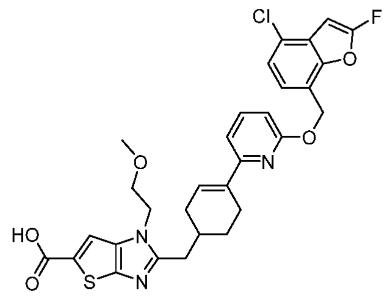
,
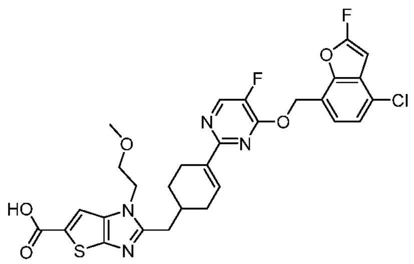
,
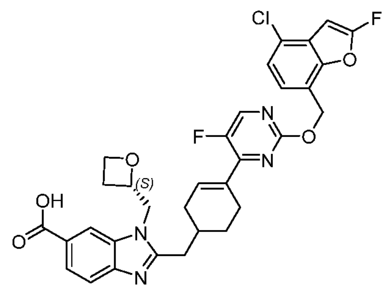
,
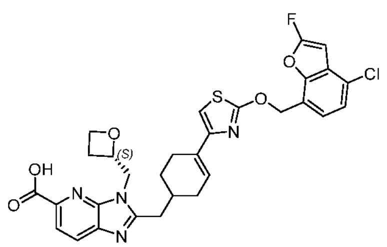
,
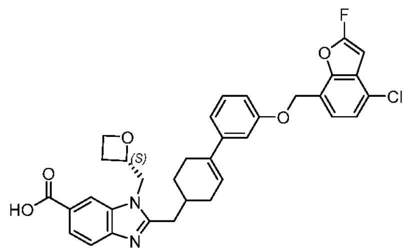
,
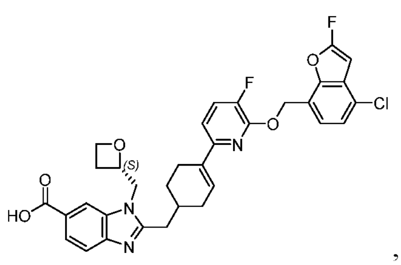
,
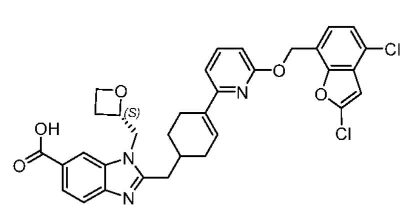
,
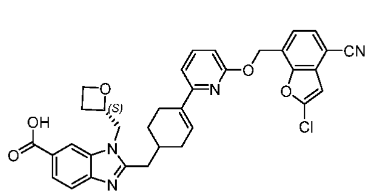
,
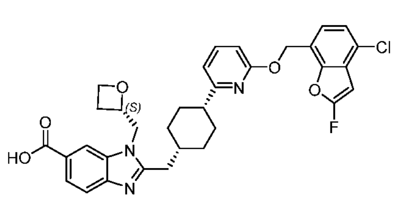
,
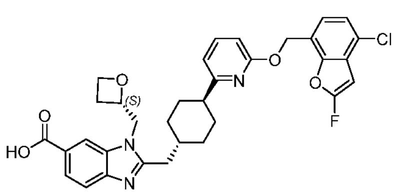
,
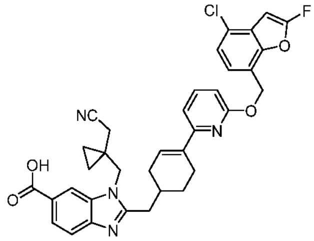
,

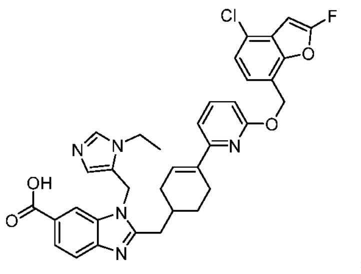,
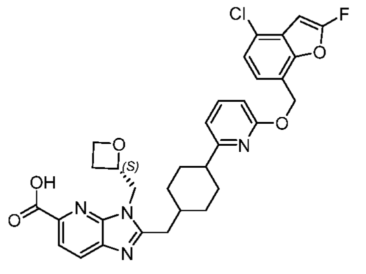,
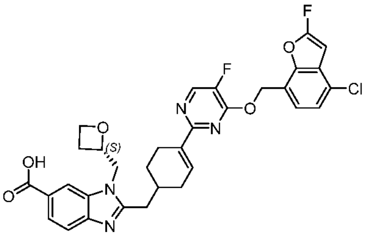,

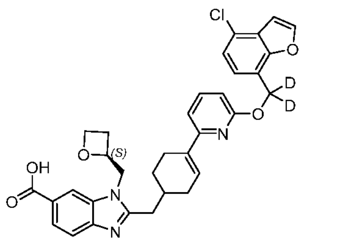,
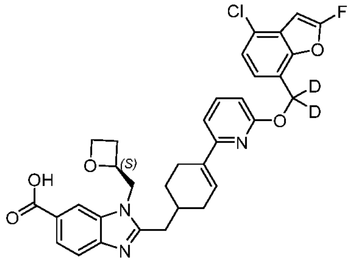,
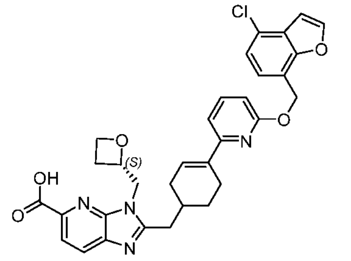,

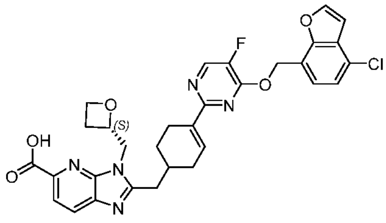,
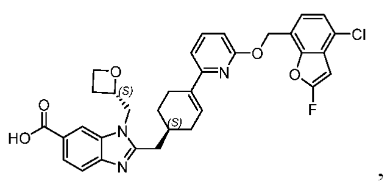,
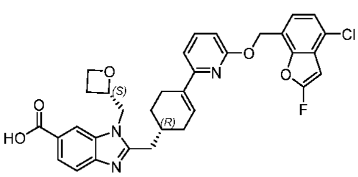,

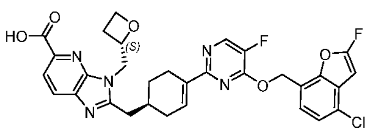,
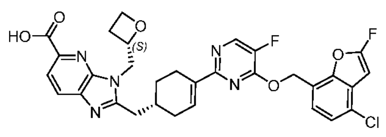,
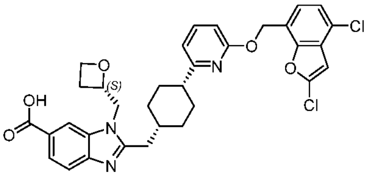,

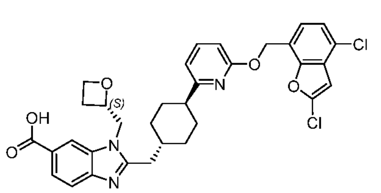,
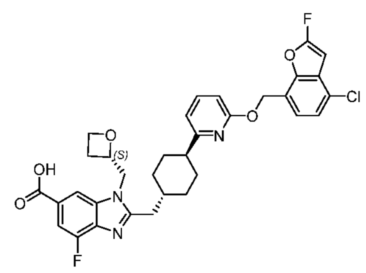,
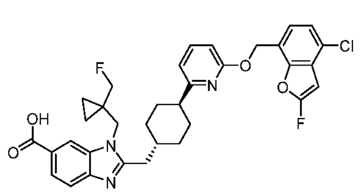,

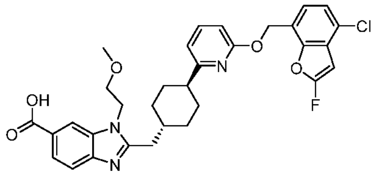,
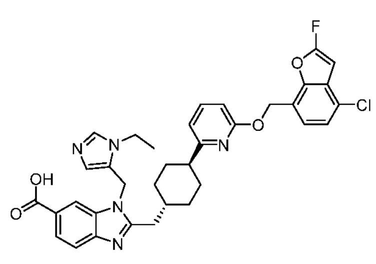,
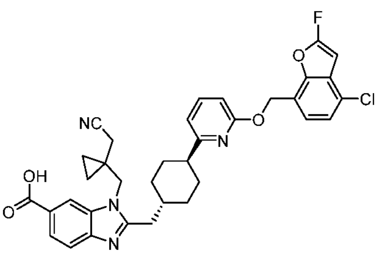,

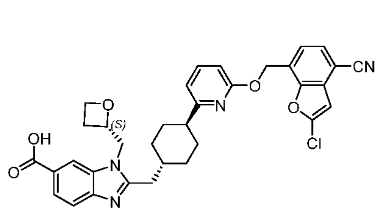 , 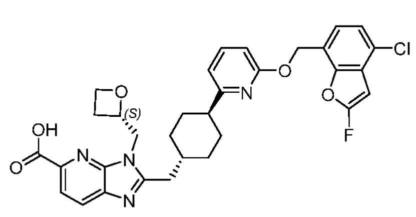 , 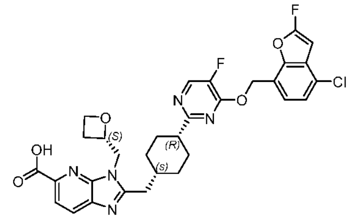 ,

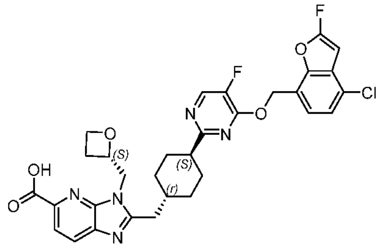 , 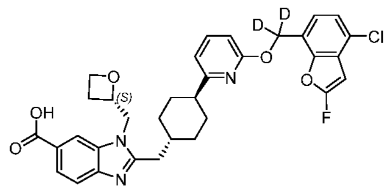 , 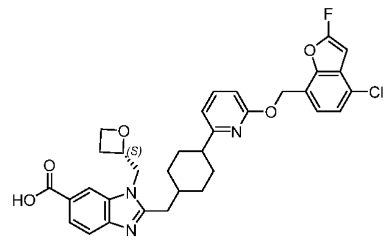 ,

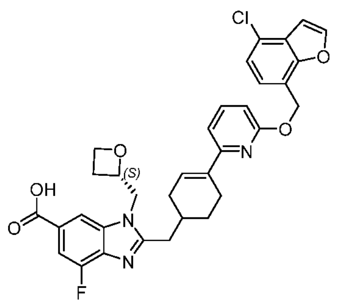 , 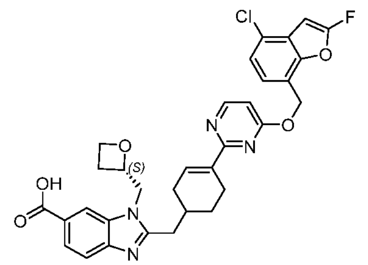 , 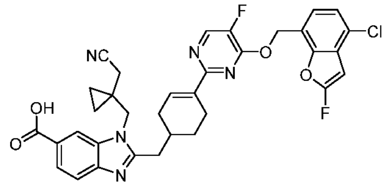 ,

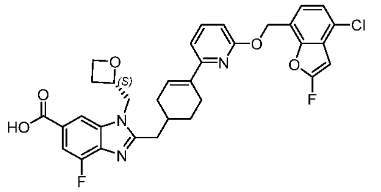 , 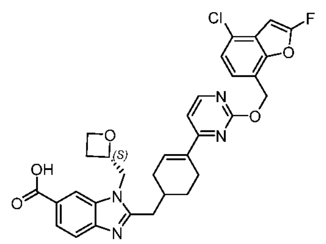 , 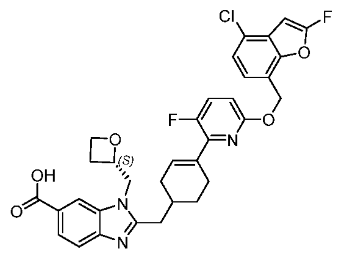 ,

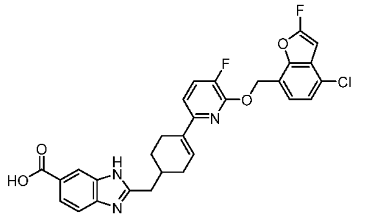 , 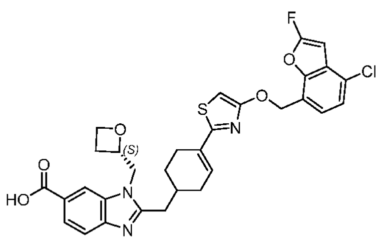 , 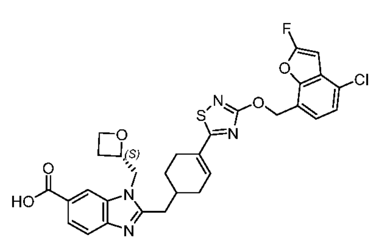 ,

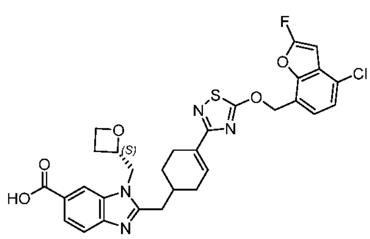 , 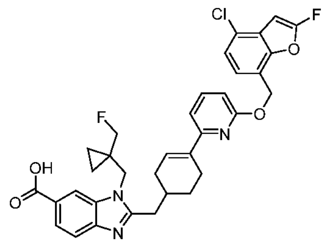 , 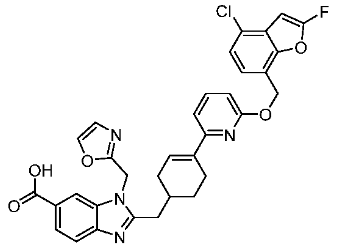 ,

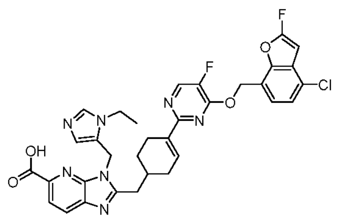 , 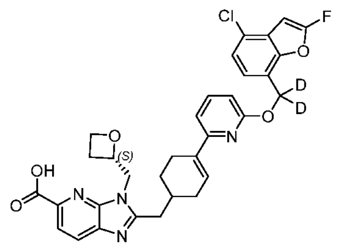 , 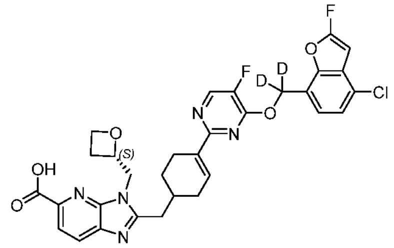 ,

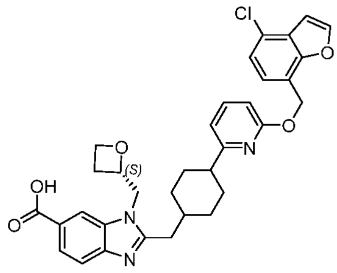 , 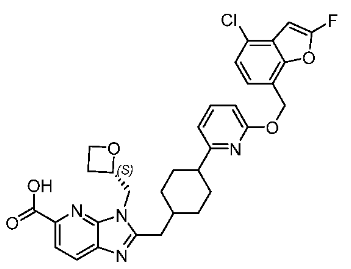 , 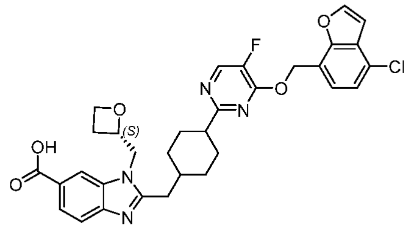 ,

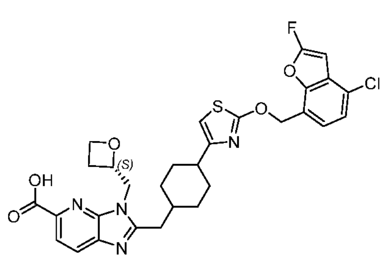 , 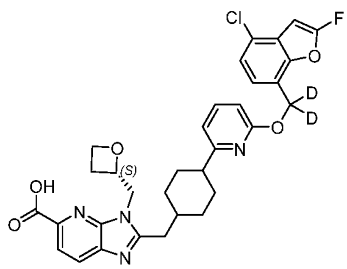 , 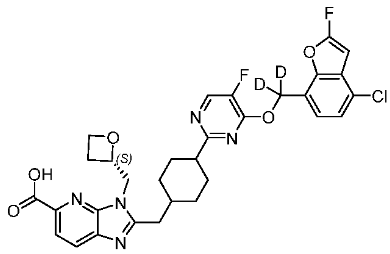 ,

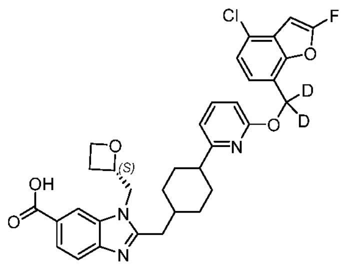 , 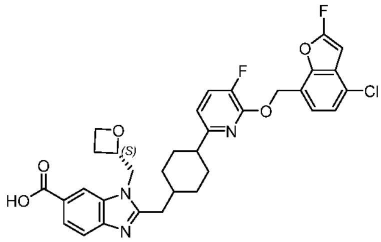 , 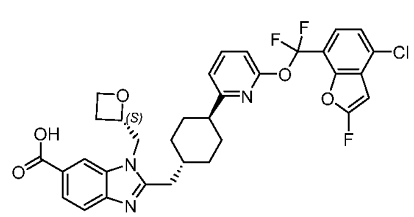 ,

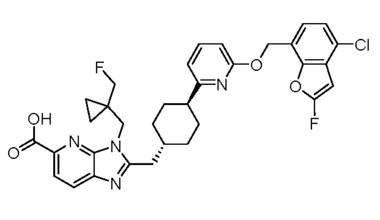 , 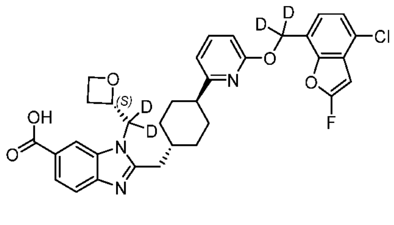 ,

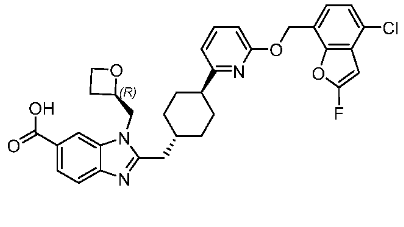 , 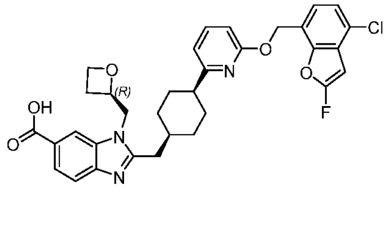 ,

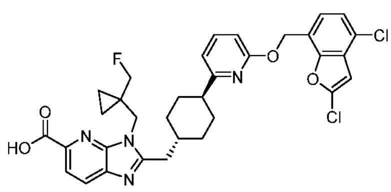 , 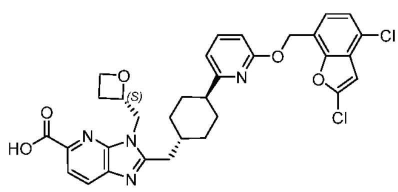 , 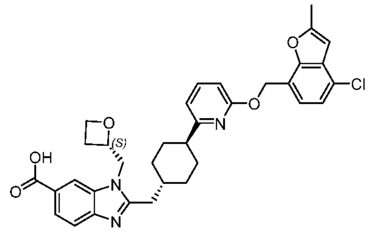 ,

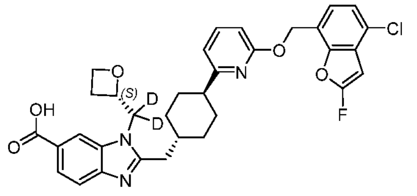 , 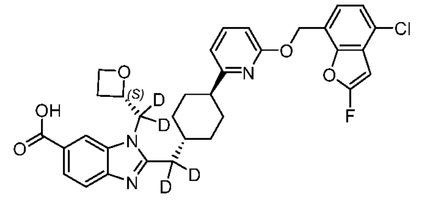 ,

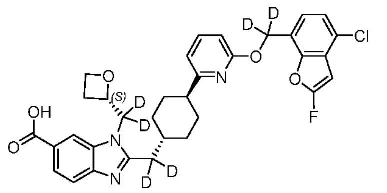 , 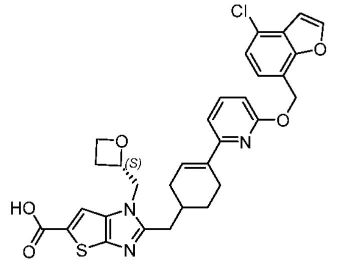 , 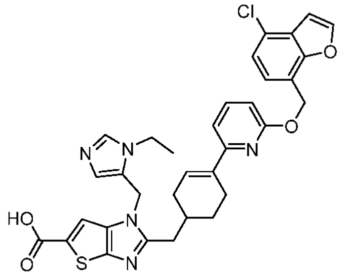 ,

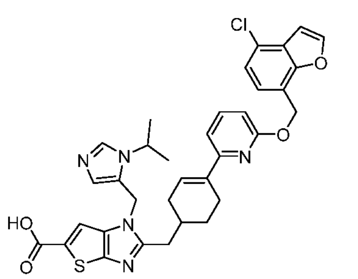 , 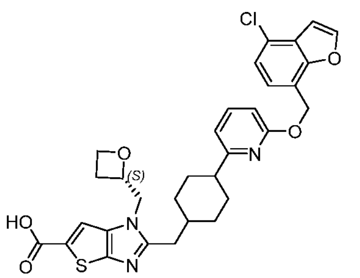 , 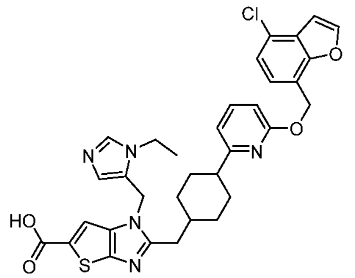 ,

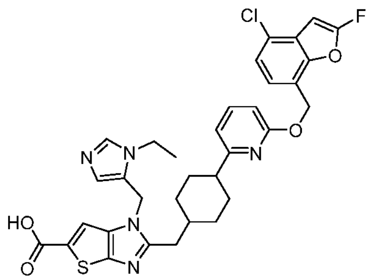 , 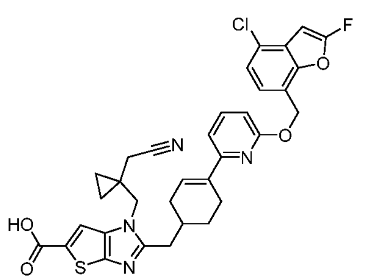 , 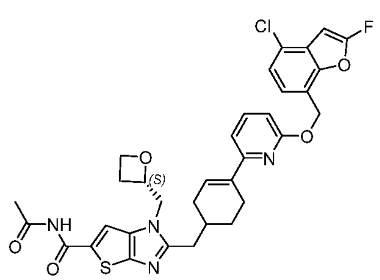 ,

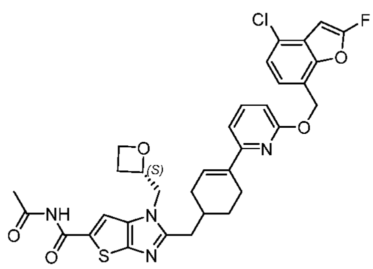 , 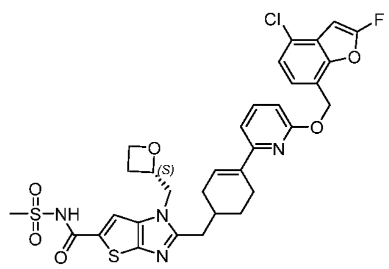 , 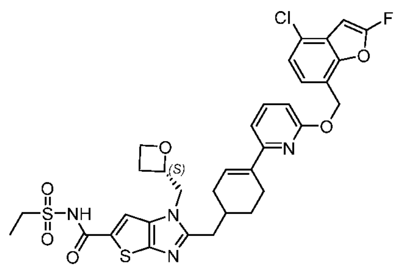 ,

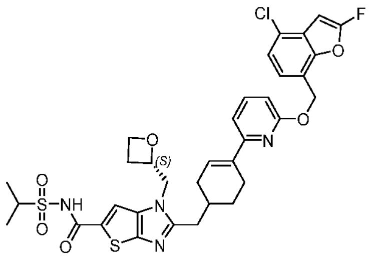
,
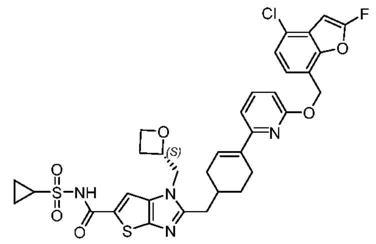
,
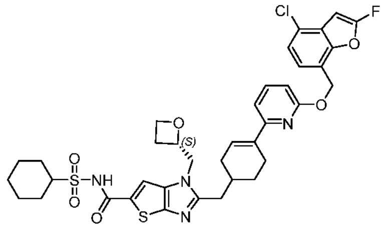
,

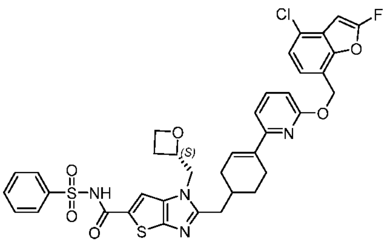
,
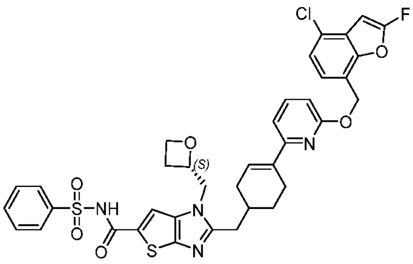
,
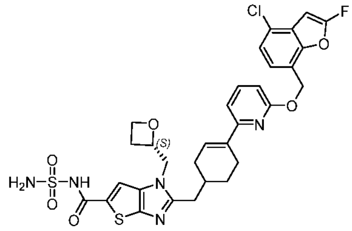
,

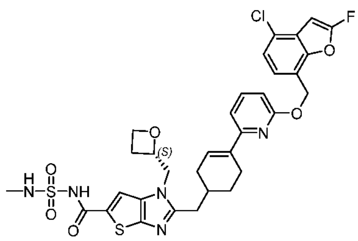
,
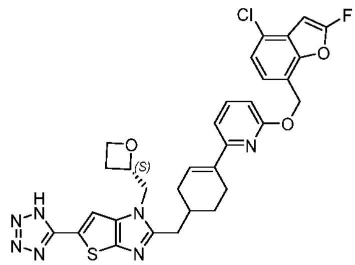
,
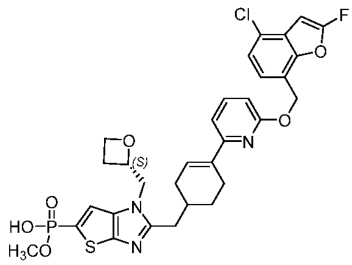
,

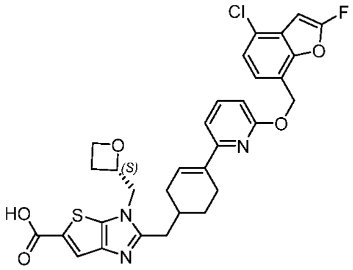
,
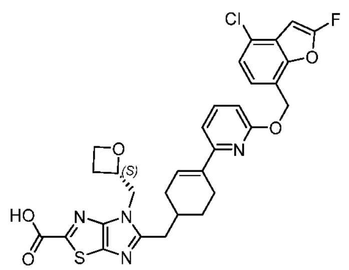
,
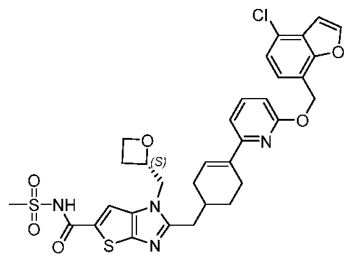
,

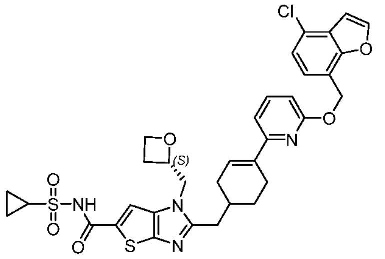
,
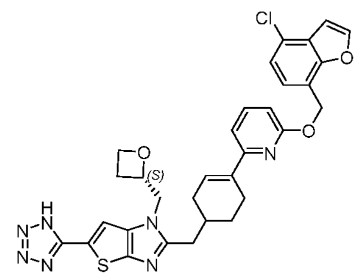
,
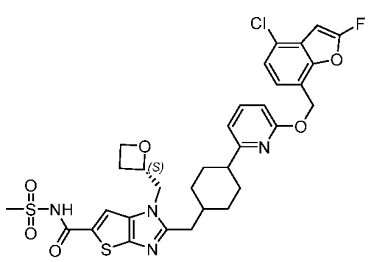
,

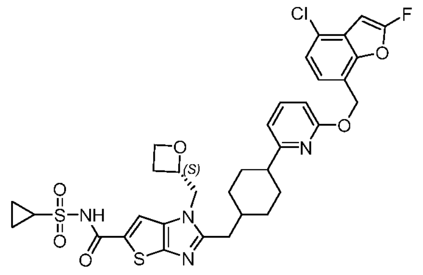
,
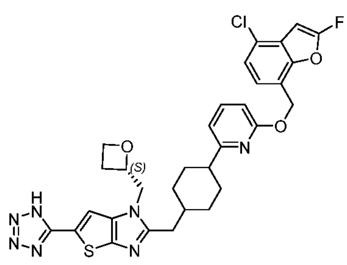
,
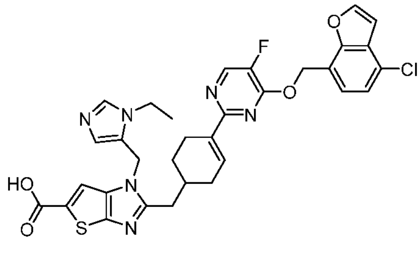
,
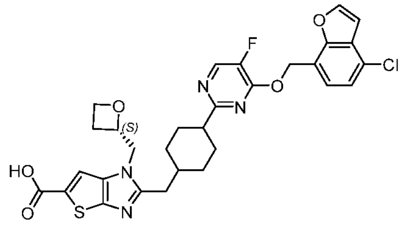
,
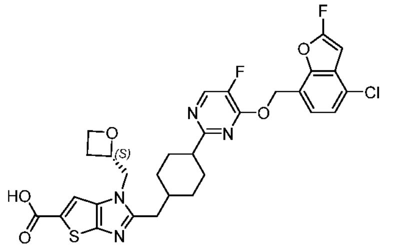
,
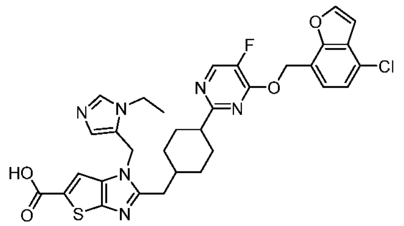
,
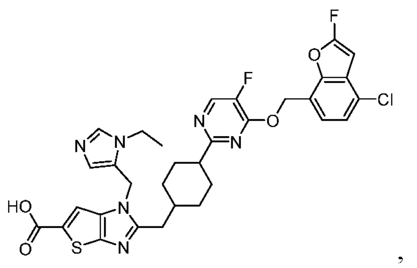
,
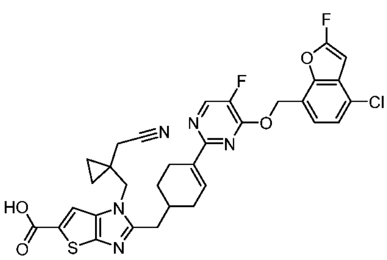
,
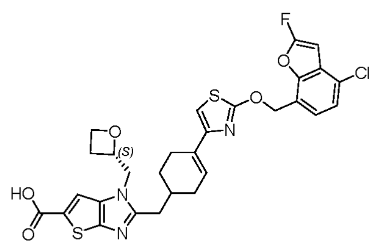
,
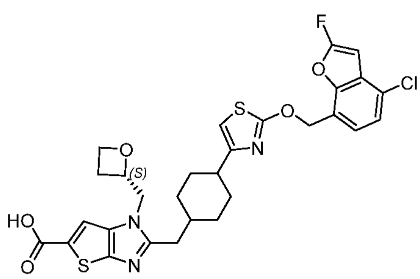
,
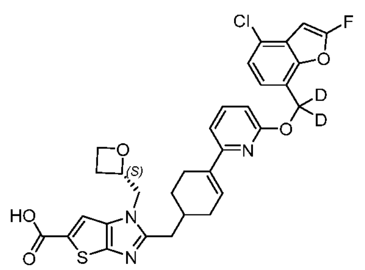
,
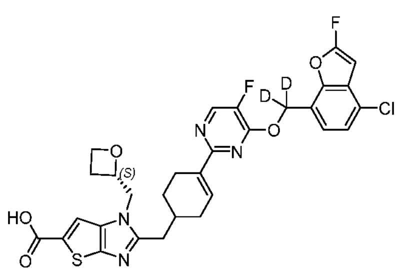
,
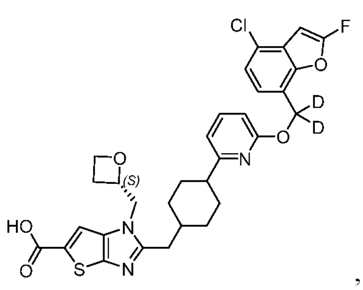
,
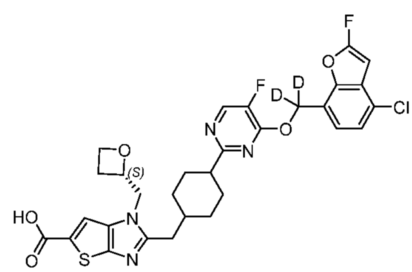
,

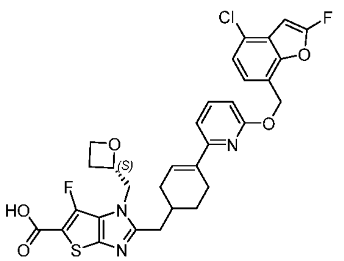, 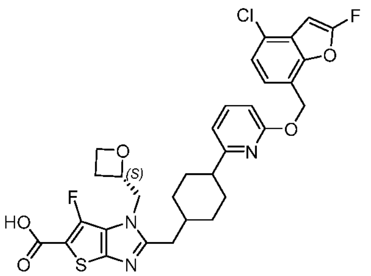, 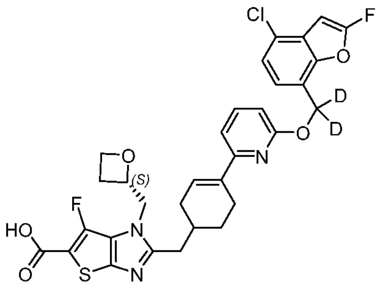,

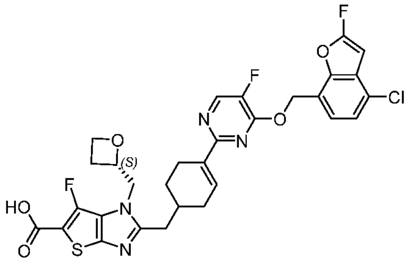, 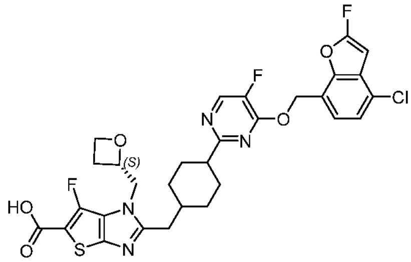,

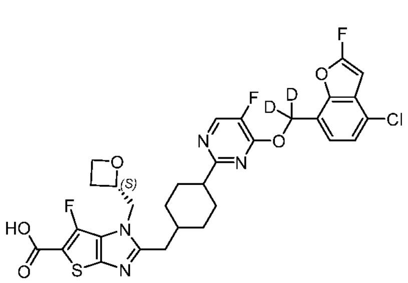, 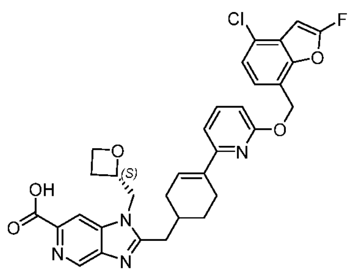, 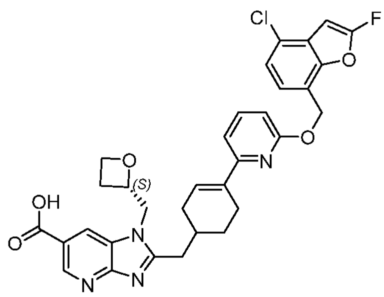,

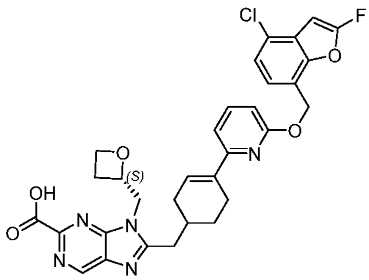, 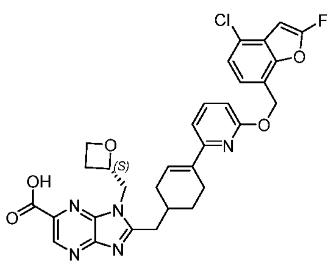, 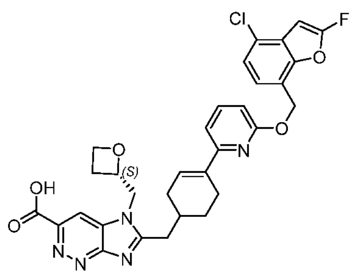,

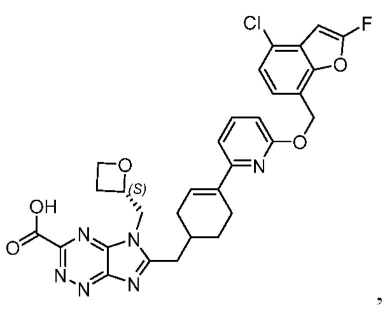, 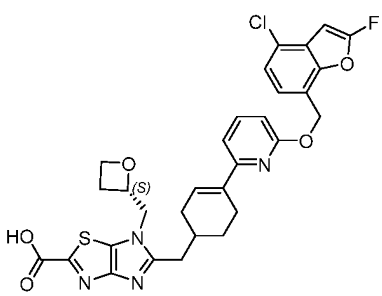, 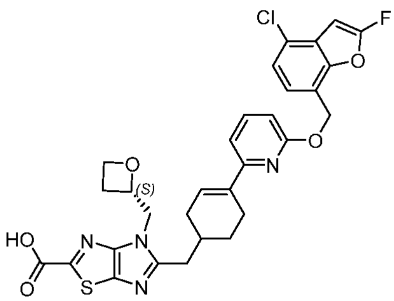.

**7.** A pharmaceutical composition comprising the compound of any one of claims 1-6, or a stereoisomer, a geometric isomer, a tautomer, an N-oxide, a hydrate, a solvate, a metabolite, a pharmaceutically acceptable salt or a prodrug thereof.

**8.** The pharmaceutical composition of claim 7 further comprising a pharmaceutically acceptable carrier, excipient,

diluent, auxiliary agent, vehicle or any combination thereof.

**9.** Use of the compound of any one of claim 1 to 6 or a stereoisomer, a geometric isomer, a tautomer, an N-oxide, a hydrate, a solvate, a metabolite, a pharmaceutically acceptable salt or a prodrug thereof or a pharmaceutical composition of claim 7 or 8 in the manufacture of a medicament for preventing, managing, treating or lessening a disease mediated by GLP-1 receptor agonist.

**10.** Use of the compound of any one of claim 1 to 6 or a stereoisomer, a geometric isomer, a tautomer, an A-oxide, a hydrate, a solvate, a metabolite, a pharmaceutically acceptable salt or a prodrug thereof or a pharmaceutical composition of claim 7 or 8 in the manufacture of a medicament for preventing and/or treating a disease associated with signaling pathways downstream of the GLP-1 receptor.

**11.** The use of claim 10, wherein the disease associated with signaling pathways downstream of the GLP-1 receptor is selected from: diabetes, diabetic retinopathy, diabetic cerebrovascular disease, diabetic neuropathy, insulin resistance, hyperglycemia, diabetic nephropathy, hypertension, cataracts, osteoporosis, hyperuricemia and infections caused by diabetes, obesity, metabolic syndrome, dyslipidemia, non-alcoholic fatty liver disease, non-alcoholic fat Hepatitis, fibrosis, heart disease, stroke, cirrhosis, liver cancer, metabolic acidosis, ketosis, cardiovascular complaints, epilepsy, atherosclerosis, Parkinson’s disease and Alzheimer’s disease.

**12.** Use of the compound of any one of claim 1 to 6 or a stereoisomer, a geometric isomer, a tautomer, an N-oxide, a hydrate, a solvate, a metabolite, a pharmaceutically acceptable salt or a prodrug thereof or a pharmaceutical composition of claim 7 or 8 in the manufacture of a medicament for promoting insulin secretion or lowering blood sugar.

**13.** Use of the compound of any one of claim 1 to 6 or a stereoisomer, a geometric isomer, a tautomer, an N-oxide, a hydrate, a solvate, a metabolite, a pharmaceutically acceptable salt or a prodrug thereof or a pharmaceutical composition of claim 7 or 8 in the manufacture of a medicament for preventing and/or treating diabetes.

**14.** The use of claim 13, wherein the diabetes is type I diabetes, type II diabetes, gestational diabetes, idiopathic type I diabetes, early-onset type II diabetes, adult-onset diabetes of the young, juvenile-onset diabetes atypical diabetes, malnutrition-related diabetes or latent autoimmune diabetes in adults.

**INTERNATIONAL SEARCH REPORT**

International application No.
**PCT/CN2023/080149**

**A. CLASSIFICATION OF SUBJECT MATTER**

C07D405/14(2006.01)i; C07D409/14(2006.01)i; C07D407/14(2006.01)i; C07D471/04(2006.01)i; C07D487/04(2006.01)i; A61K31/443(2006.01)i; A61K31/4436(2006.01)i; A61K31/4439(2006.01)i; A61K31/444(2006.01)i; A61P3/10(2006.01)i; A61P9/00(2006.01)i; A61P25/00(2006.01)i; A61P13/12(2006.01)i; A61P9/12(2006.01)i; A61P25/08(2006.01)i; A61P25/16(2006.01)i; A61P25/28(2006.01)i

According to International Patent Classification (IPC) or to both national classification and IPC

**B. FIELDS SEARCHED**

Minimum documentation searched (classification system followed by classification symbols)

C07D405/-, C07D409/-, C07D407/-, C07D471/-, C07D487/-, A61K31/-

Documentation searched other than minimum documentation to the extent that such documents are included in the fields searched

Electronic data base consulted during the international search (name of data base and, where practicable, search terms used)

CNKI, CNABS, VEN, DWPI, STN (CAPLUS, REGISTRY): 广州市联瑞制药有限公司, 广州一品红制药有限公司, 广州润霖医药科技有限公司, 咪唑, 苯并呋喃, 胰高血糖素样肽-1, 糖尿病, imidazo+, benzofuran+, Glucagon-like peptide-1, GLP-1, diabetes, 基于权利要求1的式（X）化合物的结构式检索, structural formula search on the basis of compounds of formula (X) in claim 1

**C. DOCUMENTS CONSIDERED TO BE RELEVANT**

| Category* | Citation of document, with indication, where appropriate, of the relevant passages | Relevant to claim No. |
|---|---|---|
| X | CN 113480534 A (GUANGZHOU BEBETTER MEDICINE TECHNOLOGY CO., LTD.) 08 October 2021 (2021-10-08)<br>claims 18-26 | 1-14 |
| Y | WO 2021081207 A1 (GILEAD SCIENCES, INC.) 29 April 2021 (2021-04-29)<br>claims 1 and 53-57, and description, page 89 | 1-14 |
| Y | CN 112566637 A (PFIZER INC.) 26 March 2021 (2021-03-26)<br>claims 1 and 19-20, and description, embodiment compounds 79-80 | 1-14 |
| A | CN 110325530 A (PFIZER INC.) 11 October 2019 (2019-10-11)<br>entire document | 1-14 |
| A | CN 113853371 A (QILU REGOR THERAPEUTICS INC.) 28 December 2021 (2021-12-28)<br>entire document | 1-14 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **05 July 2023** | **07 July 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

INTERNATIONAL SEARCH REPORT

International application No.
PCT/CN2023/080149

C. DOCUMENTS CONSIDERED TO BE RELEVANT

| Category* | Citation of document, with indication, where appropriate, of the relevant passages | Relevant to claim No. |
|---|---|---|
| A | US 2019382384 A1 (PFIZER) 19 December 2019 (2019-12-19)<br>entire document | 1-14 |
| A | WO 2020263695 A1 (ELI LILLY AND COMPANY) 30 December 2020 (2020-12-30)<br>entire document | 1-14 |
| A | WO 2021112538 A1 (HYUNDAI PHARM CO., LTD.) 10 June 2021 (2021-06-10)<br>entire document | 1-14 |
| A | WO 2021154796 A1 (GILEAD SCIENCES, INC.) 05 August 2021 (2021-08-05)<br>entire document | 1-14 |

Form PCT/ISA/210 (second sheet) (July 2022)

**INTERNATIONAL SEARCH REPORT**
**Information on patent family members**

International application No.
**PCT/CN2023/080149**

| Patent document cited in search report | | | Publication date (day/month/year) | Patent family member(s) | | | Publication date (day/month/year) |
|---|---|---|---|---|---|---|---|
| CN | 113480534 | A | 08 October 2021 | CN | 113480534 | B | 13 May 2022 |
| WO | 2021081207 | A1 | 29 April 2021 | AU | 2020369568 | A1 | 05 May 2022 |
| | | | | JP | 2022552735 | A | 19 December 2022 |
| | | | | US | 2021171499 | A1 | 10 June 2021 |
| | | | | CO | 2022005077 | A2 | 10 May 2022 |
| | | | | IL | 292399 | A | 01 June 2022 |
| | | | | CA | 3157525 | A1 | 29 April 2021 |
| | | | | KR | 20220092909 | A | 04 July 2022 |
| | | | | PE | 20221040 | A1 | 17 June 2022 |
| | | | | TW | 202130630 | A | 16 August 2021 |
| | | | | TWI | 790492 | B | 21 January 2023 |
| | | | | CR | 20220178 | A | 15 June 2022 |
| | | | | CL | 2022001030 | A1 | 03 February 2023 |
| | | | | BR | 112022007627 | A2 | 12 July 2022 |
| | | | | EP | 4048664 | A1 | 31 August 2022 |
| CN | 112566637 | A | 26 March 2021 | FI | 3806855 | T3 | 04 May 2023 |
| | | | | JP | 2021528394 | A | 21 October 2021 |
| | | | | JP | 7053900 | B2 | 12 April 2022 |
| | | | | US | 2021332034 | A1 | 28 October 2021 |
| | | | | IL | 279224 | A | 31 January 2021 |
| | | | | IL | 279224 | B1 | 01 March 2023 |
| | | | | ES | 2943510 | T3 | 13 June 2023 |
| | | | | RU | 2765721 | C1 | 02 February 2022 |
| | | | | BR | 112020024956 | A2 | 09 March 2021 |
| | | | | EP | 3806855 | A1 | 21 April 2021 |
| | | | | EP | 3806855 | B1 | 01 March 2023 |
| | | | | CA | 3103051 | A1 | 19 December 2019 |
| | | | | CA | 3103051 | C | 17 January 2023 |
| | | | | WO | 2019239371 | A1 | 19 December 2019 |
| | | | | AU | 2019285570 | A1 | 17 December 2020 |
| | | | | AU | 2019285570 | B2 | 24 March 2022 |
| | | | | SG | 11202011704 | XA | 30 December 2020 |
| | | | | KR | 20210020095 | A | 23 February 2021 |
| | | | | MX | 2020013624 | A | 11 August 2022 |
| | | | | DK | 3806855 | T3 | 24 April 2023 |
| | | | | DK | 3806855 | T5 | 22 May 2023 |
| | | | | PT | 3806855 | T | 03 May 2023 |
| CN | 110325530 | A | 11 October 2019 | ECSP | 19041071 | A | 30 June 2019 |
| | | | | US | 2019119255 | A1 | 25 April 2019 |
| | | | | US | 10669259 | B2 | 02 June 2020 |
| | | | | UA | 122035 | C2 | 25 August 2020 |
| | | | | IL | 267288 | A | 29 August 2019 |
| | | | | IL | 267288 | B | 30 June 2021 |
| | | | | MX | 2019007077 | A | 01 August 2019 |
| | | | | UY | 37514 | A | 31 July 2018 |
| | | | | AR | 110387 | A1 | 27 March 2019 |
| | | | | PH | 12019501360 | A1 | 10 February 2020 |
| | | | | TW | 201835066 | A | 01 October 2018 |
| | | | | TWI | 713809 | B | 21 December 2020 |
| | | | | LT | 3555064 | T | 10 January 2023 |

Form PCT/ISA/210 (patent family annex) (July 2022)

**INTERNATIONAL SEARCH REPORT**
**Information on patent family members**

International application No.
**PCT/CN2023/080149**

| Patent document cited in search report | Publication date (day/month/year) | Patent family member(s) | Publication date (day/month/year) |
|---|---|---|---|
| | | US 2020255406 A1 | 13 August 2020 |
| | | US 10851081 B2 | 01 December 2020 |
| | | CL 2019001651 A1 | 04 October 2019 |
| | | MA 56480 A | 27 April 2022 |
| | | MA 56480 B1 | 30 December 2022 |
| | | US 2018170908 A1 | 21 June 2018 |
| | | US 10208019 B2 | 19 February 2019 |
| | | AU 2017374860 A1 | 20 June 2019 |
| | | AU 2017374860 B2 | 02 September 2021 |
| | | EP 3555064 A1 | 23 October 2019 |
| | | EP 3555064 B1 | 23 November 2022 |
| | | EP 3555064 B9 | 01 March 2023 |
| | | KR 20190094433 A | 13 August 2019 |
| | | KR 102314286 B1 | 21 October 2021 |
| | | JP 6637641 B1 | 29 January 2020 |
| | | JP 2020511411 A | 16 April 2020 |
| | | US 2021047298 A1 | 18 February 2021 |
| | | US 11512070 B2 | 29 November 2022 |
| | | JP 2020063287 A | 23 April 2020 |
| | | JP 6982054 B2 | 17 December 2021 |
| | | FI 3555064 T3 | 31 January 2023 |
| | | CO 2019006046 A2 | 19 June 2019 |
| | | ZA 201904616 B | 26 May 2021 |
| | | PE 20191501 A1 | 22 October 2019 |
| | | HUE 060533 T2 | 28 March 2023 |
| | | BR 112019012211 A2 | 12 November 2019 |
| | | ES 2934789 T3 | 27 February 2023 |
| | | ES 2934789 T9 | 26 April 2023 |
| | | US 2023124938 A1 | 20 April 2023 |
| | | KR 20210127782 A | 22 October 2021 |
| | | KR 102466418 B1 | 14 November 2022 |
| | | RU 2740135 C1 | 11 January 2021 |
| | | DOP 2019000166 A | 31 July 2019 |
| | | PT 3555064 T | 20 January 2023 |
| | | CR 20190289 A | 21 August 2019 |
| | | CA 2988721 A1 | 16 June 2018 |
| | | WO 2018109607 A1 | 21 June 2018 |
| | | RS 63849 B1 | 31 January 2023 |
| | | DK 3555064 T3 | 12 December 2022 |
| | | DK 3555064 T5 | 01 May 2023 |
| | | PL 3555064 T3 | 06 March 2023 |
| | | EA 201991193 A1 | 15 January 2020 |
| | | EA 037318 B1 | 11 March 2021 |
| | | CU 20190056 A7 | 03 January 2020 |
| | | CU 24573 B1 | 13 January 2022 |
| | | SI 3555064 T1 | 28 February 2023 |
| | | GEP 20217265 B | 25 June 2021 |
| | | HRP 20221437 T1 | 03 February 2023 |
| CN 113853371 A | 28 December 2021 | US 2021179594 A1 | 17 June 2021 |
| | | US 11591321 B2 | 28 February 2023 |

Form PCT/ISA/210 (patent family annex) (July 2022)

**INTERNATIONAL SEARCH REPORT**
**Information on patent family members**

International application No.
**PCT/CN2023/080149**

| Patent document cited in search report | Publication date (day/month/year) | Patent family member(s) | Publication date (day/month/year) |
|---|---|---|---|
| | | US 2020325121 A1 | 15 October 2020 |
| | | US 10954221 B2 | 23 March 2021 |
| US 2019382384 A1 | 19 December 2019 | US 2021163455 A1 | 03 June 2021 |
| | | SG 11202011465 QA | 30 December 2020 |
| | | EP 3806955 A1 | 21 April 2021 |
| | | EP 3806955 B1 | 31 May 2023 |
| | | CO 2020015305 A2 | 08 March 2021 |
| | | MX 2020013625 A | 04 January 2022 |
| | | IL 279300 A | 31 January 2021 |
| | | CU 20200099 A7 | 06 August 2021 |
| | | KR 20210019529 A | 22 February 2021 |
| | | PH 12020552069 A1 | 31 May 2021 |
| | | UA 124371 C2 | 01 September 2021 |
| | | US 2019382388 A1 | 19 December 2019 |
| | | US 10676465 B2 | 09 June 2020 |
| | | CR 20200612 A | 20 January 2021 |
| | | US 2019382387 A1 | 19 December 2019 |
| | | US 10683281 B2 | 16 June 2020 |
| | | JP 6916968 B1 | 11 August 2021 |
| | | JP 2021521265 A | 26 August 2021 |
| | | RU 2769715 C1 | 05 April 2022 |
| | | GEP 20237453 B | 10 January 2023 |
| | | US 10934279 B2 | 02 March 2021 |
| | | ECSP 20078651 A | 29 January 2021 |
| | | AU 2019285491 A1 | 17 December 2020 |
| | | AU 2019285491 B2 | 11 November 2021 |
| | | NI 202000091 A | 23 March 2021 |
| | | UY 38261 A | 31 January 2020 |
| | | WO 2019239319 A1 | 19 December 2019 |
| | | BR 112020024470 A2 | 02 March 2021 |
| | | CL 2020003222 A1 | 30 April 2021 |
| | | MA 52882 A | 21 April 2021 |
| | | TW 202015679 A | 01 May 2020 |
| | | TWI 707683 B | 21 October 2020 |
| | | CA 3045644 A1 | 13 December 2019 |
| | | PE 20211601 A1 | 18 August 2021 |
| WO 2020263695 A1 | 30 December 2020 | US 2020407347 A1 | 31 December 2020 |
| | | US 11655242 B2 | 23 May 2023 |
| | | CL 2021003419 A1 | 23 September 2022 |
| | | KR 20220012924 A | 04 February 2022 |
| | | CR 20210599 A | 22 December 2021 |
| | | EP 3989972 A1 | 04 May 2022 |
| | | JP 2022540044 A | 14 September 2022 |
| | | JP 7256300 B2 | 11 April 2023 |
| | | CO 2021017433 A2 | 19 April 2022 |
| | | TW 202115040 A | 16 April 2021 |
| | | TWI 751585 B | 01 January 2022 |
| | | IL 288479 A | 01 January 2022 |
| | | ECSP 21093576 A | 31 January 2022 |
| | | BR 112021023923 A2 | 25 January 2022 |

Form PCT/ISA/210 (patent family annex) (July 2022)

**INTERNATIONAL SEARCH REPORT**
**Information on patent family members**

International application No.
**PCT/CN2023/080149**

| Patent document cited in search report | | | Publication date (day/month/year) | Patent family member(s) | | | Publication date (day/month/year) |
|---|---|---|---|---|---|---|---|
| | | | | CA | 3144055 | A1 | 30 December 2020 |
| | | | | AU | 2020309064 | A1 | 27 January 2022 |
| | | | | DOP | 2021000273 | A | 15 March 2022 |
| | | | | JOP | 20210337 | A1 | 30 January 2023 |
| | | | | PE | 20220590 | A1 | 22 April 2022 |
| | | | | MA | 56391 | A | 04 May 2022 |
| WO | 2021112538 | A1 | 10 June 2021 | KR | 20210069000 | A | 10 June 2021 |
| | | | | EP | 4069686 | A1 | 12 October 2022 |
| | | | | EP | 4069686 | A4 | 09 November 2022 |
| | | | | US | 2023051318 | A1 | 16 February 2023 |
| WO | 2021154796 | A1 | 05 August 2021 | US | 2022288030 | A1 | 15 September 2022 |
| | | | | JP | 2023509801 | A | 09 March 2023 |
| | | | | EP | 4097097 | A1 | 07 December 2022 |
| | | | | CA | 3168543 | A1 | 05 August 2021 |
| | | | | AU | 2021212669 | A1 | 21 July 2022 |
| | | | | KR | 20220133271 | A | 04 October 2022 |
| | | | | TW | 202140463 | A | 01 November 2021 |
| | | | | TWI | 793511 | B | 21 February 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202210219469
- WO 2018109607 A
- WO 2019239319 A
- WO 2019239371 A
- WO 2020103815 A
- WO 2020207474 A
- WO 2020263695 A
- WO 2021154796 A
- WO 2021112538 A
- WO 2021096304 A
- WO 2021096284 A
- WO 2021081207 A
- WO 2021018023 A
- WO 2021254470 A
- CN 113480534 A


### Non-patent literature cited in the description


- chemical manuals. 1994
- **THOMAS SORRELL**. Organic Chemistry. University Science Books, 1999
- **SMITH et al.** March's Advanced Organic Chemistry. John Wiley&Sons, 2007
- McGraw-Hill Dictionary of Chemical Terms. McGraw-Hill Book Company, 1984
- **ELIEL, E.** ; **WILEN, S.** Stereochemistry of Organic Compounds. John Wiley&Sons, Inc., 1994
- **BERGE et al.** *J. Pharmacol Sci*, 1977, vol. 66, 1-19
- **JERRY MARCH**. Advanced Organic Chemistiy. Wiley Interscience
- **L. W DEADY**. *Syn. Comm.*, 1977, vol. 7, 509-514
- **T. HIGUCHI** ; **V. STELLA**. Pro-drugs as Novel Delivery Systems, vol. 14
- Bioreversible Carriers in Drug Design. American Pharmaceutical Association and Pergamon Press, 1987
- **J. RAUTIO et al.** Prodrugs: Design and Clinical Applications. *Nature Review Drug Discovery*, 2008, vol. 7, 255-270
- **S. J. HECKER et al.** Prodrugs of Phosphates and Phosphonates. *Journal of Medicinal Chemistry*, 2008, vol. 51, 2328-2345